# FASCICULE DE BREVET EUROPEEN

(11) **EP 1 047 389 B1**
(45) Date de publication et mention de la délivrance du brevet: **07.07.2004**
(21) Numéro de dépôt: 99934809.7
(22) Date de dépôt: 29.07.1999
(51) Int. Cl.: A61K 7/13

(54) **COMPOSITION DE TEINTURE POUR FIBRES KERATINIQUES AVEC UN COLORANT DIRECT CATIONIQUE ET UN AGENT TENSIO-ACTIF NON-IONIQUE**
ZUSAMMENSETZUNG ZUM FÄRBEN VON KERATINFASERN MIT EINEM KATIONISCHEN DIREKTFARBSTOFF UND EINEM NICHTIONISCHEN GRENZFLÄCHENAKTIVEN STOFF
DYEING COMPOSITION FOR KERATINOUS FIBRES WITH DIRECT CATIONIC COLOURING AGENT AND A NON-IONIC SURFACTANT

(30) Priorité: 24.08.1998 FR 9810659
(43) Date de publication de la demande: 02.11.2000
(73) Titulaire: L'OREAL, 75008 Paris (FR)
(72) Inventeur: LANG, Gérard, F-95390 Saint Prix (FR); COTTERET, Jean, F-78480 Verneuil sur Seine (FR)
(74) Mandataire: Miszputen, Laurent
(86) Numéro de dépôt international: PCT/FR1999/001875
(87) Numéro de publication internationale: WO 2000/010519

(56) Documents cités:
- EP-A- 0 531 943
- EP-A- 0 711 542
- EP-A- 0 850 637
- EP-A- 0 850 638
- WO-A-95/01772
- WO-A-95/15144
- WO-A-99/20234
- WO-A-99/20235
- DE-U- 29 504 690
- FR-A- 2 140 205
- FR-A- 2 189 006
- FR-A- 2 282 860
- FR-A- 2 285 851

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un agent tensio-actif non-ionique particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la ténacité, en terme de résistance aux diverses agressions que peuvent subir les cheveux (lumière, intempéries, shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations puissantes et peu sélectives et résistant bien néanmoins aux diverses agressions que peuvent subir les cheveux, en associant au moins un agent tensio-actif anionique particulier à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, (i)au moins un colorant direct cationique dont la structure répond aux formules (I) à (IV) définies ci-après, caractérisée par le fait qu'elle contient en outre (ii)au moins un agent tensio-actif non-ionique particulier.

(i) Le colorant direct cationique utilisable selon la présente invention est un composé choisi parmi ceux de formules (I), (II), (III), (III'), (IV) suivantes
**a) les composés de formule (I) suivante :** 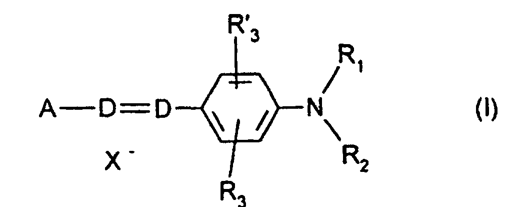 dans laquelle:
   D représente un atome d'azote ou le groupement -CH,
   R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₃ et R'₃, identiques ou différents, représentent un atome d'hydrogène ou d'halogène choisi parmi le chlore, le brome, l'iode et le fluor, un radical cyano, alkyl en C₁-C₄ , alcoxy en C₁-C₄ ou acétyloxy,
   X⁻ représente un anion de préférence choisi parmi le chlorure, le méthyl sulfate et l'acétate,
   A représente un groupement choisi par les structures A1 à A18 suivantes : 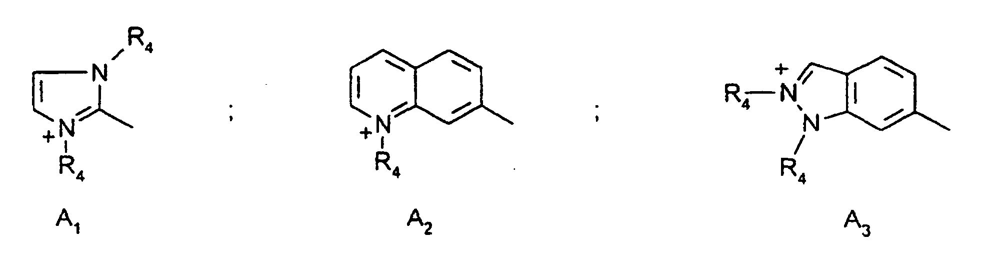 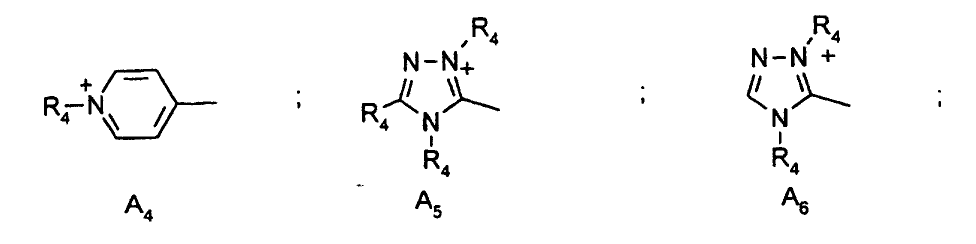 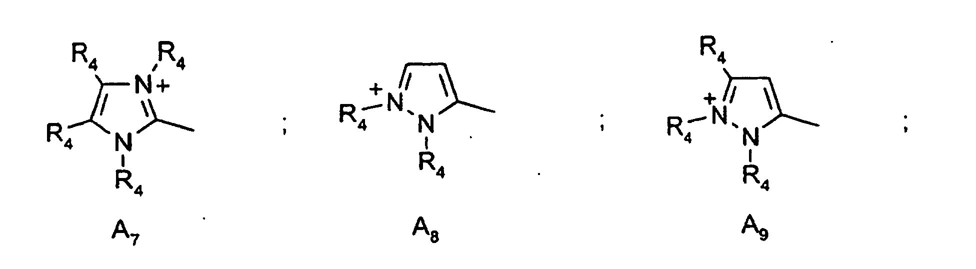 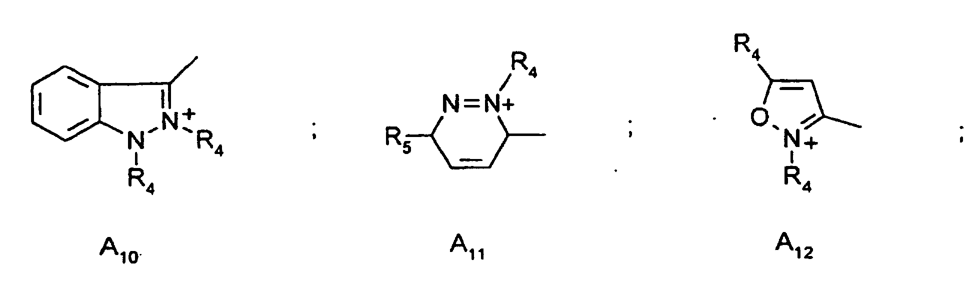 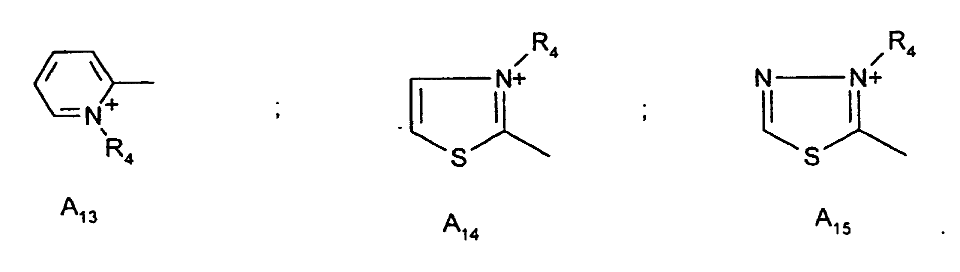 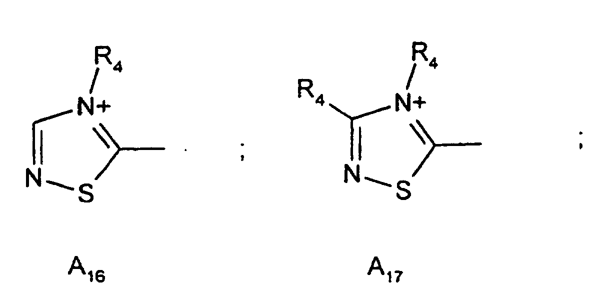 et 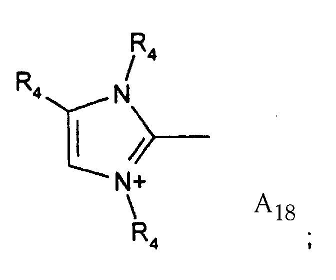
   dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 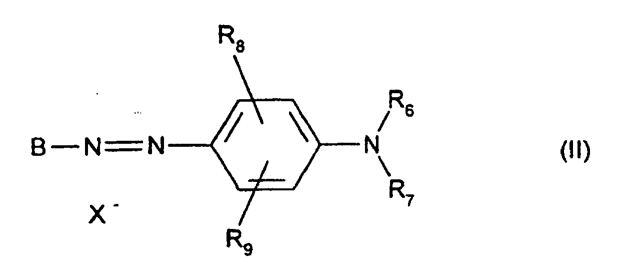 dans laquelle:
   R₆ représente un atome d'hydrogène ou un radical alkyle en C₁-C₄,
   R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X représente un anion de préférence choisi parmi le chlorure, le méthyl sulfate et l'acétate,
   B représente un groupement choisi par les structures B1 à B6 suivantes : 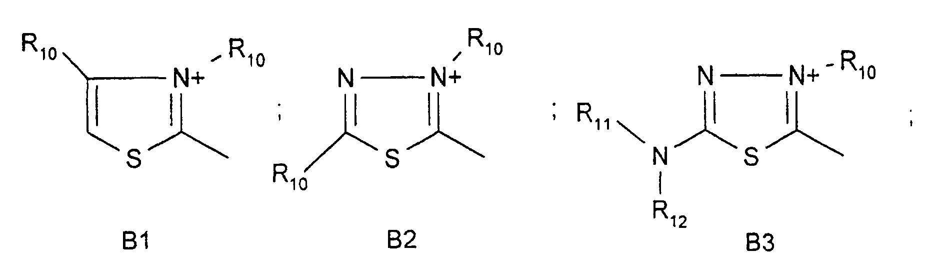 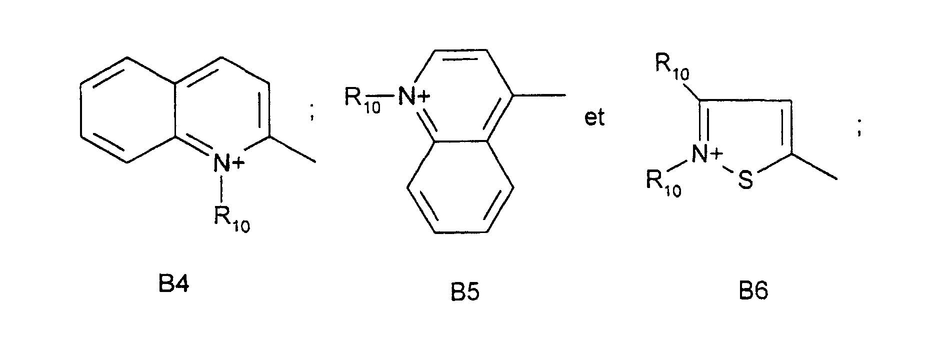
   dans lesquelles R₁₀ représente un radical alkyle en C₁-C₄, R₁₁ et R₁₂, identiques ou différents, représentent un atome d'hydrogène ou un radical alkyle en C₁-C₄ ,
**c) les composés de formules (III) et (III') suivantes :** 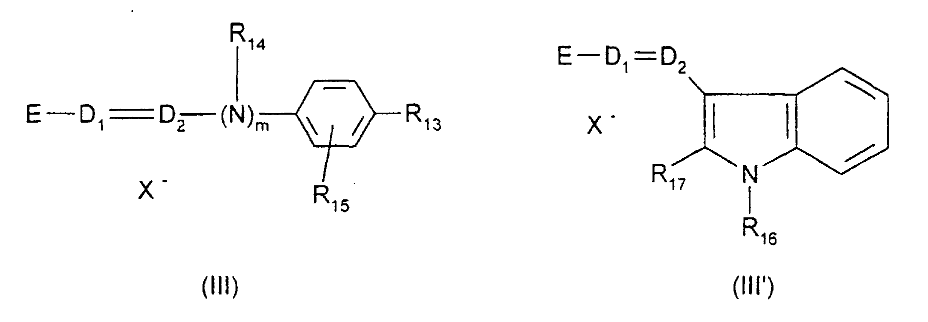 dans lesquelles :
   R₁₃ représente un atome d'hydrogène, un radical alcoxy en C₁-C₄, un atome d'halogène tel que le brome, le chlore, l'iode ou le fluor ou un radical amino,
   R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₁₅ représente un atome d'hydrogène ou d'halogène tel que le brome, le chlore, l'iode ou le fluor,
   R₁₆ et R₁₇, identiques ou différents, représentent un atome d'hydrogène ou un radical alkyle en C₁-C₄,
   D₁ et D₂, identiques ou différents, représentent un atome d'azote ou le groupement -CH,
   m = 0 ou 1,
   étant entendu que lorsque R₁₃ représente un groupement amino non substitué, alors D₁ et D₂ représentent simultanément un groupement -CH et m = 0,
   X⁻ représente un anion de préférence choisi parmi le chlorure, le méthyl sulfate et l'acétate,
   E représente un groupement choisi par les structures E1 à E8 suivantes : 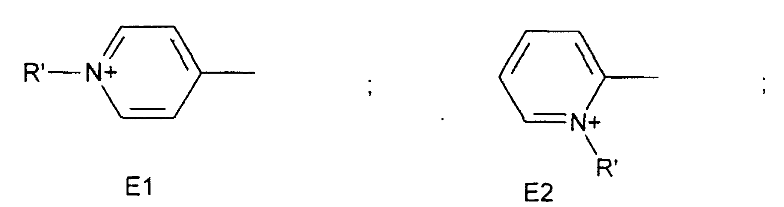 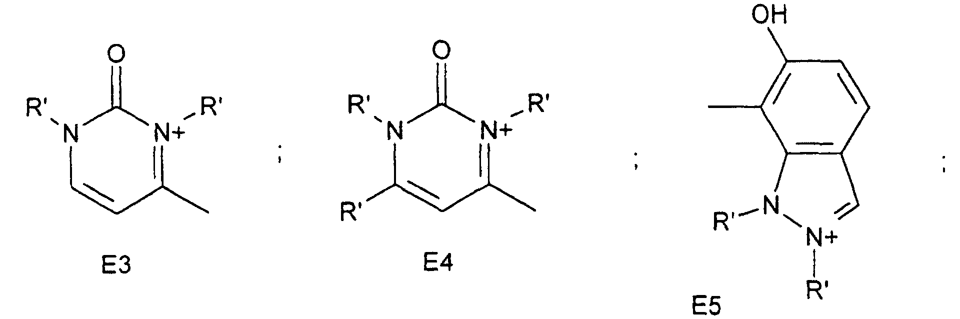 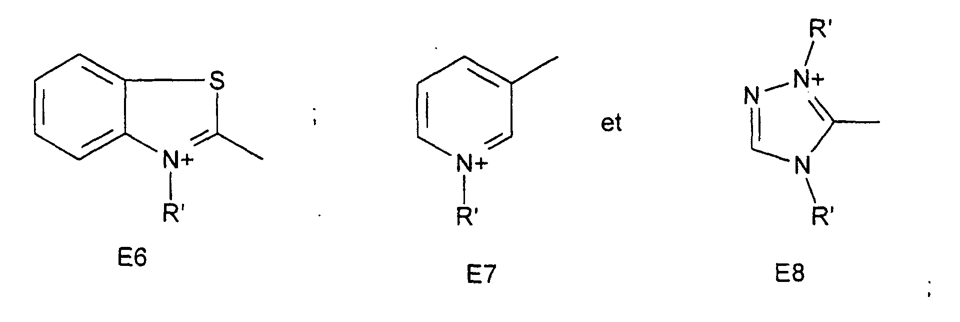
   dans lesquelles R' représente un radical alkyle en C₁-C₄ ;
   lorsque m = 0 et que D₁ représente un atome d'azote, alors E peut également désigner un groupement de structure E9 suivante : 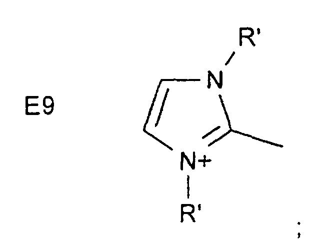 dans laquelle R' représente un radical alkyle en C₁-C₄.
**d) les composés de formule (IV) suivante :**

   G-N=N-J (IV)

   dans laquelle :
   **le symbole G** représente un groupement choisi parmi les structures G₁ à G₃ suivantes : 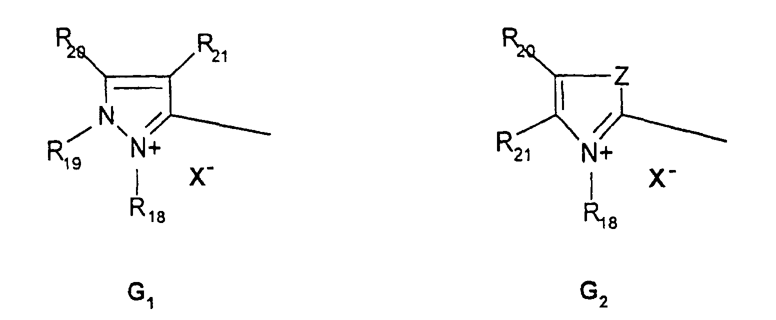 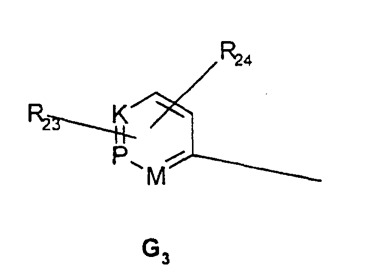 structures G₁ à G₃ dans lesquelles,
   R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
   R₁₉ désigne un radical alkyle en C₁-C₄ ou un radical phényle;
   R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂;
   R₂₀ peut désigner en outre un atome d'hydrogène;
   Z désigne un atome d'oxygène, de soufre ou un groupement -NR₁₉;
   M représente un groupement -CH, -CR (R désignant alkyle en C₁-C₄),
      ou -NR₂₂(X⁻)r;
   K représente un groupement -CH, -CR (R désignant alkyle en C₁-C₄),
      ou -NR₂₂(X⁻)r;
   P représente un groupement -CH, -CR (R désignant alkyle en C₁-C₄),
      ou -NR₂₂(X⁻)ᵣ; r désigne zéro ou 1;
   R₂₂ représente un atome O⁻, un radical alcoxy en C₁-C₄, ou un radical alkyle en C₁-C₄;
   R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄ , un radical -NO₂;
   X⁻ représente un anion de préférence choisi parmi le chlorure, l'iodure, le méthyl sulfate, l'éthyl sulfate, l'acétate et le perchlorate;
   sous réserve que,
   si R₂₂ désigne O⁻, alors r désigne zéro;
   si K ou P ou M désignent -N-alkyle C₁-C₄ X⁻ , alors R₂₃ ou R₂₄ est différent d'un atome d'hydrogène;
   si K désigne -NR₂₂(X-)ᵣ , alors M= P= -CH, -CR;
   si M désigne -NR₂₂(X-)ᵣ , alors K= P= -CH, -CR;
   si P désigne -NR₂₂(X⁻), alors K= M et désignent -CH ou -CR;
   si Z désigne un atome de soufre avec R₂₁ désignant alkyle en C₁-C₄ , alors R₂₀ est différent d'un atome d'hydrogène;
   si Z désigne -NR₁₉ avec R₁₉ désignant alkyle en C₁-C₄ , alors au moins l'un des radicaux R₁₈, R₂₀ ou R₂₁ du groupement de structure G₂ est différent d'un radical alkyle en C₁-C₄;
   **le symbole J** représente :
   - (a) un groupement de structure J₁ suivante : 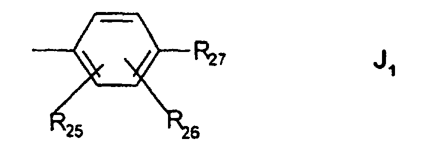 structure J₁ dans laquelle,
      R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec
      R₂₆ un cycle à 5 ou 6 chaînons contenant ou non un ou plusieurs hétéroatomes choisis parmi l'azote, l'oxygène ou le soufre;
      R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
      R₂₇ représente un atome d'hydrogène, un radical -OH, un radical -NHR₂₈, un radical -NR₂₉R₃₀;
      R₂₈ représente un atome d'hydrogène, un radical alkyle en C₁-C₄, un radical monohydroxyalkyle en C₁-C₄, polyhydroxyalkyle en C₂-C₄, un radical phényle;
      R₂₉ et R₃₀, identiques ou différents, représentent un radical alkyle en C₁-C₄ , un radical monohydroxyalkyle en C₁-C₄, polyhydroxyalkyle en C₂-C₄;
   - **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 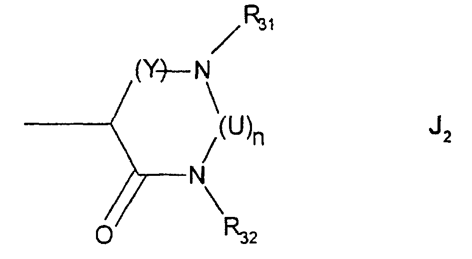 structure J₂ dans laquelle,
      R₃₁ et R₃₂, identiques ou différents, représentent un atome d'hydrogène , un radical alkyle en C₁-C₄ un radical phényle;
      Y désigne le radical -CO- ou le radical 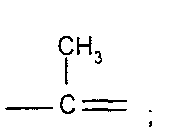
      n = 0 ou 1, avec, lorsque n désigne 1, U désigne le radical -CO- .

Dans les structures (I) à (IV) définies ci-dessus le groupement alkyle ou alcoxy en C₁-C₄ désigne de préférence méthyle, éthyle, butyle, méthoxy, éthoxy.

Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954. Ceux de formule (IV) utilisables dans les compositions tinctoriales conformes à l'invention, sont des composés connus et sont décrits par exemple dans les demandes de brevets FR-2189006, FR-2285851 et FR-2140205 et ses certificats d'addition.

Parmi les colorants directs cationiques de formule (I) utilisables dans les compositions tinctoriales conformes à l'invention, on peut plus particulièrement citer les composés répondant aux structures (I1) à (I54) suivantes : 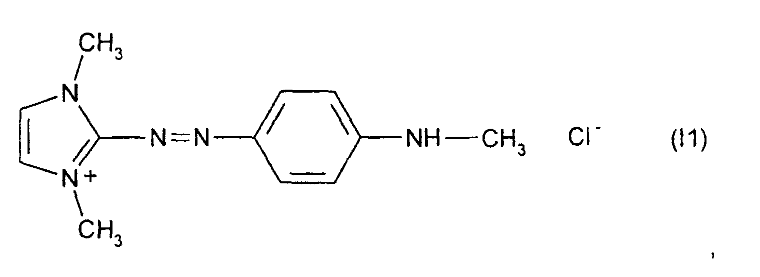 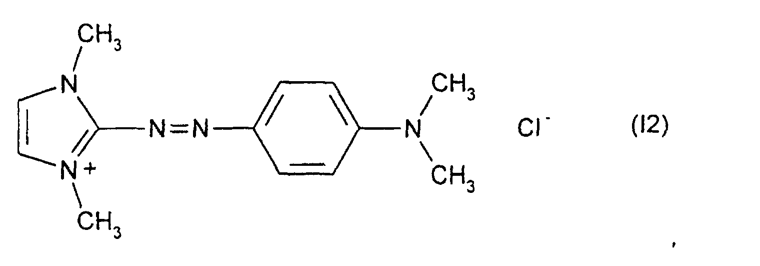 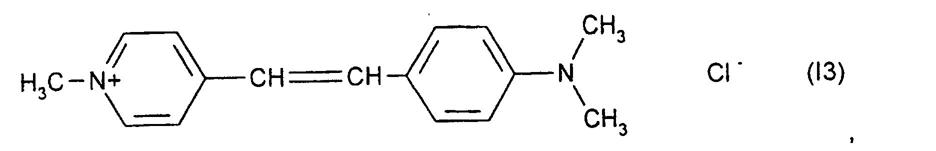 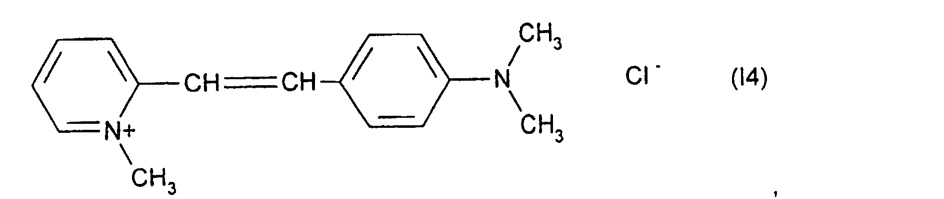 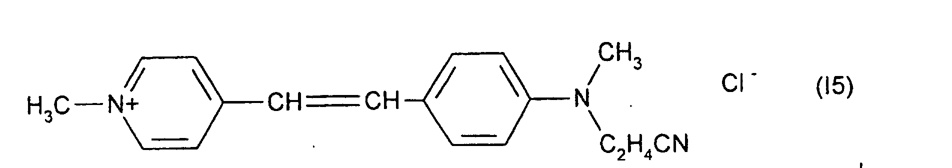 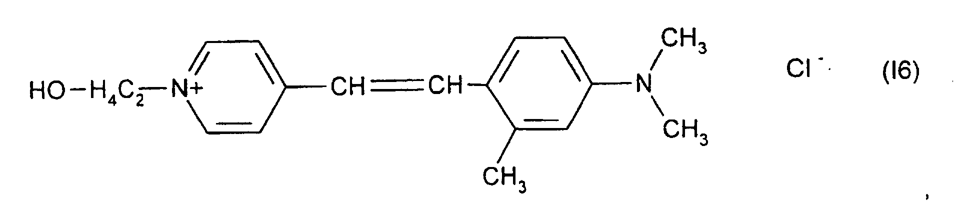 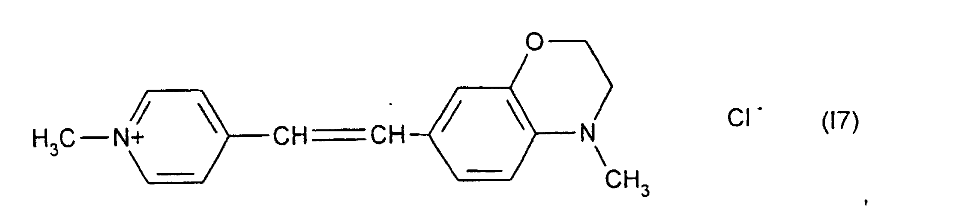 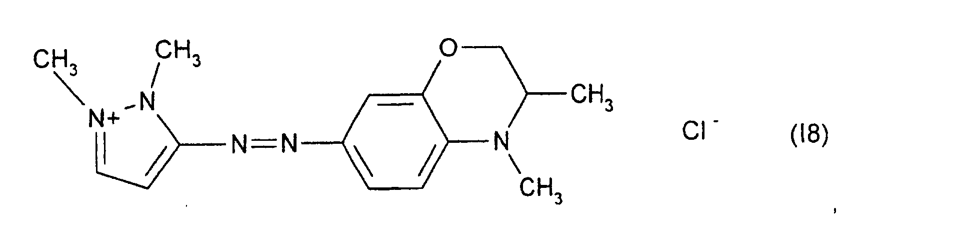 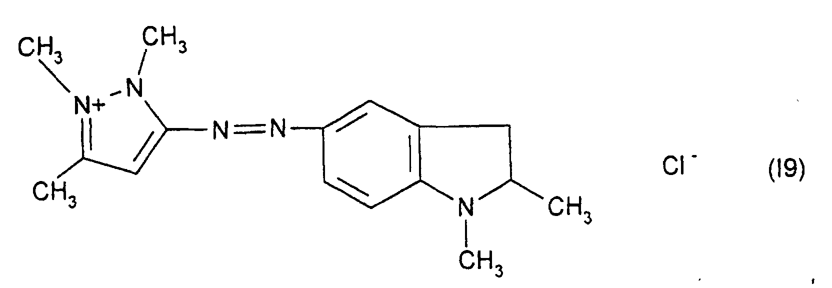 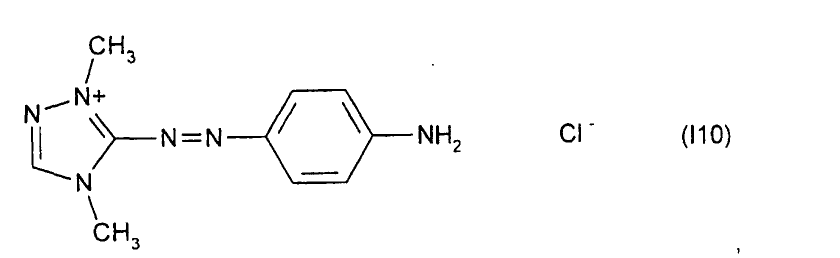 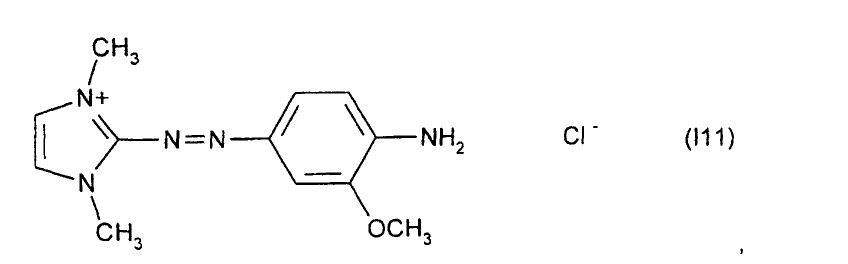 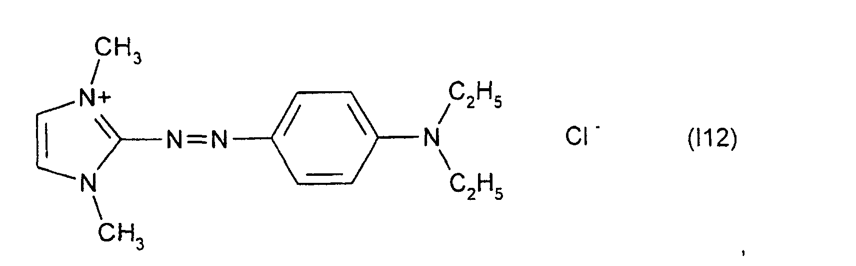 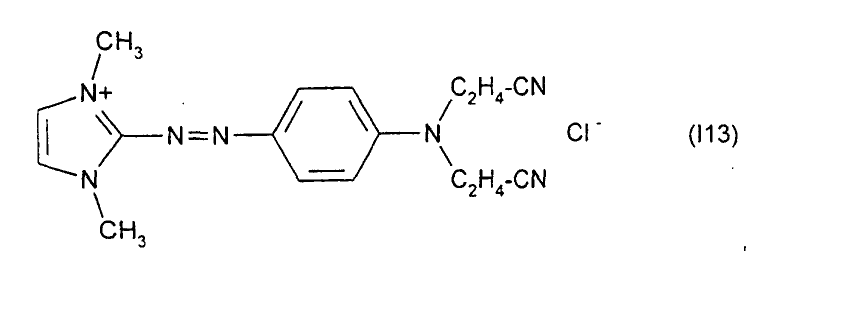 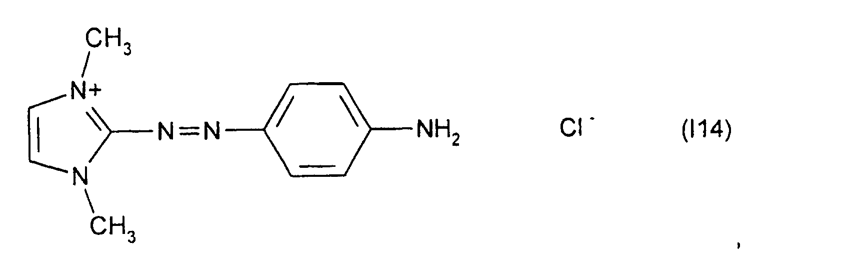 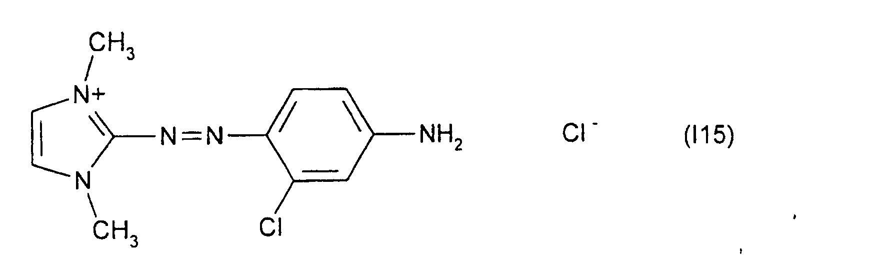 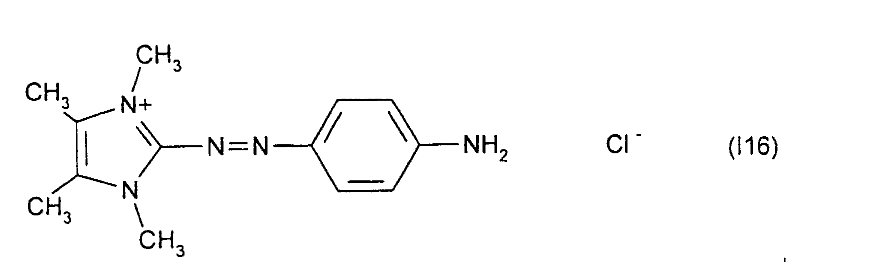 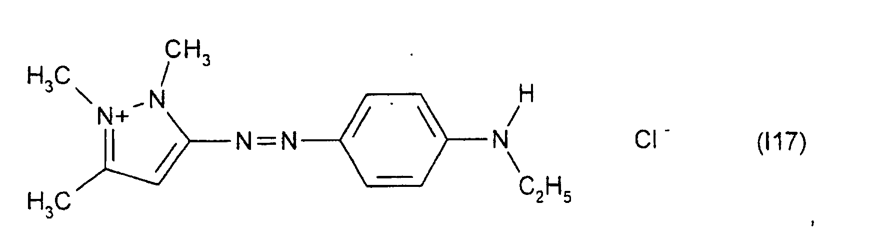 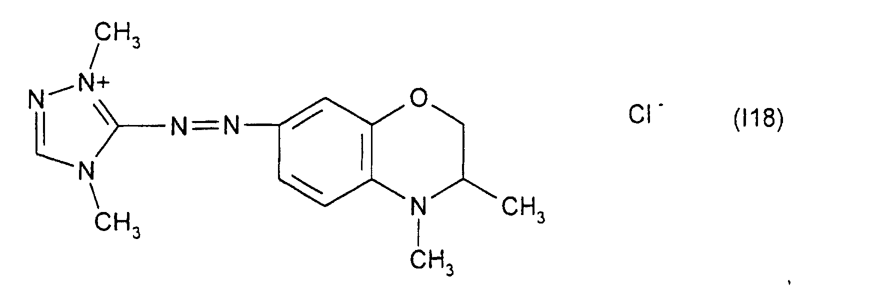 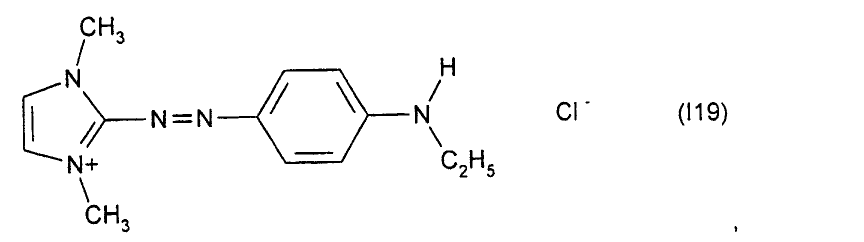 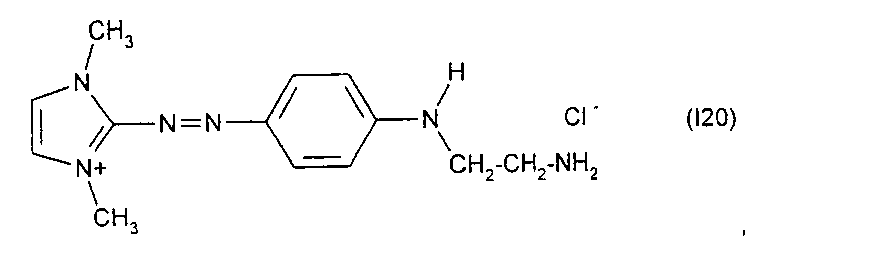 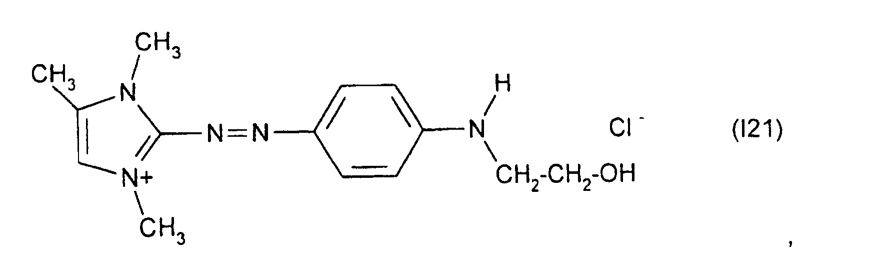 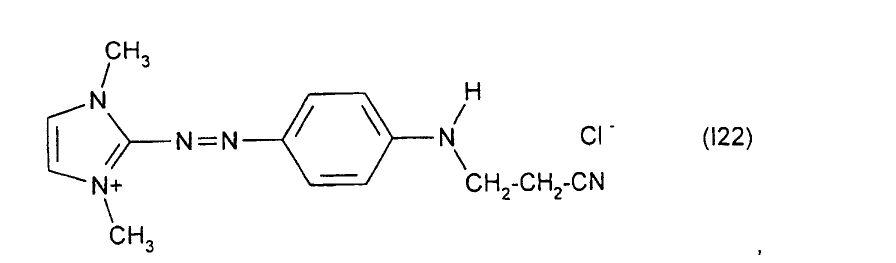 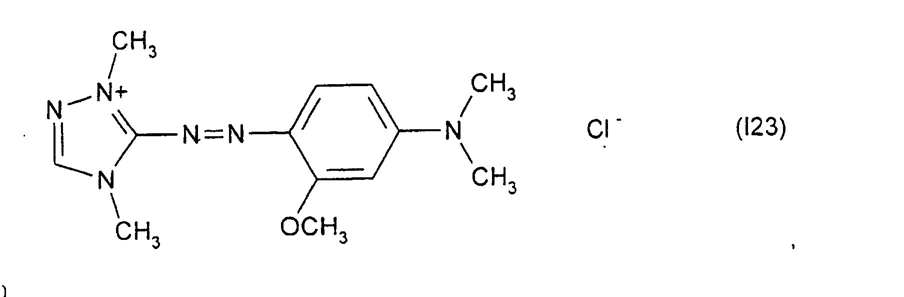 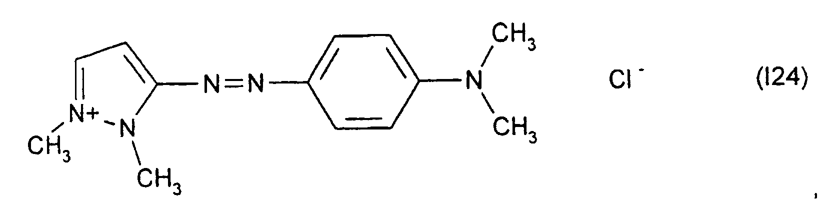 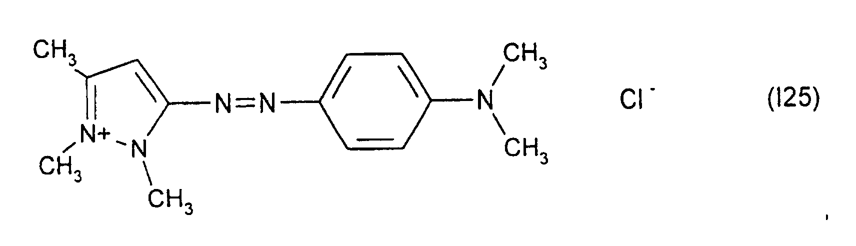 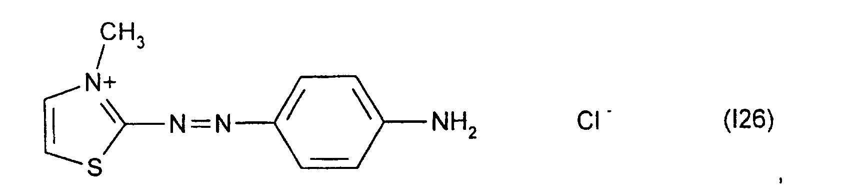 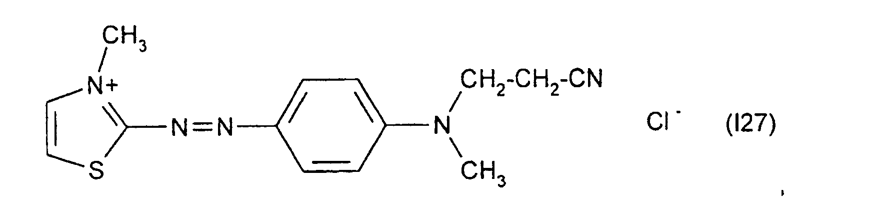 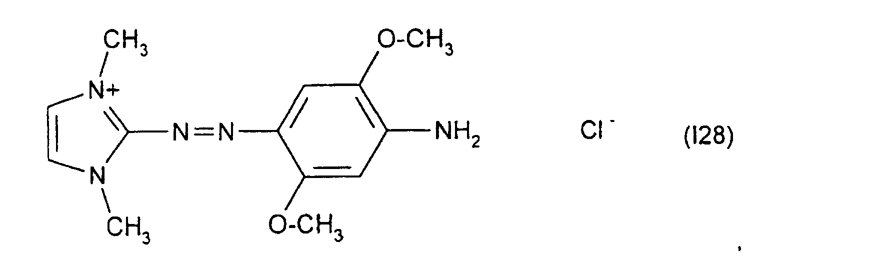 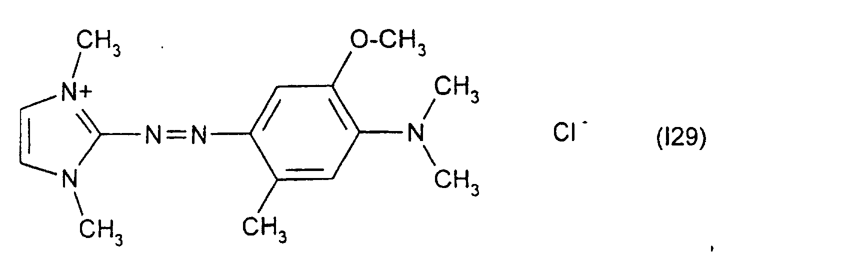 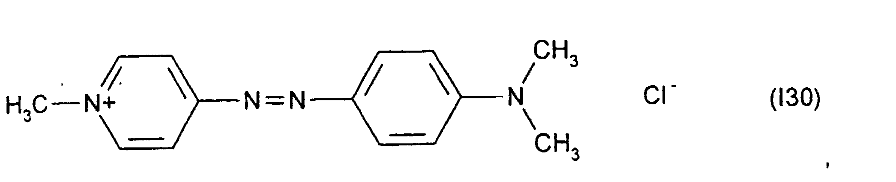 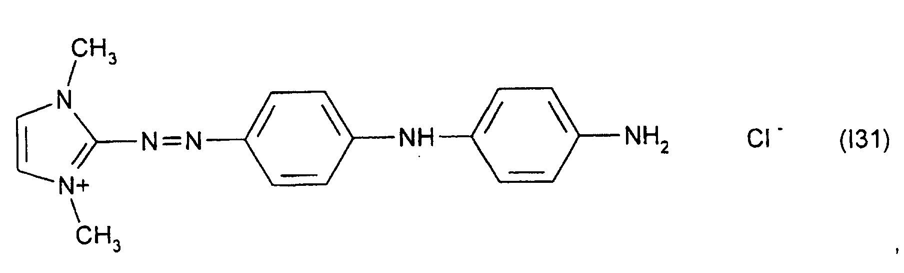 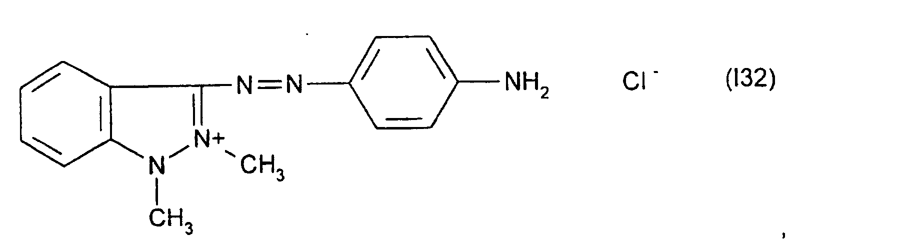 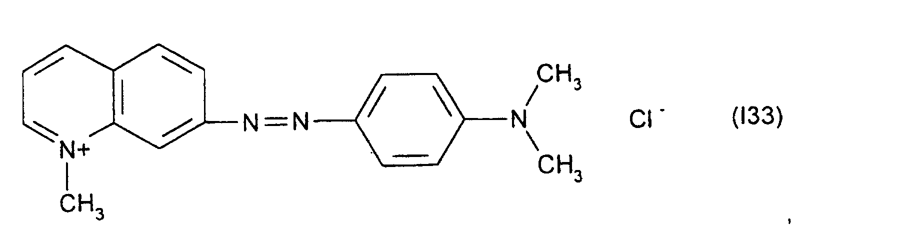 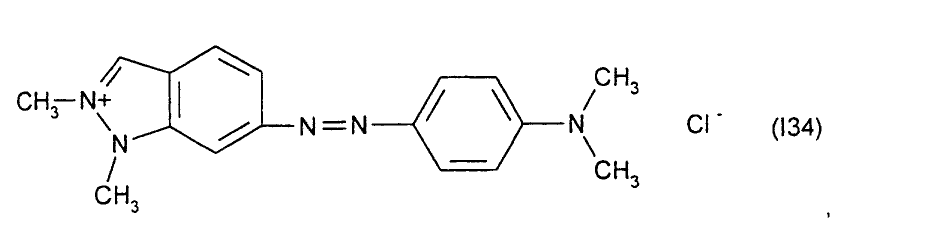 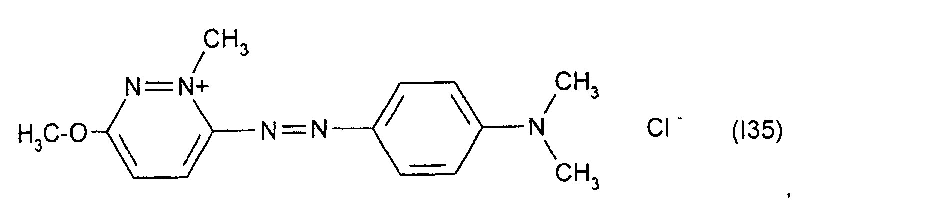 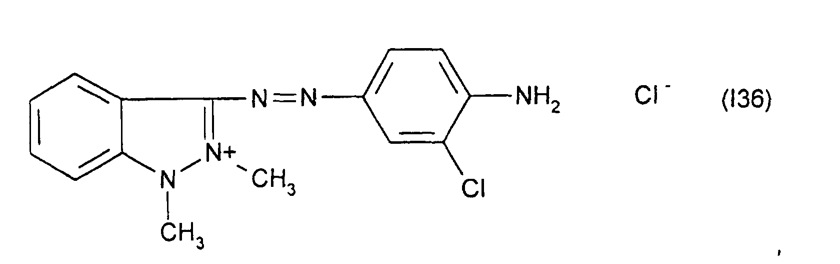 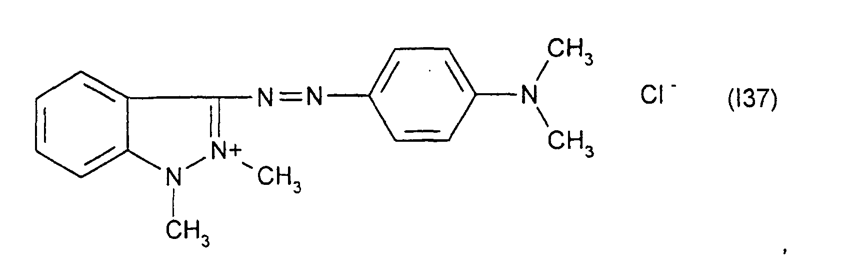 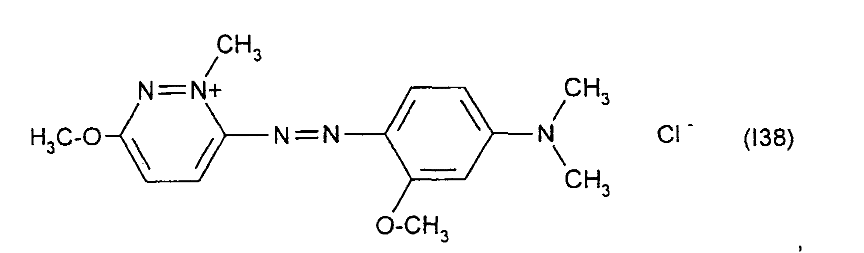 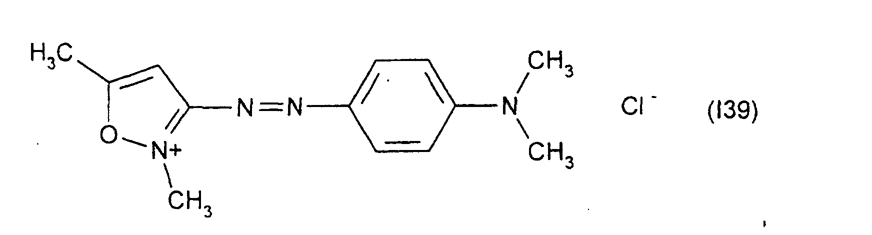 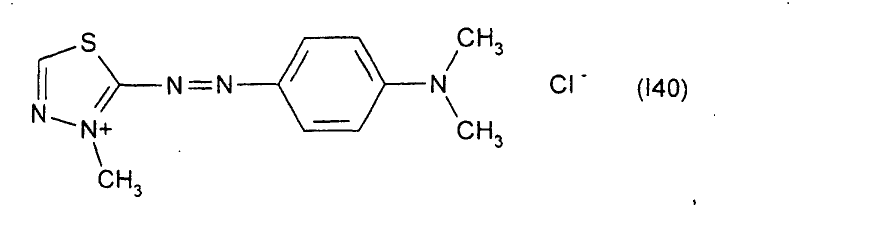 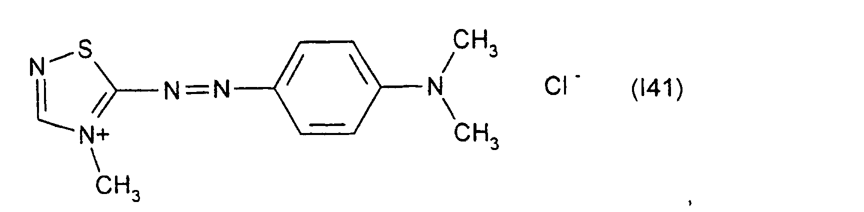 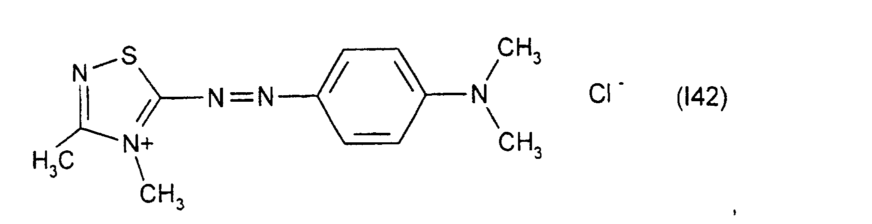 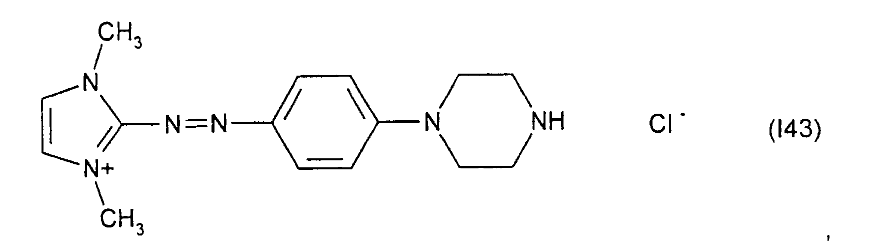 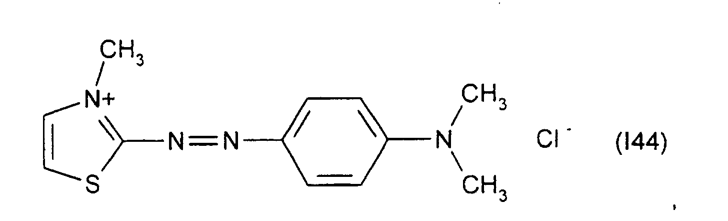 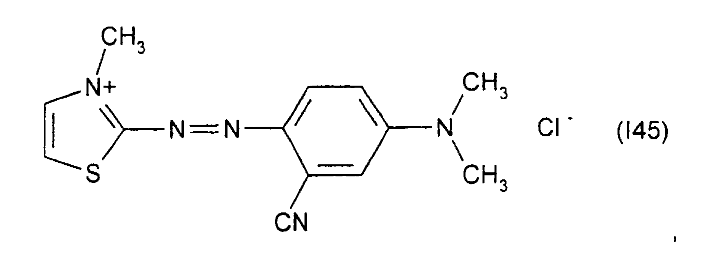 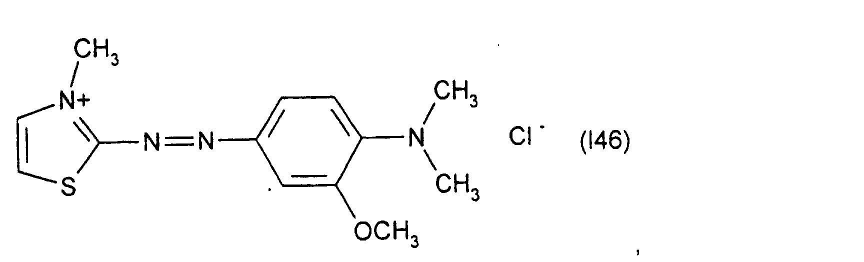 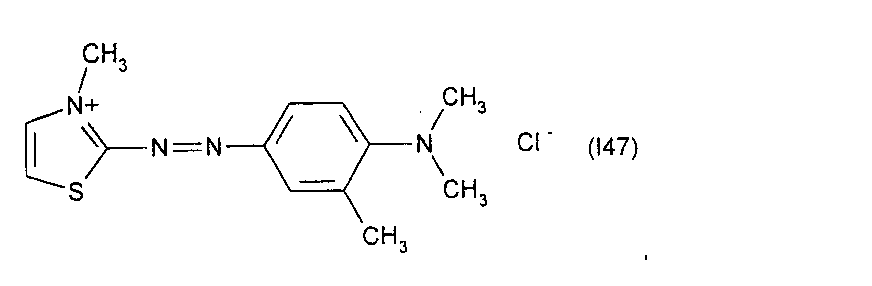 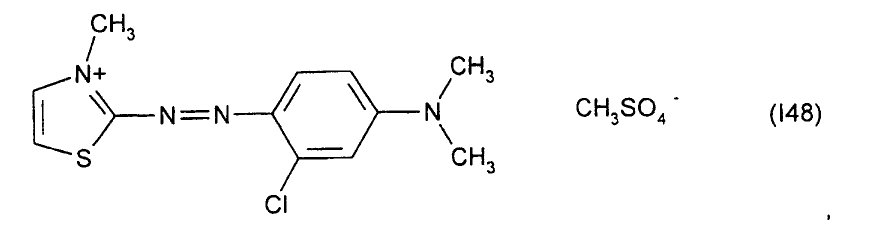 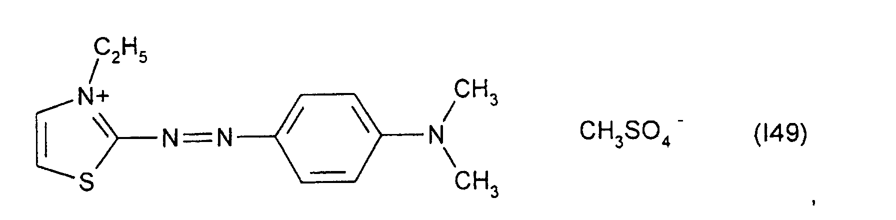 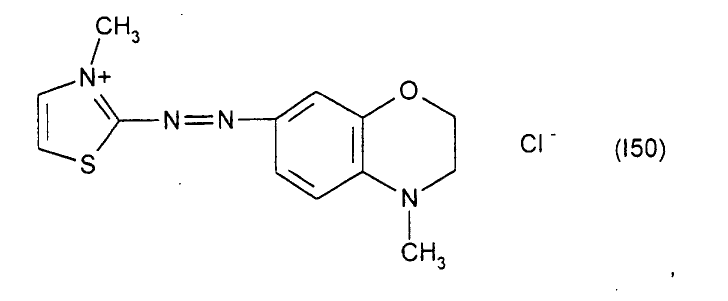 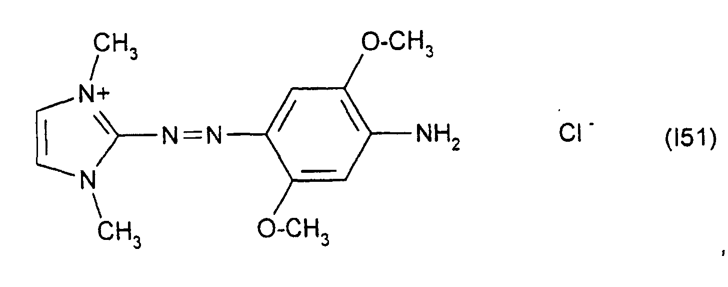 et

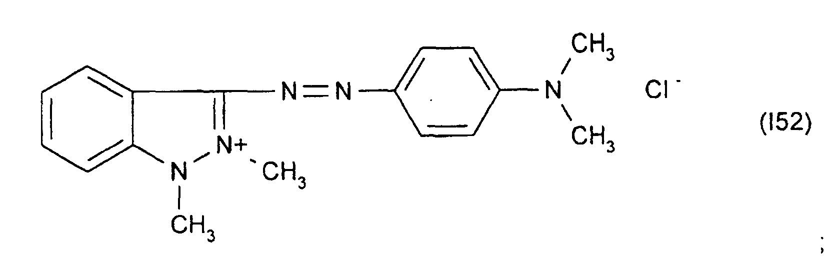

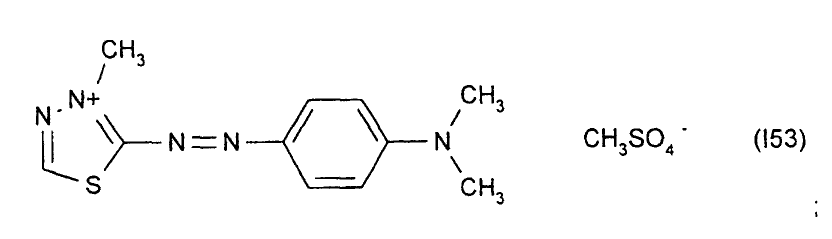

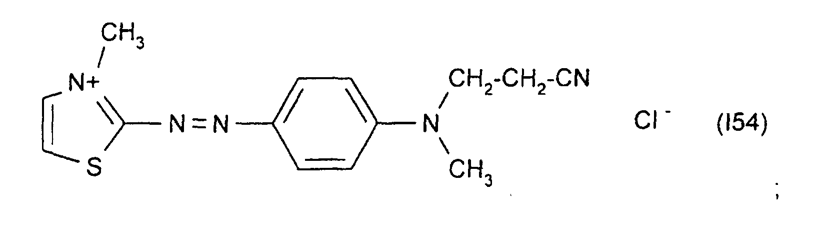

Parmi les composés de structures (I1) à (154) décrits ci-dessus, on préfère tout particulièrement les composés répondant aux structures (I1), (12), (I14) et (I31)

Parmi les colorants directs cationiques de formule (II) utilisables dans les compositions tinctoriales conformes à l'invention, on peut plus particulièrement citer les composés répondant aux structures (II1) à (II9) suivantes : 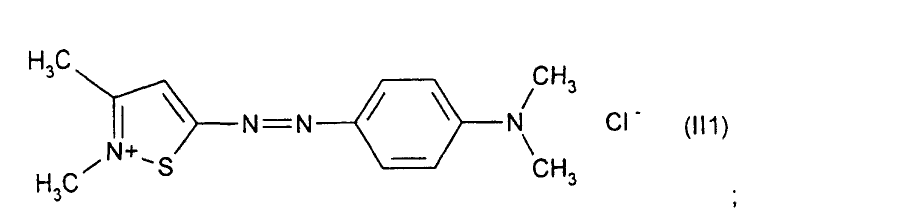 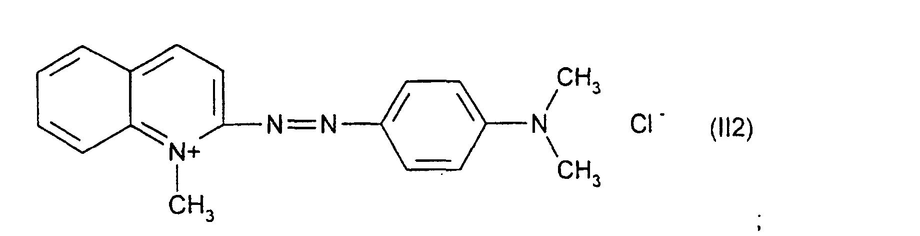 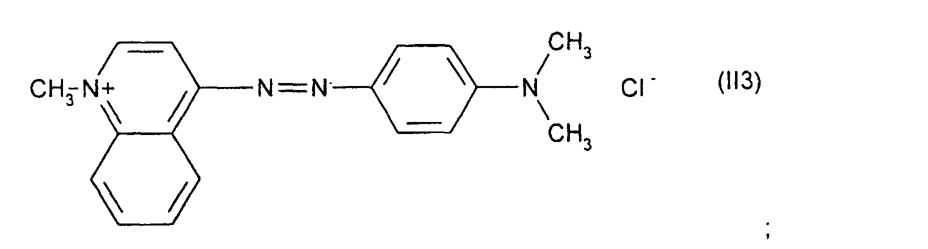 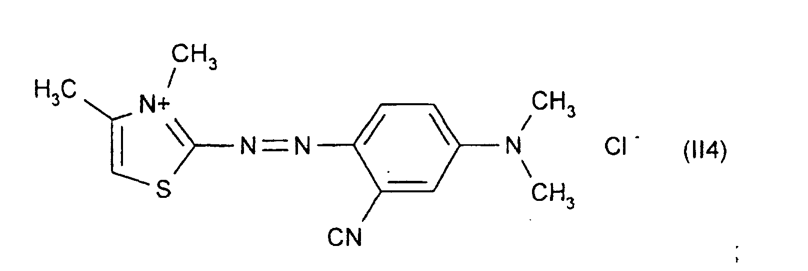 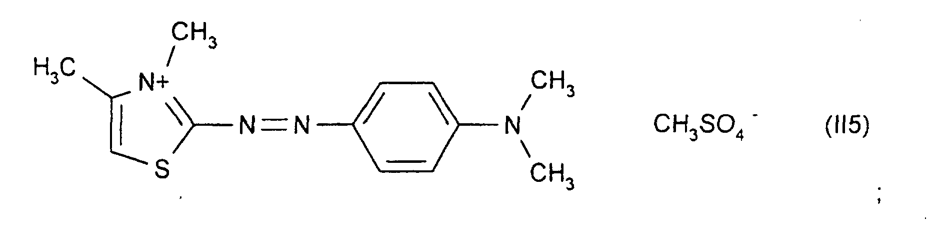 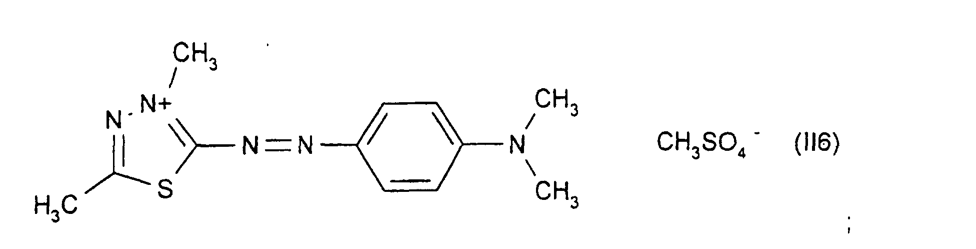 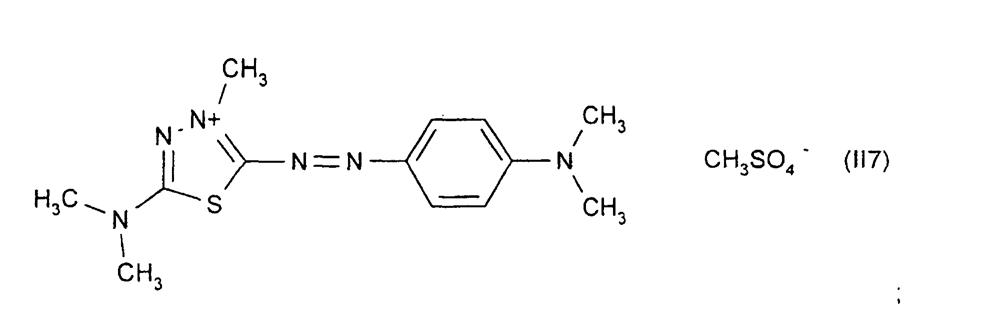 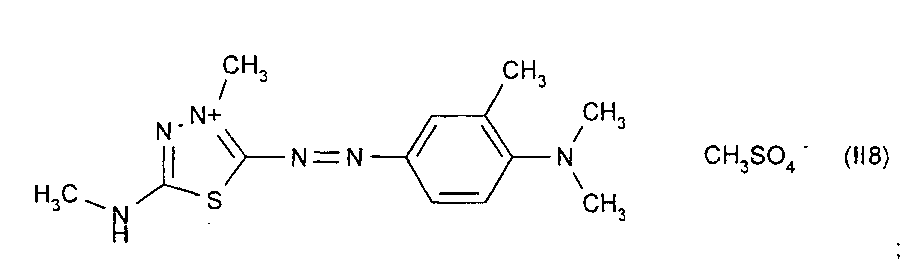 et

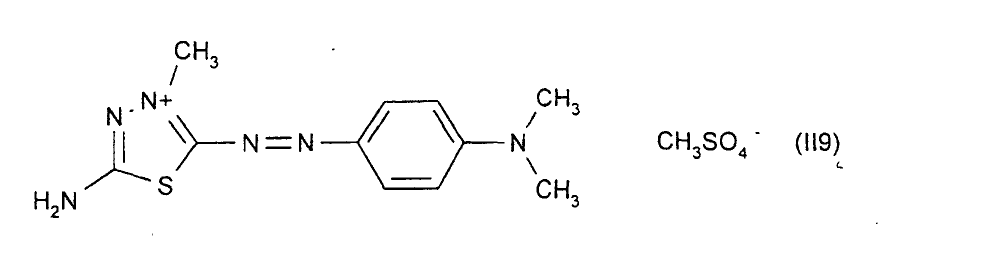

Parmi les colorants directs cationiques de formule (III), utilisables dans les compositions tinctoriales conformes à l'invention, on peut plus particulièrement citer les composés répondant aux structures (III1) à (III18) suivantes : 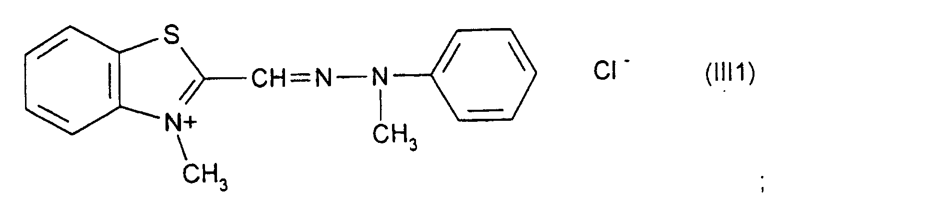 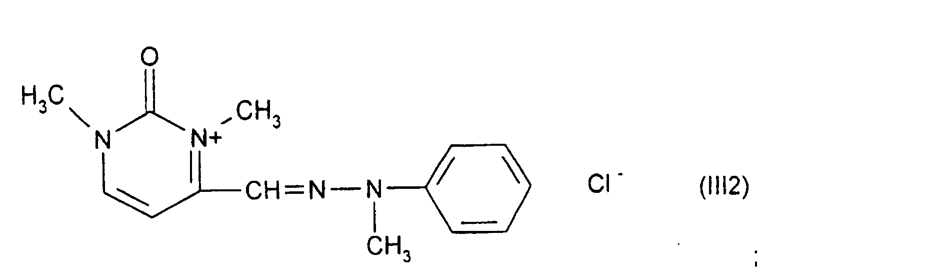 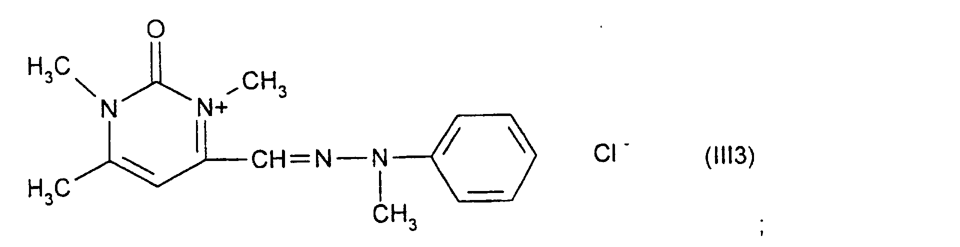 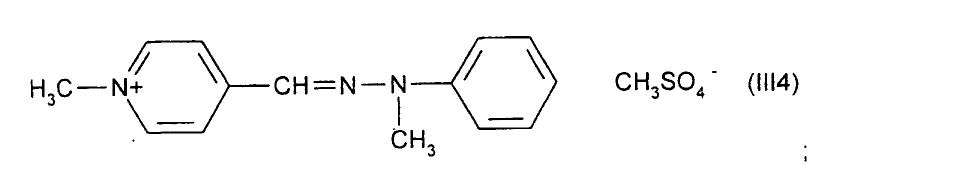 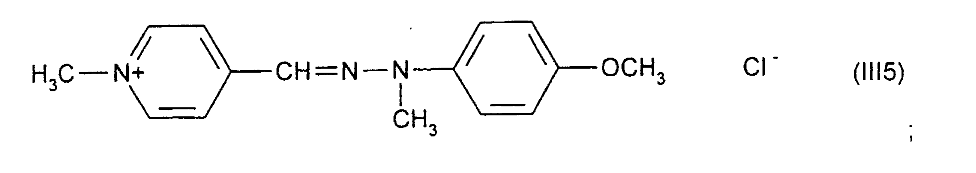 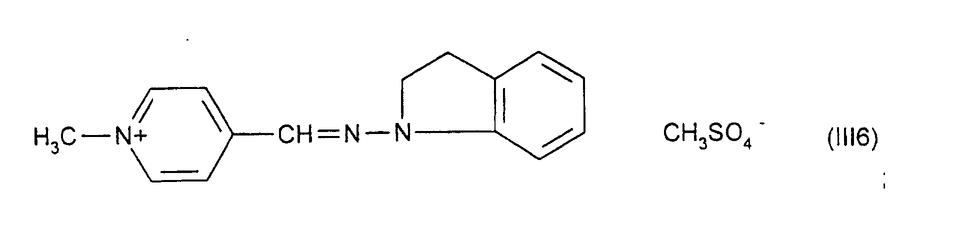 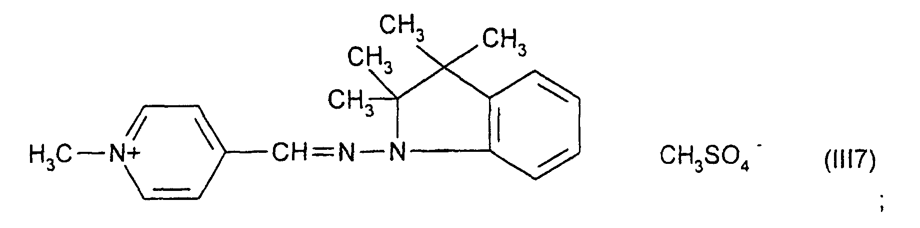 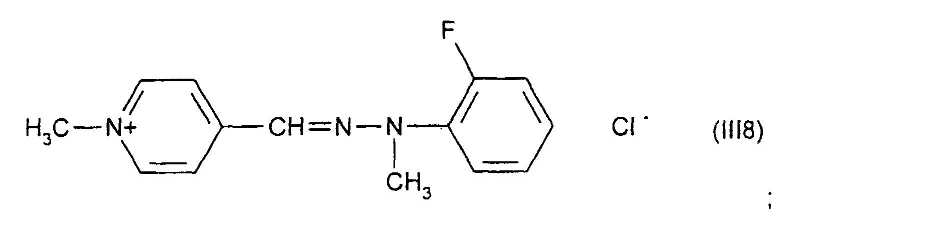 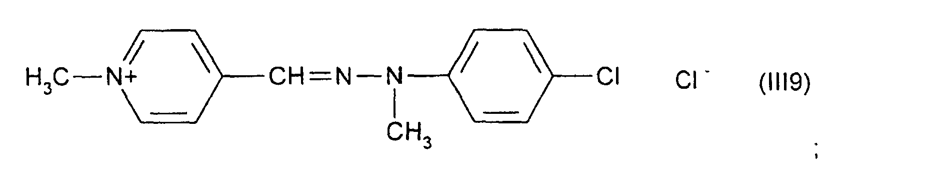 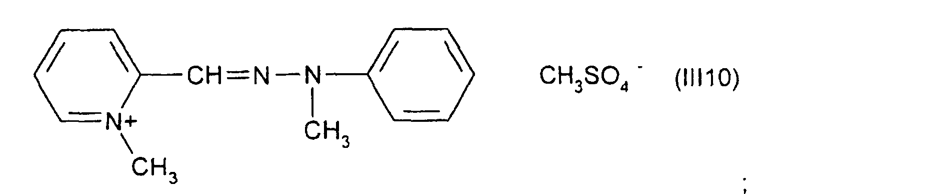 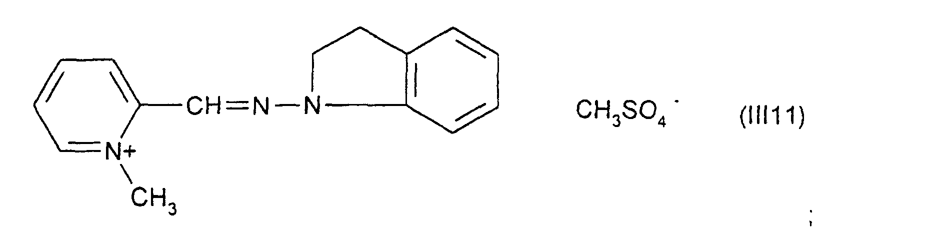 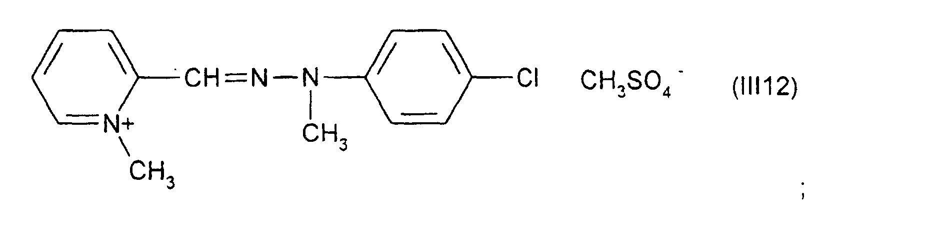 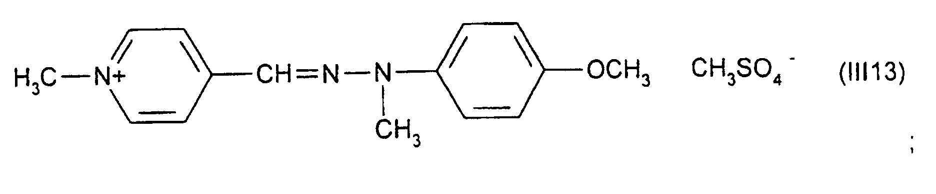 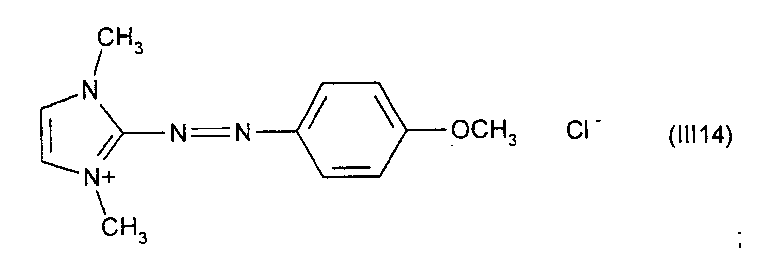 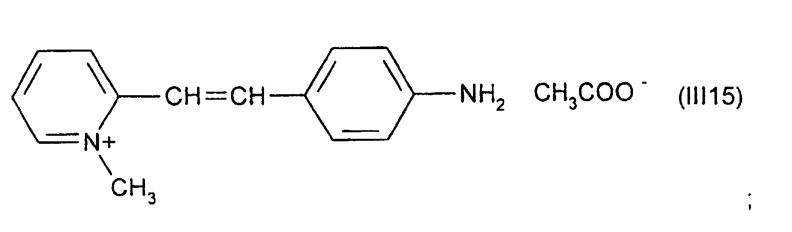 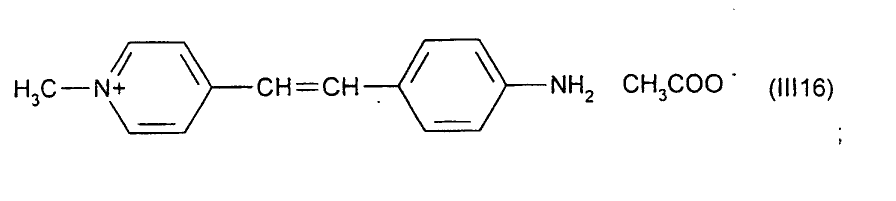 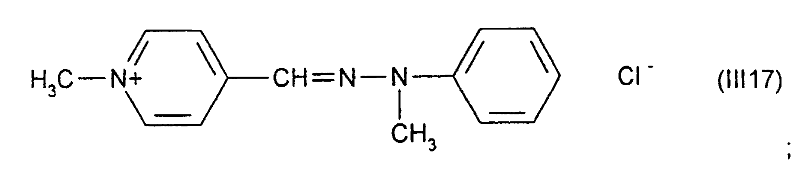 et

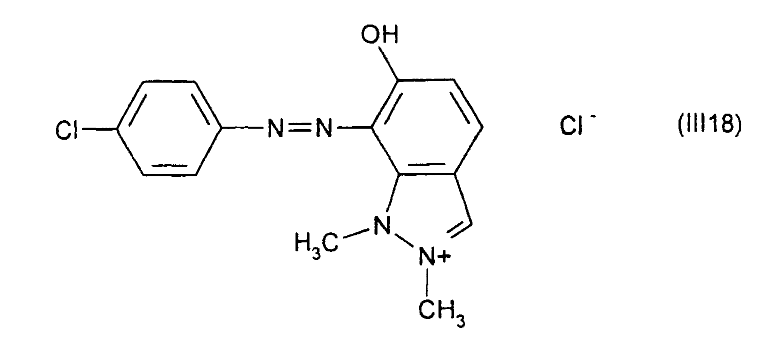

Parmi les composés particuliers de structures (III1) à (III18) décrits ci-dessus, on préfère tout particulièrement les composés répondant aux structures (III4), (III5) et (III13).

Parmi les colorants directs cationiques de formule (III'), utilisables dans les compositions tinctoriales conformes à l'invention, on peut plus particulièrement citer les composés répondant aux structures (III'1) à (III'3) suivantes : 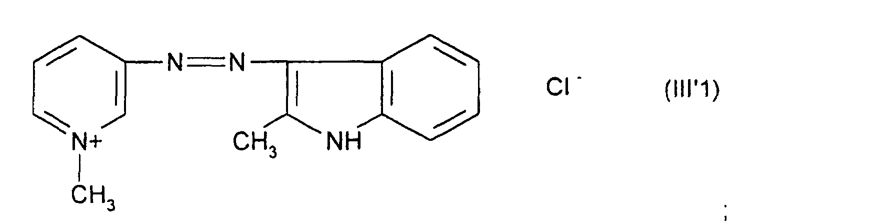 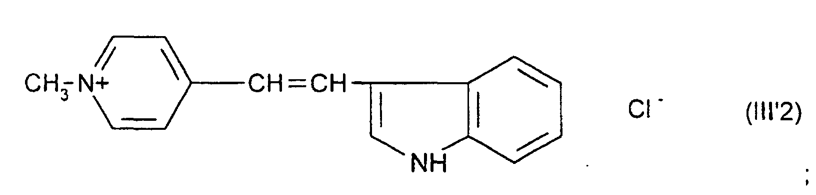 et

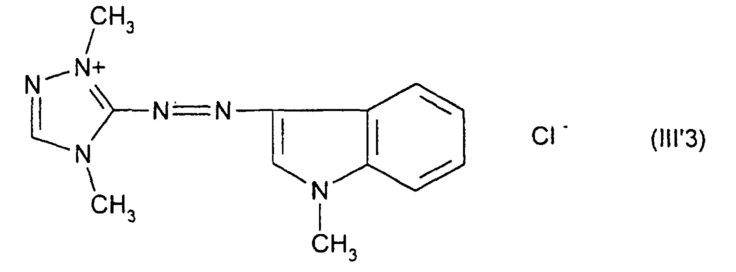

Parmi les colorants directs cationiques de formule (IV) utilisables dans les compositions tinctoriales conformes à l'invention, on peut citer plus particulièrement les composés de structures (IV)₁ à (IV)₇₇ suivantes :

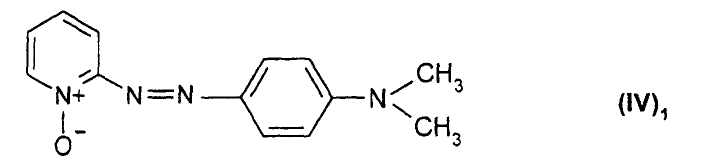

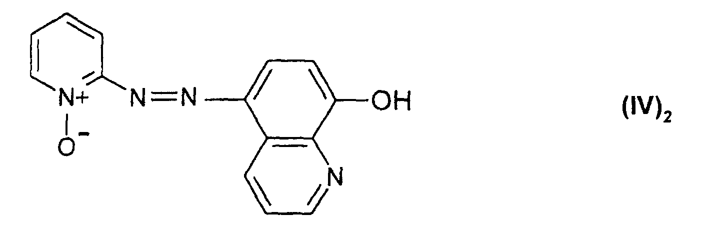

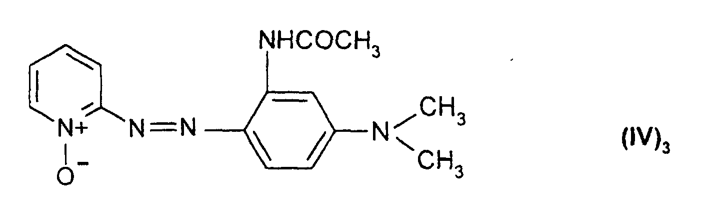

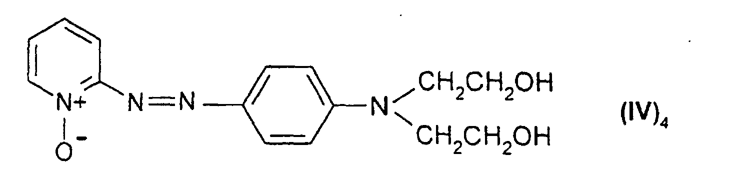

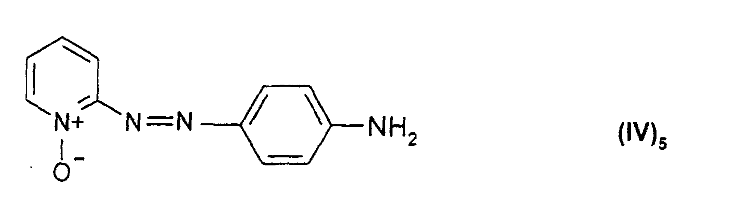

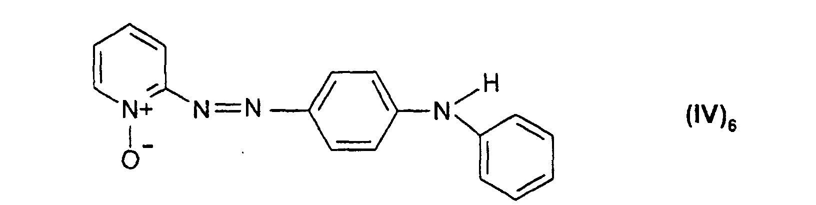

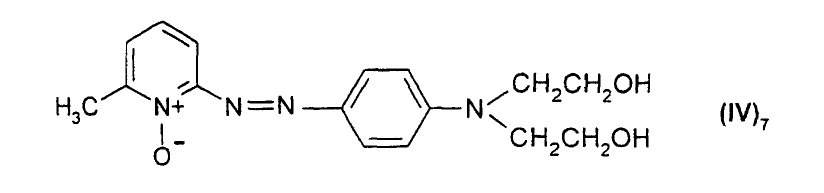

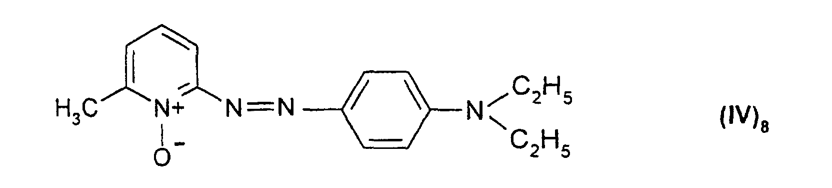

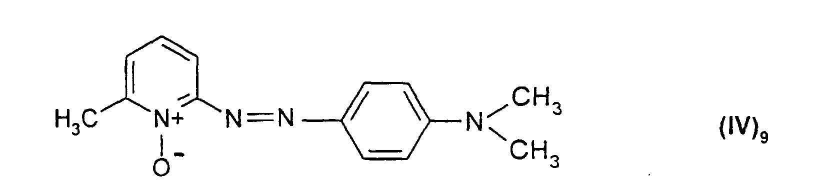

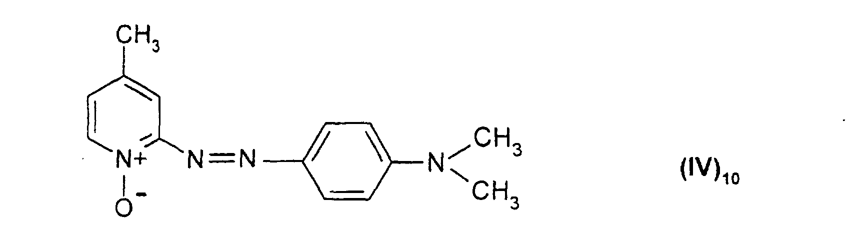

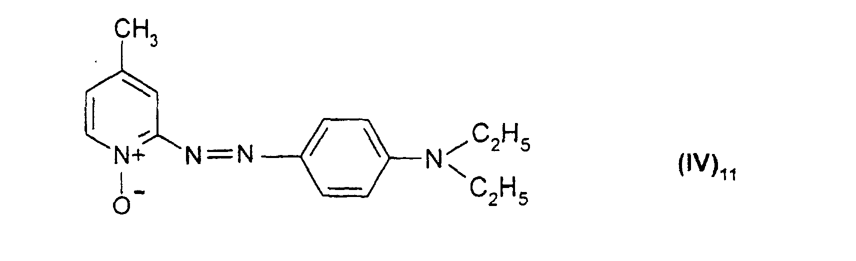

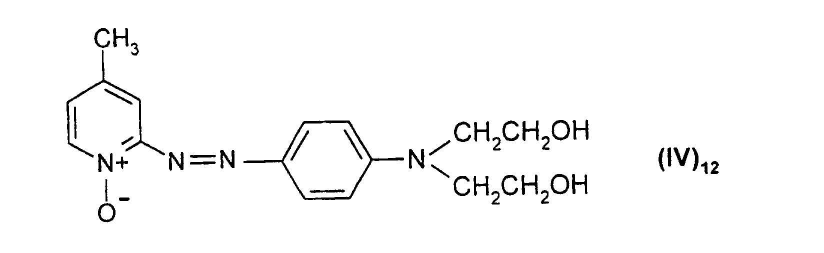

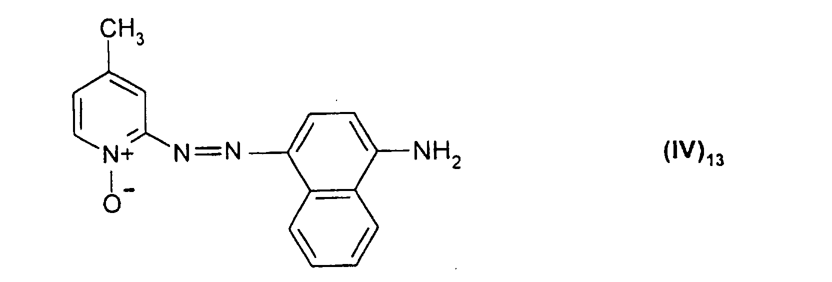

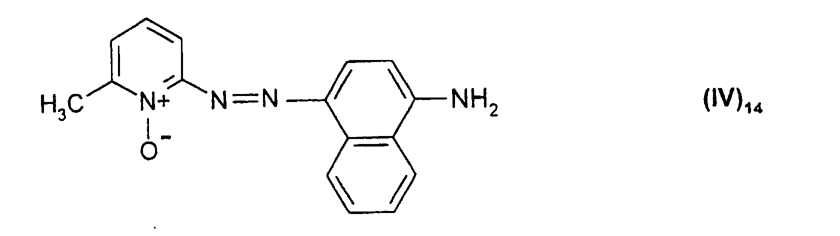

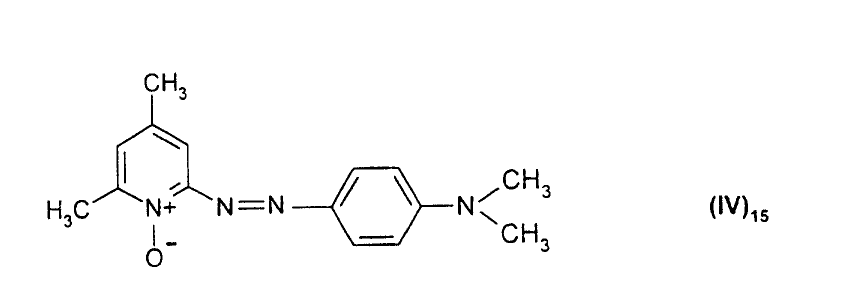

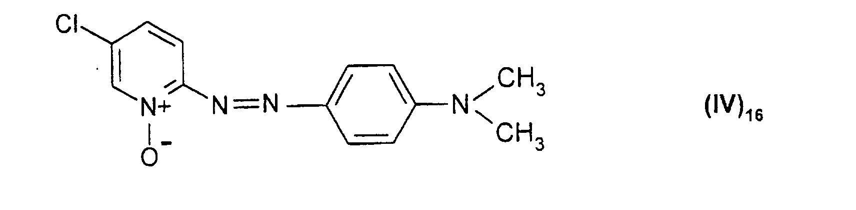

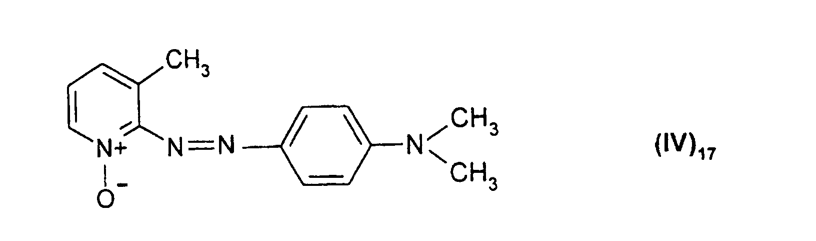

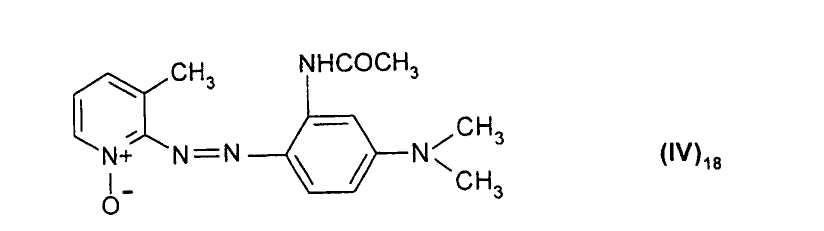

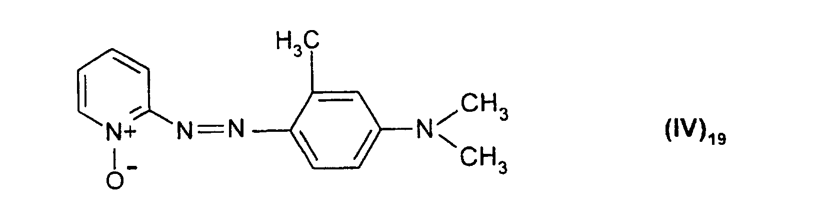

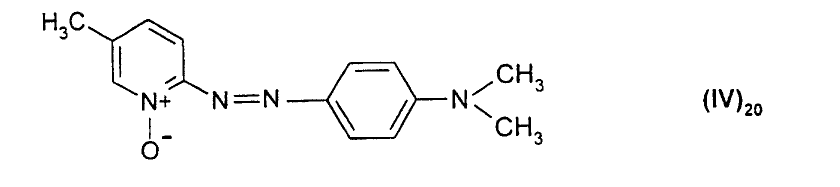

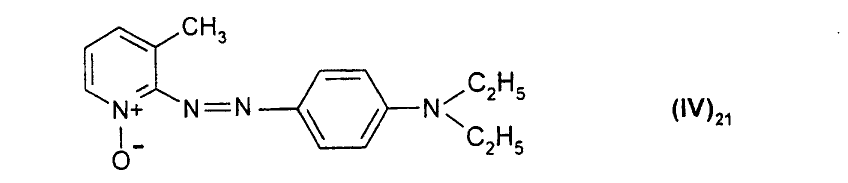

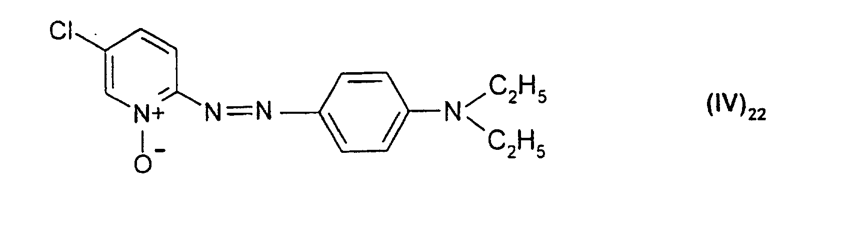

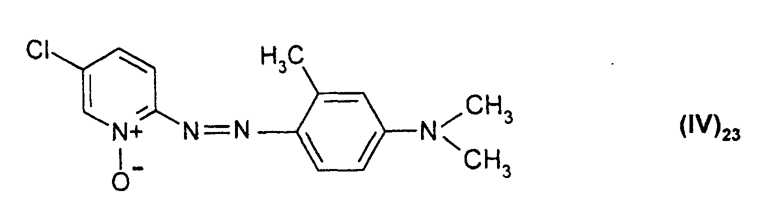

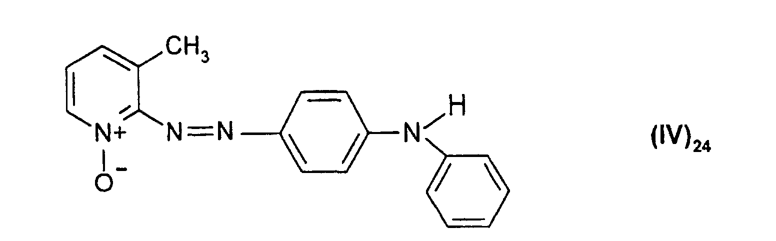

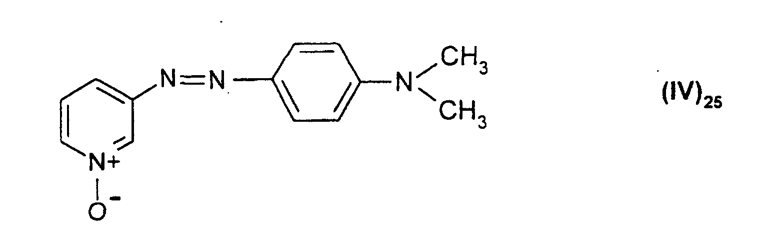

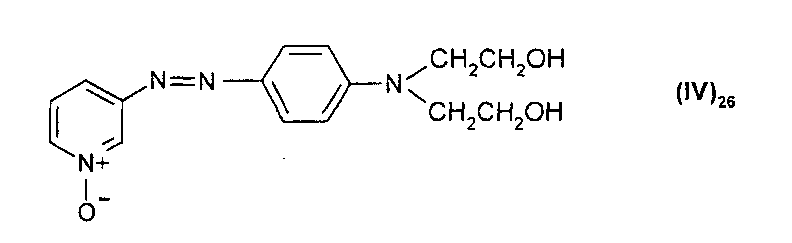

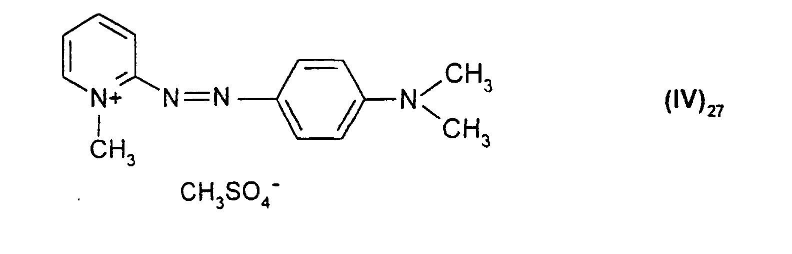

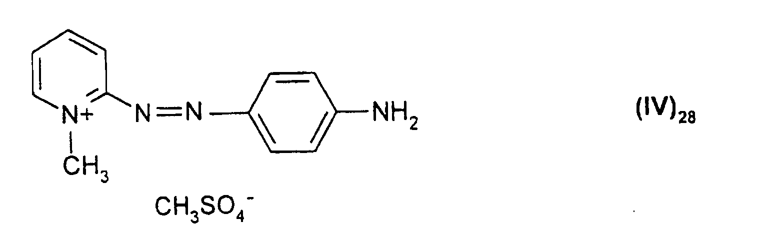

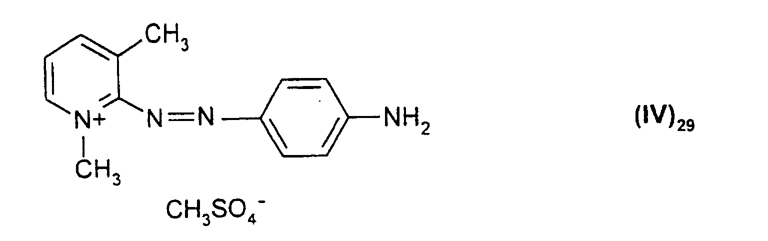

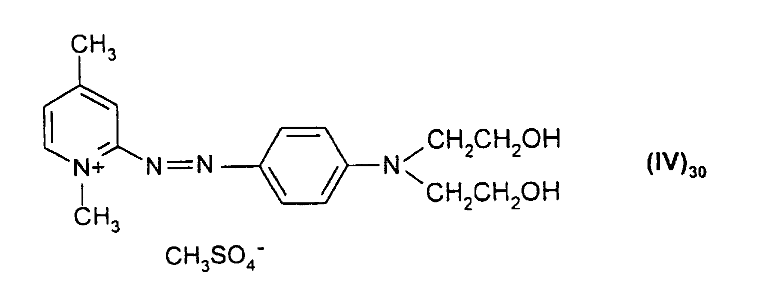

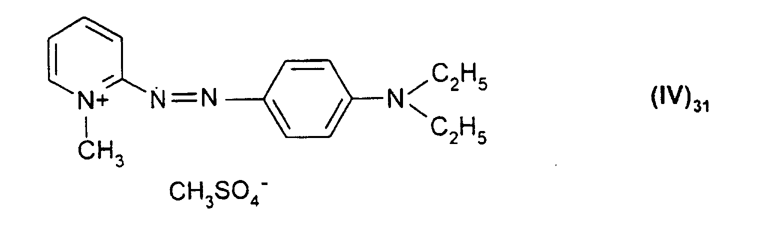

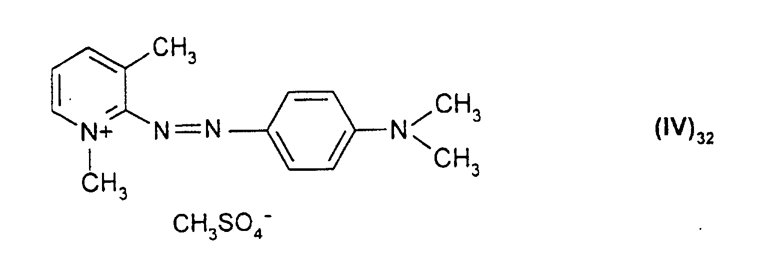

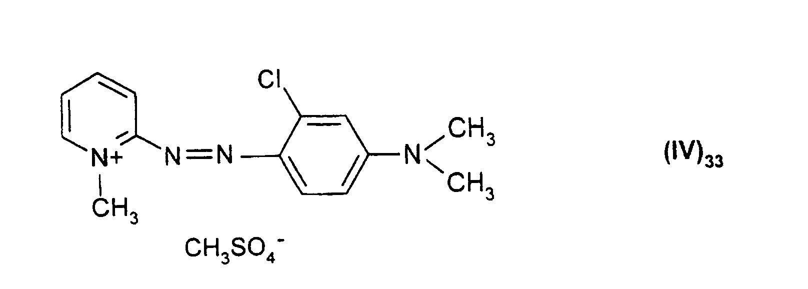

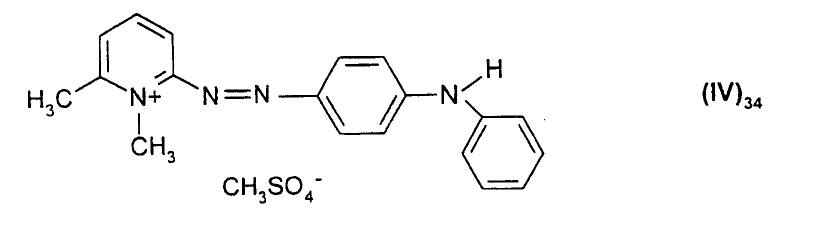

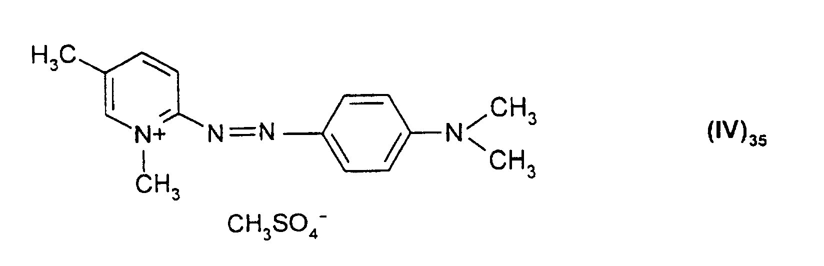

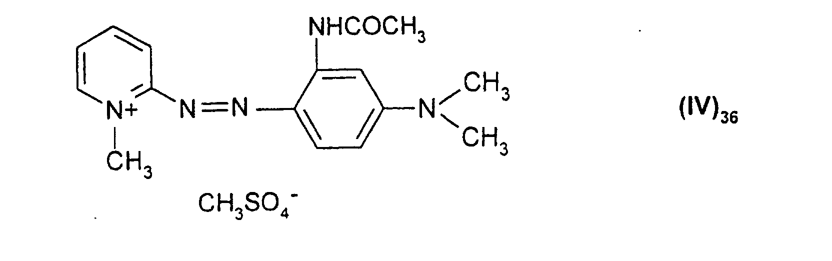

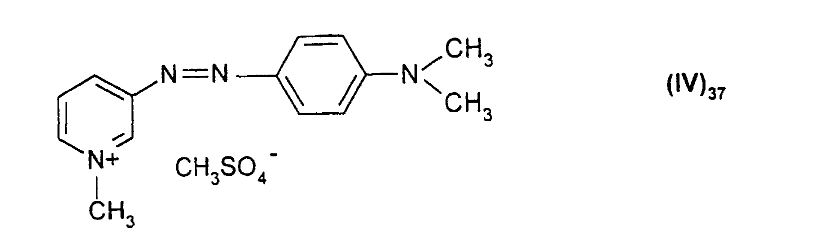

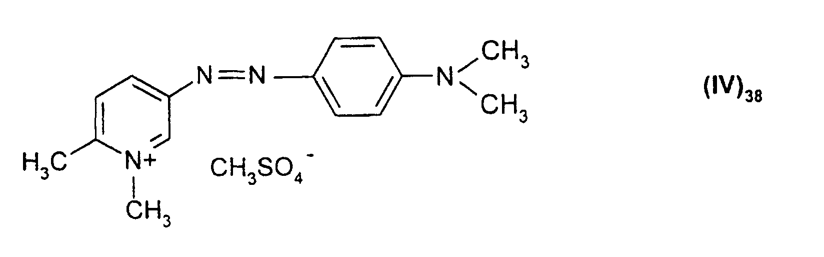

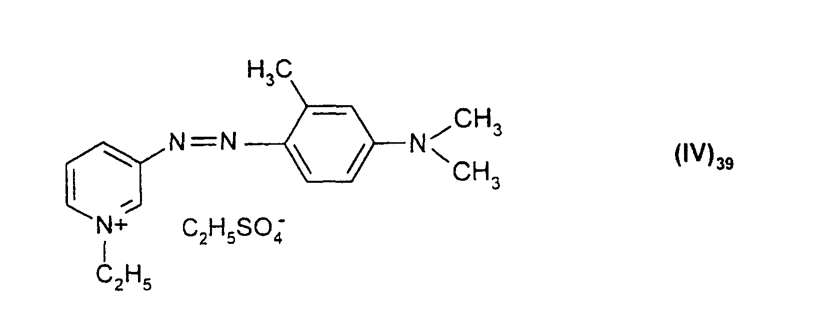

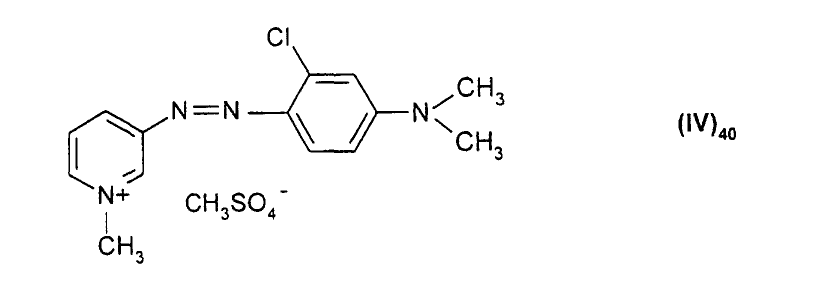

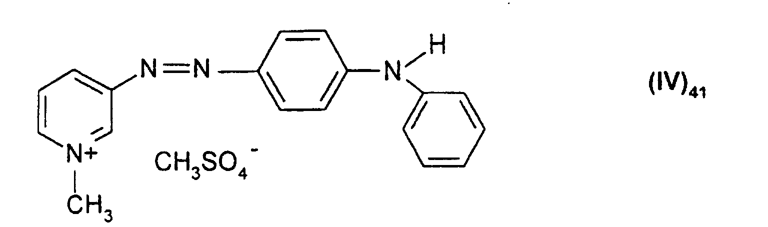

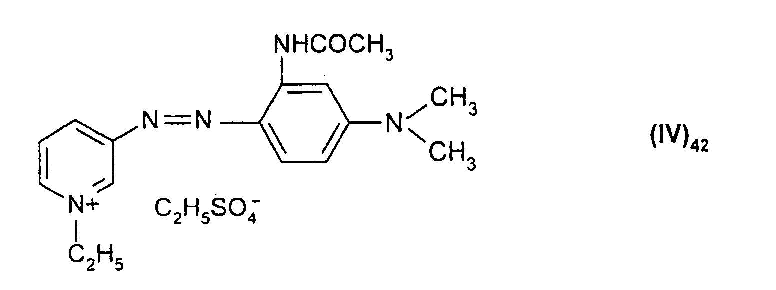

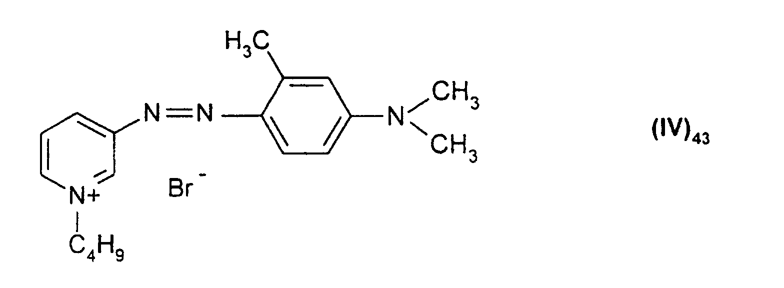

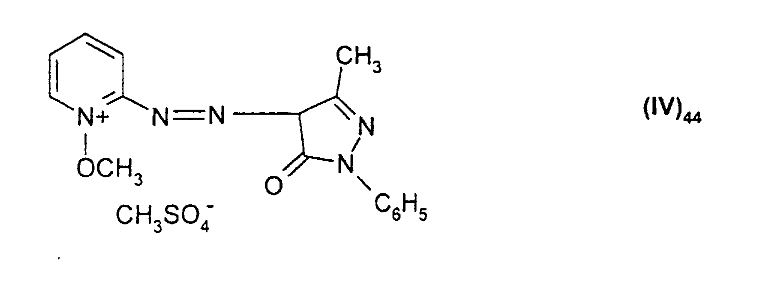

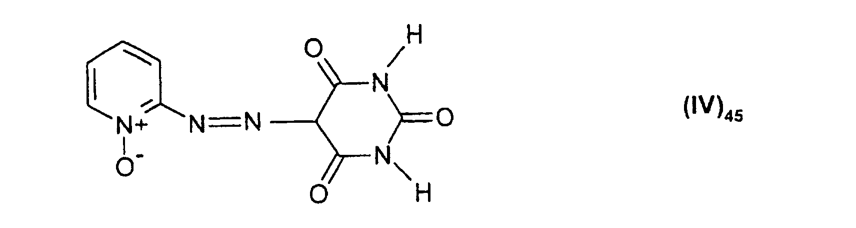

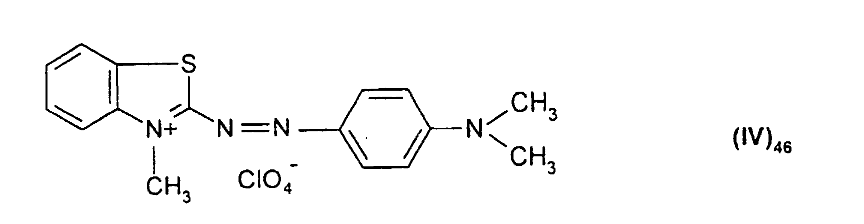

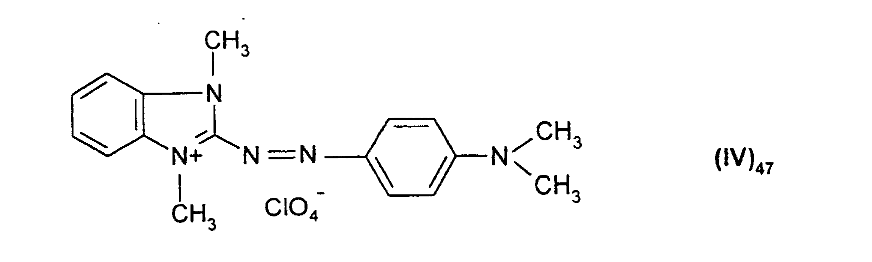

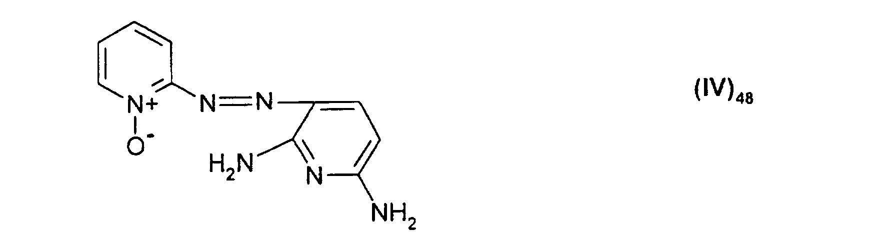

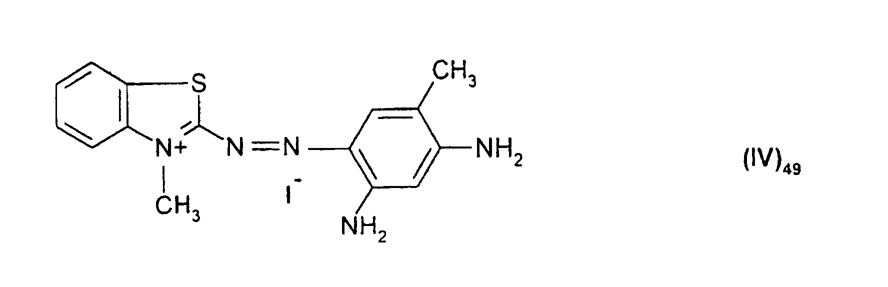

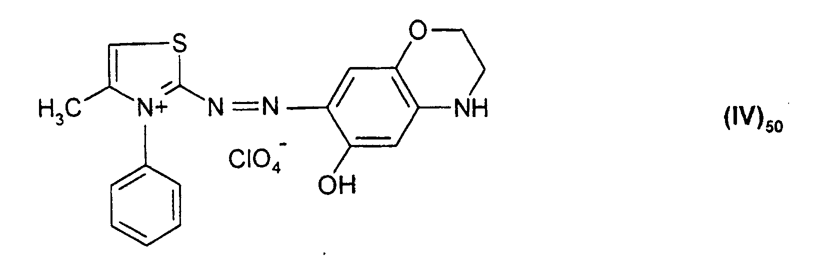

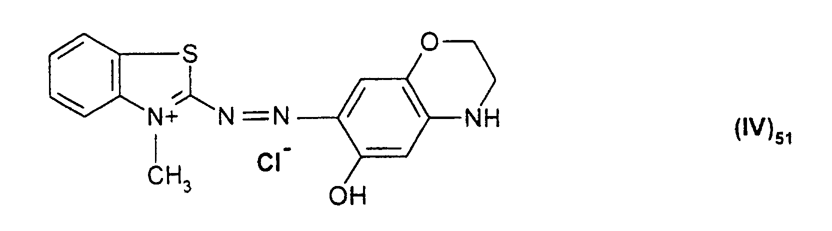

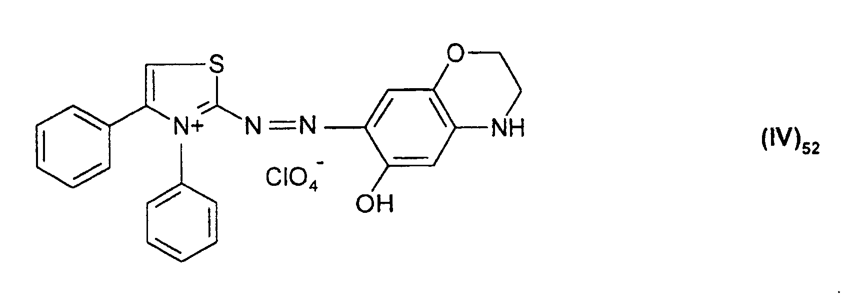

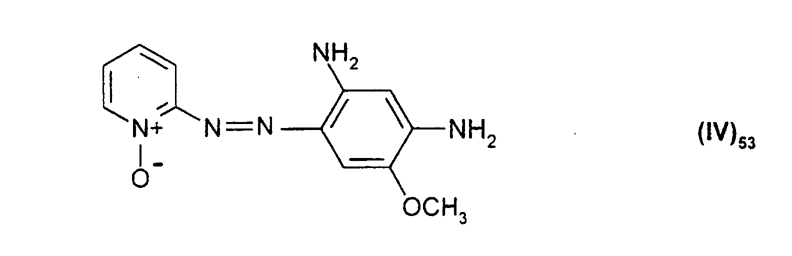

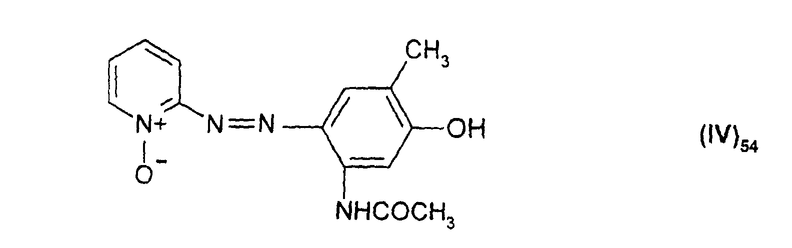

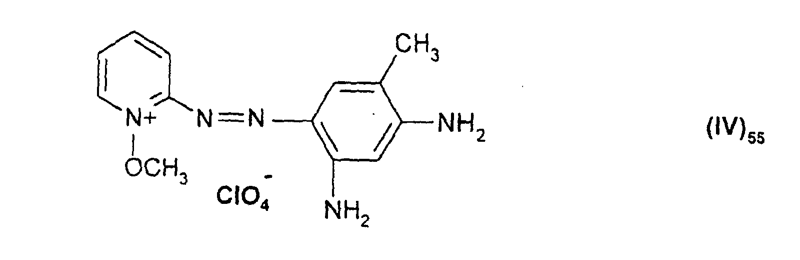

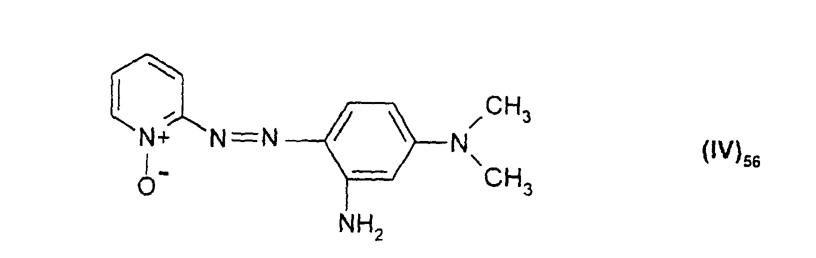

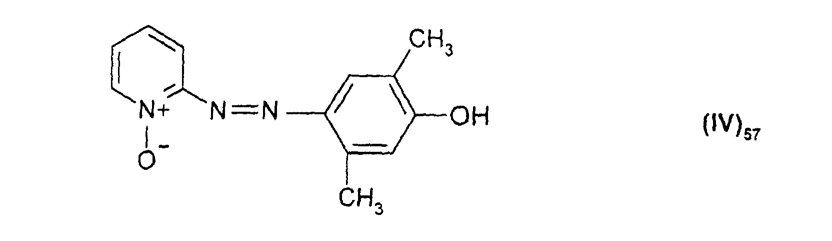

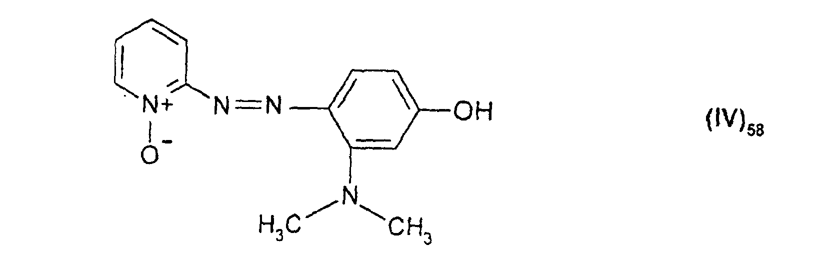

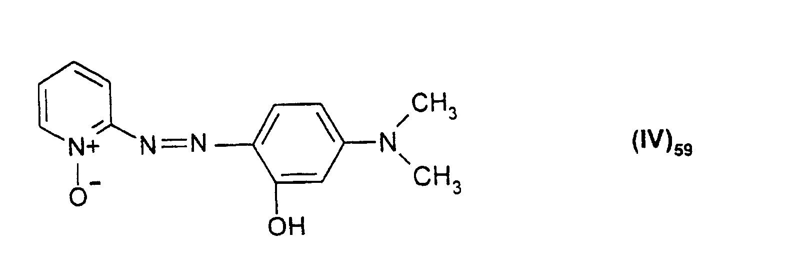

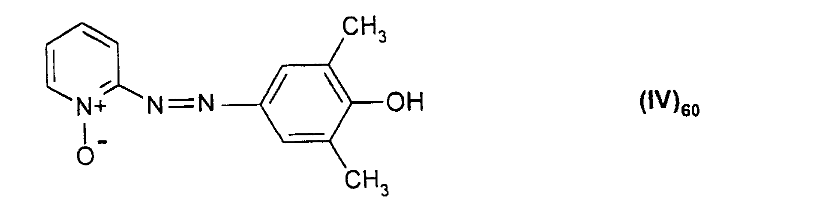

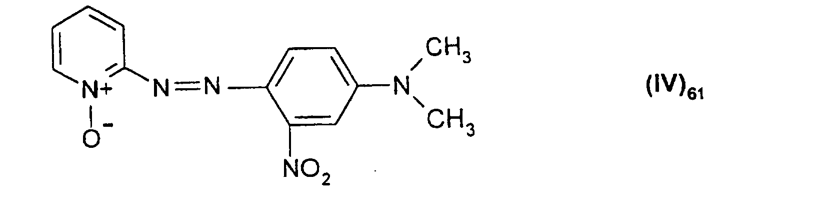

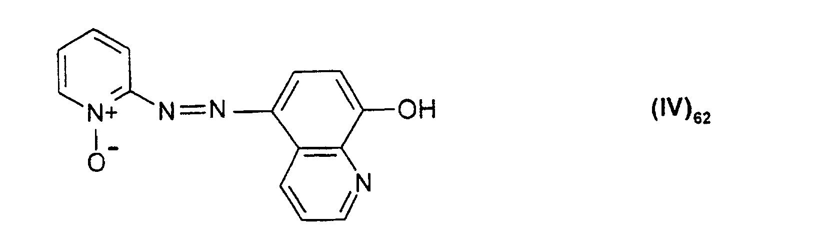

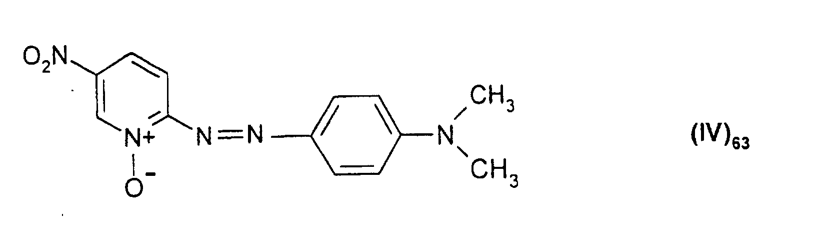

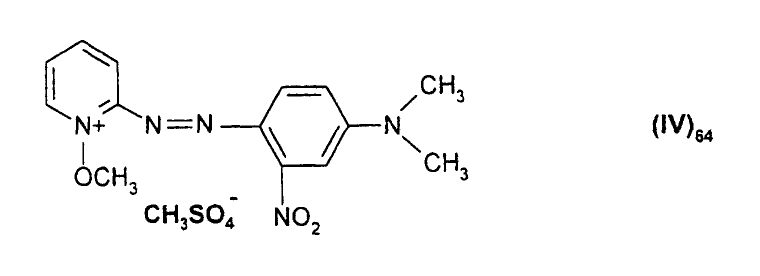

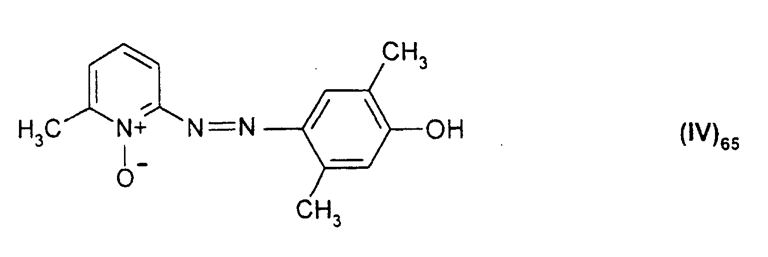

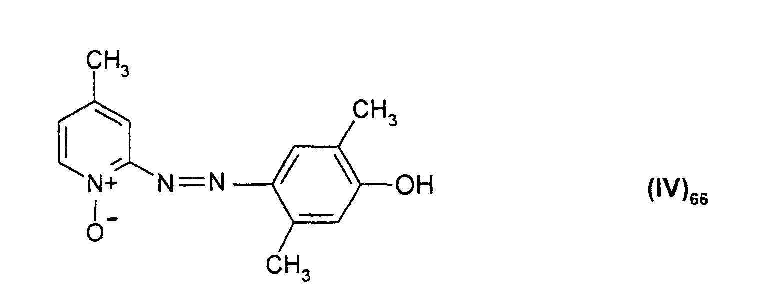

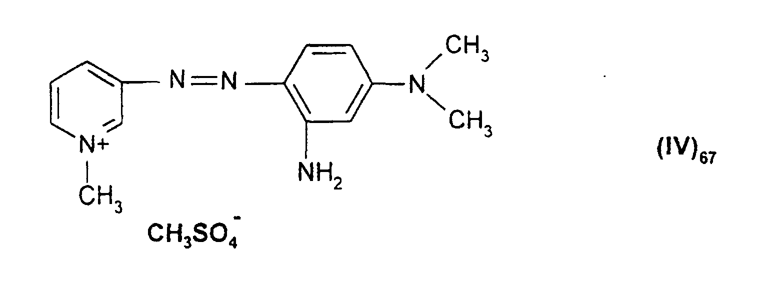

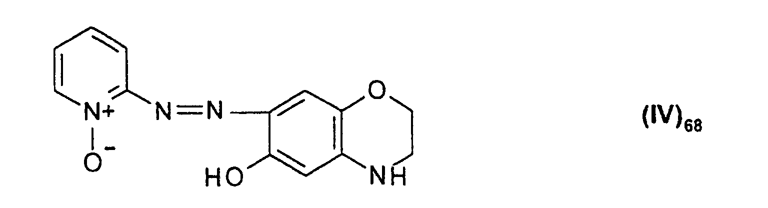

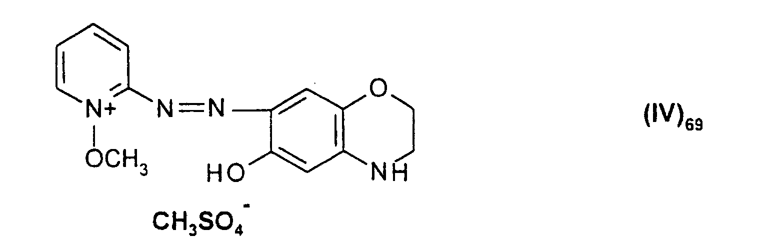

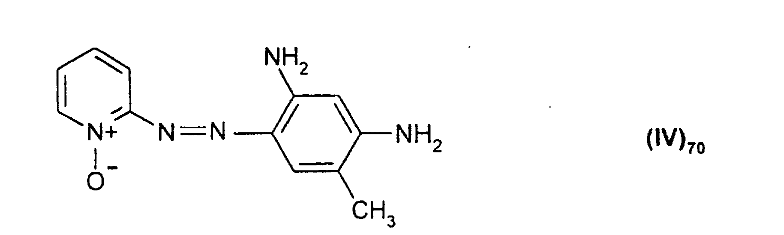

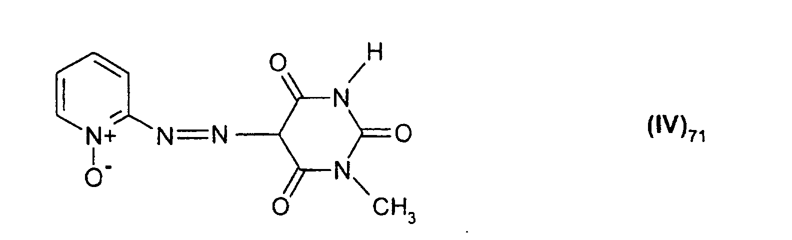

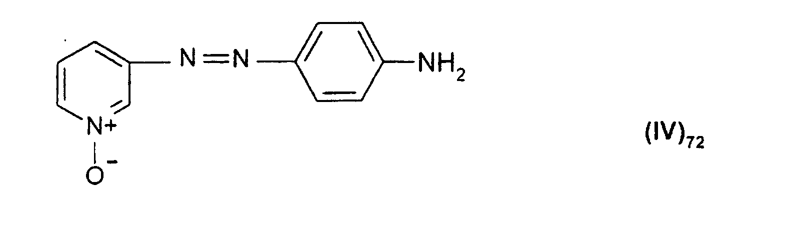

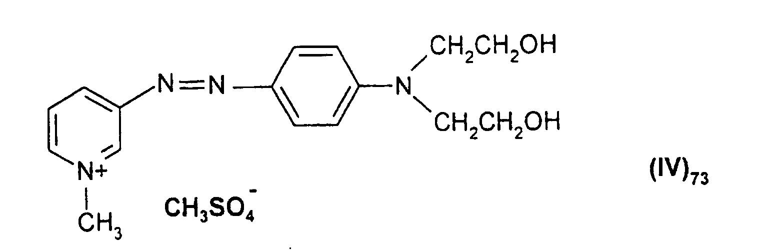

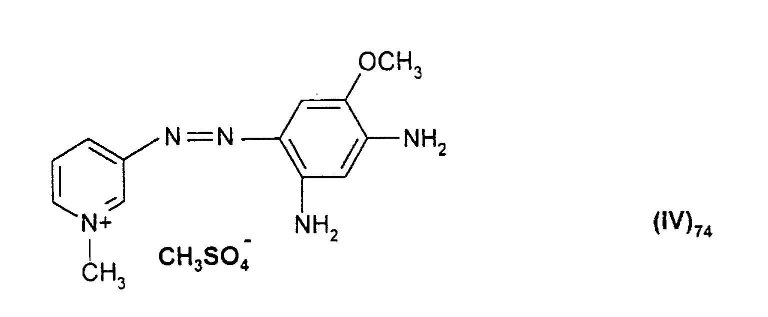

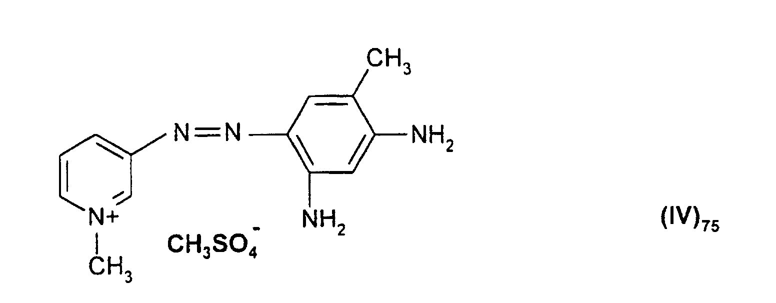

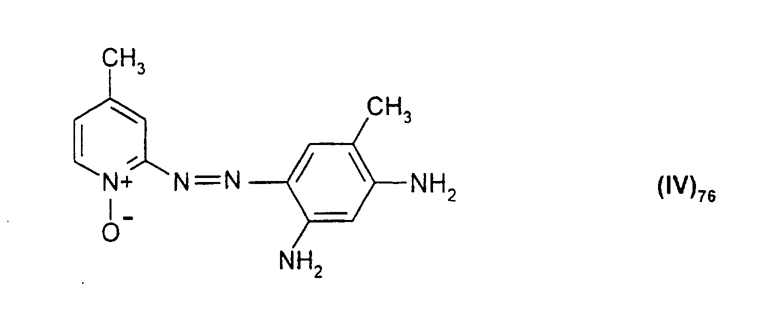

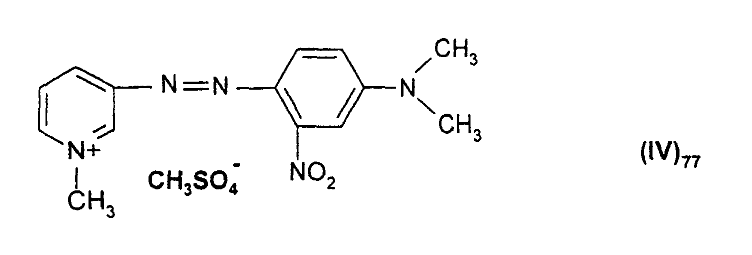

Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.

### (ii) L'agent tensio-actif non-ionique utilisable selon la présente invention est choisi dans le groupe constitué par :

**(ii)**_{**1**} - les alkylpolyglucosides;
**(ii)**_{**2**} - les esters d'acides gras de sucre ou d'alkylsucre;
**(ii)**_{**3**} - les sucramides gras ;
**(ii)**_{**4**}- leurs mélanges.

Les agents tensio-actifs non-ioniques de type alkylglucoside (ii)₁ utilisés dans la présente invention sont des produits bien connus en soi qui peuvent être plus particulièrement représentés par la formule générale (V) suivante : 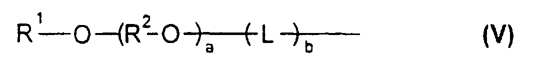 dans laquelle,
R¹ désigne un radical alkyle et/ou alcényle linéaire ou ramifié comportant environ de 8 à 24 atomes de carbone, un radical alkylphényle dont le groupe alkyle linéaire ou ramifié comporte environ de 8 à 24 atomes carbone, R² désigne un radical alkylène comportant de 2 à 4 atomes de carbone, L désigne un sucre réduit comportant de 5 à 6 atomes de carbone, a désigne une valeur allant de 0 à 10, et b désigne une valeur allant de 1 à 15.

Des alkylpolyglucosides préférés selon la présente invention sont des composés de formule (V) dans laquelle R¹ désigne plus particulièrement un radical alkyle et/ou alcényle linéaire ou ramifié comportant de 9 à 14 atomes de carbone, a désigne une valeur allant de 0 à 3 et plus particulièrement encore égale à zéro, L désigne le glucose, le fructose ou le galactose. Le degré de polymérisation (S) du saccharide, i.e. la valeur de b dans la formule (V), peut aller de 1 à 15. Selon l'invention, on préfère les sucres réduits contenant 80%, ou plus, de sucres dont le degré de polymérisation (S) prend une valeur allant de 1 à 4.

Des composés de formule (V) sont notamment représentés par les produits vendus par la société HENKEL sous la dénomination APG, tels que les produits APG 300, APG 350, APG 500, APG 550, APG 625, APG base 10-12; les produits vendus par la société SEPPIC sous les dénominations TRITON CG 110 (ou ORAMIX CG 110) et TRITON CG 312 (ou ORAMIX NS 10); ceux vendus par la société B.A.S.F. sous la dénomination LUTENSOL GD 70, ceux vendus par la société HENKEL sous les dénominations PLANTAREN 1200, PLANTAREN 1300, PLANTAREN 2000, et PLANTACARE 2000, PLANTACARE 818, PLANTACARE 1200.

Les agents tensio-actifs non-ioniques de type ester d'acide gras de sucre ou d'alkylsucre **(ii)**_{**2**} utilisés dans la présente invention sont des esters d'acides gras en C₄-C₂₂ de sucre ou d'alkylsucre dont on peut notamment citer:
- **(ii)**_{**2**}**(a)** les esters d'alkyl(C₁-C₄)glucoside, tels que :
   - le monostéarate de méthylglucoside, comme le produit vendu sous la dénomination GRILLOCOSE IS par la société GRILLOWERKE;
   - le sesquistéarate de méthylglucoside, comme le produit vendu sous la dénomination GLUCATE SS par la société AMERCHOL;
   - le décanoate d'éthyl-6-glucoside, comme le produit vendu sous la dénomination BIOSURF 10 par la société NOVO;
   - le mélange de mono et dicocoate (82/7) d'éthyl-6-glucoside, comme le produit vendu sous la dénomination BIOSURF COCO par la société NOVO;
   - le mélange de mono et dilaurate (84/8) d'éthyl-6-glucoside, comme le produit vendu sous la dénomination BIOSURF 12 par la société NOVO;
   - les monoesters d'acide gras en C₁₂-C₁₈ du butylglucoside, tels que le monococoate de butylglucoside, comme le produit vendu sous les dénominations REWOPOL V3101 ou REWOSAN V3101 et le monococoate de butylglucoside polyoxyéthyléné avec 3 moles d'oxyde d'éthylène, comme le produit vendu sous la dénomination REWOPOL V3122 par la société REWO.
- **(ii)**_{**2**}**(b)** les esters de glucose tels que:
   - l'O-hexadécanoyl-6-αD-glucose, l'O-octanoyl-6-D-glucose, l'O-oleyl-6-D-glucose, l'O-linoleyl-6-D-glucose, qui sont des composés connus pouvant être préparés, par exemple, à partir du chlorure d'acide correspondant et du D-glucose, selon la méthode décrite par E. REINEFELD et Coll. ; "Die Stärke" n°6 - pages 181-189, - 1968.
- **(ii)**_{**2**}**(c)** les monoesters de saccharose, tels que:
   - le monolaurate de saccharose, comme le produit vendu sous la dénomination GRILLOTEN LES 65, et,
   - le monococoate de saccharose vendu sous la dénomination GRILLOTEN LES 65K, par la société GRILLO-WERKE.

Les agents tensio-actifs non-ioniques de type sucramide gras **(ii)**_{**3**} utilisés dans la présente invention sont des composés comportant au moins une fonction amide et incluant au moins une portion sucre ou dérivée de sucre et au moins une chaîne grasse; de tels composés peuvent, par exemple, résulter de l'action d'un acide gras ou d'un dérivé d'acide gras sur la fonction amine d'un aminosucre, ou de l'action d'une amine grasse sur un sucre comportant une fonction acide carboxylique (libre ou sous forme de lactone) ou dérivée d'acide carboxylique ou encore une fonction carbonyle, et ceci éventuellement en présence de co-réactifs adéquats.

Les sucramide gras **(ii)**_{**3**} utilisés sont choisis de préférence parmi:
**(ii)**_{**3**}**(a)** les aldonamides N-substitués et,
**(ii)**_{**3**}**(b)** les amides d'acides gras polyhydroxylés ou leurs mélanges.
**(ii)**_{**3**}**(a)** Les aldonamides N-substitués utilisables selon l'invention peuvent être choisis parmi ceux décrits dans la demande de brevet EP-A-550 106 dont le contenu fait partie intégrante de la présente description.

Parmi ceux-ci, on peut citer :
- les lactobionamides N-substitués, les maltobionamides N-substitués, les cellobionamides N-substitués, les mellibionamides N-substitués et les gentiobionamides N-substitués tels que :
   (i) les N-alkyl lactobionamides, N-alkyl maltobionamides, les N-alkyl cellobionamides, les N-alkyl mellibionamides ou les N-alkyl gentiobionamides mono- ou disubstitués par un groupe hydrocarboné aliphatique, linéaire ou ramifié, saturé ou insaturé, pouvant contenir des hétéroatomes ayant de préférence jusqu'à 36 atomes de carbone, plus préférentiellement jusqu'à 24 atomes de carbone et encore plus particulièrement de 8 à 18 (par exemple méthyle, éthyle, amyle, hexyle, heptyle, nonyle, décyle, undécyle, dodecyle, tridécyle, tétradécyle, pentadécyle, hexadécyle, heptadécyle, octadécyle ; allyle, undécenyle, oléyle, linoléyle, propényle, heptènyle), par un groupe hydrocarboné aromatique (par exemple benzyle, aniline, benzyle substitué, phényléthyle, phénoxyéthyle, vinylbenzyle) ou des groupes cycloaliphatiques (par exemple cyclopentyle, cyclohexyle) ;
   (ii) les esters de N-lactobionyl aminoacide, où l'aminoacide peut désigner notamment : alanine, valine, glycine, lysine, leucine, arginine, acide aspartique, acide glutamique, thréonine, serine, cystéine, histidine, tyrosine, méthionine ou bien peut être choisi par exemple parmi la β-alanine, la sarcosine, l'acide gamma-aminobutyrique, l'ornithine, la citrulline ou leurs équivalents ; lesdits esters de N-lactobionyl aminoacides étant monosubstitués par un groupe de formule : 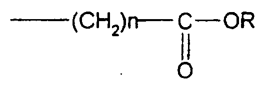 où R est un groupe hydrocarboné aliphatique pouvant contenir jusqu'à 36 atomes de carbones et n est un entier supérieur à 1, ainsi que les esters de N-maltobionyl aminoacide, les esters de N-mellibionyl aminoacide, les esters de N-cellobionyl aminoacide, les esters de N-gentiobionyl aminoacide correspondants,
   (iii) les N-(alkyloxy)alkyl-lactobionamides mono ou disubstitués par un groupe -(CH₂)ₙOR' où R' est un groupe hydrocarboné alipatique, aromatique ou cycloaliphatique tel que défini dans le paragraphe (i) ;
   (iv) les N-(polyalkyloxy)alkyl lactobionamides, les N-(polyalkyloxy)alkyl maltobionamides, les N-(polyalkyloxy)alkyl cellobionamides, les N-(polyalkyloxy)alkyl mellibionamides ou les N-(polyalkyloxy)alkyl gentiobionamides qui sont mono ou disubstitués par un groupe - R₁-(OR₁)ₙR₁R₂ où R₁ est un groupe alkylène tel que éthylène, propylène, ou leurs mélanges, n est un entier supérieur à 1, R₂ est un groupe lactobionamide, maltobionamide, cellobionamide, melliobionamide ou gentiobionamide.

**(ii)**_{**3**}**(b)** Les amides gras polyhydroxylés conformes à la présente invention sont choisis de préférence parmi ceux décrits dans le brevet EP-B-550 656 dont le contenu fait partie intégrante de la description et répondant à la formule (VI) suivante : 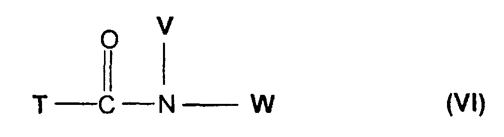 dans laquelle,
T désigne un groupe hydrocarboné en C₅-C₃₁, de préférence une chaîne alkyle ou alcényle linéaire en C₇-C₁₅ ou leurs mélanges ;
**V** désigne hydrogène, un radical hydrocarboné en C₁-C₄, 2-hydroxy éthyle, 2-hydroxypropyle ou leurs mélanges, de préférence un alkyle en C₁-C₄ tel que méthyle, éthyle, propyle, isopropyle, N-butyle et plus particulièrement méthyle ;
**W** désigne un groupe polyhydroxyhydrocarboné ayant une chaîne linéaire hydrocarbonée avec au moins 3 groupes hydroxyles directement liés à la chaîne, ou un dérivé alcoxylé dudit groupe (de préférence éthoxylé ou propoxylé).
**W** est de préférence un dérivé de sucre réducteur obtenu par réaction d'amination réductrice et plus préférentiellement un groupe glycityle. Parmi les sucres réducteurs, on peut citer le glucose, le maltose, le lactose, le galactose, le mannose, et le xylose.
Plus préférentiellement encore, W est choisi parmi les groupes de formules suivantes :
-CH₂-(CHOH)ₙ-CH₂OH ;
-CH-(CH₂OH)-(CHOH)ₙ₋₁-CH₂OH ;
-CH₂-(CHOH)₂(CHOR')(CHOH)-CH₂OH dans lesquelles n est un nombre entier allant de 3 à 5, R' est hydrogène, un monosaccharide cyclique ou aliphatique ou l'un de ses dérivés alcoxylés,
et parmi lesquels on préfère encore un groupe glycityle dans lequel n est égal à 4, et en particulier le groupe -CH₂-(CHOH)₄-CH₂OH.
Le groupe T-CON= peut être par exemple cocamide, stéaramide, oléamide, lauramide, myristiramide, capricamide, palmitamide, amide de suif.

Selon la présente invention, on préfère utiliser plus particulièrement à titre d'agents tensio-actifs non-ioniques , les alkylpolyglucosides **(ii)**_{**1**}.

Le ou les agents tensio-actifs non-ioniques (ii) utilisés selon l'invention, représentent de préférence de 0,05 à 30 % en poids environ du poids total de la composition tinctoriale et encore plus préférentiellement de 0,1 à 15 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 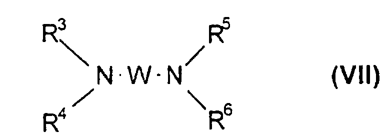 dans laquelle W est un reste propylène éventuellement substitué par un groupement hydroxyle ou un radical alkyle en C₁-C₆ ; R³, R⁴, R⁵ et R⁶, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques

Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et de l'agent tensio-actif non-ionique (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant l'agent tensio-actif non-ionique.

Lorsque l'association du colorant direct cationique (i) et de l'agent tensio-actif non-ionique (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydo-réductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant l'agent tensio-actif non-ionique (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant. dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant l'agent tensio-actif non-ionique tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse

Les exemples qui suivent sont destinés à illustrer l'invention sans pour autant en limiter la portée.

### EXEMPLES

### EXEMPLES 1 à 9 :

On a préparé les neuf compositions de teinture directe réunies dans le tableau suivant :
*(toutes teneurs exprimées en grammes)*

| **EXEMPLES N°→** | **1** | **2** | **3** | **4** | **5** | **6** | **7** | **8** | **9** |
|---|---|---|---|---|---|---|---|---|---|
| Colorant direct cationique de formule (IV)₁₀ | 0,12 | | | | | | | | |
| Colorant direct cationique de formule(IV)₂₇ | | 0,10 | | | | | | | |
| Colorant direct cationique de formule (IV)₁₇ | | | 0,10 | | | | | | |
| Colorant direct cationique de formule (IV)₄₁ | | | | 0,11 | | | | | |
| Colorant direct cationique de formule (IV)₂₅ | | | | | 0,12 | | | | |
| Colorant direct cationique de formule (I1) | | | | | | 0,10 | | | 0,10 |
| Colorant direct cationique de formule (I2) | | | | | | 0,10 | | | |
| Colorant direct cationique de formule (I14) | | | | | | | 0,20 | | |
| Colorant direct cationique de formuler (I31) | | | | | | | | 0,15 | |
| Alkylpolyglucoside vendu sous la dénomination ORAMIX CG110 par la société SEPPIC | | 8,0 | 8,0 | | | | | | 8,0 |
| N-décanoyl-N-méthylglucamine ** | 8,0 | | | | | | 8,0 | | |
| O-hexadecanoyl-6-αD-glucose | | | | 8,0 | | 8,0 | | | |
| N-cocolactobionamide | | | | | 8,0 | | | 8.0 | |
| Ethanol | 10 | 10 | 10 | 10 | 10 | 10 | 10 | 10 | 10 |
| 2-amino-2-méthyl-1-propanol qs | PH 9 | pH9 | pH9 | pH9 | pH9 | pH9 | pH9 | pH9 | pH9 |
| Eau déminéralisée qsp | 100 | 100 | 100 | 100 | 100 | 100 | 100 | 100 | 100 |

| | | | | | | | | | |
|---|---|---|---|---|---|---|---|---|---|
| **N-décanoyl N-méthylglucamine [amide d'acide gras polyhydroxylé de formule: C₉H₁₉-CO-N(CH₃)-CH₂-(CHOH)₄-CH₂OH] | | | | | | | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| **Exemples** | **Nuances obtenues** |
|---|---|
| 1 | Rouge puissant |
| 2 | Pourpre puissant |
| 3 | Orangé puissant |
| 4 | Rouge puissant |
| 5 | Orangé puissant |
| 6 | Rouge puissant |
| 7 | Orangé puissant |
| 8 | Violet puissant |
| 9 | Rouge puissant |

## Revendications

1. Composition pour la teinture des fibres kératiniques et en particulier des fibres kératiniques humaines telles que les cheveux, renfermant dans un milieu approprié pour la teinture, **(i)**au moins un colorant direct cationique choisi parmi les composés de formules **(I)**, (II), (III), (III'), (IV) suivantes:
**a) les composés de formule (I) suivante :** 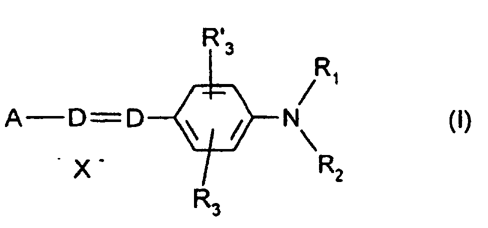 dans laquelle:
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyfoxy,
X⁻ représente un anion de préférence choisi parmi le chlorure, le méthyl sulfate et l'acétate,
A représente un groupement choisi par les structures A1 à A18 suivantes : 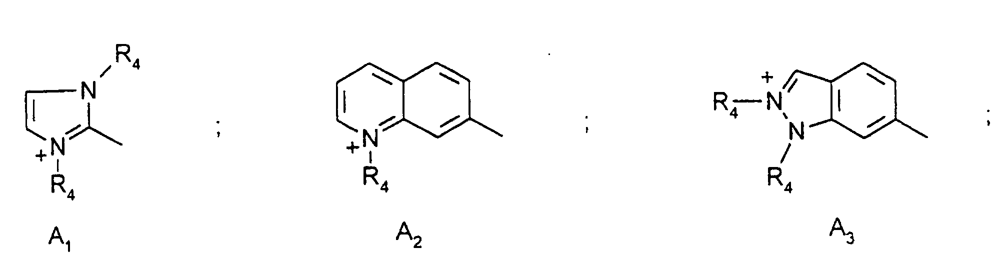 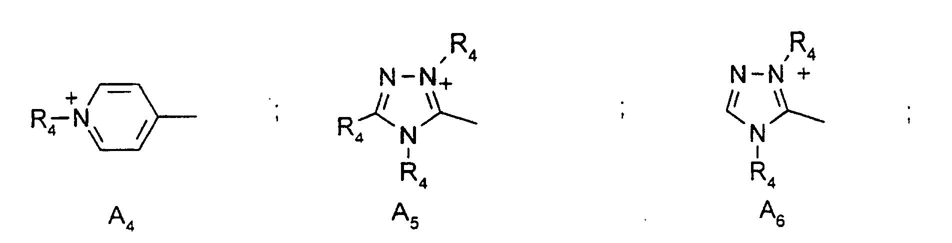 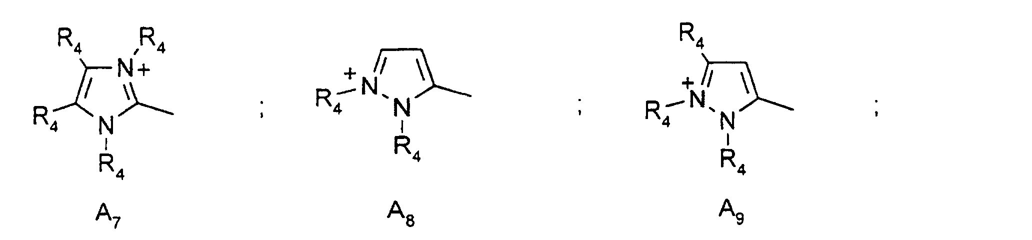 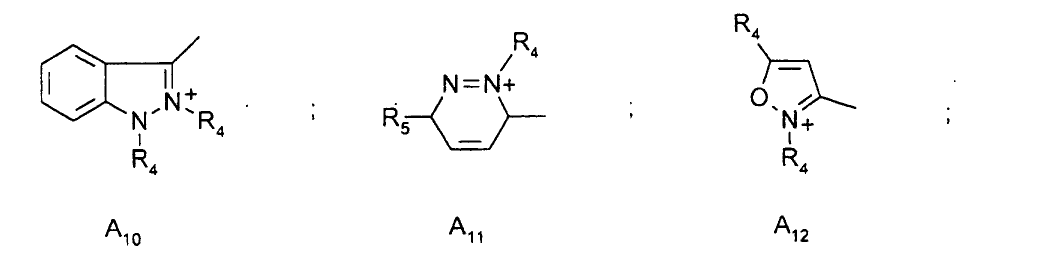 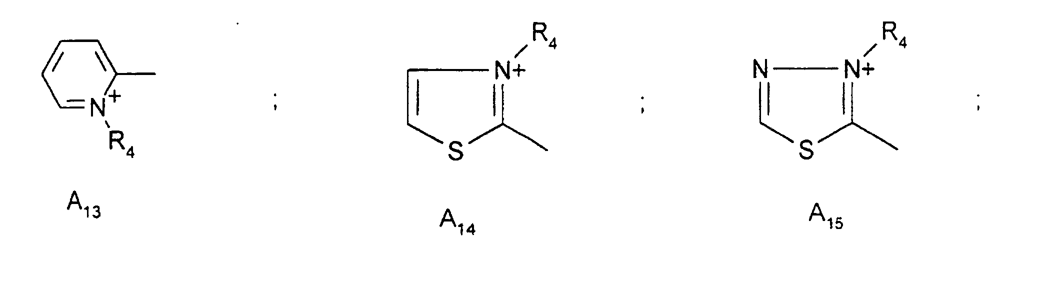 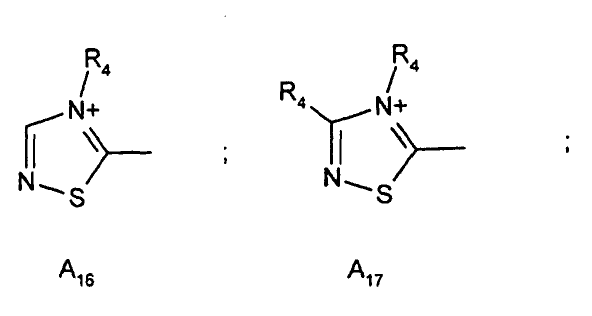 et 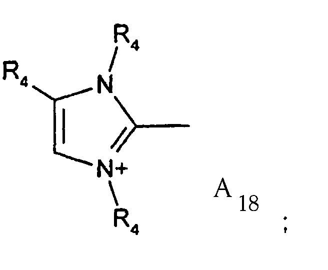
dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 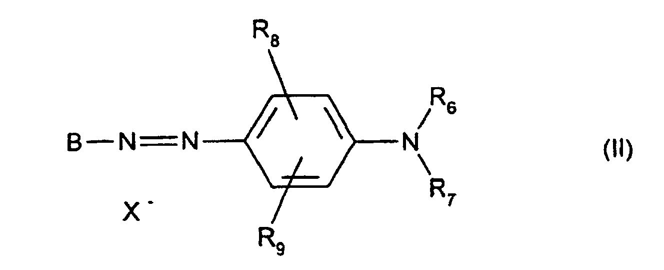 dans laquelle:
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X ⁻ représente un anion de préférence choisi parmi le chlorure, le méthyl sulfate et l'acétate,
B représente un groupement choisi par les structures B1 à B6 suivantes : 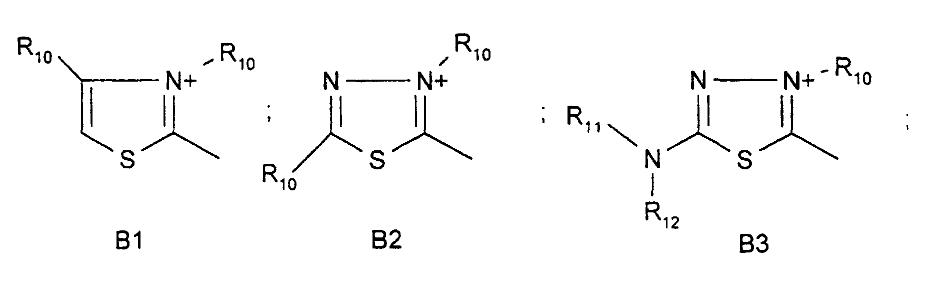 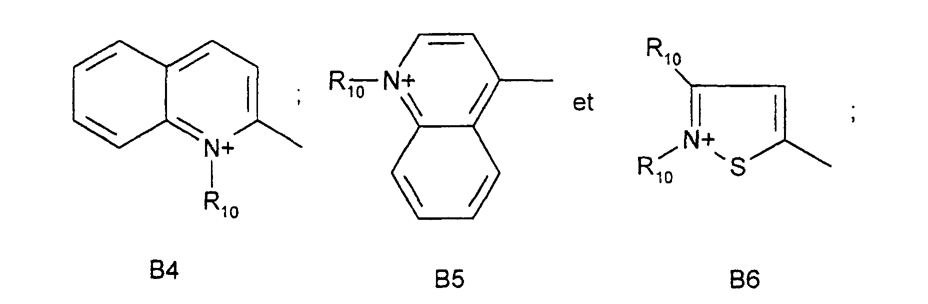 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) les composés de formules (III) et (III') suivantes :** 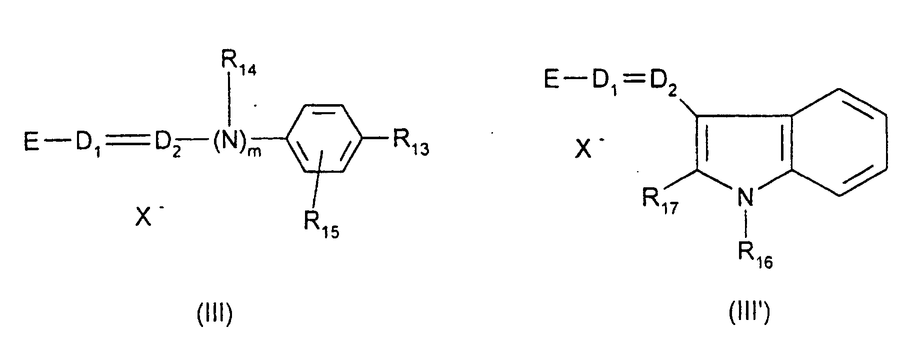 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X ⁻ représente un anion de préférence choisi parmi le chlorure, le méthyl sulfate et l'acétate,
E représente un groupement choisi par les structures E1 à E8 suivantes : 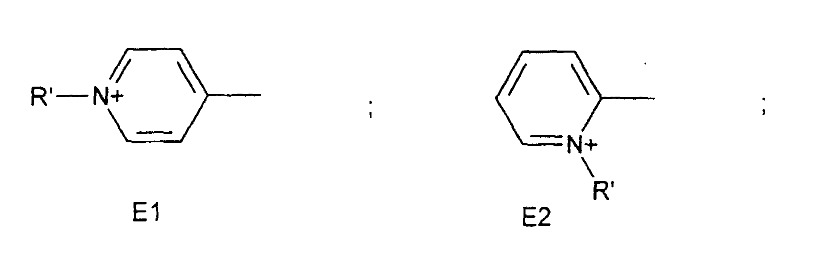 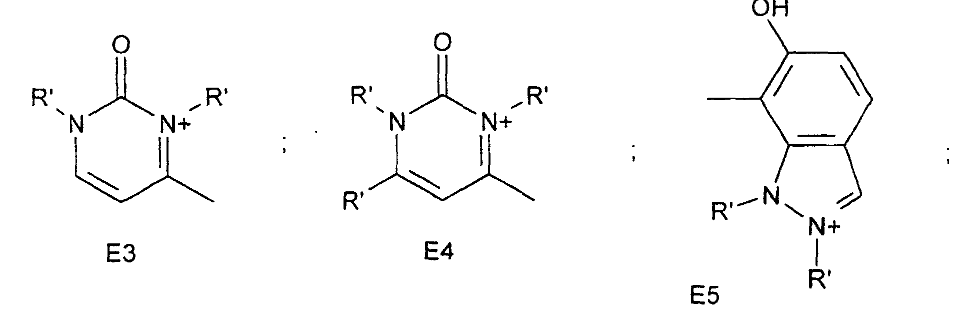 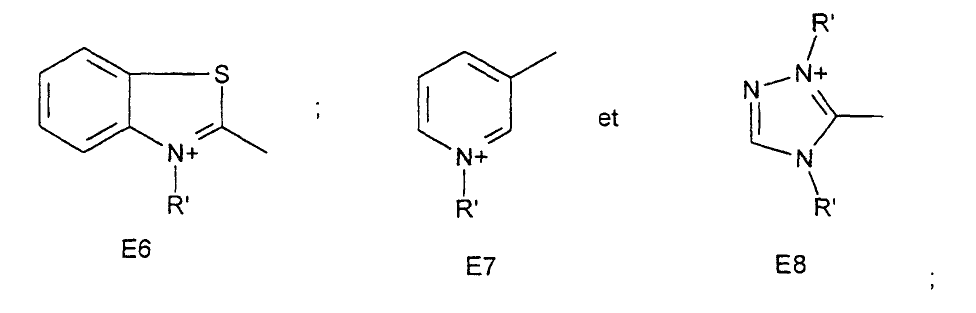
dans lesquelles R' représente un radical alkyle en C₁-C₄ ;
lorsque m = 0 et que D₁ représente un atome d'azote, alors E peut également désigner un groupement de structure E9 suivante : 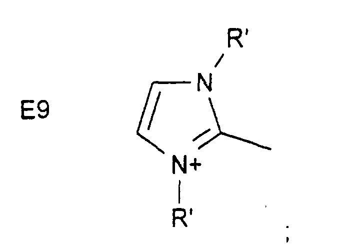 dans laquelle R' représente un radical alkyle en C₁-C₄.
**d) les composés de formule (IV) suivante :**
G-N=N-J (IV)
dans laquelle :
**le symbole G** représente un groupement choisi parmi les structures G₁ à G₃ suivantes . 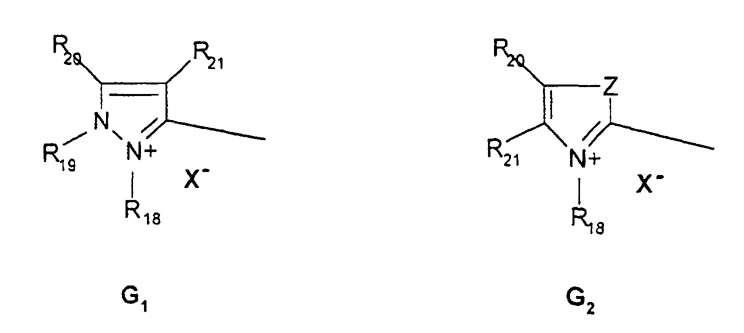 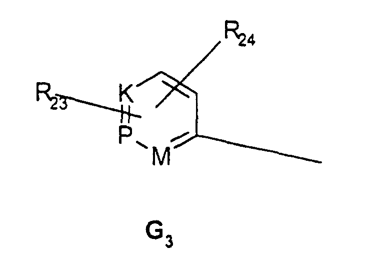 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄ , un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂,
R₂₀ peut désigner en outre un atome d'hydrogène;
Z désigne un atome d'oxygène, de soufre ou un groupement -NR₁₉;
M représente un groupement -CH, -CR (R désignant alkyle en C₁-C₄),
ou -NR₂₂(X⁻)r;
K représente un groupement -CH, -CR (R désignant alkyle en C₁-C₄),
ou -NR₂₂(X⁻)r;
P représente un groupement -CH, -CR (R désignant alkyle en C₁-C₄).
ou -NR₂₂(X⁻)ᵣ; r désigne zéro ou 1;
R₂₂ représente un atome O⁻, un radical alcoxy en C₁-C₄, ou un radical alkyle en C₁-C₄;
R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄ , un radical -NO₂;
X⁻ représente un anion de préférence choisi parmi le chlorure, l'iodure, le méthyl sulfate, l'éthyl sulfate, l'acétate et le perchlorate;
sous réserve que,
si R₂₂ désigne O⁻, alors r désigne zéro;
si K ou P ou M désignent -N-alkyle C₁-C₄ X⁻ , alors R₂₃ ou R₂₄ est différent d'un atome d'hydrogène;
si K désigne -NR₂₂(X-)ᵣ , alors M= P= -CH, -CR;
si M désigne -NR₂₂(X⁻)ᵣ , alors K= P= -CH, -CR;
si P désigne -NR₂₂(X⁻)ᵣ , alors K= M et désignent -CH ou -CR;
si Z désigne un atome de soufre avec R₂₁ désignant alkyle en C₁-C₄, alors R₂₀ est différent d'un atome d'hydrogène;
si Z désigne -NR₁₉ avec R₁₉ désignant alkyle en C₁-C₄ alors au moins l'un des radicaux R₁₈, R₂₀ ou R₂₁ de G₂ est différent d'un radical alkyle en C₁-C₄;
**le symbole J** représente :
- **(a)** un groupement de structure J₁ suivante : 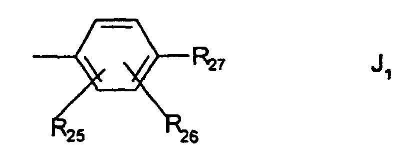 structure J₁ dans laquelle,
R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec
R₂₆ un cycle à 5 ou 6 chaînons contenant ou non un ou plusieurs hétéroatomes choisis parmi l'azote, l'oxygène ou le soufre;
R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
R₂₇ représente un atome d'hydrogène, un radical -OH, un radical -NHR₂₈, un radical -NR₂₉R₃₀;
R₂₈ représente un atome d'hydrogène, un radical alkyle en C₁-C₄, un radical monohydroxyalkyle en C₁-C₄, polyhydroxyalkyle en C₂-C₄, un radical phényle;
R₂₉ et R₃₀, identiques ou différents, représentent un radical alkyle en C₁-C₄, un radical monohydroxyalkyle en C₁-C₄, polyhydroxyalkyle en C₂-C₄;
- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 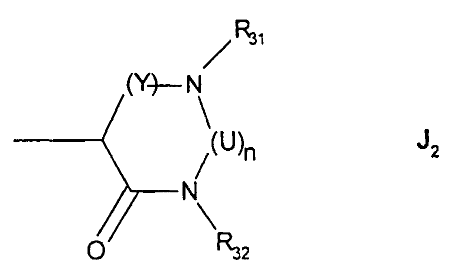 structure J₂ dans laquelle,
R₃₁ et R₃₂, identiques ou différents, représentent un atome d'hydrogène , un radical alkyle en C₁-C₄ , un radical phényle;
Y désigne le radical -CO- ou le radical 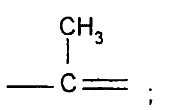
n = 0 ou 1, avec, lorsque n désigne 1, U désigne le radical -CO- .
ladite composition étant **caractérisée par le fait qu'**elle contient en outre (ii)au moins un agent tensio-actif non-ionique choisi dans le groupe comprenant :
**(ii)**_{**1**} - les alkylpolyglucosides;
**(ii)**_{**2**} - les esters d'acides gras de sucre ou d'alkylsucre;
**(ii)**_{**3**} - les sucramides gras ;
**(ii)**_{**4**}- leurs mélanges.

2. Composition selon la revendication 1, **caractérisée par le fait que** les colorants directs cationiques de formule (I) sont choisis parmi les composés répondant aux structures (I1) à (I54) suivantes : 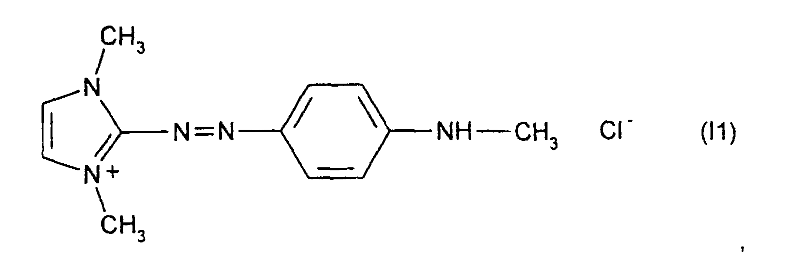 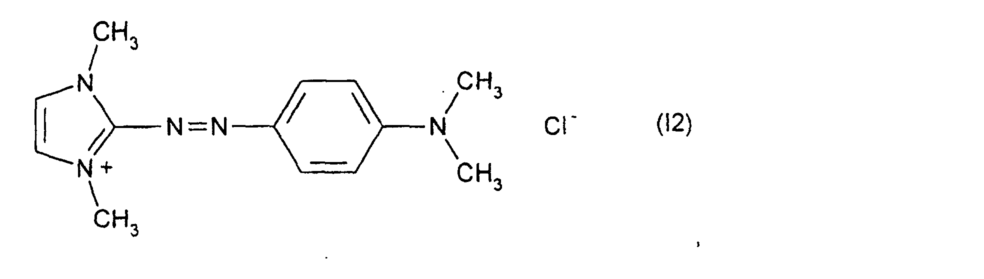 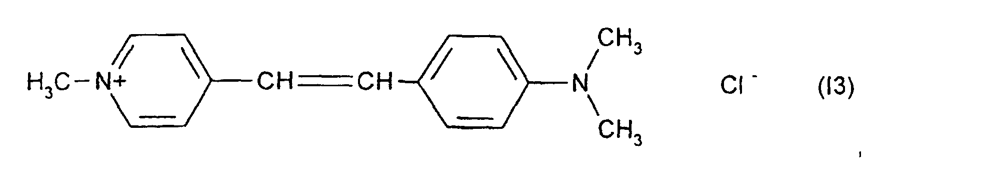 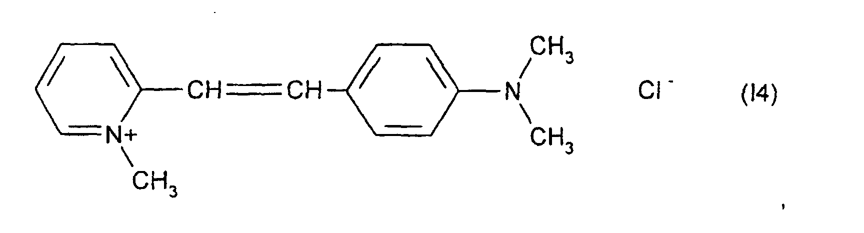 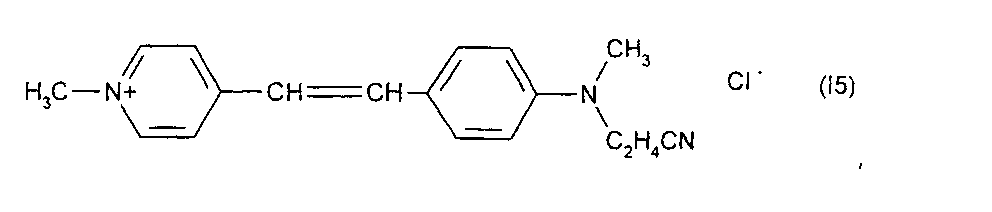 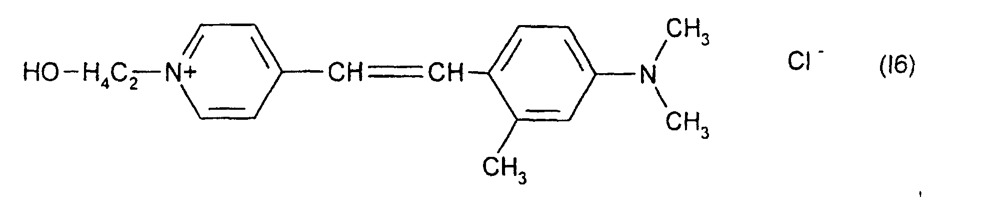 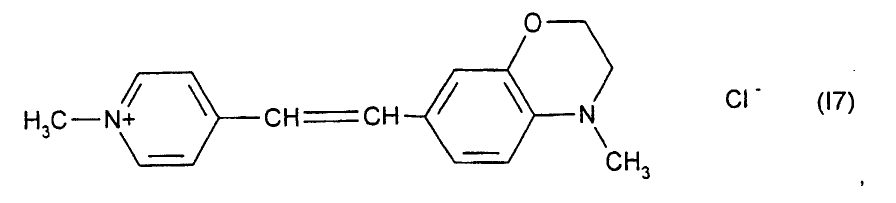 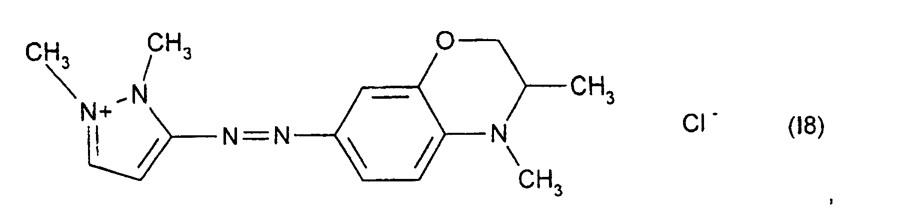 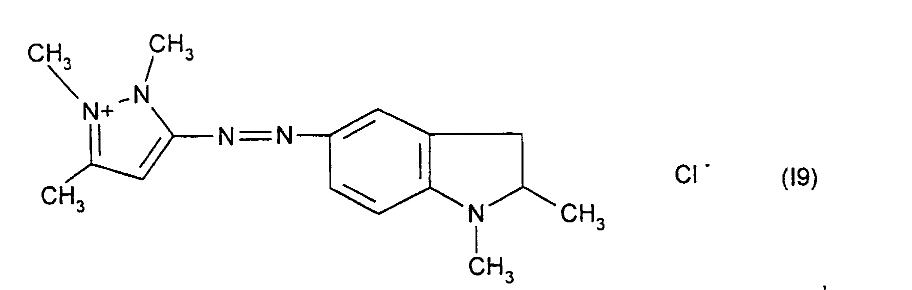 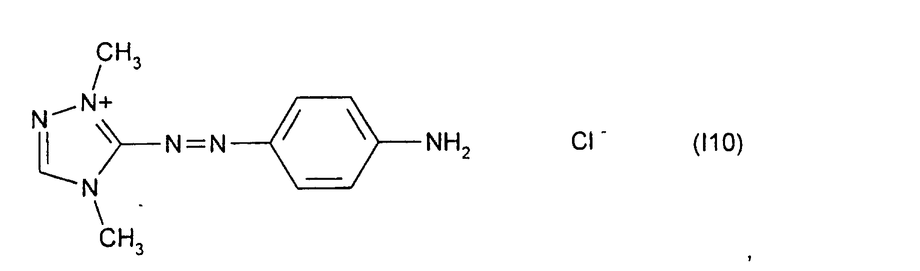 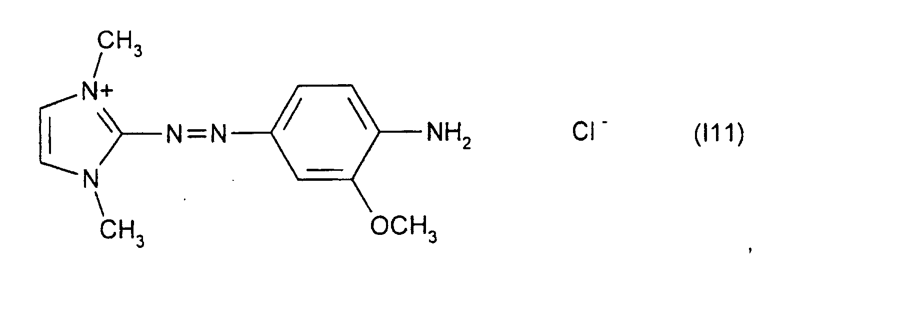 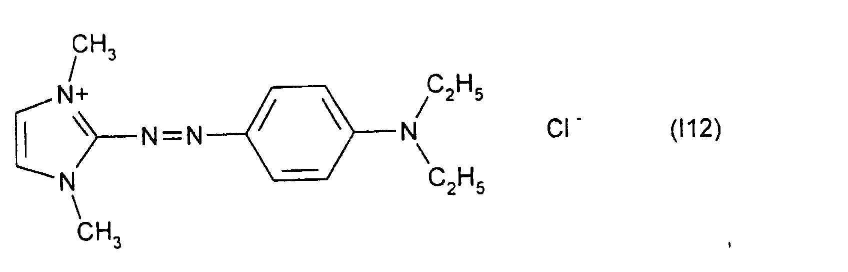 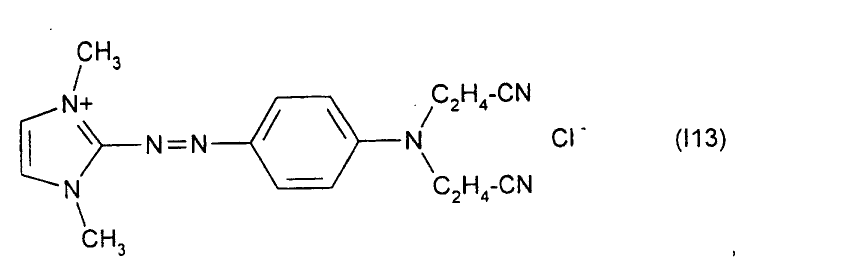 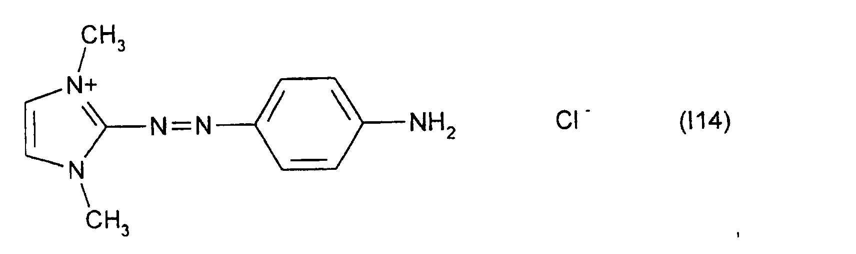 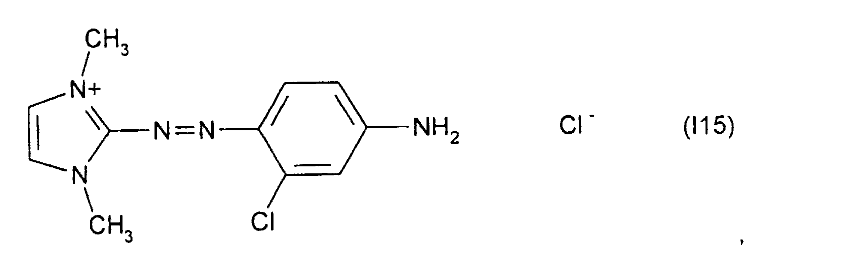 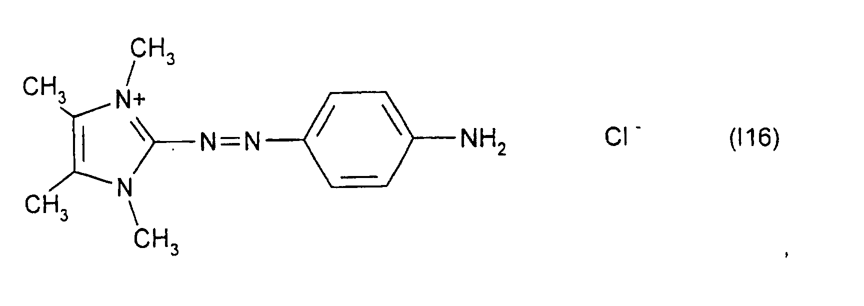 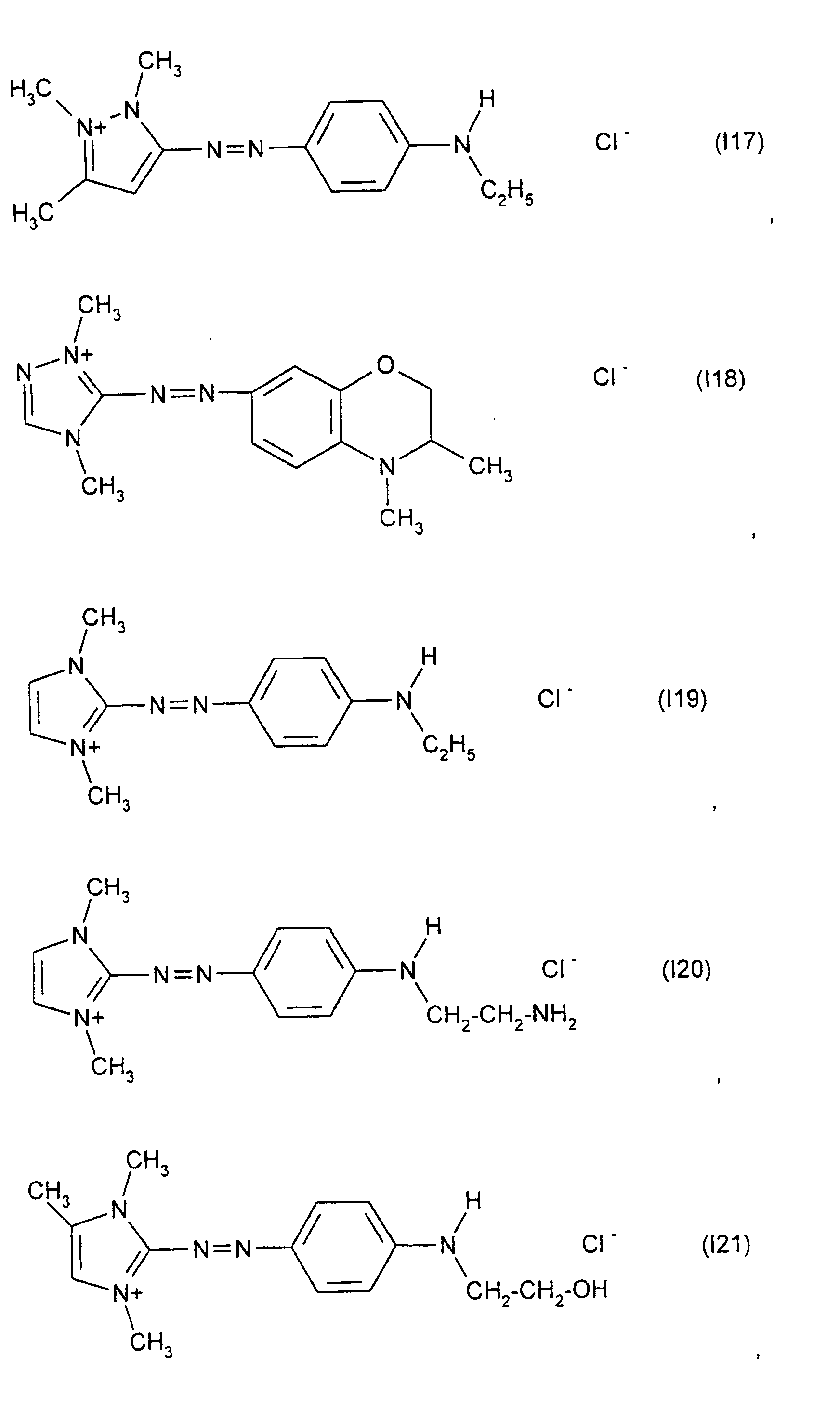 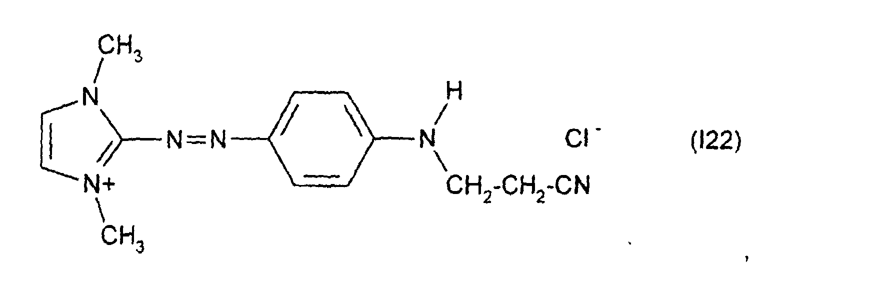 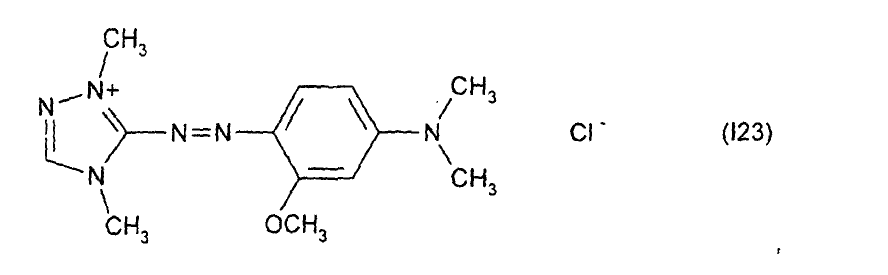 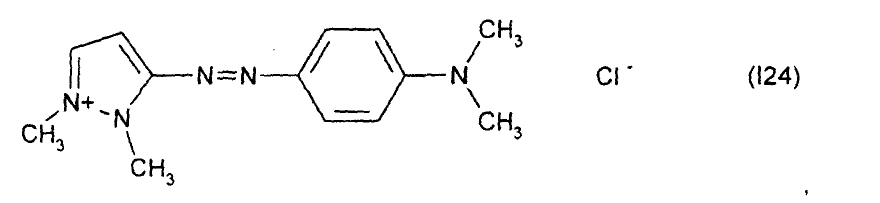 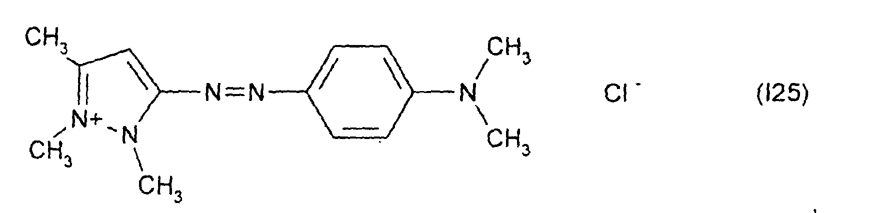 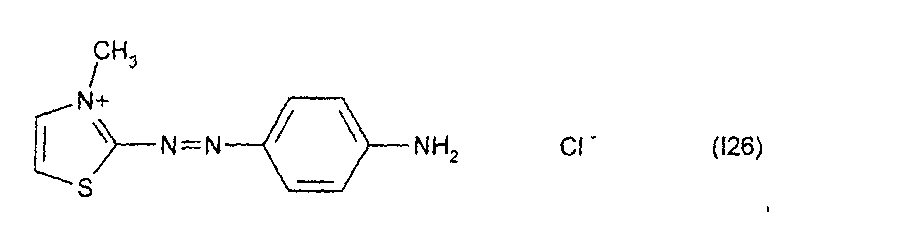 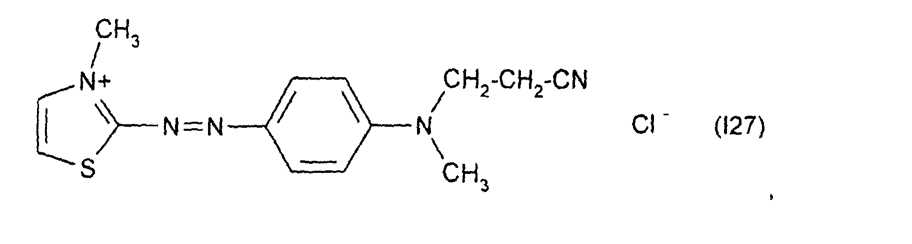 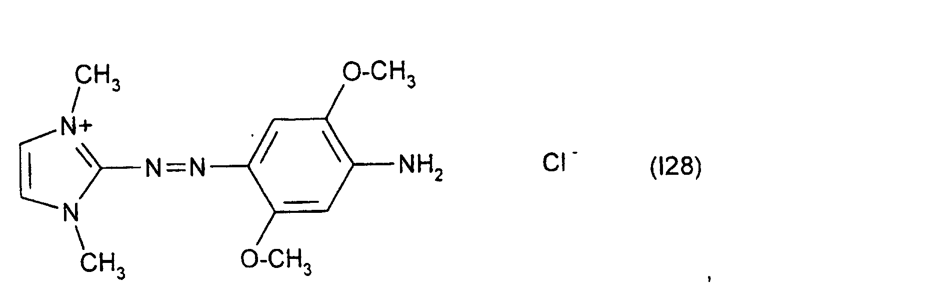 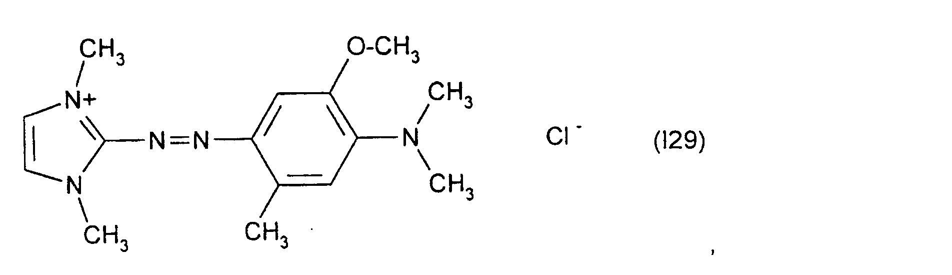 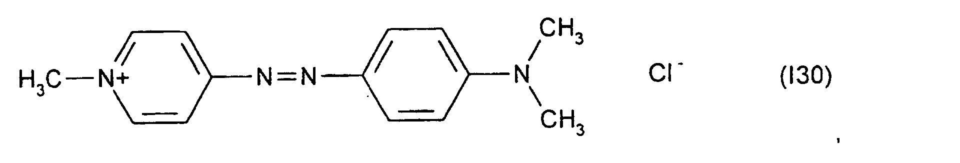 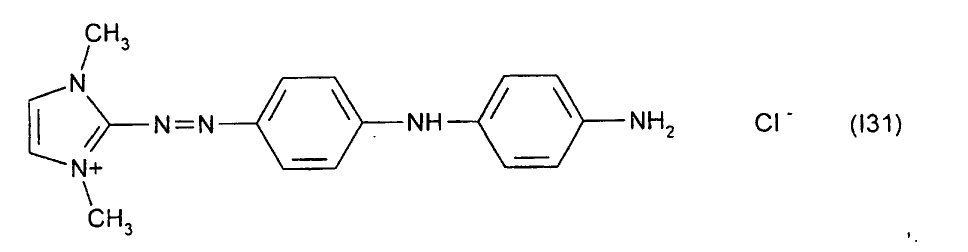 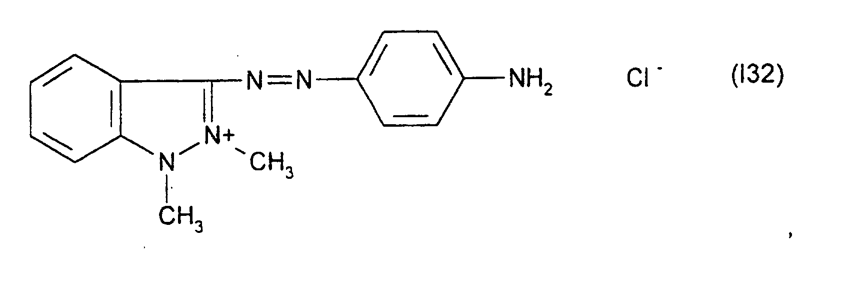 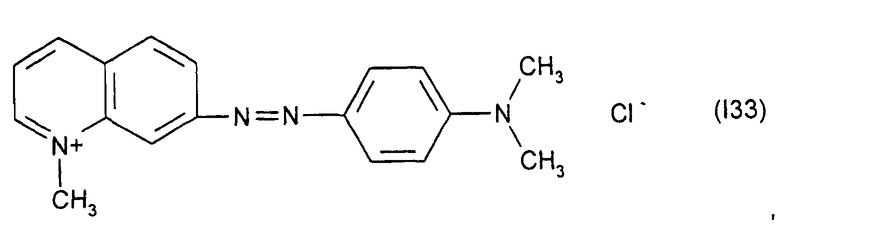 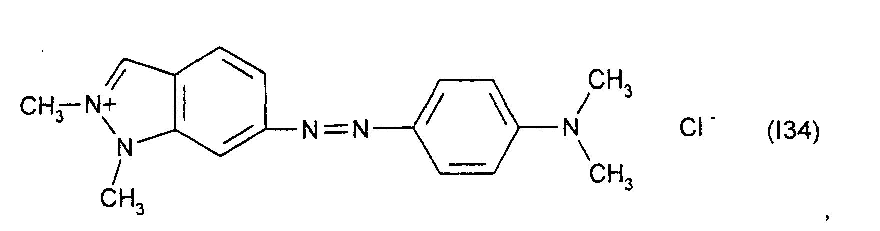 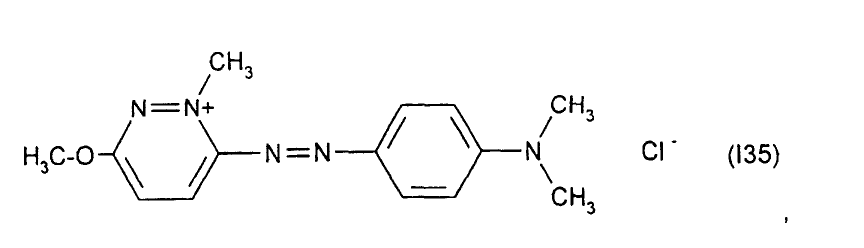 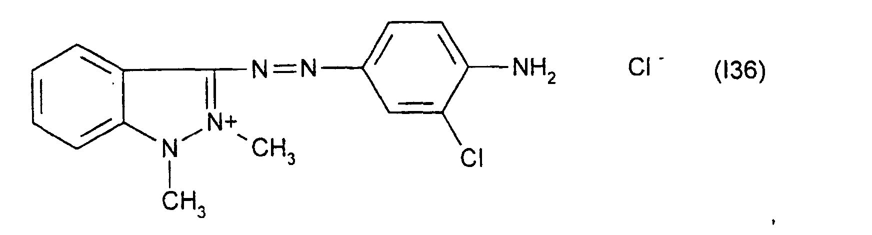 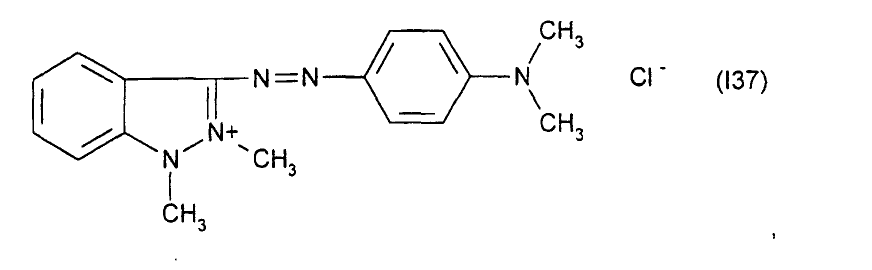 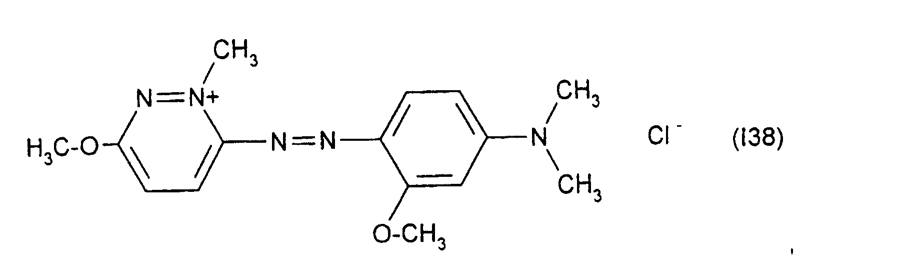 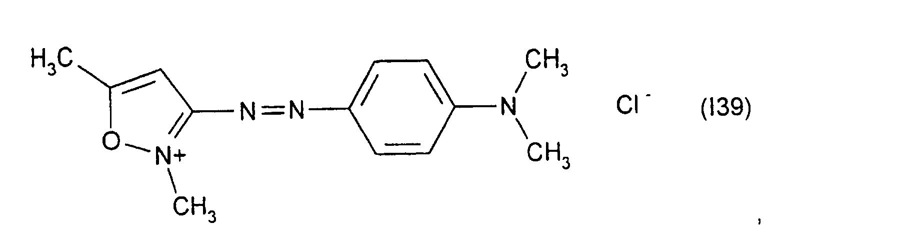 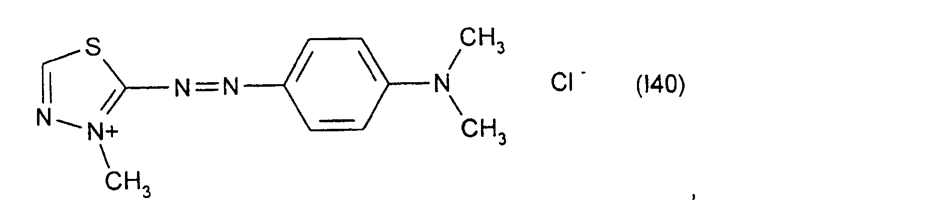 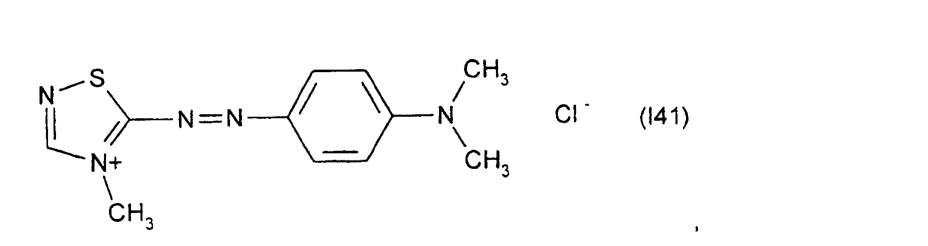 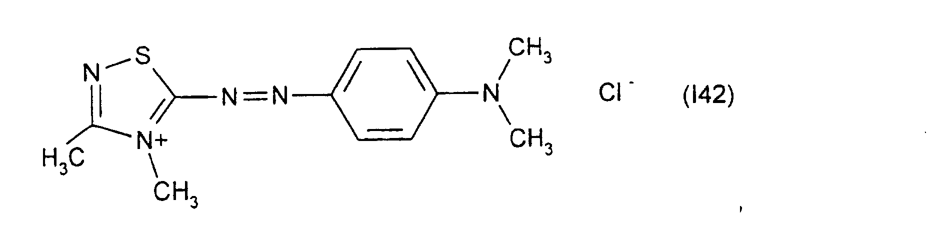 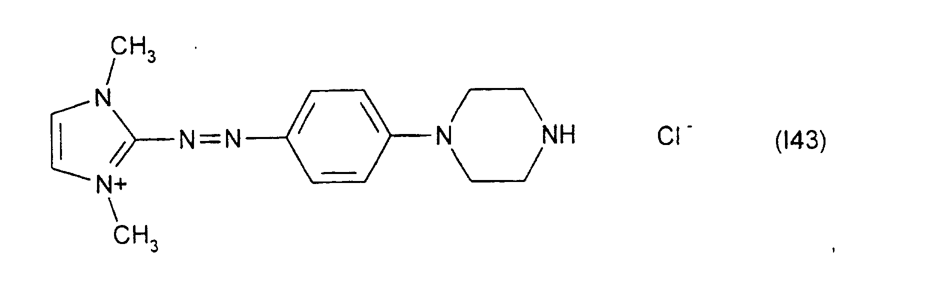 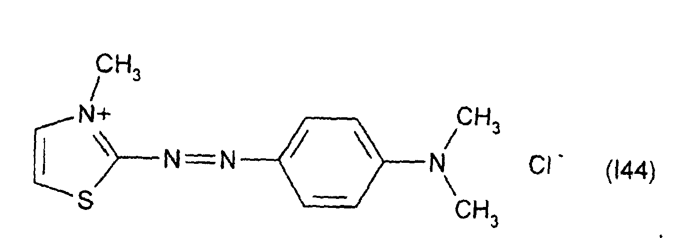 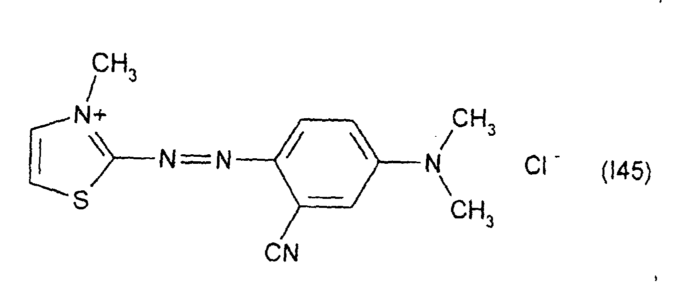 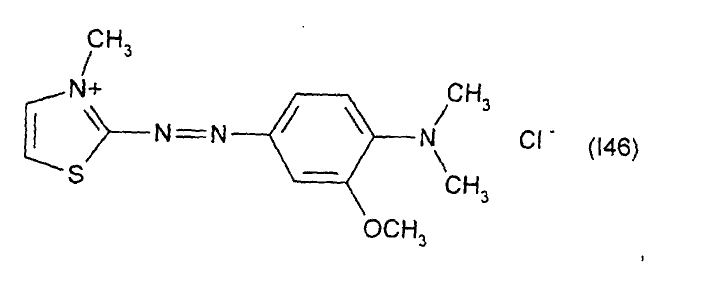 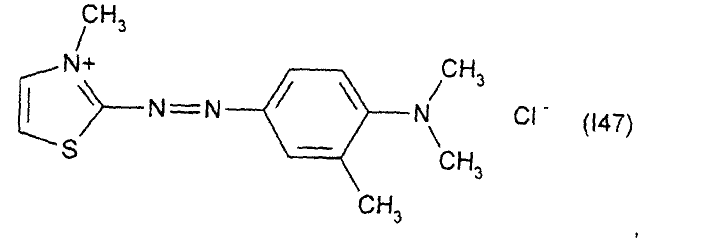 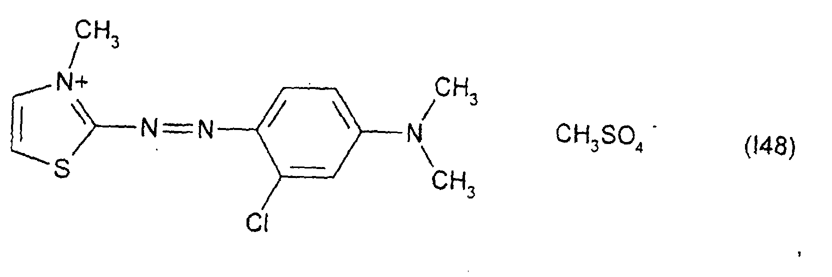 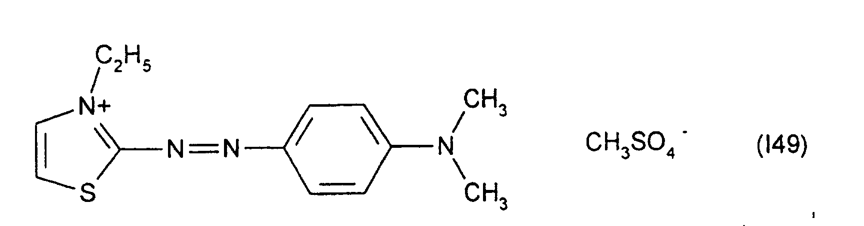 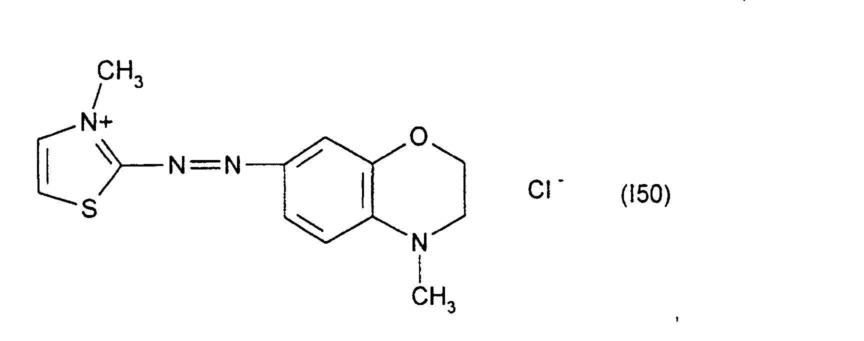 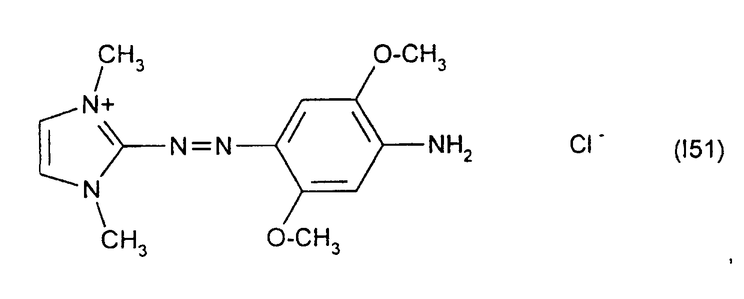 et

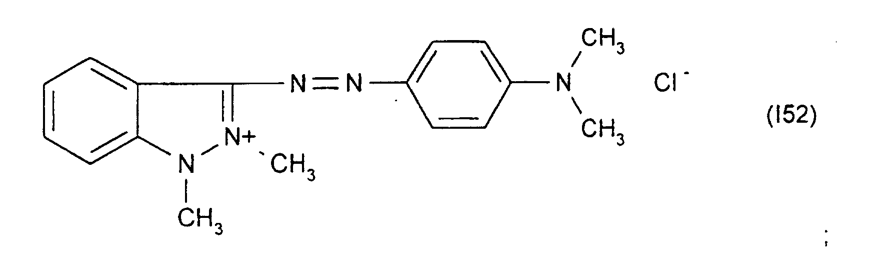

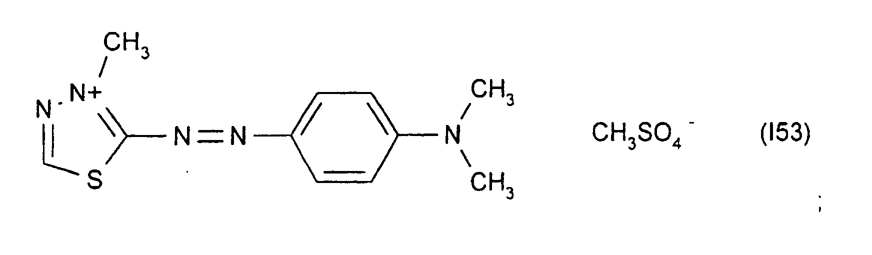

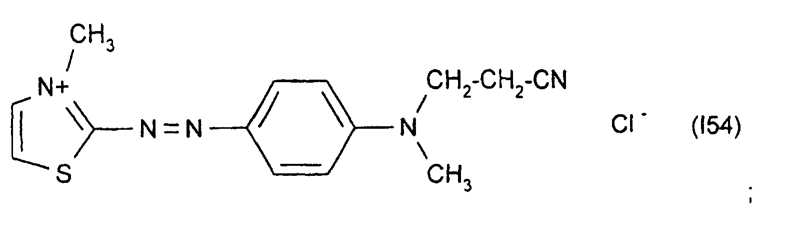

3. Composition selon la revendication 2, **caractérisée par le fait que** les colorants directs cationiques répondent aux structures (I1), (I2), (I14), et (I31).

4. Composition selon la revendication 1, **caractérisée par le fait que** les colorants directs cationiques de formule (II) sont choisis parmi les composés répondant aux structures (II1) à (II9) suivantes : 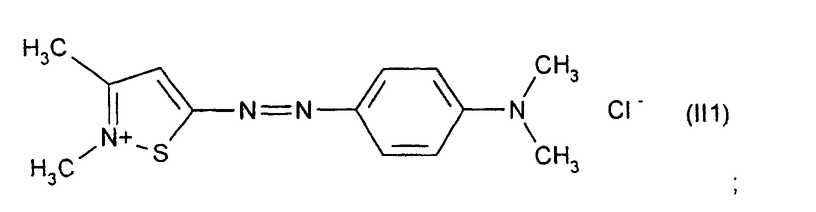 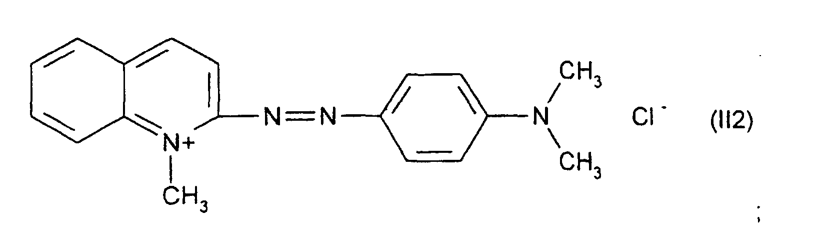 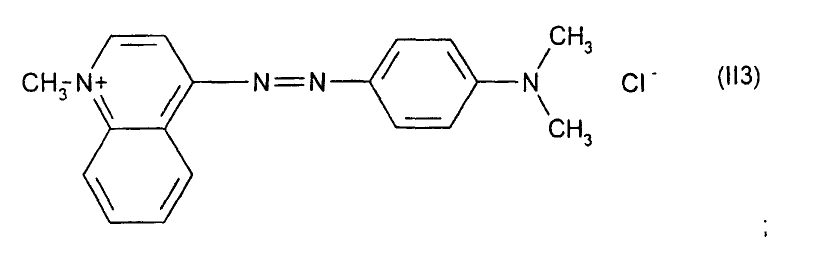 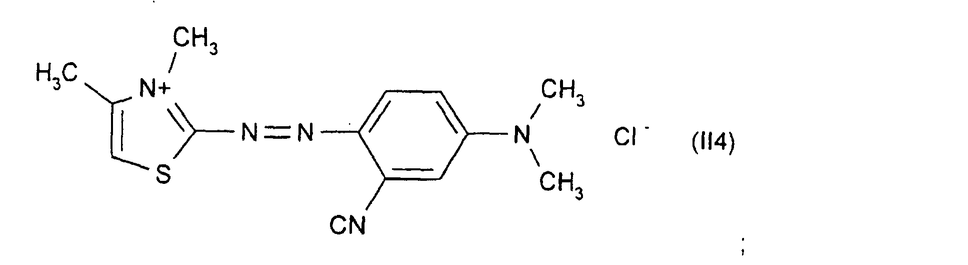 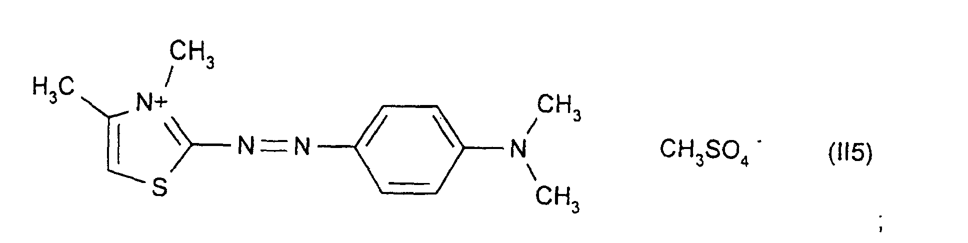 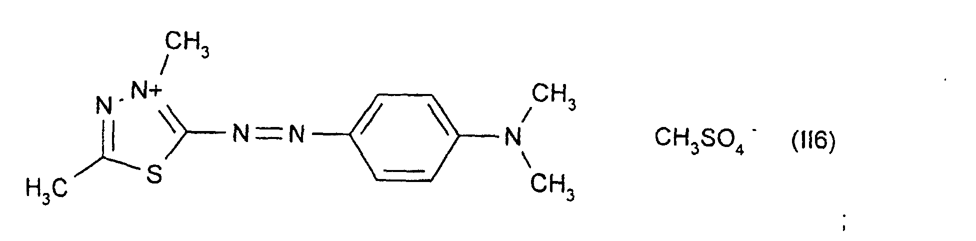 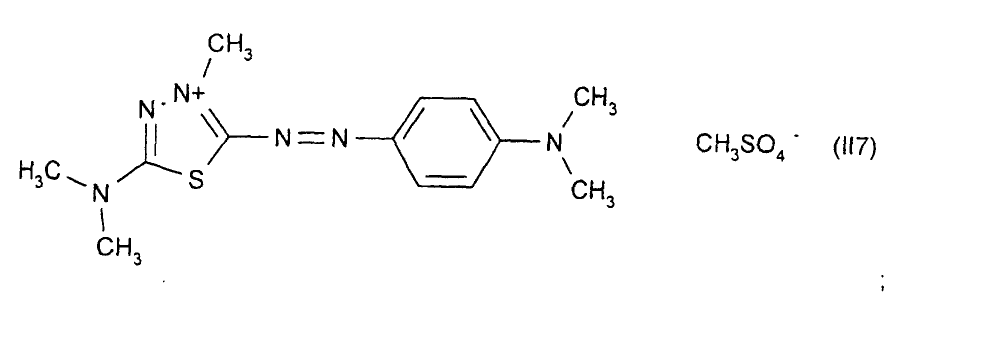 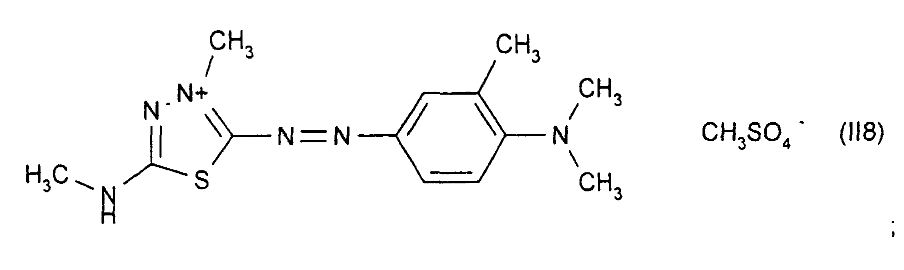 et

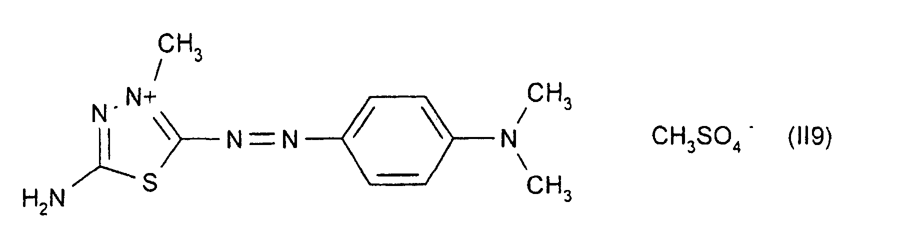

5. Composition selon la revendication 1, caracténsée par le fait que les colorants directs cationiques de formule (I11) sont choisis parmi les composés répondant aux structures (III1) à (III18) suivantes: 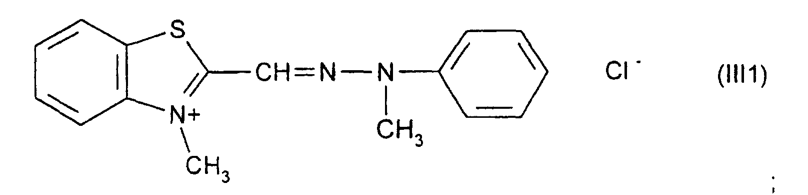 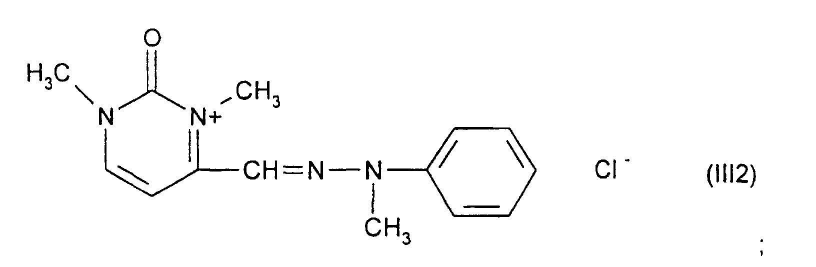 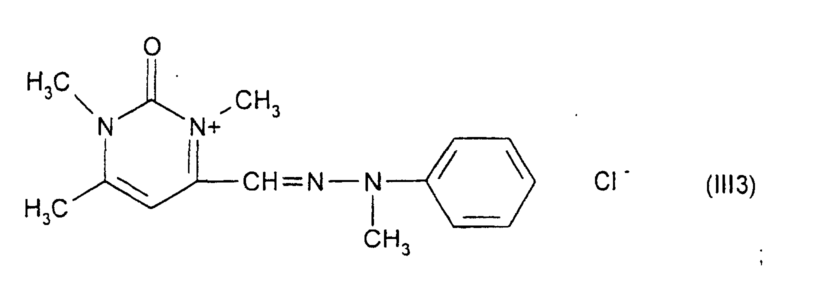 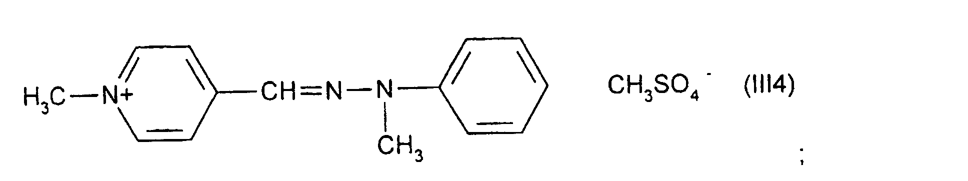 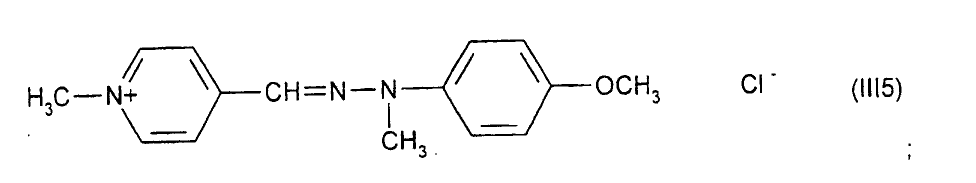 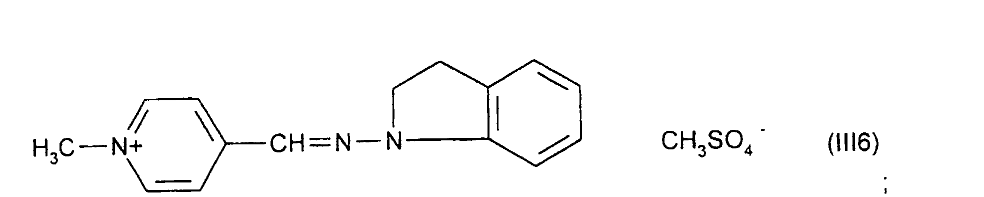 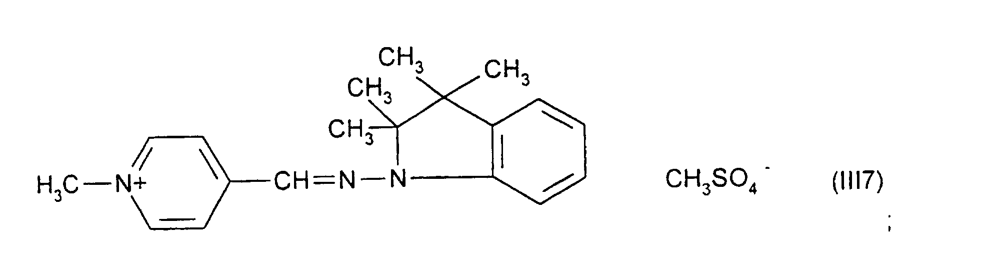 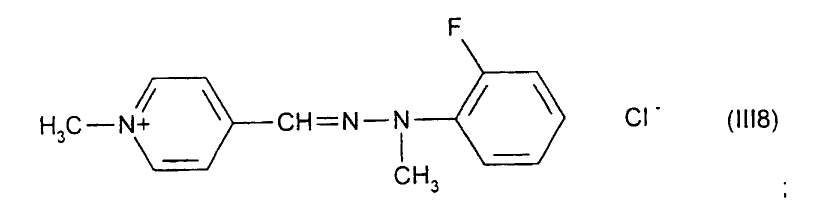 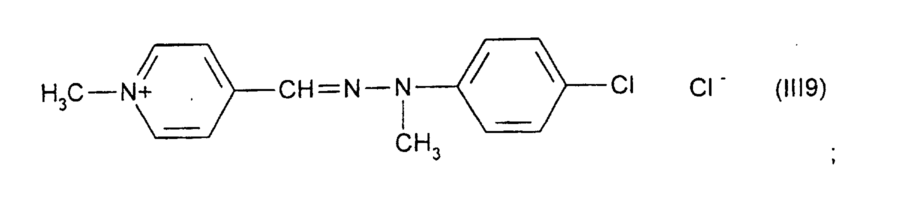 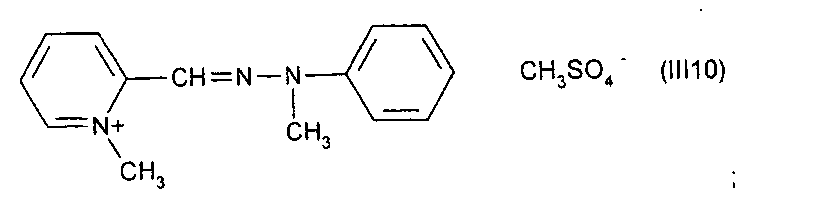 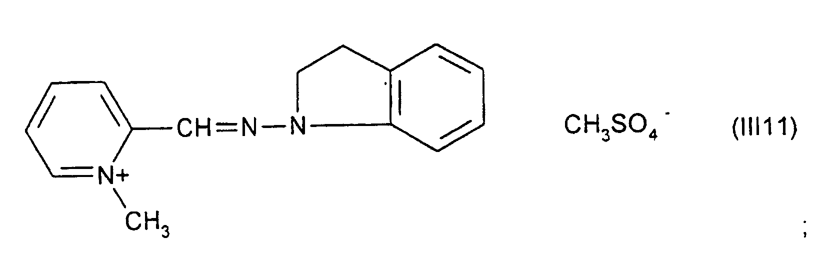 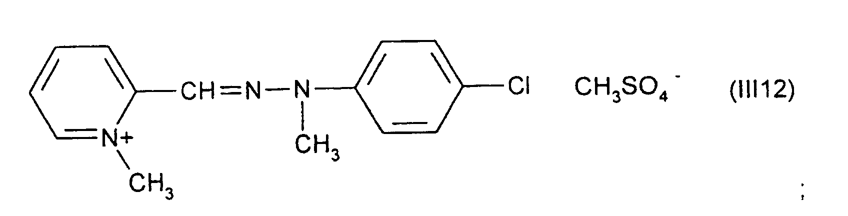 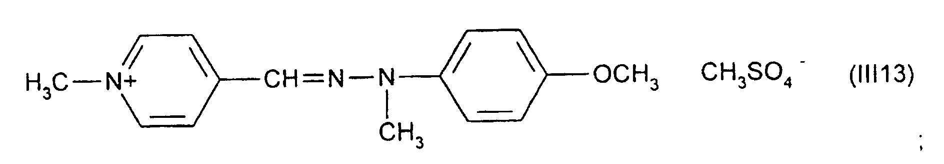 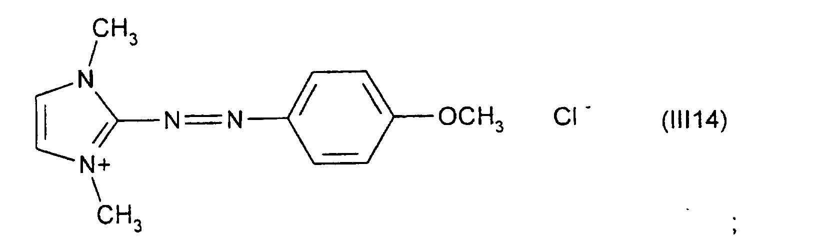 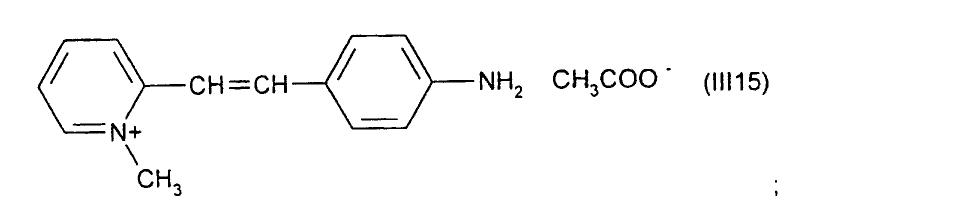 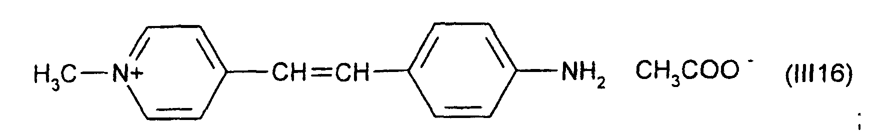 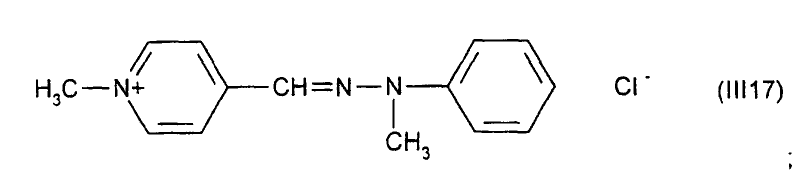 et

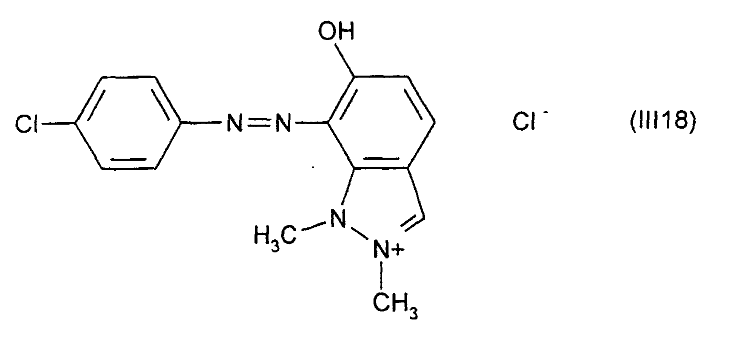

6. Composition selon la revendication 5, **caractérisée par le fait que** les colorants directs cationiques de formule (III) sont choisis parmi les composés répondant aux structures (III4), (III5) et (III13).

7. Composition selon la revendication 1, **caractérisée par le fait que** les colorants directs cationiques de formule (III') sont choisis parmi les composés répondant aux structures (III'1) à (III'3) suivantes : 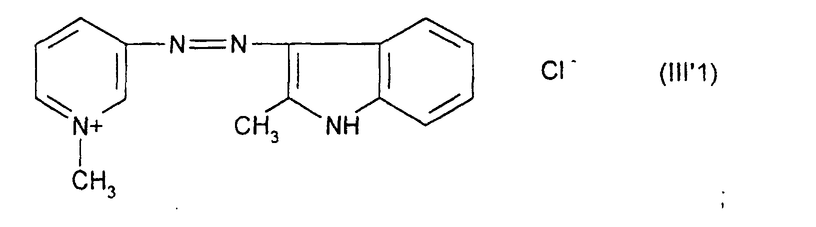 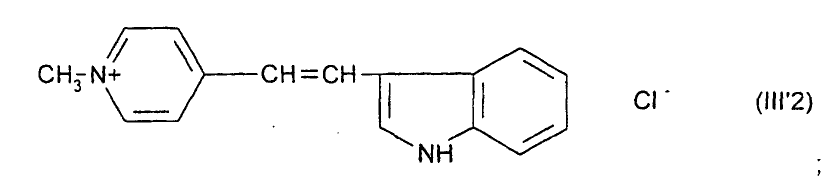 et

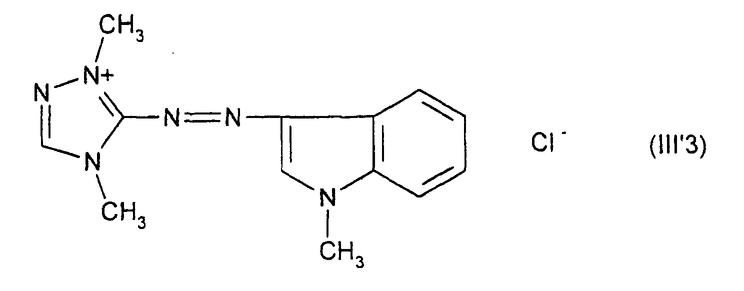

8. Composition selon la revendication 1, **caractérisée par le fait que** les colorants directs cationiques de formule (IV) sont choisis parmi les composés répondant aux structures (IV)₁ à (IV)₇₇ suivantes :

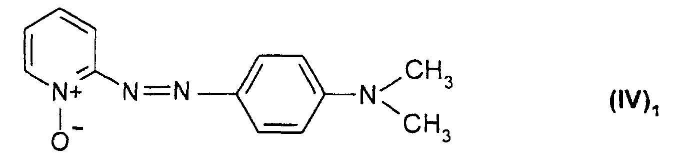

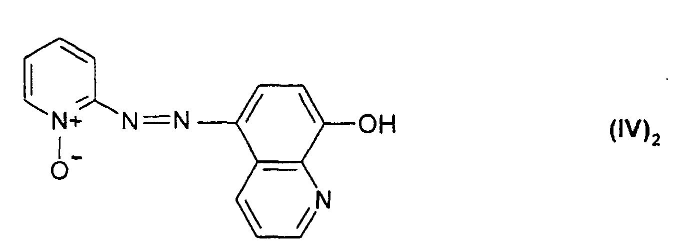

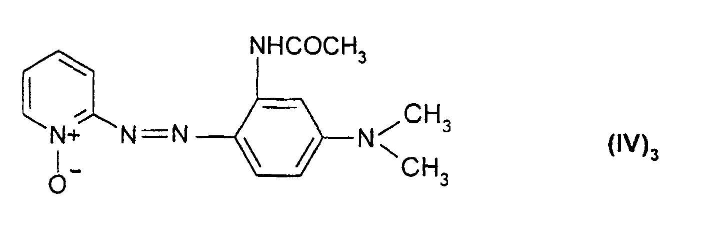

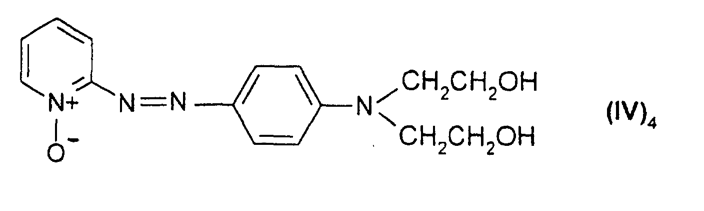

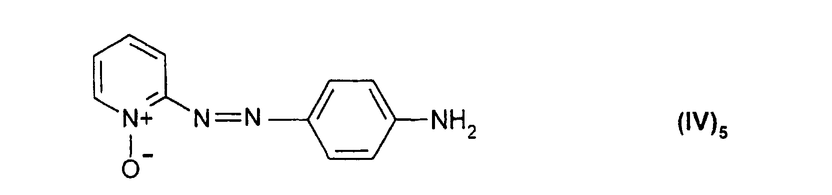

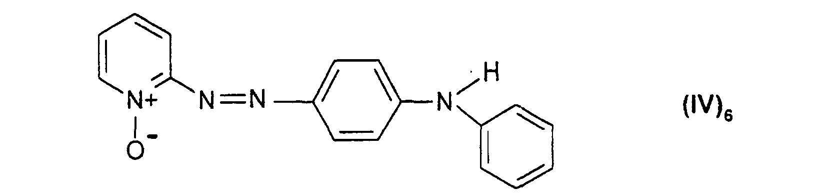

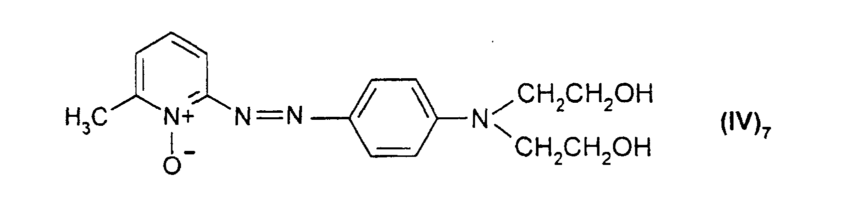

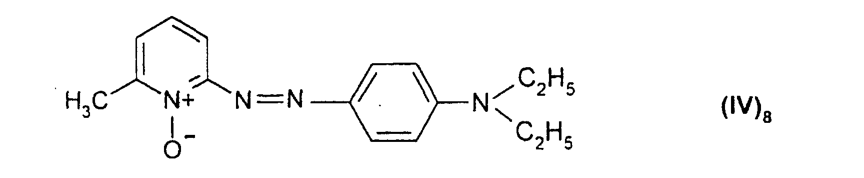

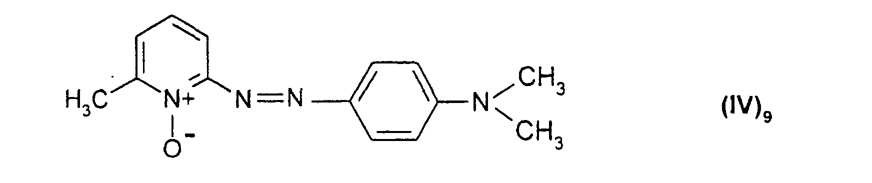

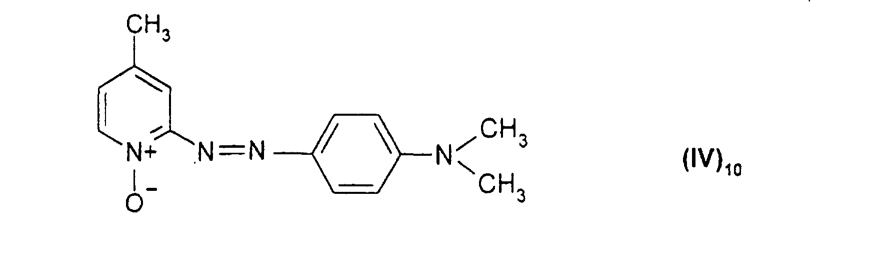

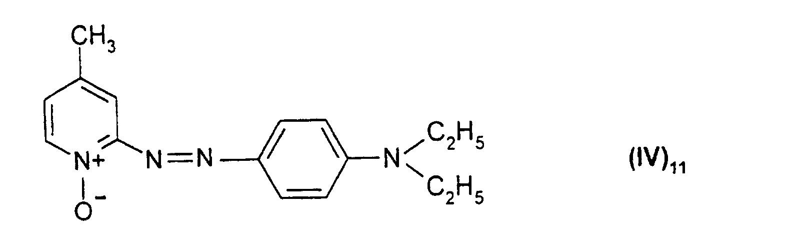

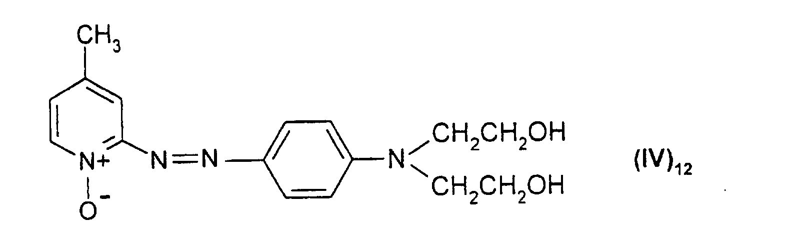

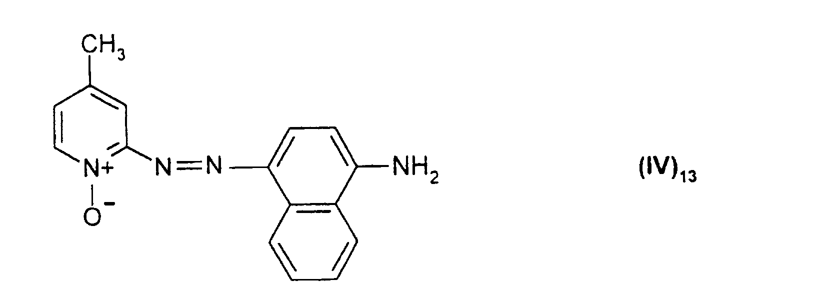

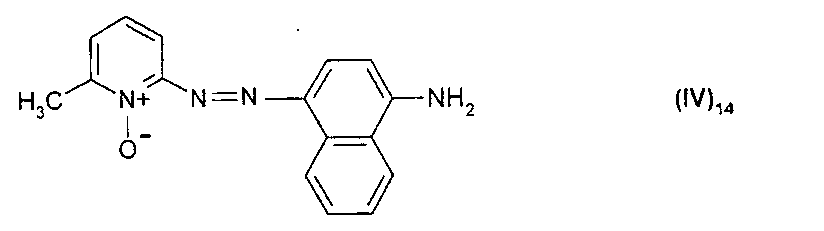

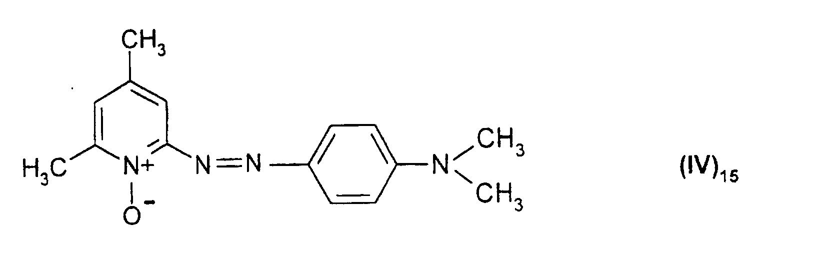

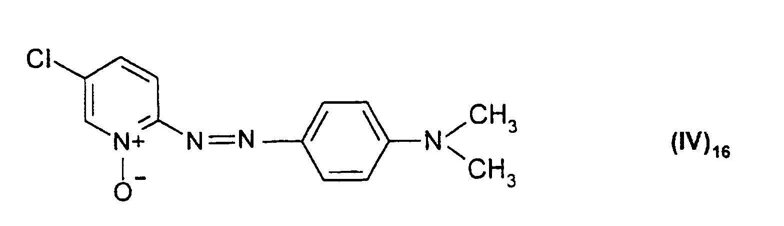

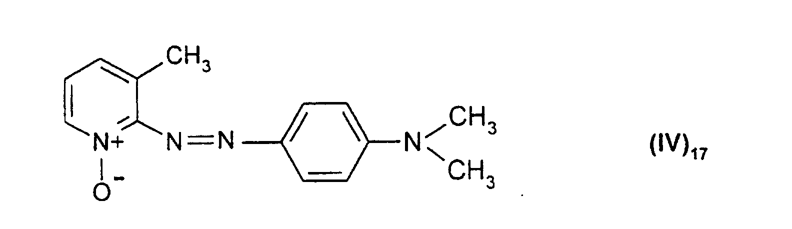

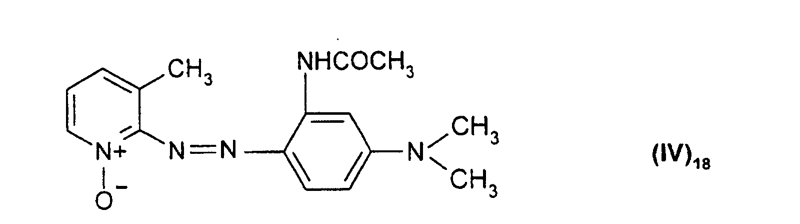

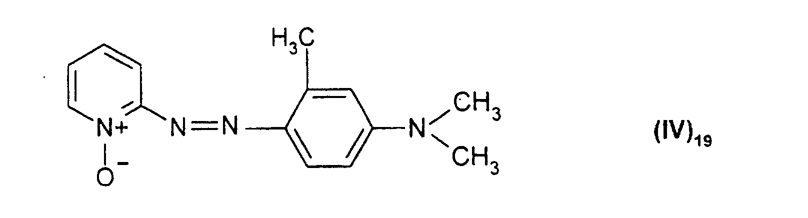

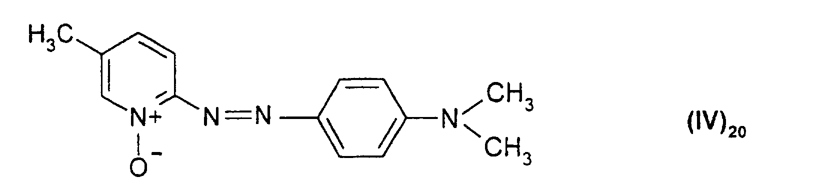

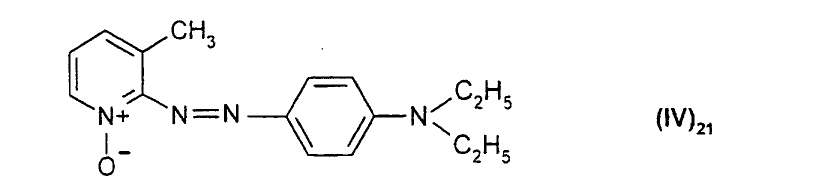

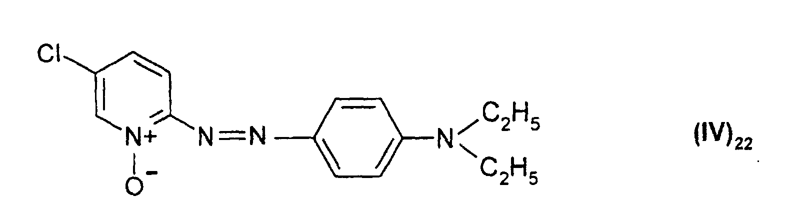

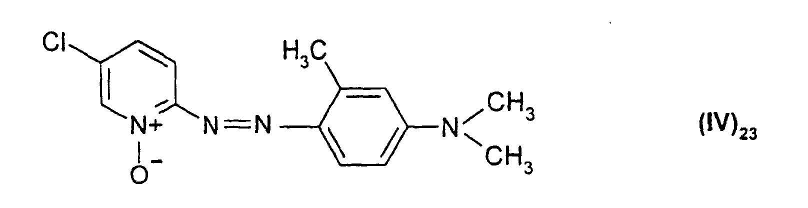

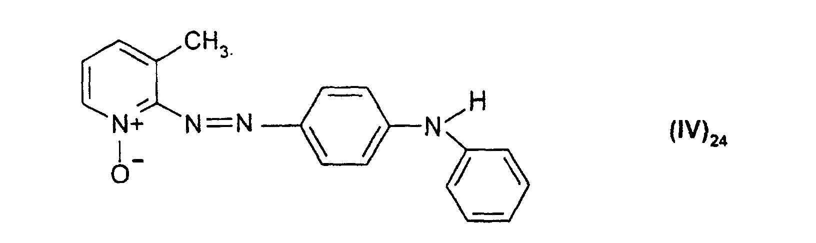

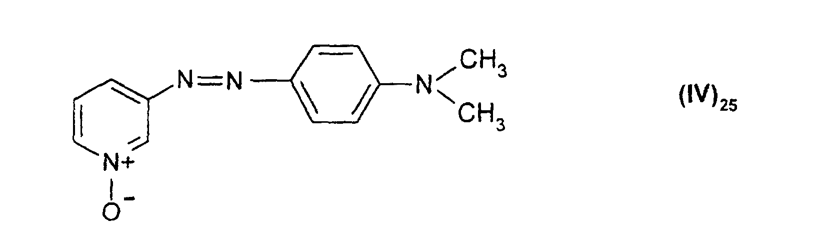

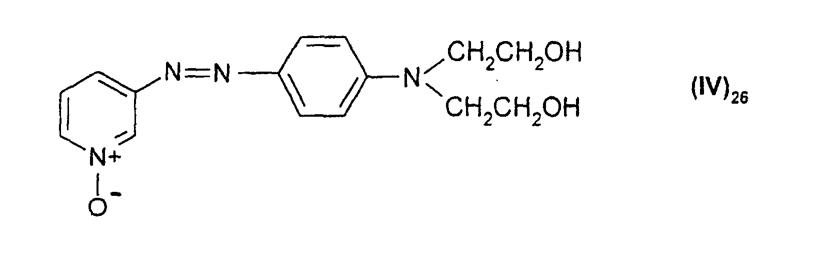

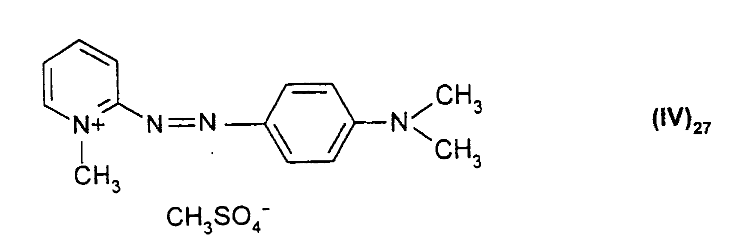

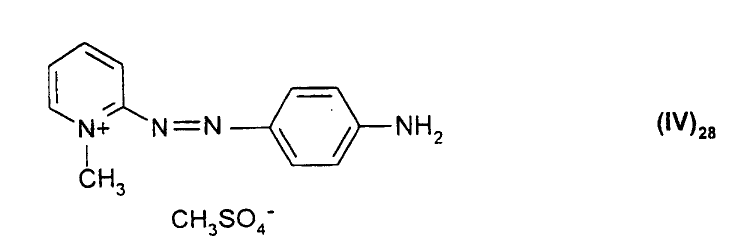

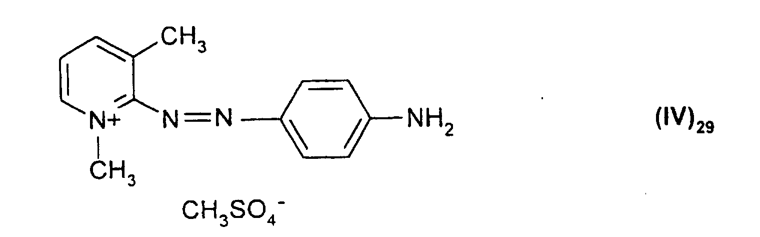

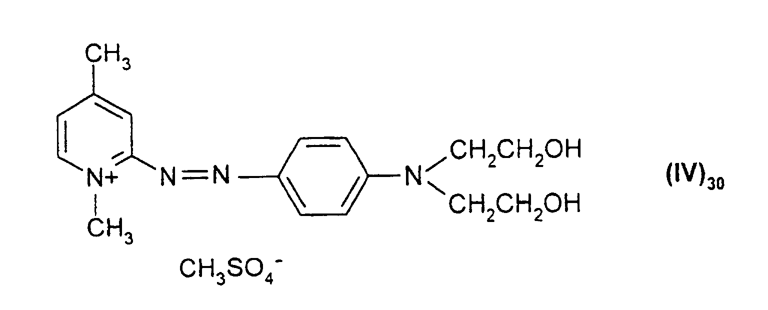

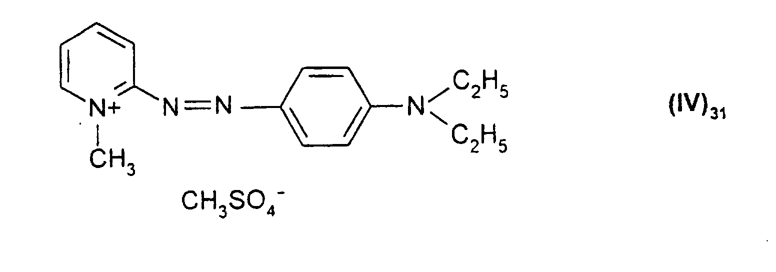

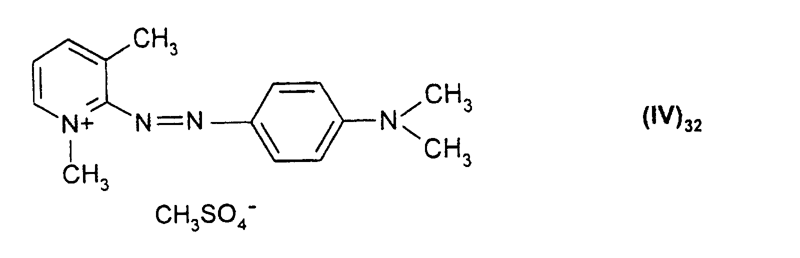

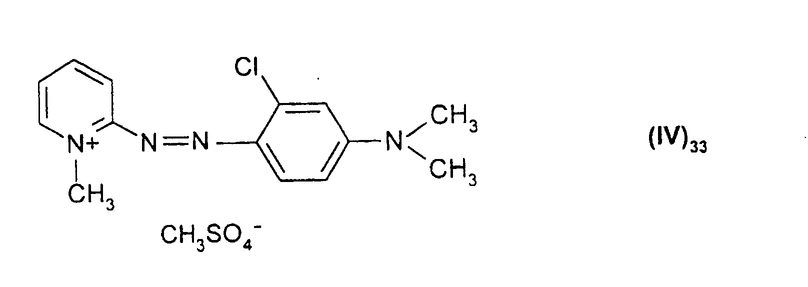

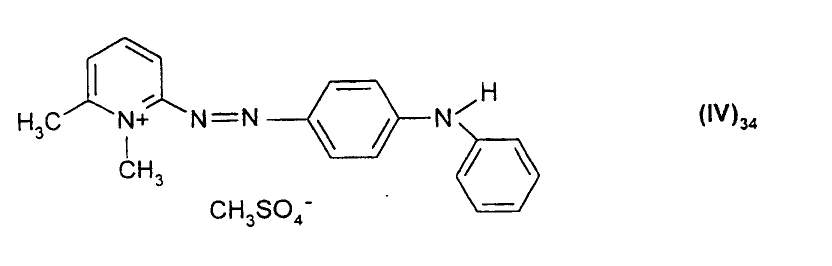

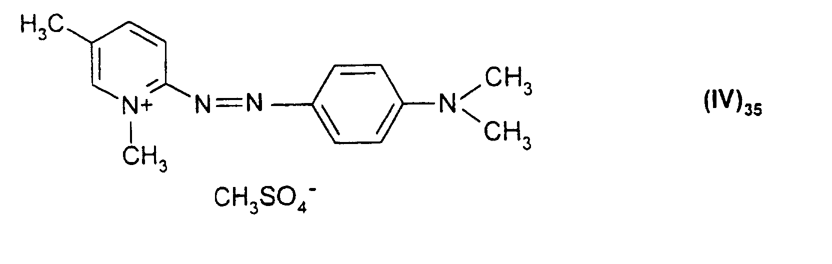

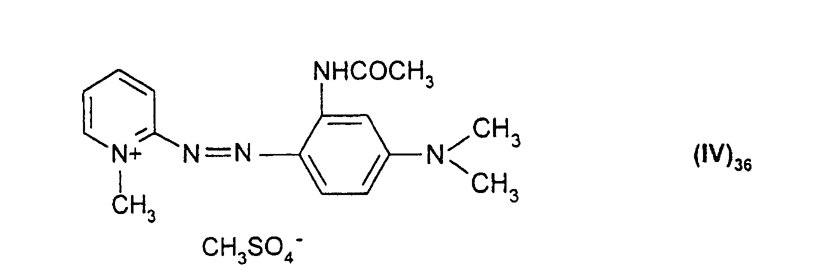

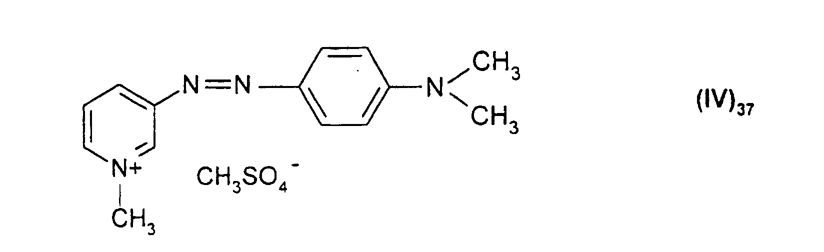

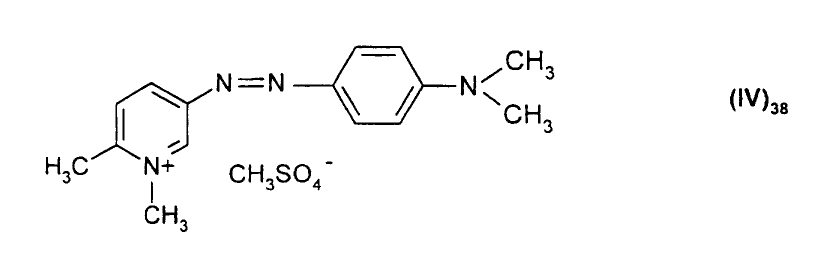

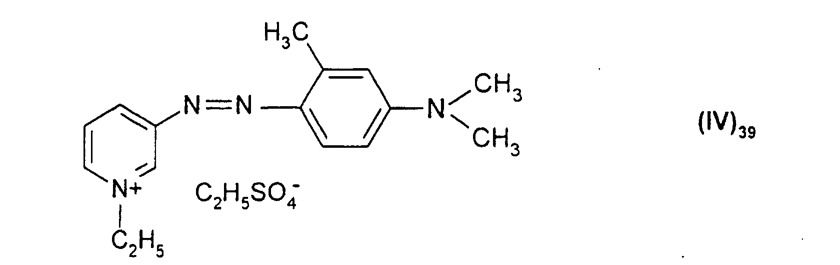

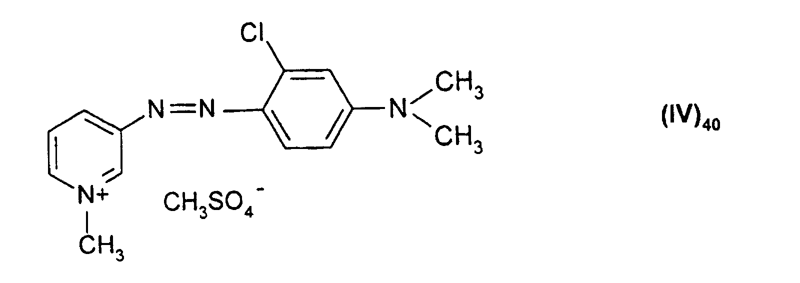

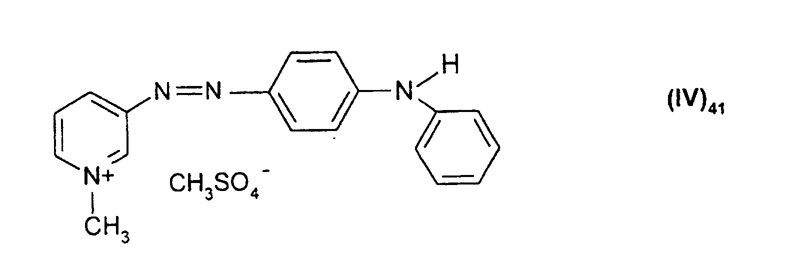

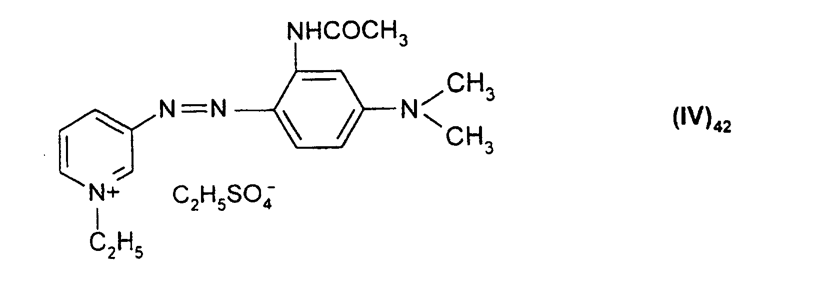

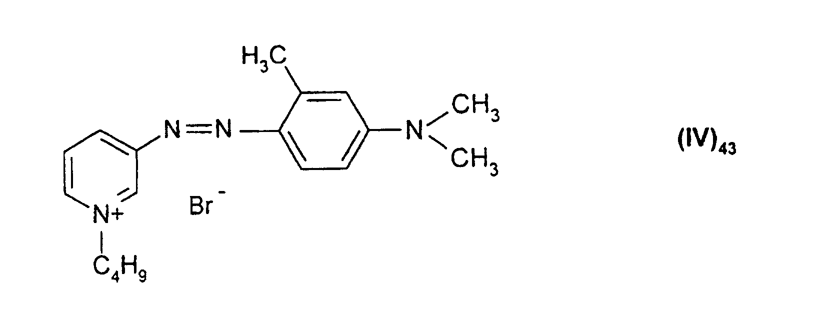

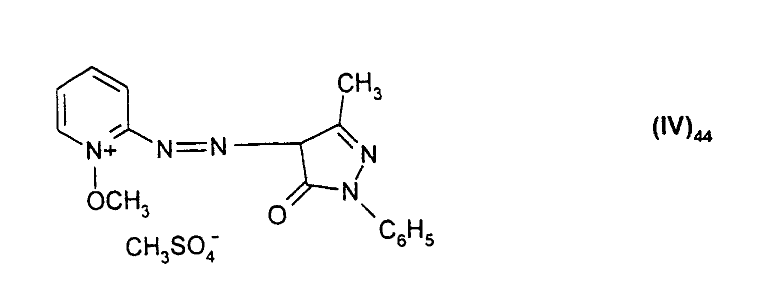

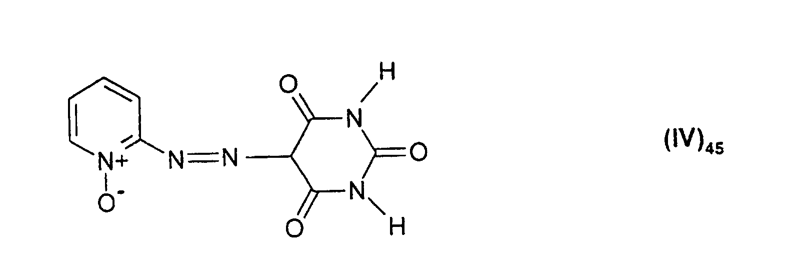

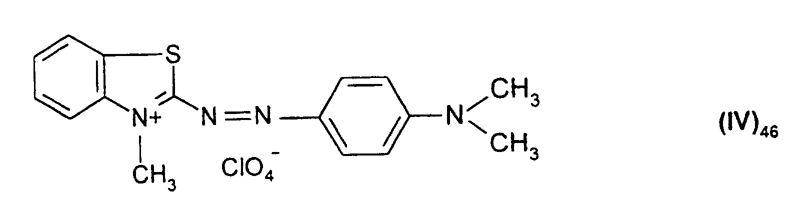

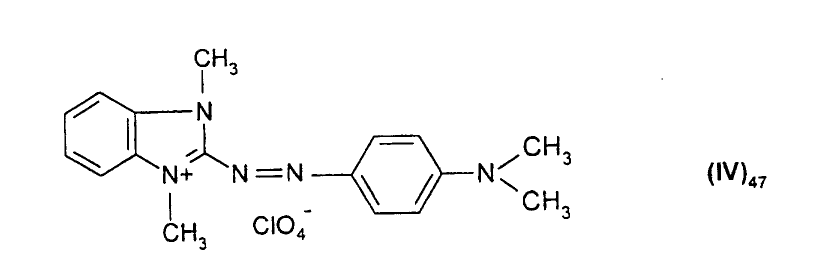

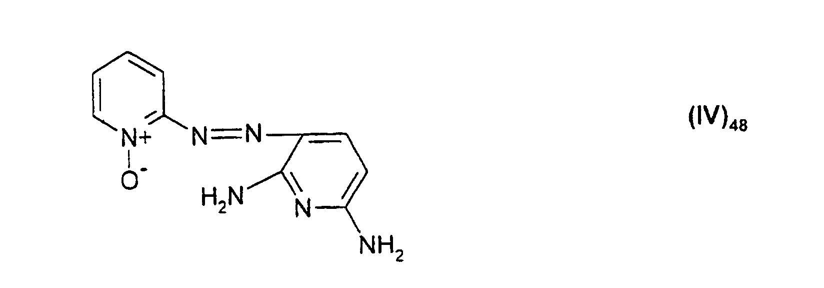

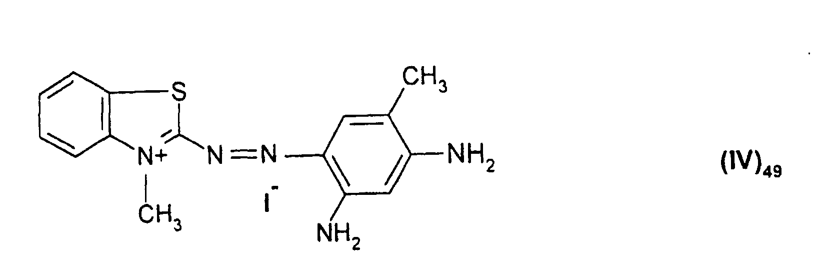

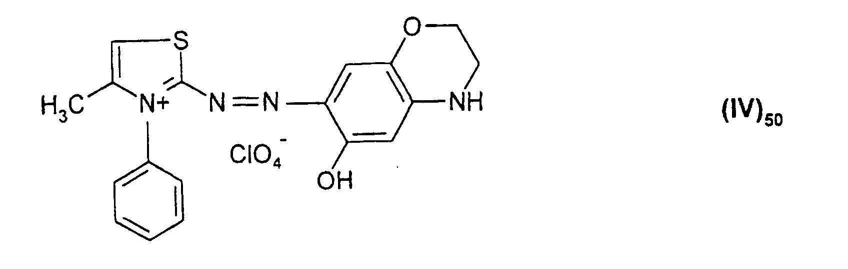

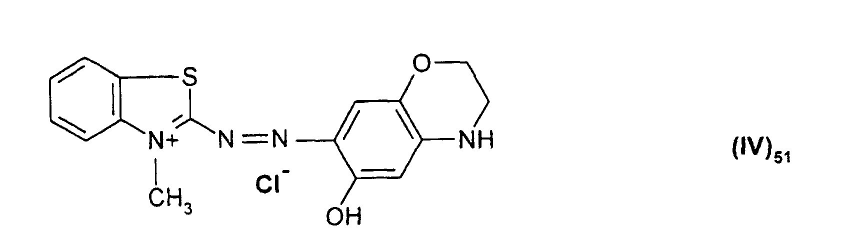

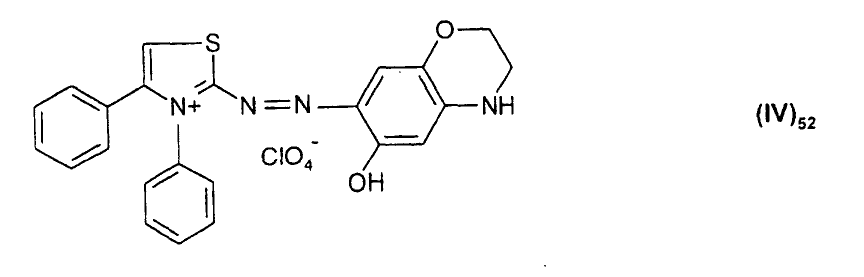

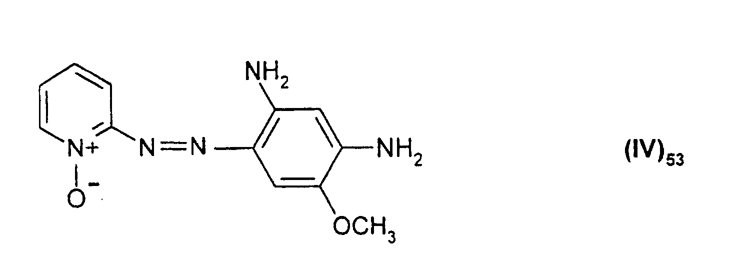

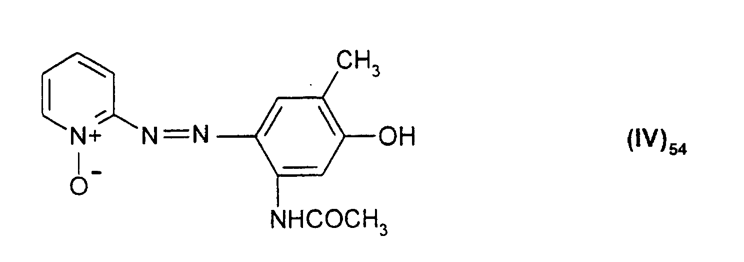

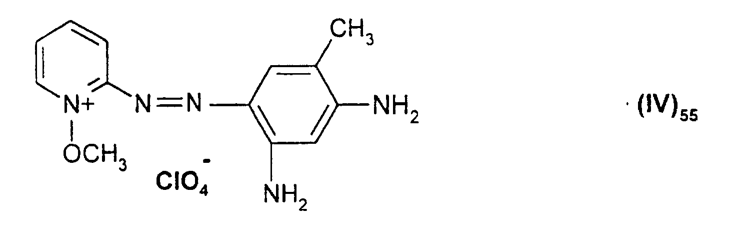

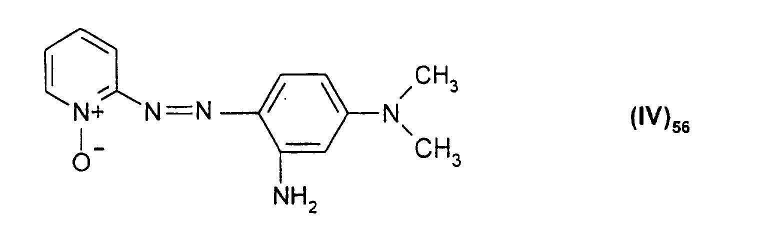

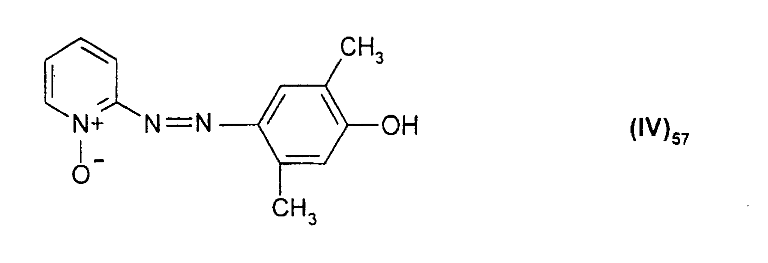

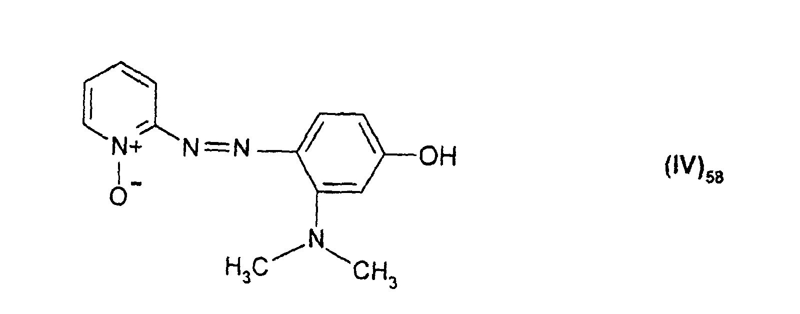

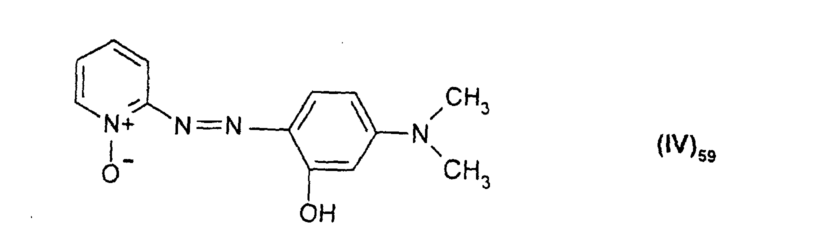

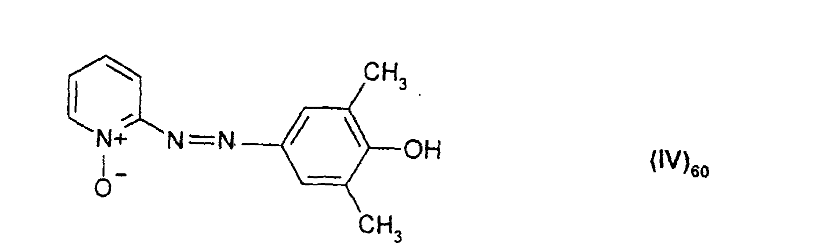

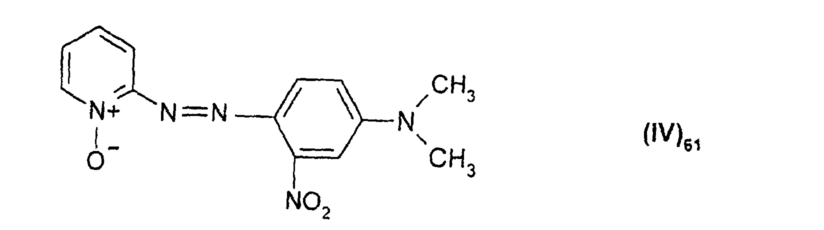

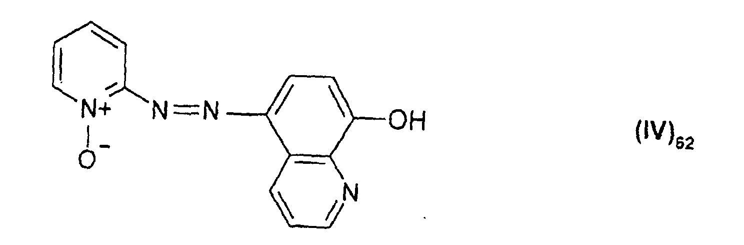

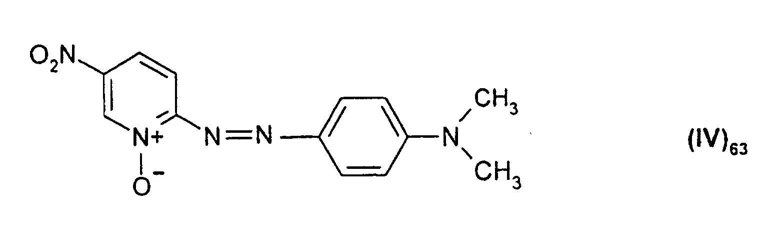

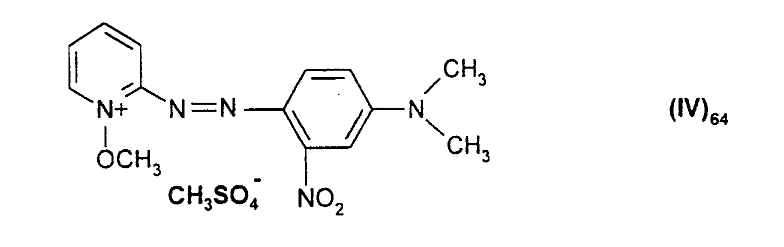

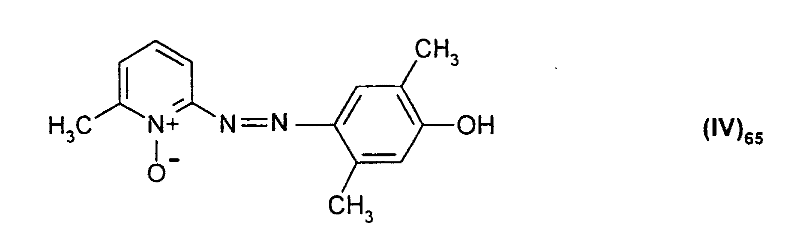

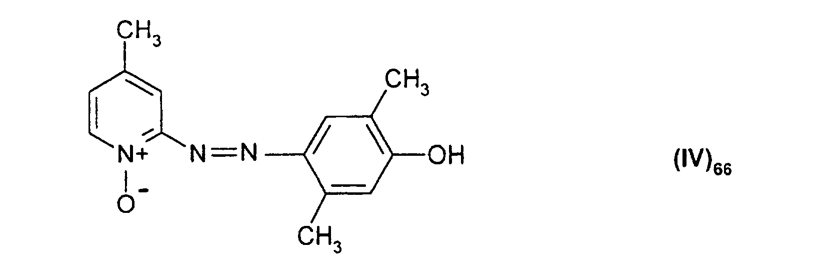

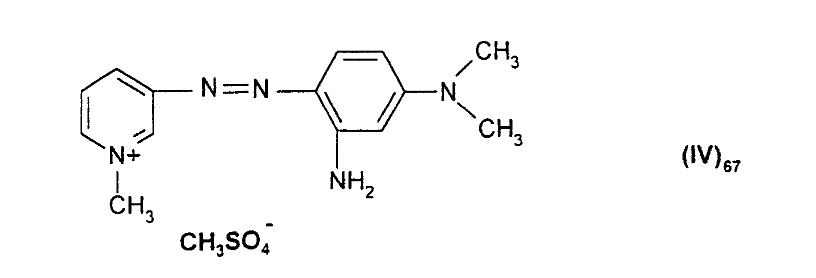

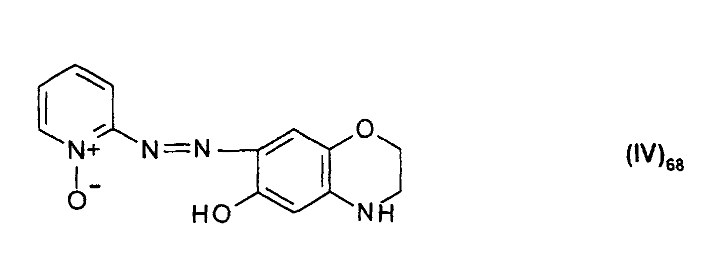

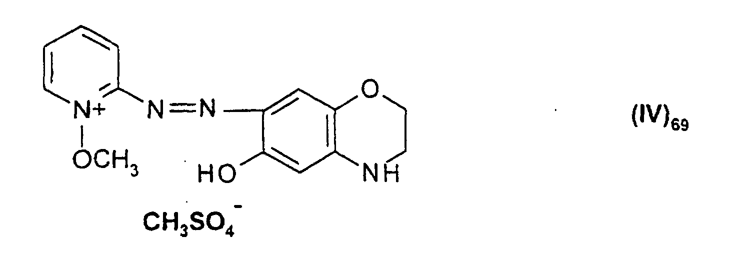

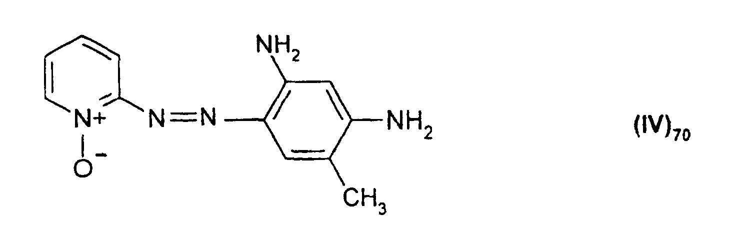

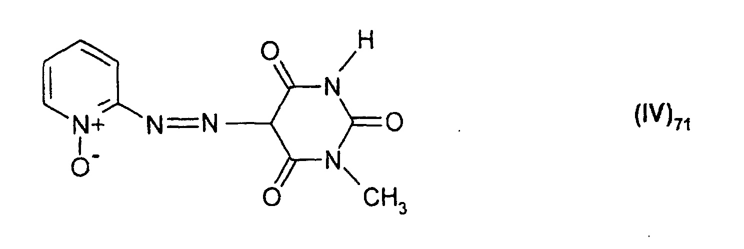

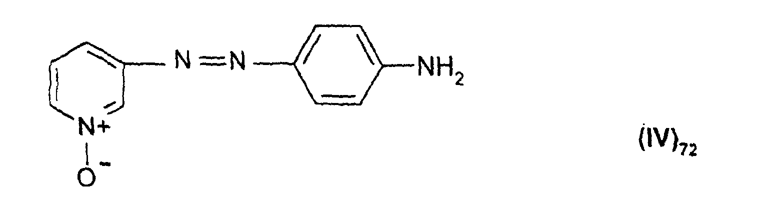

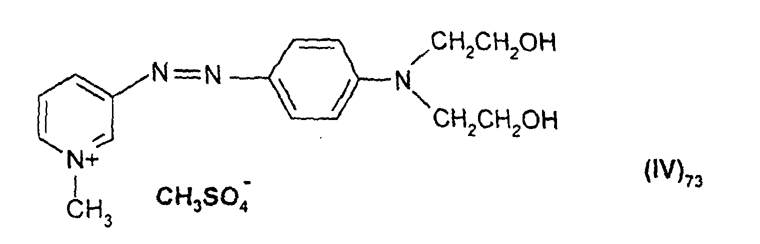

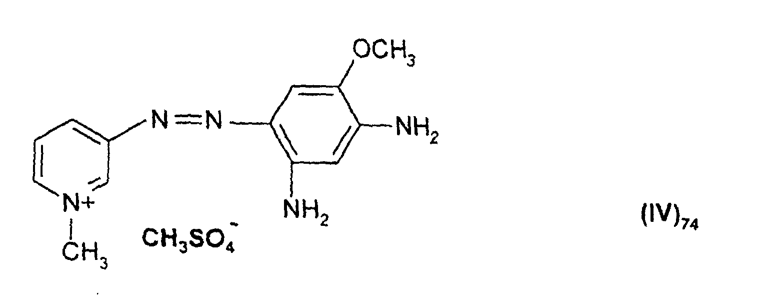

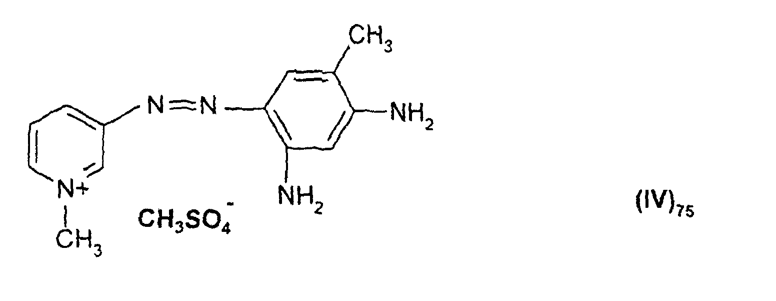

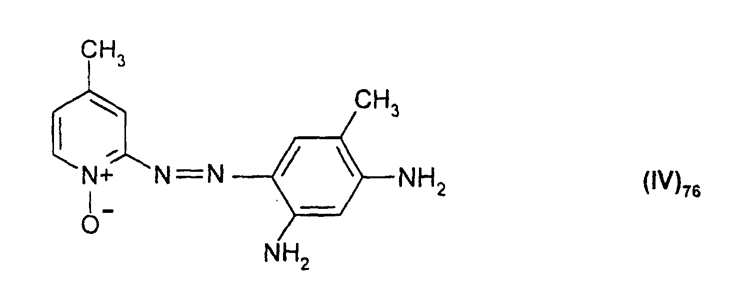

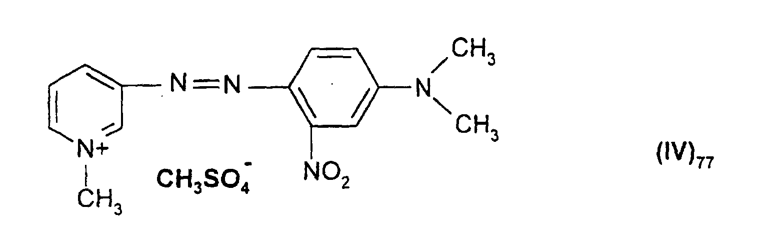

9. Composition selon l'une quelconque des revendications précédentes, **caractérisée par le fait que** le ou les colorants directs cationiques de formules (I), (II), (III), (III') ou (IV) représentent de 0,001 à 10 % en poids du poids total de la composition

10. Composition selon la revendication 9, **caractérisée par le fait que** le ou les colorants directs cationiques de formules (I), (II), (III) (III') ou (IV) représentent de 0,005 à 5 % en poids du poids total de la composition.

11. Composition selon l'une quelconque des revendications précédentes, **caractérisée par le fait que** l'agent tensio-actif non-ionique (ii) de type alkylpolyglucoside est un composé de formule (V) suivante : 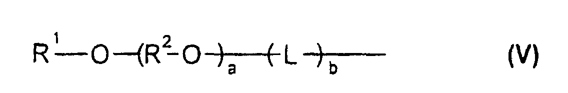 dans laquelle,
R¹ désigne un radical alkyle et/ou alcényle linéaire ou ramifié comportant environ de 8 à 24 atomes de carbone, un radical alkylphényle dont le groupe alkyle linéaire ou ramifié comporte environ de 8 à 24 atomes carbone, R² désigne un radical alkylène comportant de 2 à 4 atomes de carbone, L désigne un sucre réduit comportant de 5 à 6 atomes de carbone, a désigne une valeur allant de 0 à 10, et b désigne une valeur allant de 1 à 15.

12. Composition selon la revendication 11, **caractérisée par le fait que** dans la formule (V), R¹ désigne un radical alkyle et/ou alcényle linéaire ou ramifié comportant de 9 à 14 atomes de carbone, a désigne zéro, L désigne le glucose, b désigne une valeur allant de 1 à 4.

13. Composition selon l'une quelconque des revendications précédentes **caractérisée par le fait que** l'agent tensio-actif non-ionique (ii) de type ester d'acide gras de sucre ou d'alkylsucre **(ii)**_{**2**} est un ester d'acide gras en C₄-C₂₂ de sucre ou un ester d'acide gras en C₄-C₂₂ d'alkyl(C₁-C₄)sucre

14. Composition selon la revendication 13, **caractérisée par le fait qu'**il s'agit de monoesters d'acide gras en C₄-C₂₂ de glucose ou de saccharose.

15. Composition selon la revendication 13, **caractérisée par le fait qu'**il s'agit d'ester d'acide gras en C₄-C₂₂ de méthylglucoside, d'éthylglucoside ou de butylglucoside.

16. Composition selon la revendication 15, **caractérisée par le fait qu'**il s'agit d'esters d'acides gras en C₁₂-C₁₈ de butylglucoside, ou du monostéarate de méthylglucoside.

17. Composition selon l'une quelconque des revendications précédentes, **caractérisée par le fait que** l'agent tensio-actif non-ionique de type sucramide gras (ii)₃ est choisi parmi : **(ii)**_{**3**}**(a)** les aldonamides N-substitués et **(ii)**_{**3**}**(b)** les amides d'acides gras polyhydroxylés ou leurs mélanges.

18. Composition selon la revendication 17, **caractérisée par le fait que** les aldonamides N-substitués **(ii)**_{**a**}**(a)** sont choisis parmi :
(i) les lactobionamides N-substitués, les maltobionamides N-substitués, les cellobionamides N-substitués, les mellibionamides N-substitués et les gentiobionamides N-substitués;
(ii) les esters de N-lactobionyl aminoacide, les esters de N-maltobionylaminoacide, les esters de N-mellibionylaminoacide, les esters de N-cellobionylaminoacide, et les esters de N-gentiobionylaminoacide;
(iii) les N-(alkyloxy)alkyl-lactobionamides;
(iv) les N-(polyalkyloxy)alkyl lactobionamides, N-(polyalkyloxy)alkyl maltobionamides, N-(polyalkyloxy)alkyl cellobionamides, N-(polyalkyloxy)alkyl mellibionamides ou les N-(polyalkyloxy)alkyl gentiobionamides.

19. Composition selon la revendication 17, **caractérisée par le fait que** l'amide d'acide gras polyhydroxylé **(ii)**_{**3**}**(b)** est un composé de formule (VI) suivante : 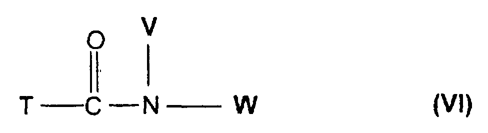 dans laquelle,
**T** désigne un groupe hydrocarboné en C₅-C₃₁, de préférence une chaîne alkyle ou alcényle linéaire en C₇-C₁₅ ou leurs mélanges ;
**V** désigne hydrogène, un radical hydrocarboné en C₁-C₄, 2-hydroxy éthyle, 2-hydroxypropyle ou leurs mélanges, de préférence un alkyle en C₁-C₄ et plus particulièrement méthyle ;
**W** désigne un groupe polyhydroxyhydrocarboné ayant une chaîne linéaire hydrocarbonée avec au moins 3 groupes hydroxyles directement liés à la chaîne, ou un dérivé alcoxylé dudit groupe.

20. Composition selon la revendication 19, **caractérisée par le fait que W** désigne un dérivé de sucre réducteur obtenu par réaction d'amination réductrice et de préférence un groupe glycityle.

21. Composition selon la revendication 19 ou 20, **caractérisée par le fait que W** est choisi parmi les groupes de formules suivantes :
-CH₂-(CHOH)ₙ-CH₂OH ;
-CH-(CH₂OH)-(CHOH)ₙ₋₁-CH₂OH :
-CH₂-(CHOH)₂(CHOR')(CHOH)-CH₂OH dans lesquelles n est un nombre entier allant de 3 à 5, R' désigne hydrogène, un monosaccharide cyclique ou aliphatique ou l'un de ses dérivés alcoxylés.

22. Composition selon la revendication 21, **caractérisée par le fait qu'**il s'agit du groupe -CH₂-(CHOH)₄-CH₂OH.

23. Composition selon l'une quelconque des revendications 19 à 22, **caractérisée par le fait que** dans la formule (VI), le groupe T-CON= est choisi parmi : cocamide, stéaramide, oléamide, lauramide, myristiramide, capricamide, palmitamide ou amide de suif.

24. Composition selon l'une quelconque des revendications précédentes, **caractérisée par le fait que** le ou les agents tensio-actifs non-ioniques (ii) représentent de 0,05 à 30 % en poids du poids total de la composition tinctoriale.

25. Composition selon la revendication 24, **caractérisée par le fait que** le ou les agents tensio-actifs non-ioniques représentent de 0,1 à 15 % en poids du poids total de la composition tinctoriale.

26. Composition selon l'une quelconque des revendications précédentes, **caractérisée par le fait que** le milieu approprié pour la teinture (ou support) est constitué par de l'eau ou par un mélange d'eau et d'au moins un solvant organique.

27. Composition selon l'une quelconque des revendications précédentes, **caractérisée par le fait qu'**elle présente un pH compris entre 2 et 11, et de préférence entre 5 et 10.

28. Composition selon l'une quelconque des revendications précédentes, **caractérisée par le fait qu'**elle est destinée à la teinture d'oxydation et qu'elle contient une ou plusieurs bases d'oxydation choisie parmi les paraphényfènediamines, les bis-phénylalkylènediamines, les para-aminophénols, les ortho-aminophénols et les bases hétérocycliques.

29. Composition selon la revendication 28, **caractérisée par le fait que** la ou les bases d'oxydation représentent 0,0005 à 12 % en poids du poids total de la composition tinctoriale.

30. Composition selon la revendication 29, **caractérisée par le fait que** la ou les bases d'oxydation représentent 0,005 à 6 % en poids du poids total de la composition tinctoriale.

31. Composition selon l'une quelconque des revendications 28 à 30, **caractérisée par le fait qu'**elle renferme un ou plusieurs coupleurs choisis parmi les métaphénytenediannnes, les méta-aminophénols, les métadiphénols et les coupleurs hétérocycliques.

32. Composition selon la revendication 31, **caractérisée par le fait que** le ou les coupleurs représentent de 0,0001 à 10 % en poids du poids total de la composition tinctoriale.

33. Composition selon la revendication 32, **caractérisée par le fait que** le ou les coupleurs représentent de 0,005 à 5 % en poids du poids total de la composition tinctoriale.

34. Composition selon l'une quelconque des revendications précédentes, **caractérisée par le fait qu'**elle est destinée à la teinture directe éclaircissante ou la teinture d'oxydation et qu'elle renferme alors au moins un agent oxydant.

35. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 34, pendant un temps suffisant pour développer la coloration désirée, après quoi on rince, on lave éventuellement au shampooing, on rince à nouveau et on sèche.

36. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 34, pendant un temps suffisant pour développer la coloration désirée, sans rinçage final.

37. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant l'agent tensio-actif non-ionique (ii) tel que défini dans les revendications précédentes.

38. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant l'agent tensio-actif non-ionique (ii) tel que défini dans les revendications précédentes.

39. Dispositif à plusieurs compartiments ou "kit" de teinture à plusieurs compartiments, **caractérisé par le fait qu'**un premier compartiment renferme la composition (A1) ou (A2) telle que définie à la revendication 37 ou 38 et un second compartiment renferme la composition (B1) ou (B2) telle que définie à la revendication 37 ou 38.

## Patentansprüche

1. Zusammensetzung zum Färben von Keratinfasern und insbesondere menschlichen Keratinfasern, wie dem Haar, die in einem zum Färben geeigneten Medium enthält: (i) mindestens eine kationische Direktfarbstoff, die unter den Verbindungen der folgenden Formeln (I), (II), (III), (III') und (IV) ausgewählt ist:
a) Verbindungen der folgenden Formel (I): 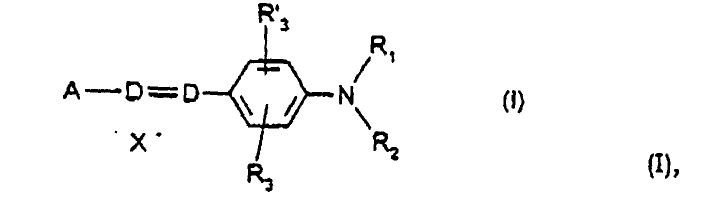 worin bedeuten:
D ein Stickstoffatom oder die Gruppe -CH, die Gruppen R₁ und R₂, die gleich oder verschieden sind, ein
Wasserstoffätom; eine C₁₋₄-Alkylgruppe, die mit einer Gruppe -CN, -OH oder -NH₂ substituiert sein kann; oder sie bilden mit einem Kohlenstoffatom des Benzolrings einen gegebenenfalls sauerstoff- oder stickstoffhaltigen Heterocyclus, der mit einer oder mehreren C₁₋₄-Alkylgruppen substituiert sein kann; oder eine 4'-Aminophenylgruppe,
die Gruppen R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder Acetyloxy,
X⁻ ein Anion, das vorzugsweise unter Chlorid, Methylsulfat und Acetat ausgewählt ist,
A eine Gruppe, die unter den folgenden Strukturen A1 bis A18 ausgewählt ist: 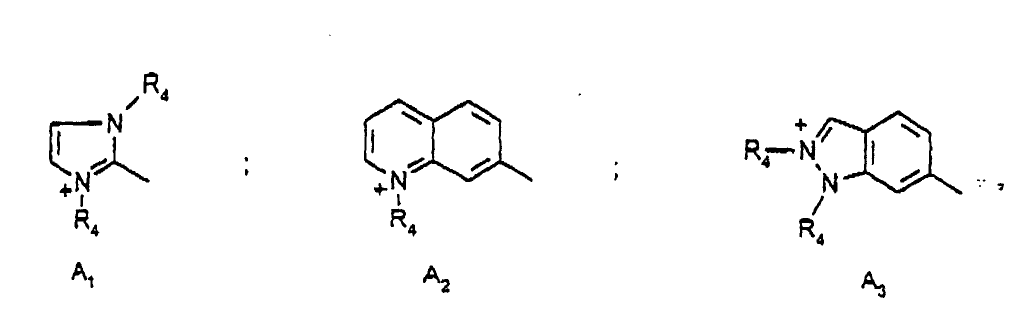 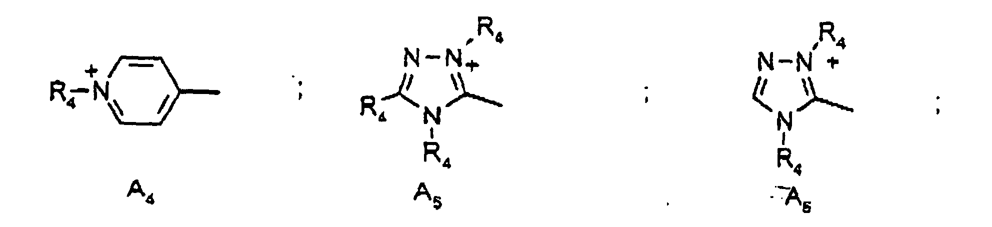 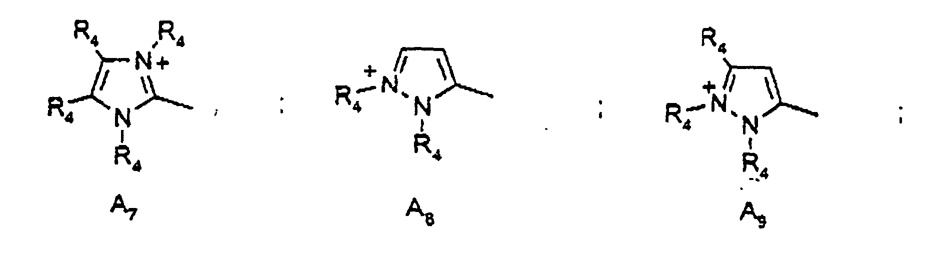 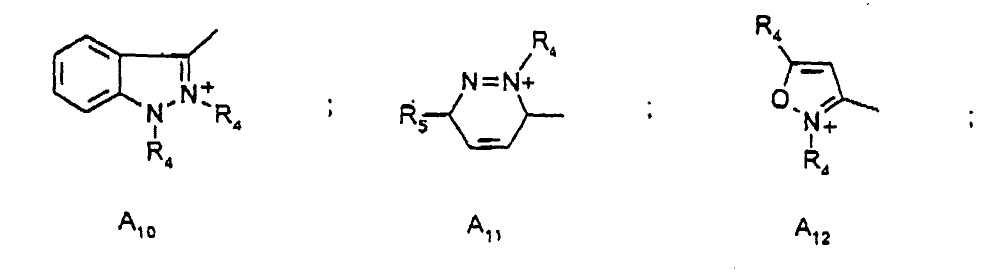 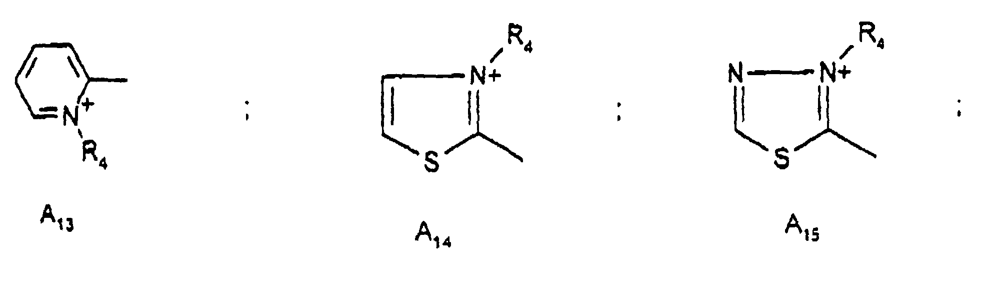 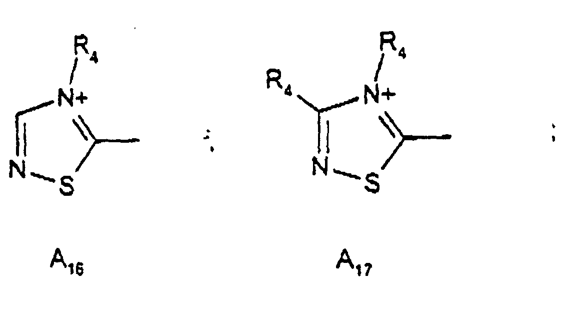 und 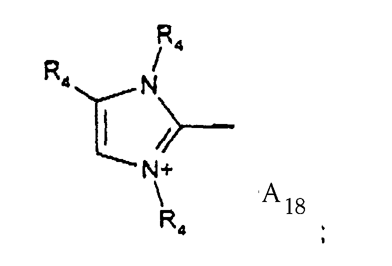
worin die Gruppe R₄ eine C₁₋₄-Alkylgruppe bedeutet, die mit einer Hydroxygruppe substituiert sein kann und R₅ eine C₁₋₄-Alkoxygruppe bedeutet, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
b) Verbindungen der folgenden Formel (II): 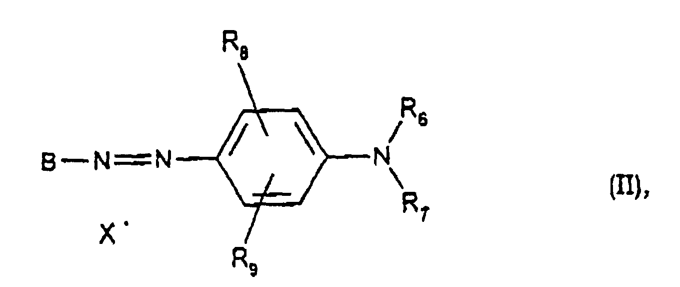 worin bedeuten:
R₆ ein Wasserstoffatom oder eine C₁₋₄-Alkylgruppe,
R₇ ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstvffhaltigen und/oder stickstoffhaltigen Heterocyclus, der mit einer C₁₋₄-Alkylgruppe substituiert sein kann,
die Gruppen R₈ und R₉, die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
X⁻ ein Anion, das vorzugsweise unter Chlorid, Methylsulfat und Acetat ausgewählt ist,
B eine Gruppe, die unter den folgenden Strukturen B1 bis B6 ausgewählt ist: 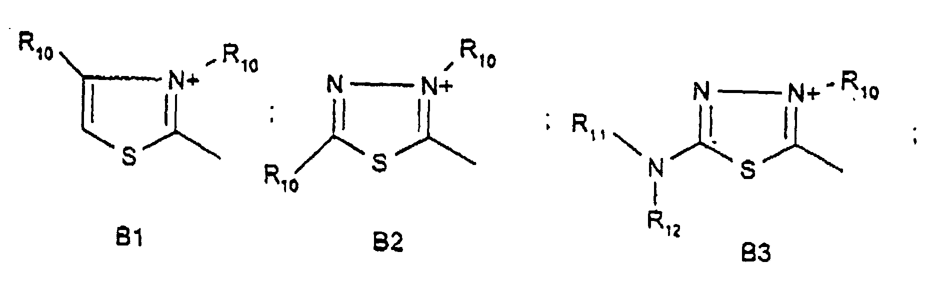 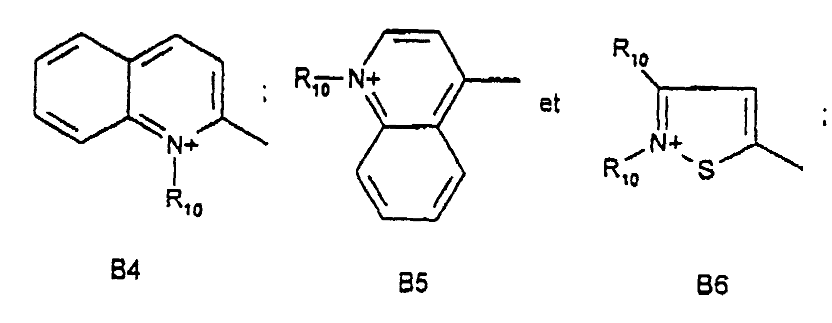
worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten;
c) Verbindungen der folgenden Formeln (III) und (III'): 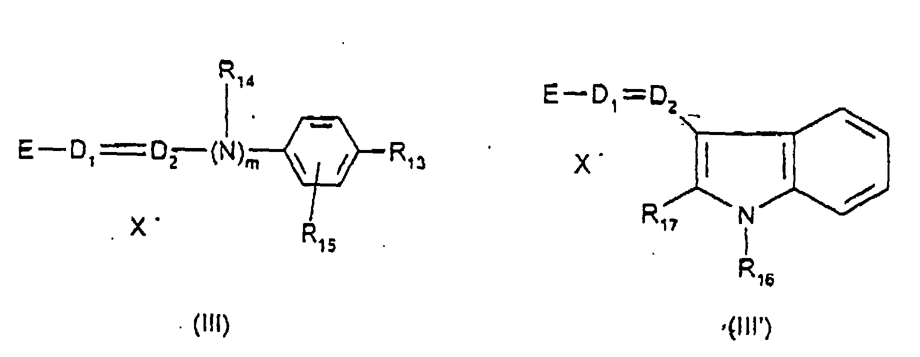 worin bedeuten:
R₁₃ ein Wasserstoffatom, eine C₁₋₄-Alkoxygruppe, ein Halogenatom, wie Brom, Chlor, Iod oder Fluor, oder eine Aminogruppe,
R₁₄ ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig ist und/oder mit einer oder mehreren C₁₋₄-Alkylgruppen substituiert ist;
R₁₅ ein Wasserstoffatom oder ein Halogenatom, wie Brom, Chlor, Iod oder Fluor,
die Gruppen R₁₆ und R₁₇, die identisch oder voneinander verschieden sind, Wasserstoff oder C₁₋₄-Alkyl,
die Gruppen D₁ und D₂, die gleich oder verschieden sind, ein Stickstoffatom oder die Gruppe -CH,
m = 0 oder 1, mit der Maßgabe, dass die Gruppen D₁ und D₂ gleichzeitig -CH bedeuten und m = 0, wenn R₁₃ eine unsubstituierte Aminogruppe ist,
X⁻ ein Anion, das vorzugsweise unter Chlorid, Methylsulfat und Acetat ausgewählt ist,
E eine Gruppe, die unter den folgenden Strukturen E1 bis E8 ausgewählt ist: 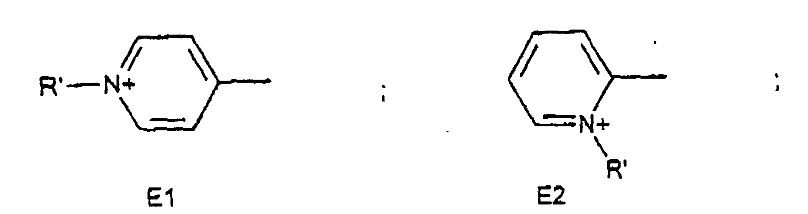 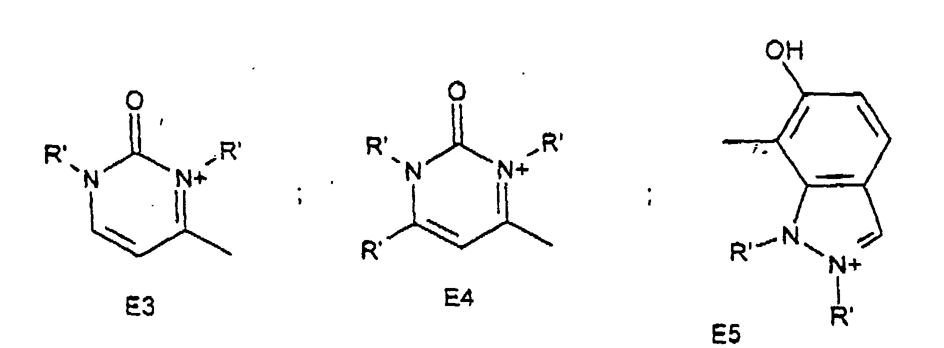 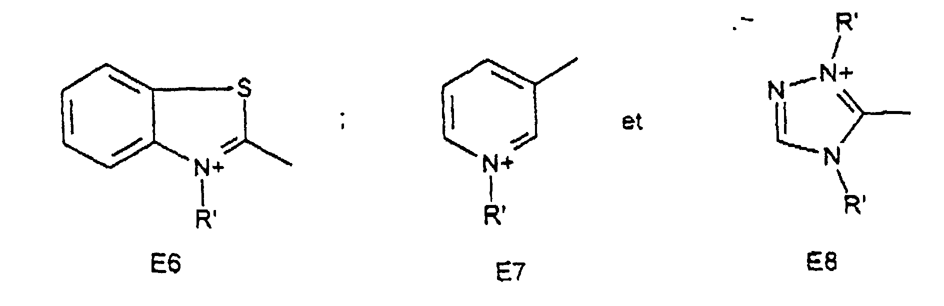
wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 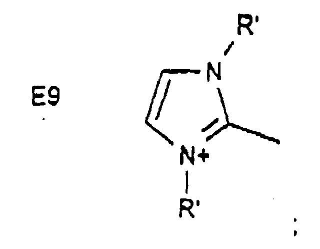 wobei R' eine C₁₋₄-Alkylgruppe bedeutet;
d) Verbindungen der folgenden Formel (IV):
G-N=N-J (IV)
worin bedeuten:
das Symbol G eine Gruppe, die unter den folgenden Strukturen G₁ bis G₃ ausgewählt ist: 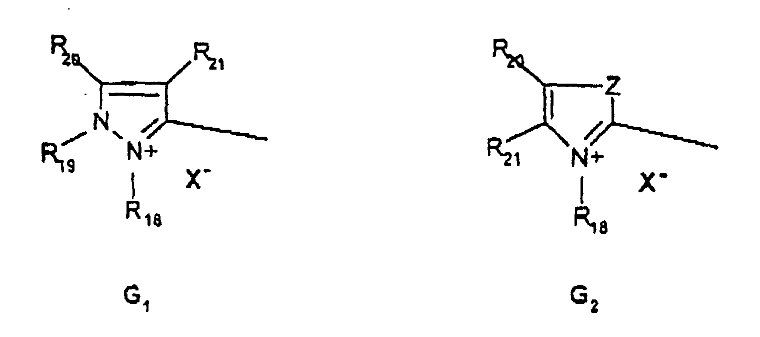 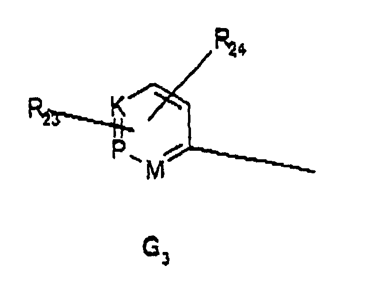 wobei in den Strukturen G₁ bis G₃ bedeuten:
R₁₈ C₁₋₄-Alkyl oder eine Phenylgruppe, die mit einer C₁₋₄-Alkylgruppe oder einem Halogenatom substituiert sein kann, das unter Chlor, Brom, Iod und Fluor ausgewählt ist;
R₁₉ C₁₋₄-Alkyl oder Phenyl;
R₂₀ und R₂₁, die gleich oder verschieden sind, C₁₋₄-Alkyl, Phenyl oder R₂₀ und R₂₁ bilden in G₁ gemeinsam einen Benzolring, der mit einer oder mehreren Gruppen C₁₋₄-Alkyl, C₁₋₄-Alkoxy oder NO₂ substituiert ist, oder bilden in G₂ gemeinsam einen Benzolring, der gegebenenfalls mit einer oder mehreren Gruppen C₁₋₄-Alkyl, C₁₋₄-Alkoxy oder NO₂ substituiert ist;
wobei R₂₀ außerdem ein Wasserstoffatom bedeuten kann;
Z ein Sauerstoffatom, ein Schwefelatom oder eine Gruppe -NR₁₉;
M -CH, -CR (wobei R eine C₁₋₄-Alkylgruppe ist), oder -NR₂₂(X⁻)ᵣ;
K -CH, -CR (wobei R eine C₁₋₄-Alkylgruppe bedeutet) oder -NR₂₂(X⁻ )ᵣ;
P eine Gruppe -CH, -CR (wobei R eine C₁₋₄-Alkylgruppe bedeutet) oder -NR₂₂(X⁻)ᵣ; wobei r Null oder 1 ist;
R₂₂ ein Atom O⁻, C₁₋₄-Alkoxy oder C₁₋₄-Alkyl;
R₂₃ und R₂₄, die gleich oder verschieden sind, ein Wasserstoffatom, ein Halogenatom, das unter Chlor, Brom, Iod und Fluor ausgewählt ist, eine C₁₋₄-Alkylgruppe, eine C₁₋₄-Alkoxygruppe oder die Gruppe -NO₂;
X⁻ ein Anion, das vorzugsweise unter Chlorid, Iodid, Methylsulfat, Ethylsulfat, Acetat und Perchlorat ausgewählt ist;
mit der Maßgabe, dass
wenn R₂₂ O⁻ bedeutet, r Null ist;
wenn, K oder P oder M -N(C₁₋₄)alkyl X⁻ bedeutet, R₂₃ oder R₂₄ von Wasserstoff verschieden ist;
wenn K -NR₂₂(X⁻)ᵣ bedeutet, M = P = -CH, -CR;
wenn M -NR₂₂(X-)ᵣ bedeutet, K = P = -CH, -CR;
wenn P -NR₂₂(X⁻)ᵣ bedeutet, K = M = -CH oder -CR bedeutet;
wenn Z ein Schwefelatom und R₂₁ C₁₋₄-Alkyl ist, R₂₀ von Wasserstoff verschieden ist;
wenn Z -NR₁₉ und R₁₉ C₁₋₄-Alkyl bedeutet, mindestens eine der Gruppen R₁₈, R₂₀ oder R₂₁ der Struktur G₂ von einer C₁₋₄-Alkylgruppe verschieden ist;
das Symbol J:
- (a) eine Gruppe der folgenden Struktur J₁: 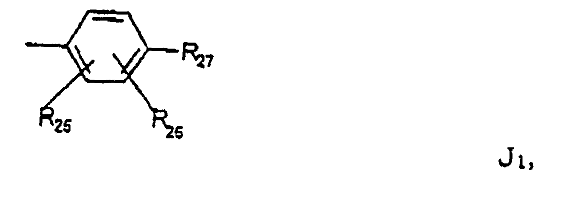 wobei in der Struktur J₁ bedeuten:
R₂₅ ein Wasserstoffatom, ein Halogenatom, das unter Chlor, Brom, Iod und Fluor ausgewählt ist, C₁₋₄-Alkyl, C₁₋₄-Alkoxy, -OH, -NO₂, -NHR₂₈, -NR₂₉R₃₀, -NHCOalkyl (C₁₋₄) oder R₂₅ bildet mit R₂₆ einen 5- oder 6-gliedrigen Ring, der gegebenenfalls ein oder mehrere Heteroatome enthält, die unter Stickstoff, Sauerstoff oder Schwefel ausgewählt sind;
R₂₆ ein Wasserstoffatom, ein Halogenatom, das unter Chlor, Brom, Iod und Fluor ausgewählt ist, C₁₋₄-Alkyl, C₁₋₄-Alkoxy oder R₂₆ bildet mit R₂₇ oder R₂₈ einen 5- oder 6-gliedrigen Ring, der gegebenenfalls ein oder mehrere Heteroatome enthält, die unter Stickstoff, Sauerstoff oder Schwefel ausgewählt sind;
R₂₇ Wasserstoff, -OH, -NHR₂₈ oder -NR₂₉R₃₀;
Raa Wasserstoff, C₁₋₄-Alkyl, C₁₋₄-Monohydroxyalkyl, C₂₋₄-Polyhydroxyalkyl oder Phenyl;
R₂₉ und R₃₀, die gleich oder verschieden sind, C₁₋₄-Alkyl, C₁₋₄-Monohydroxyalkyl oder C₂₋₄-Polyhydroxyalkyl;
- (b) eine stickstoffhaltige, 5- oder 6-gliedrige heterocyclische Gruppe, die weitere Heteroatome und/ oder carbonylhaltige Gruppen enthalten und mit einer oder mehreren Gruppen C₁₋₄-Alkyl, Amino oder Phenyl substituiert sein kann, insbesondere eine Gruppe der folgenden Struktur J₂: 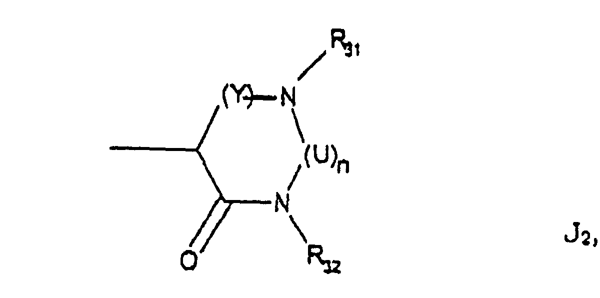 wobei in der Struktur J₂ bedeuten:
R₃₁, und R₃₂, die gleich oder verschieden sind, Wasserstoff; C₁₋₄-Alkyl oder Phenyl;
Y die Gruppe -CO- oder die Gruppe 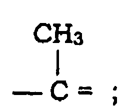
n = 0 oder 1, wobei U die Gruppe -CO- bedeutet, wenn n 1 ist,
wobei die Zusammensetzung **dadurch gekennzeichnet ist, dass** sie ferner enthält:
(ii) mindestens einen nichtionischen grenzflächenaktiven Stoff, der ausgewählt ist unter:
(ii)₁ - den Alkylpolyglycosiden;
(ii)₂ - den Zuckerfettsäureestern oder Alkylzuckerfettsäureestern;
(ii)₃ - den Zuckeramiden mit Fettkette;
(ii)₄ - deren Gemischen.

2. Zusammensetzung nach Anspruch 1, **dadurch gekennzeichnet, dass** die kationischen Direktfarbstoffe der Formel (I) unter den Verbindungen der folgenden Strukturen (I1) bis (I54) ausgewählt sind:

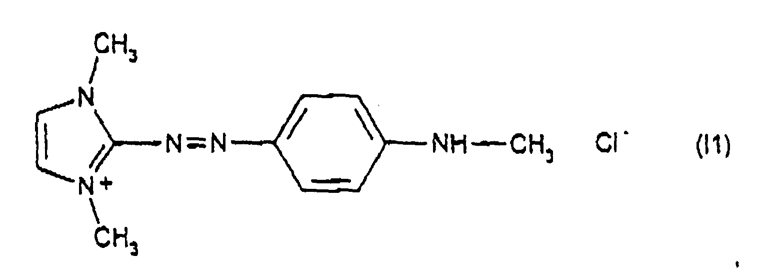

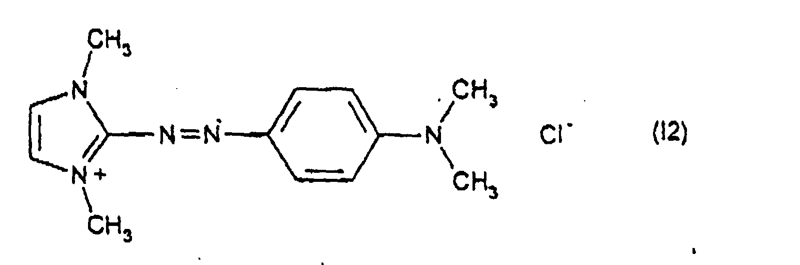

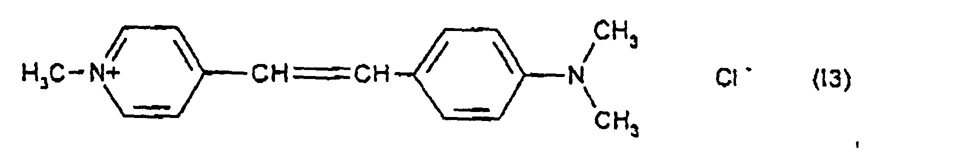

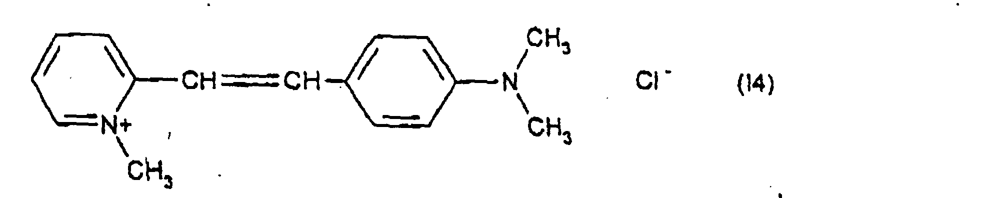

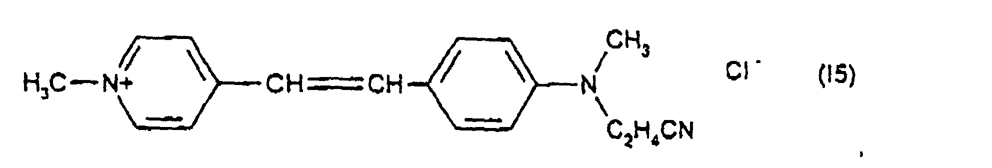

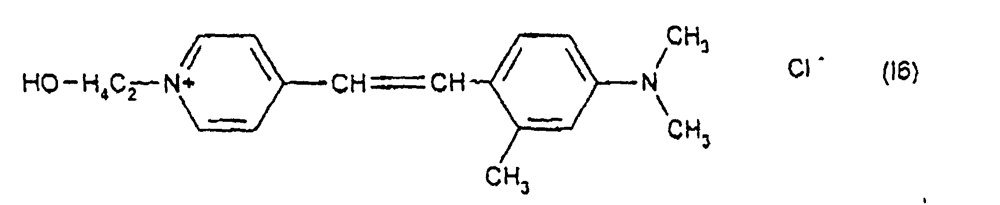

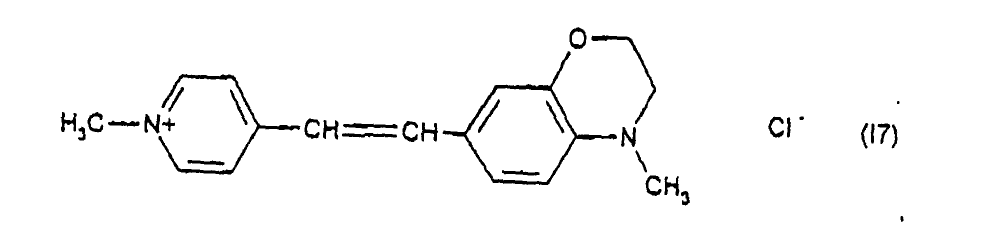

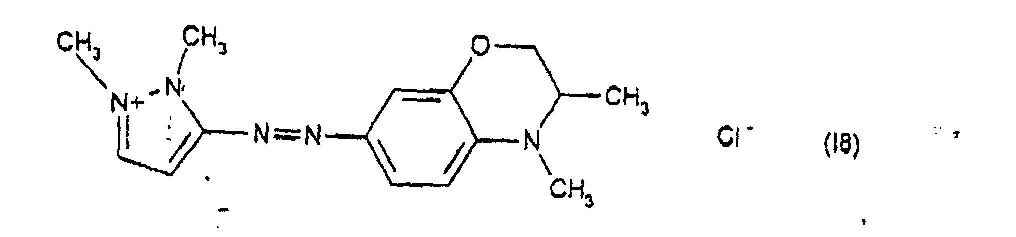

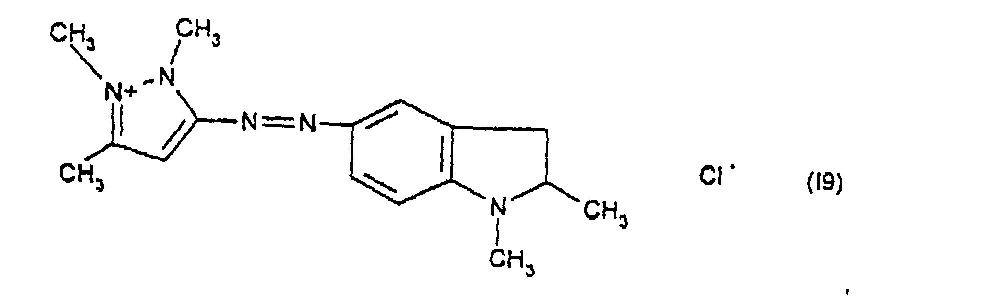

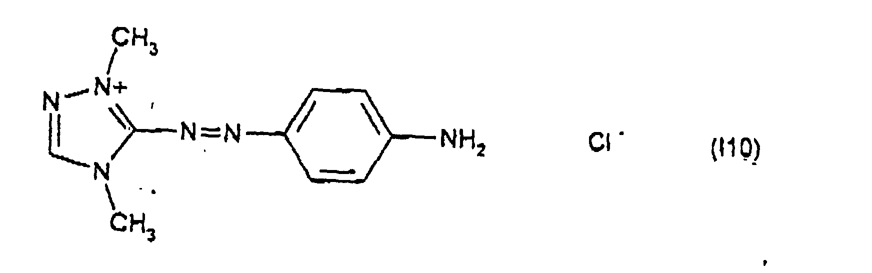

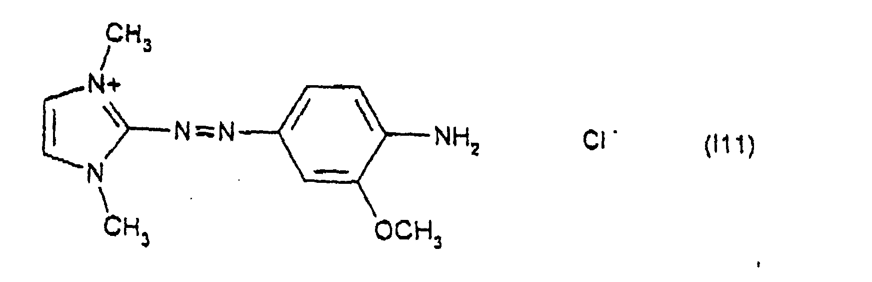

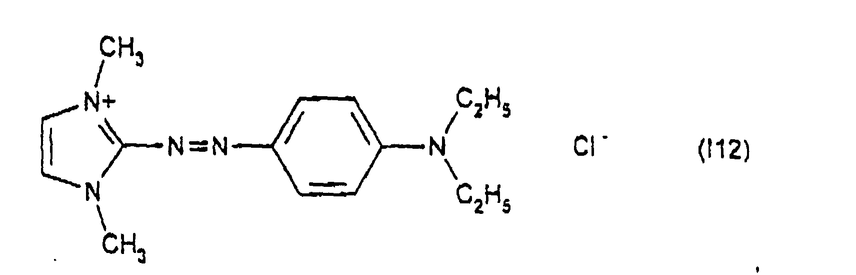

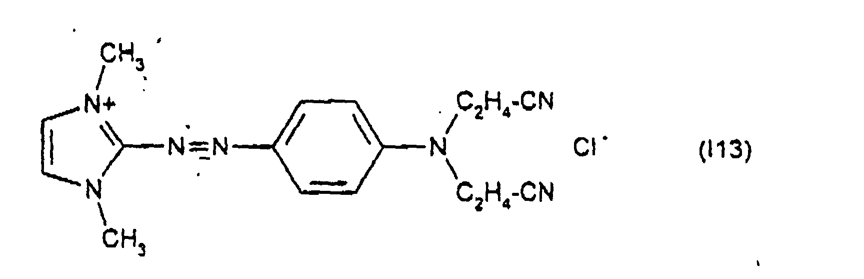

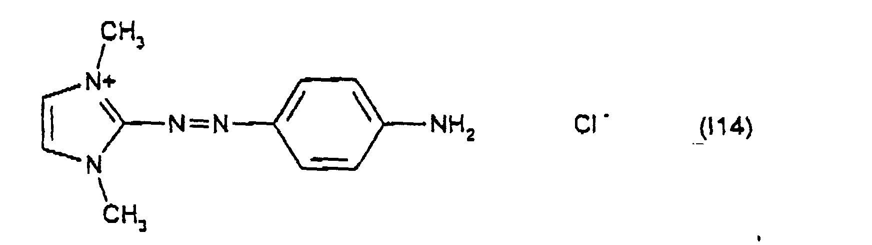

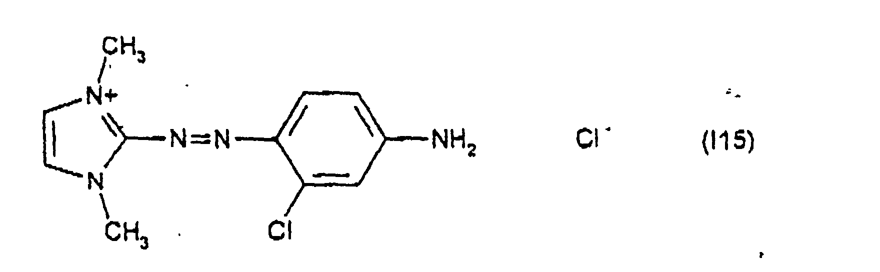

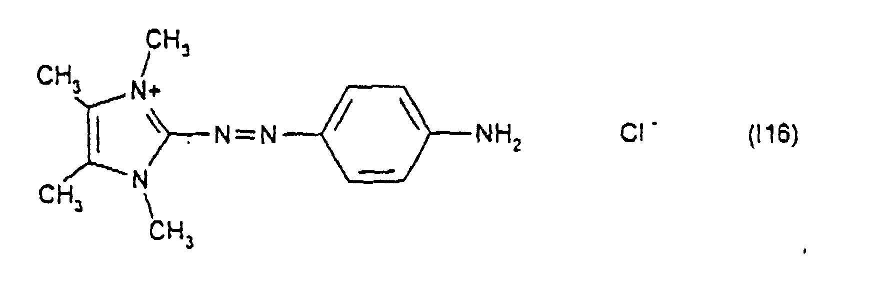

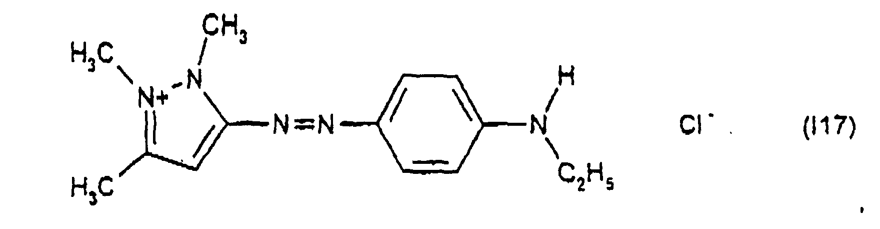

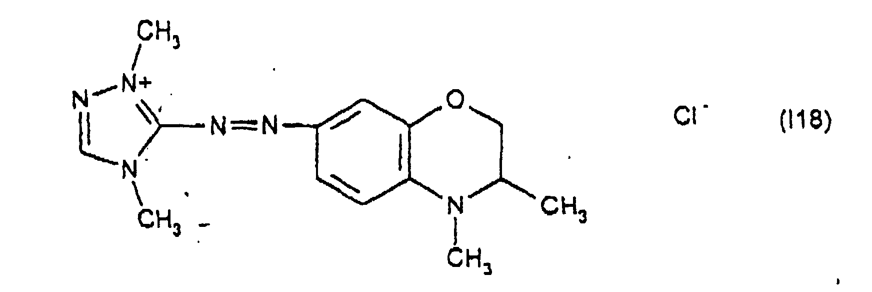

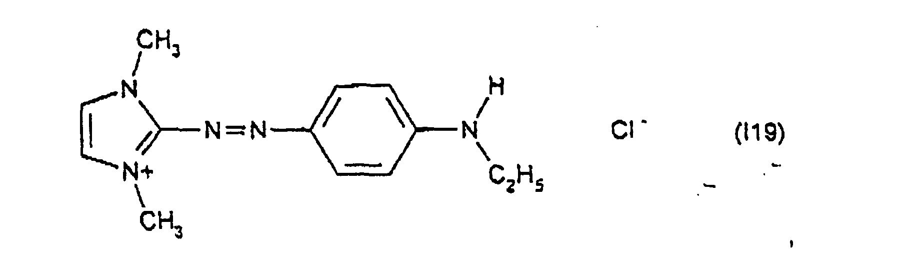

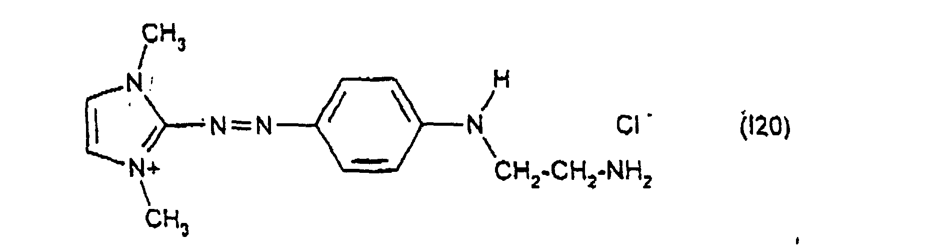

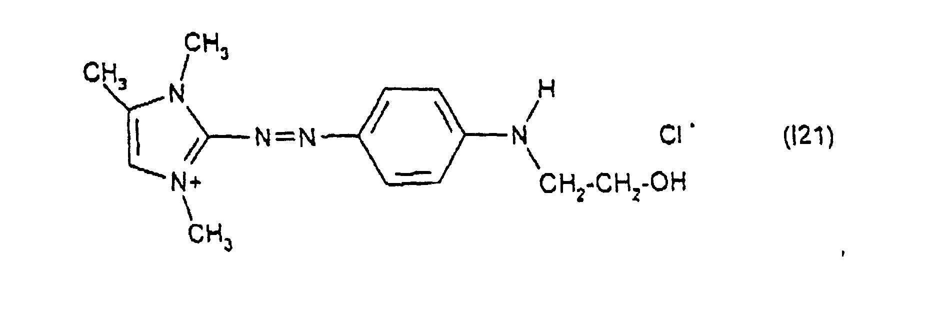

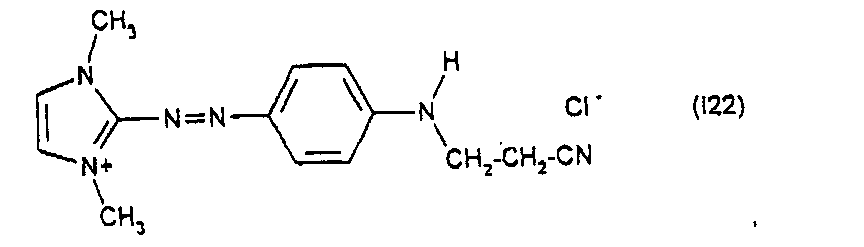

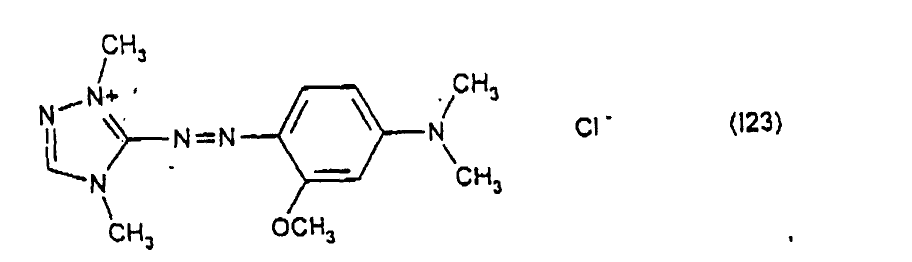

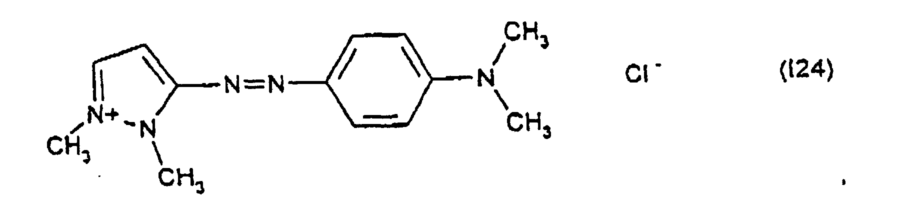

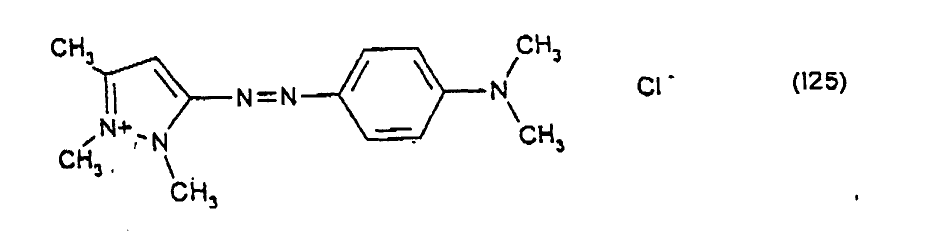

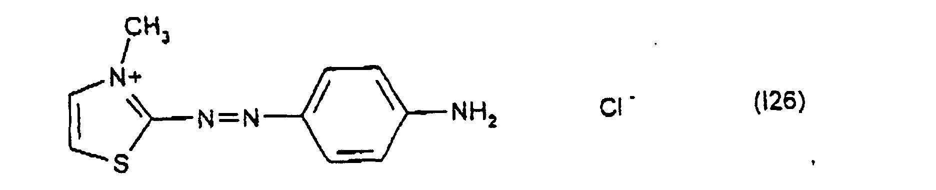

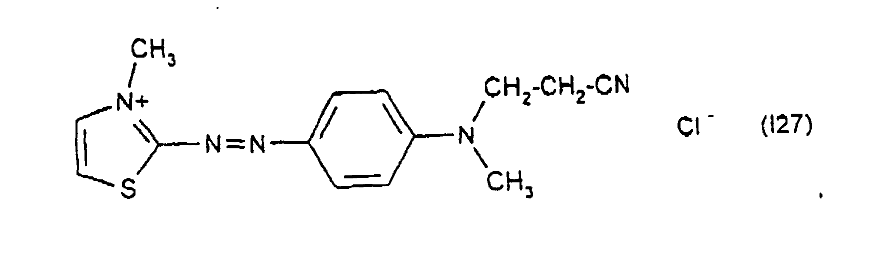

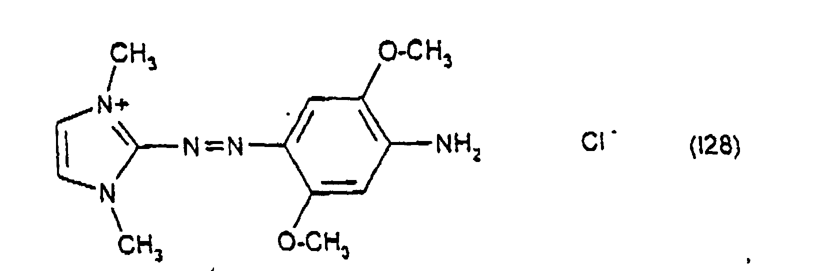

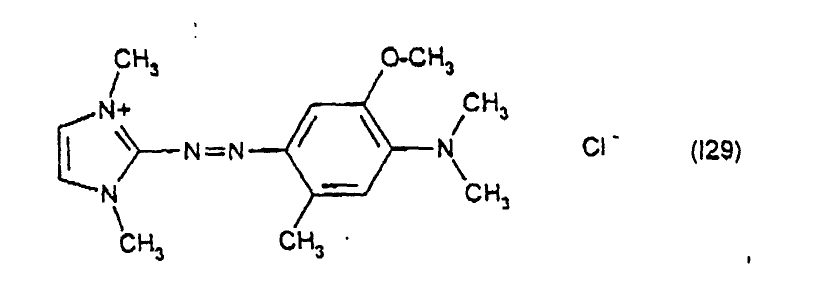

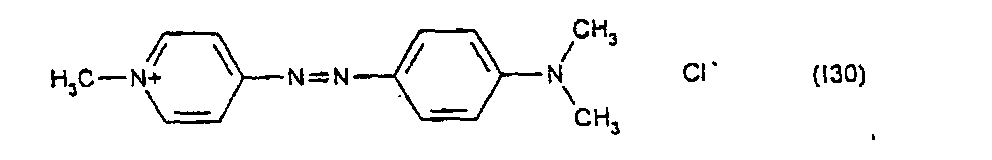

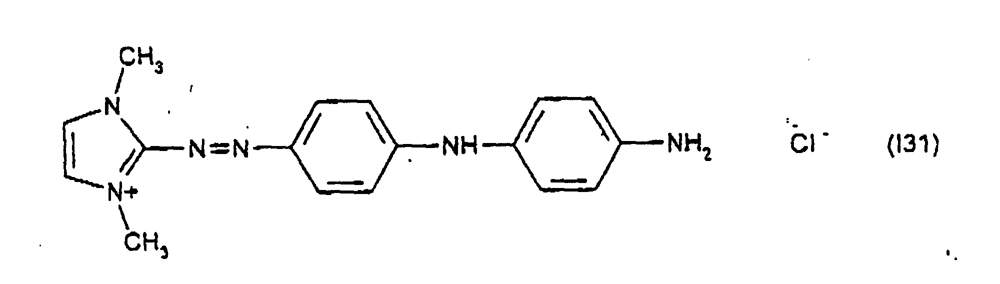

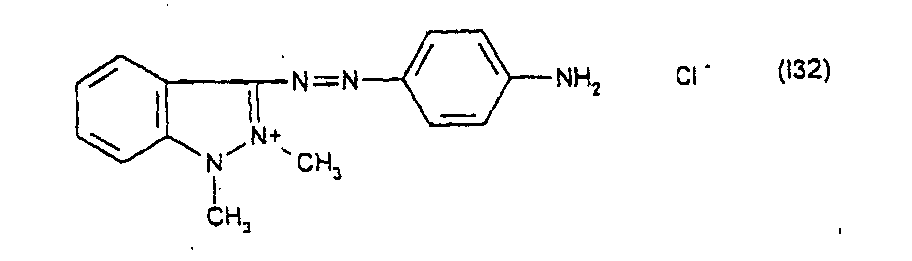

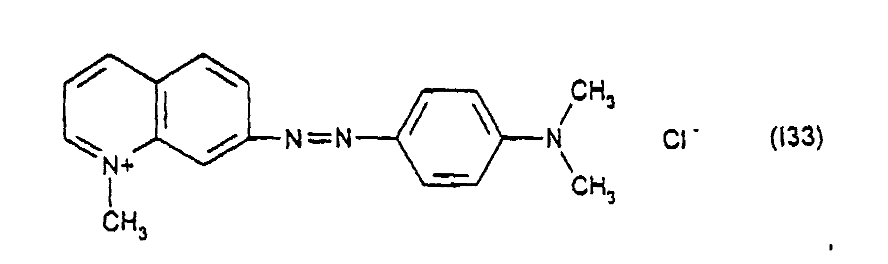

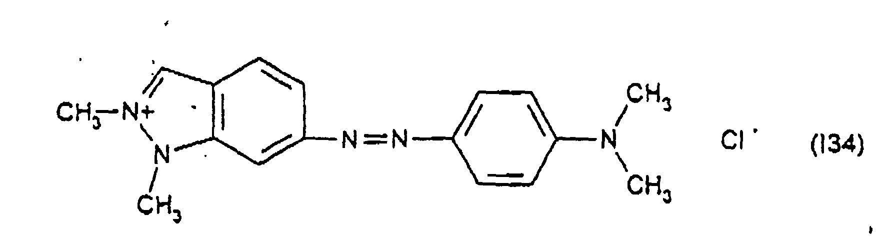

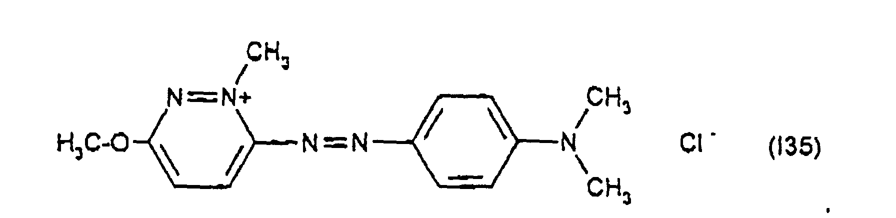

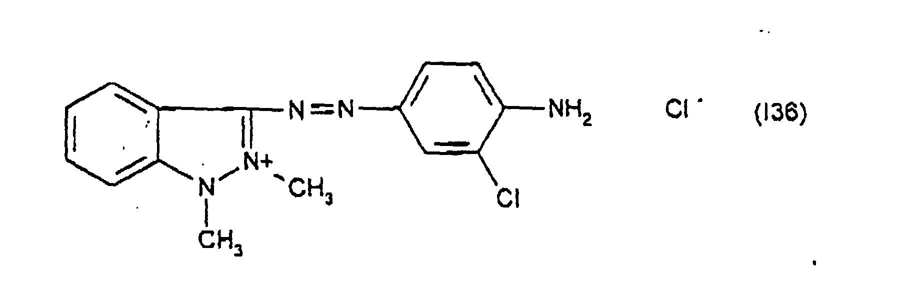

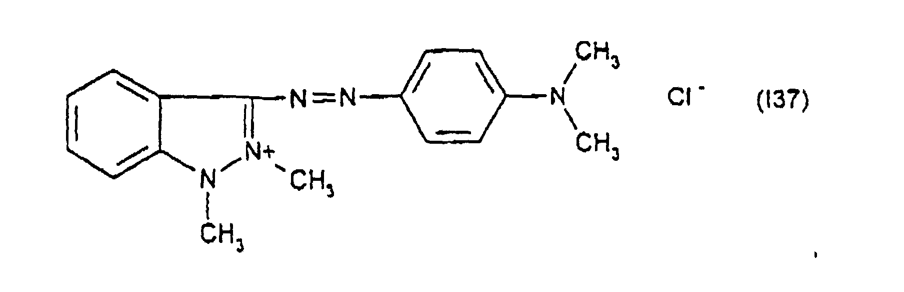

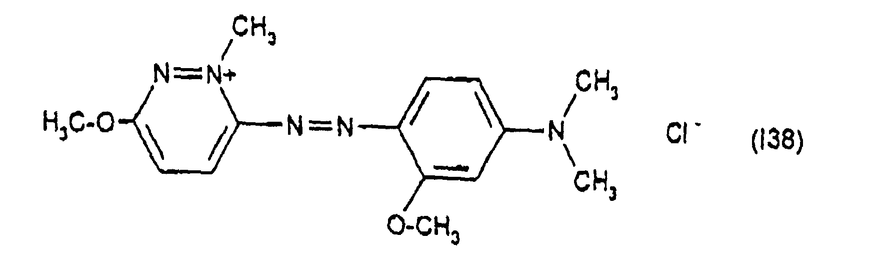

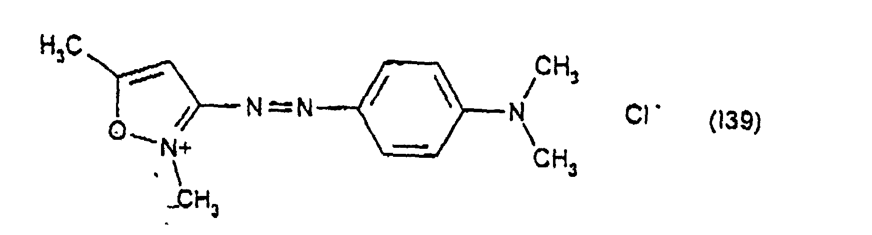

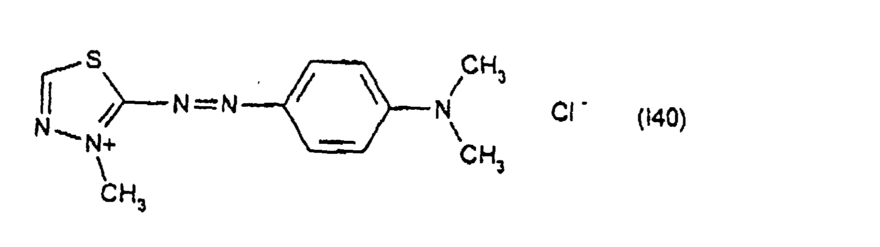

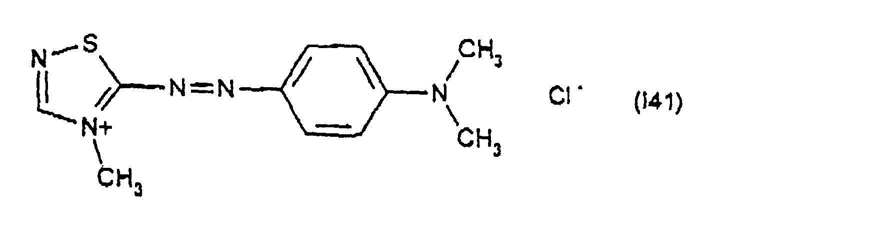

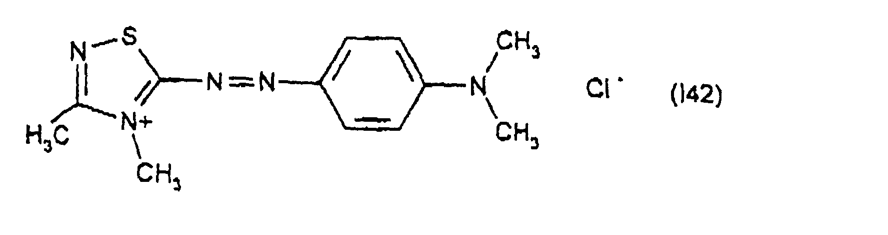

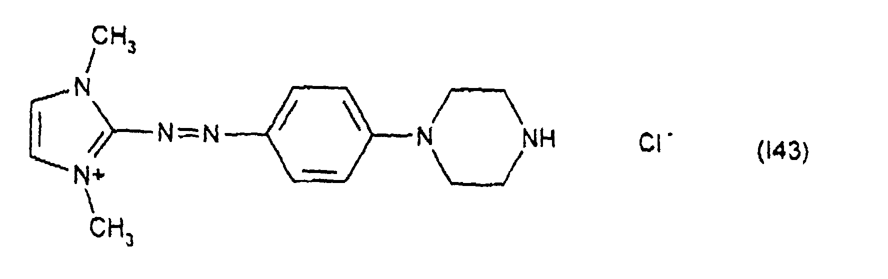

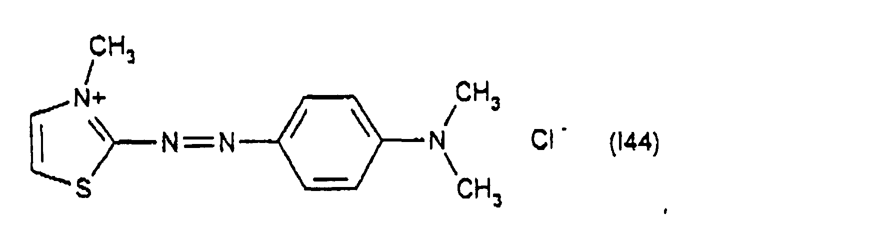

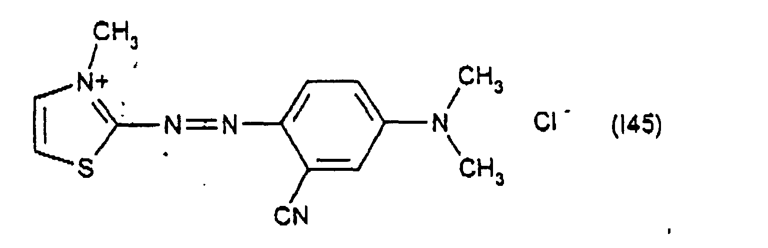

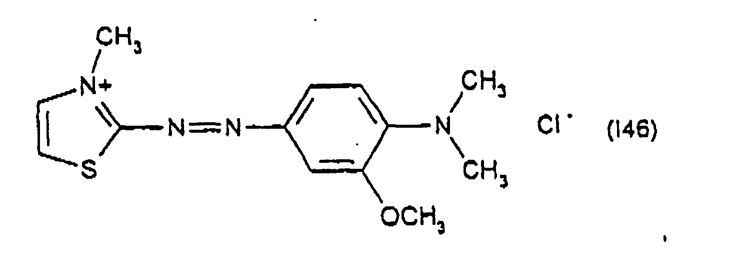

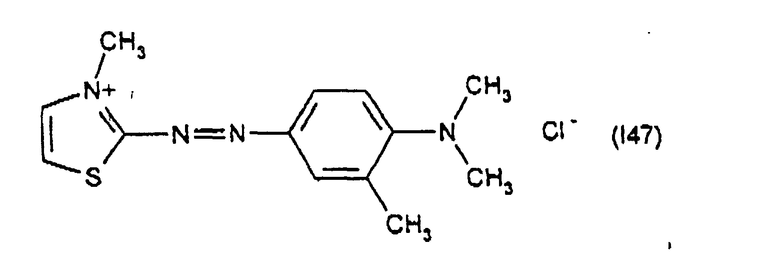

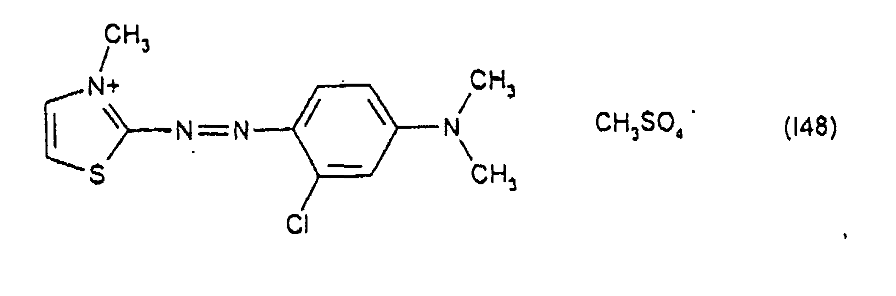

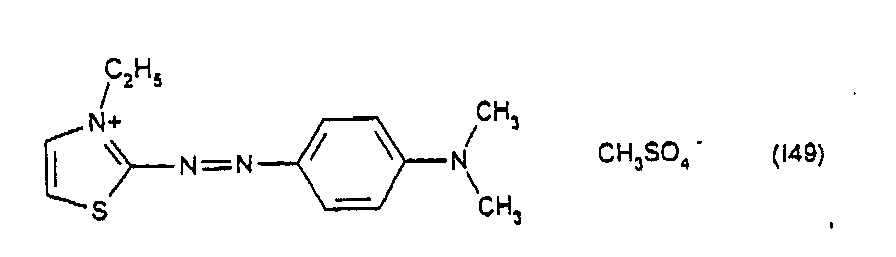

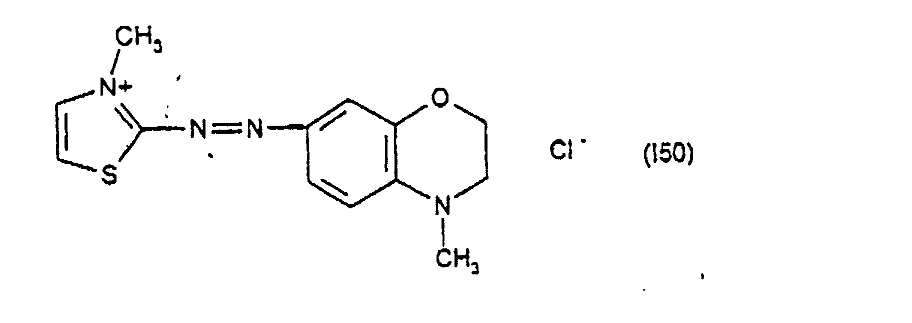

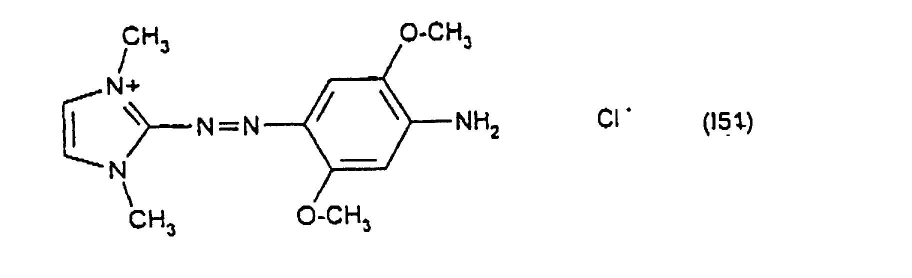

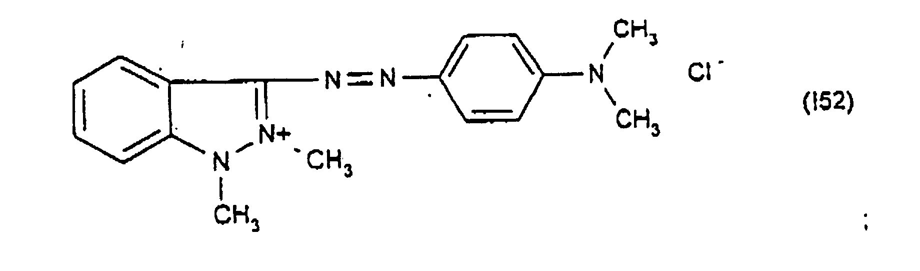

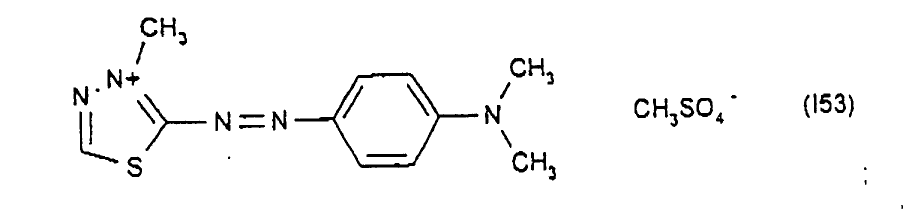

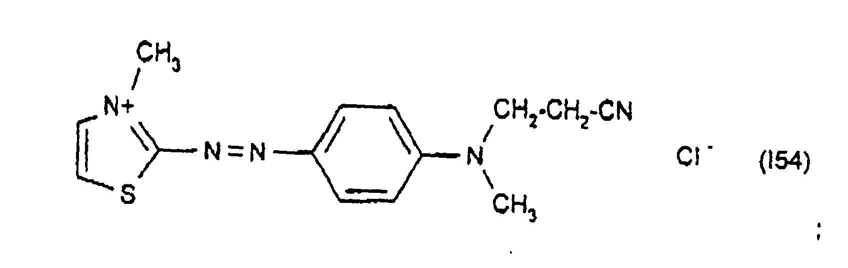

3. Zusammensetzung nach Anspruch 2, **dadurch gekennzeichnet, dass** die kationischen Direktfarbstoffe den Strukturen (I1), (I2), (I14) und (I31)-entsprechen.

4. Zusammensetzung nach Anspruch 1, **dadurch gekennzeichnet, dass** die kationischen Direktfarbstoffe der Formel (II) unter den Verbindungen der folgenden Strukturen (II1) bis (II19) ausgewählt sind:

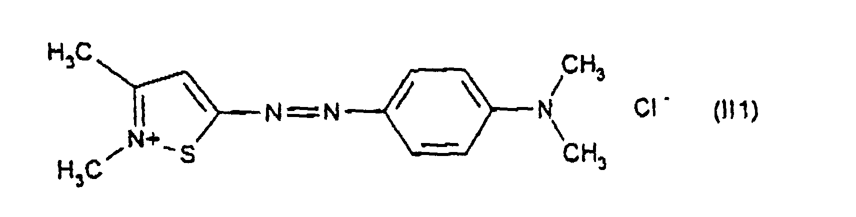

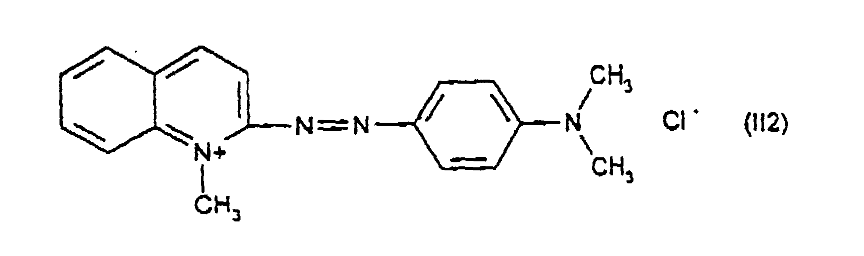

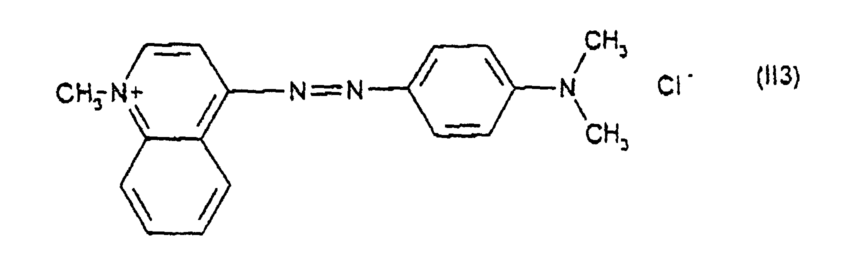

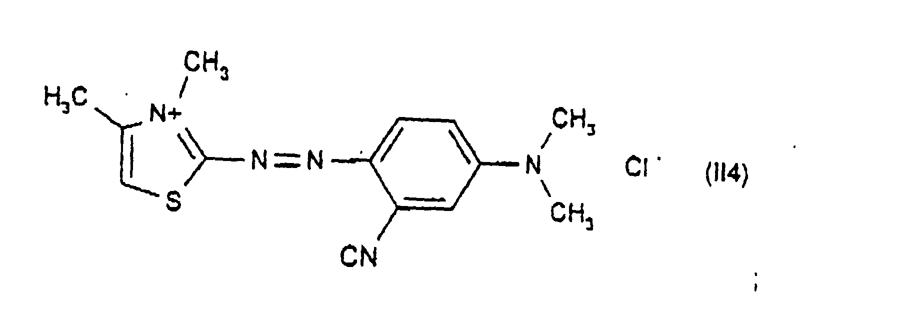

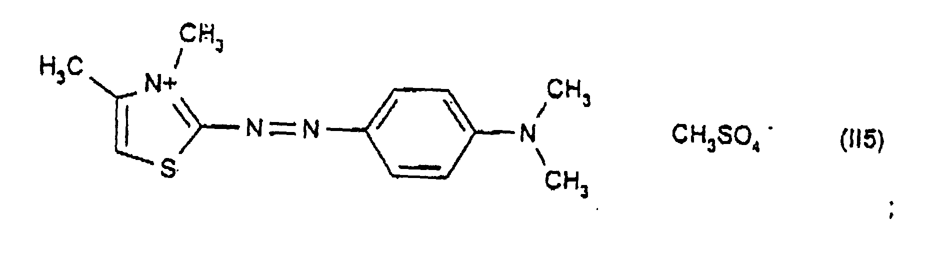

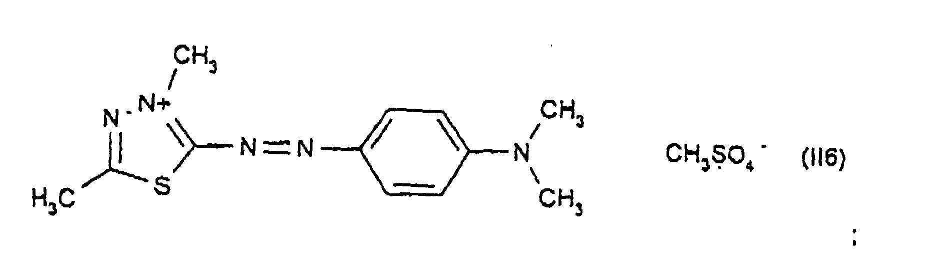

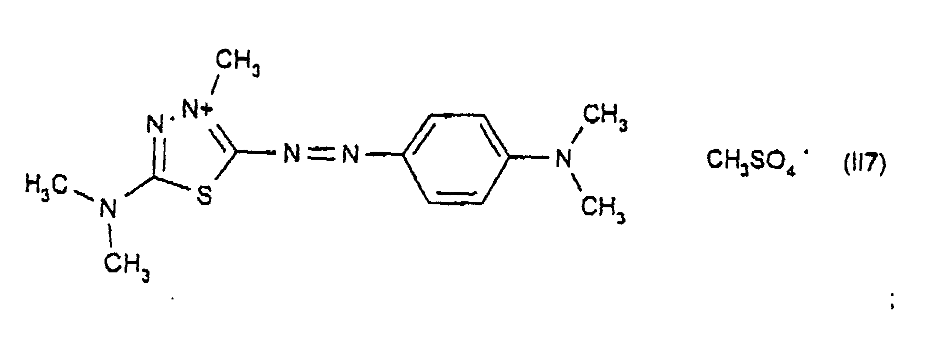

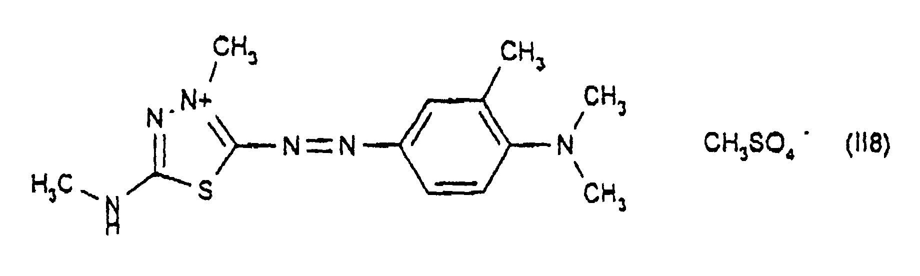

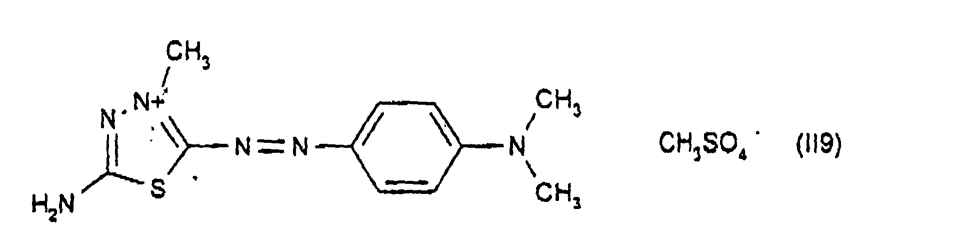

5. Zusammensetzung nach Anspruch 1, **dadurch gekennzeichnet, dass** die kationischen Direktfarbstoffe der Formel (III) unter den Verbindungen der folgenden Strukturen (III1) bis (III18) ausgewählt sind:

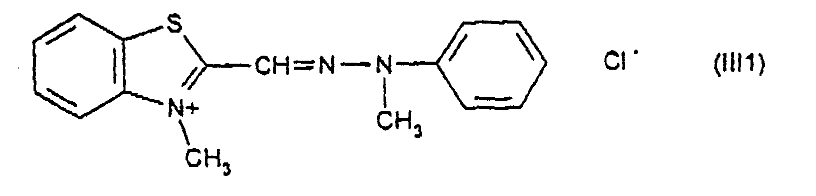

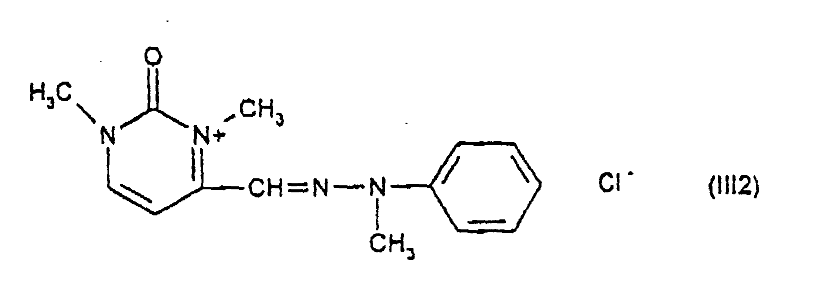

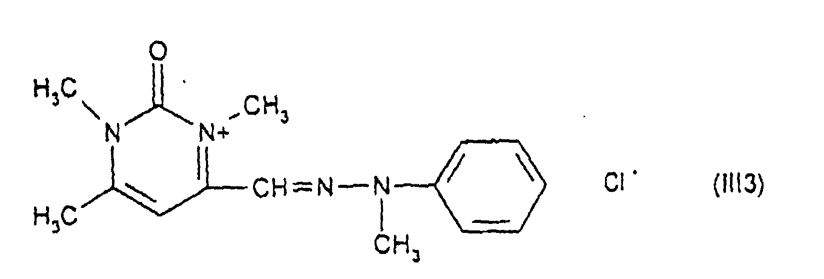

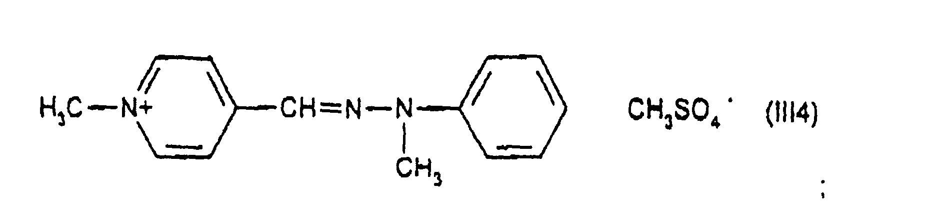

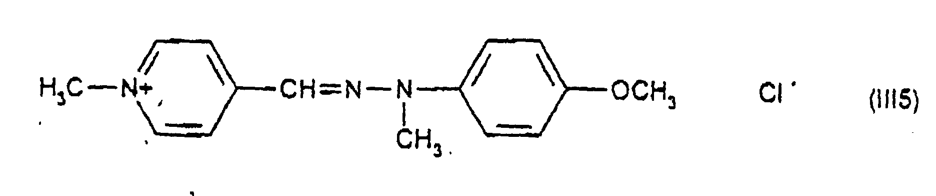

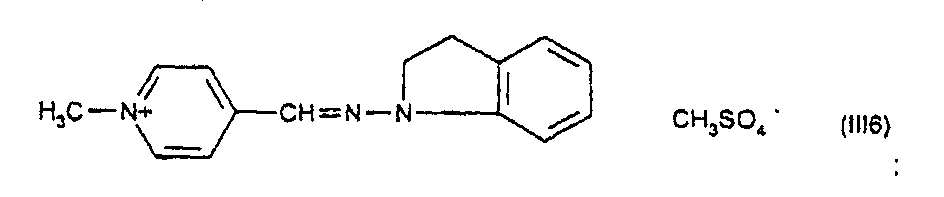

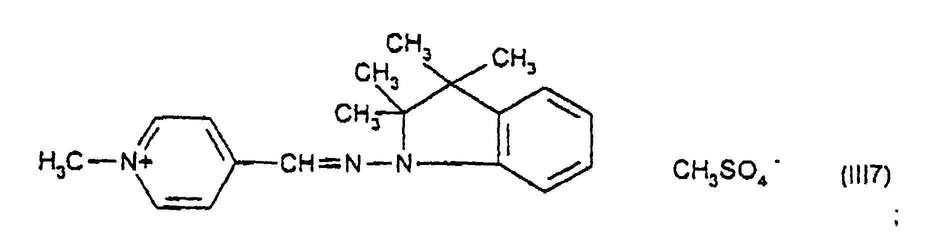

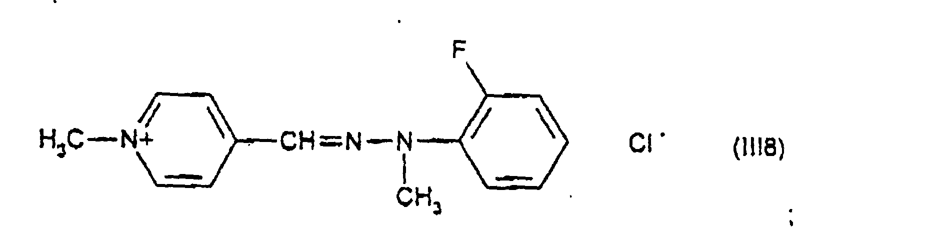

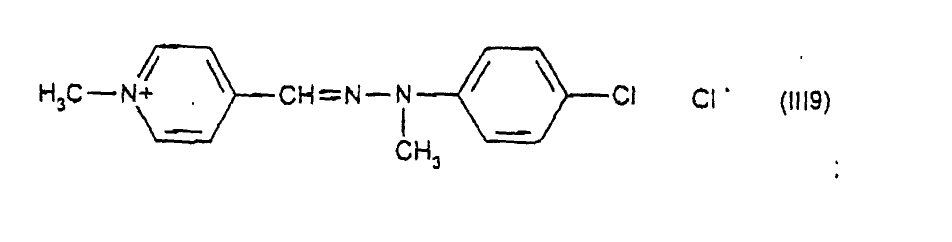

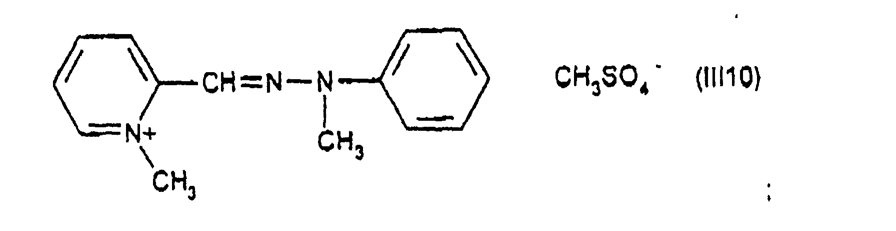

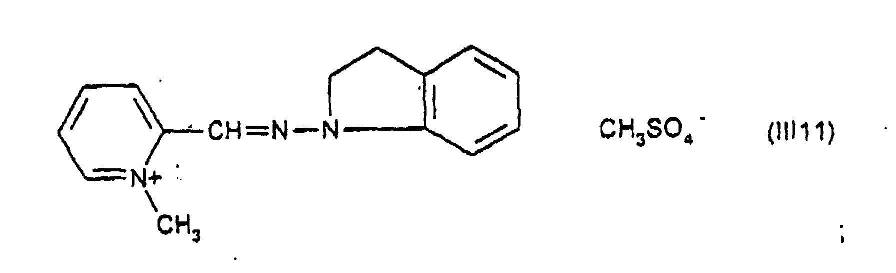

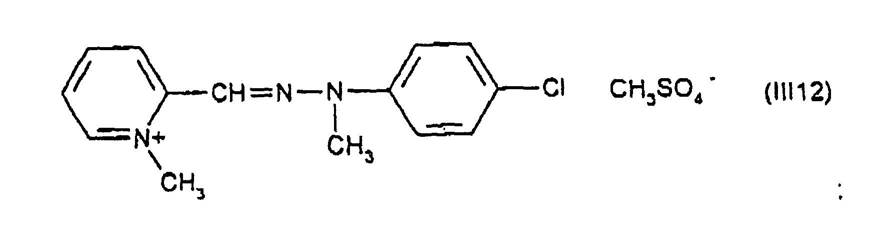

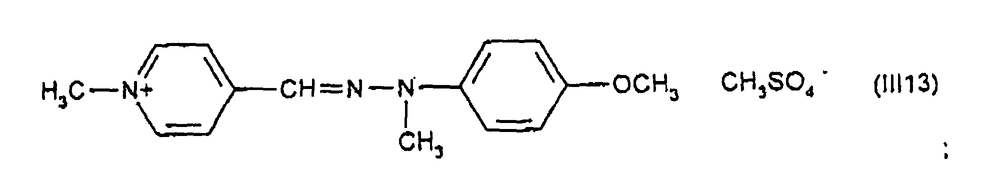

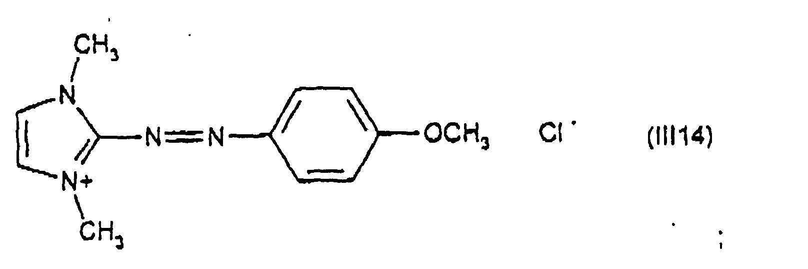

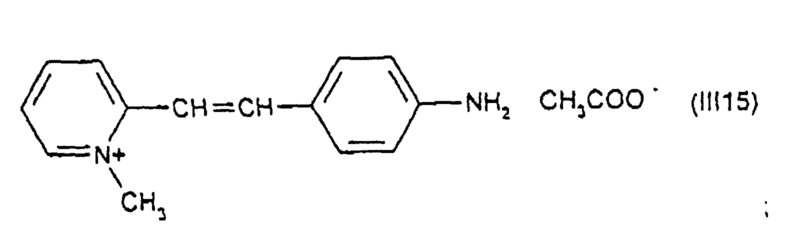

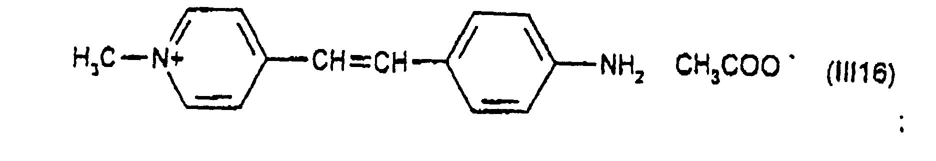

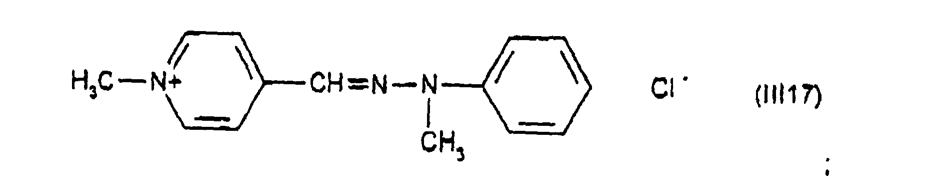

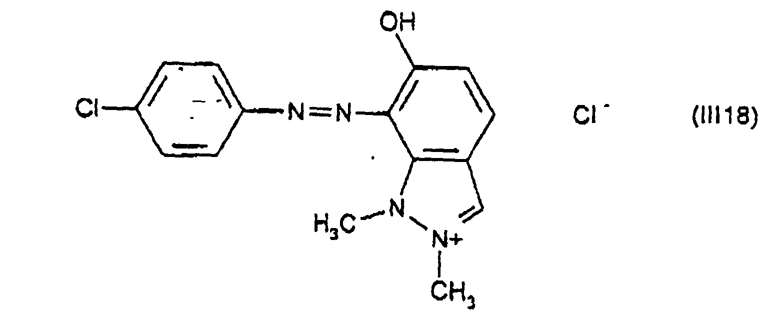

6. Zusammensetzung nach Anspruch 5, **dadurch gekennzeichnet, dass** die kationischen Direktfarbstoffe der Formel (III) den Strukturen (III4) , (III5) und (III13) entsprechen.

7. Zusammensetzung nach Anspruch 1, **dadurch gekennzeichnet, dass** die kationischen Direktfarbstoffe der Formel (III') unter den Verbindungen der folgenden Strukturen (III'1) bis (III'3) ausgewählt sind:

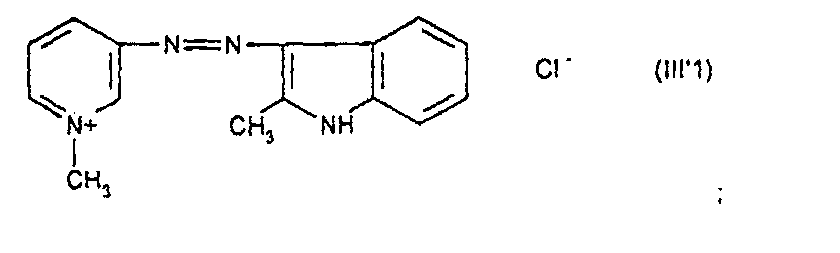

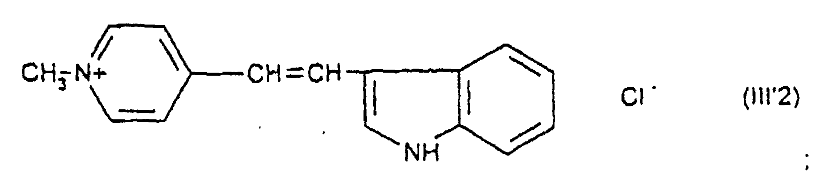

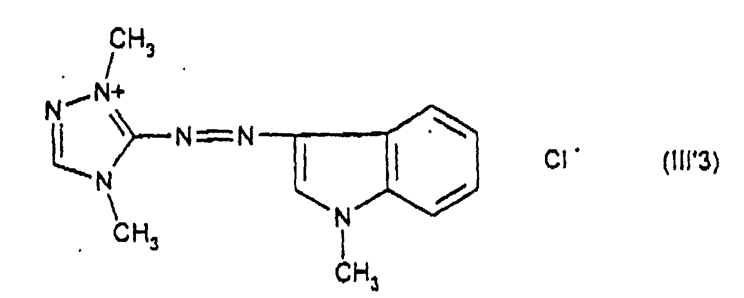

8. Zusammensetzung nach Anspruch 1, **dadurch gekennzeichnet, dass** die kationischen Direktfarbstoffe der Formel (IV) unter den Verbindungen der folgenden Formeln (IV)₁ bis (IV)₇₇ ausgewählt sind:

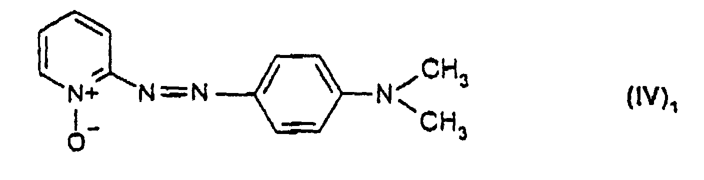

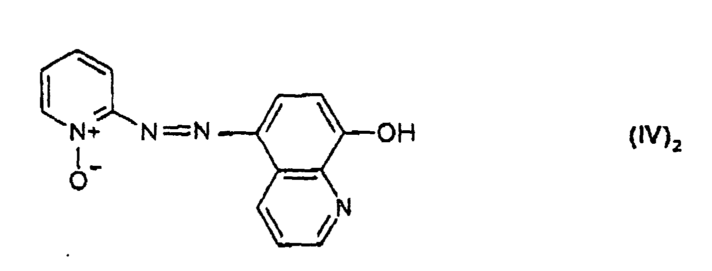

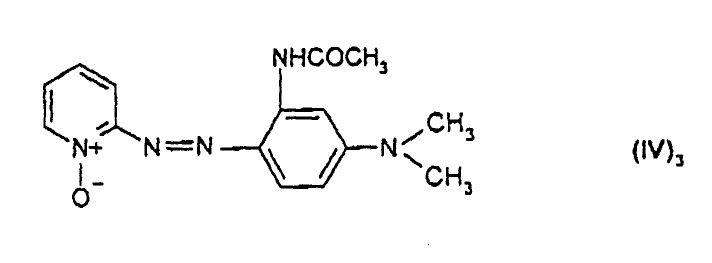

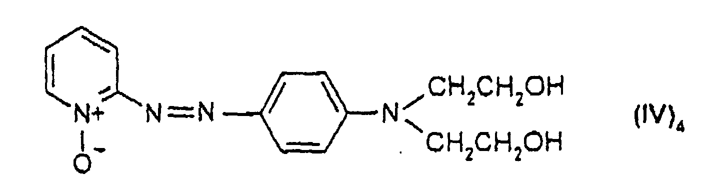

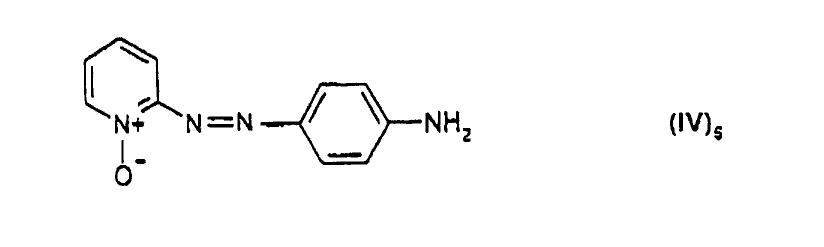

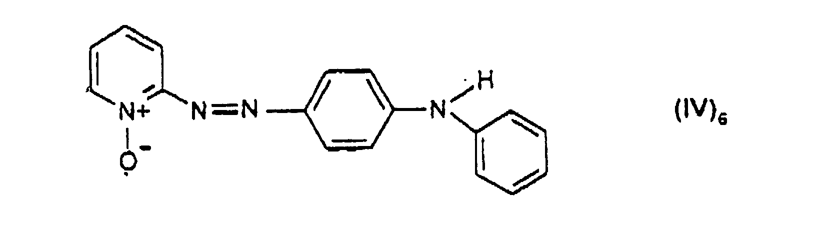

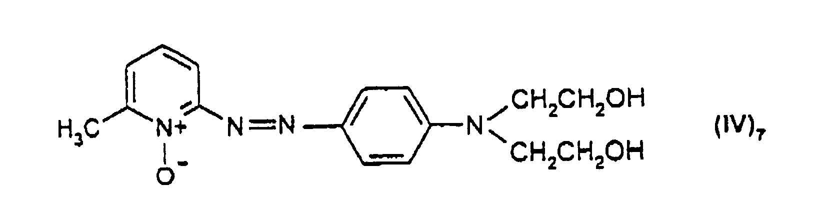

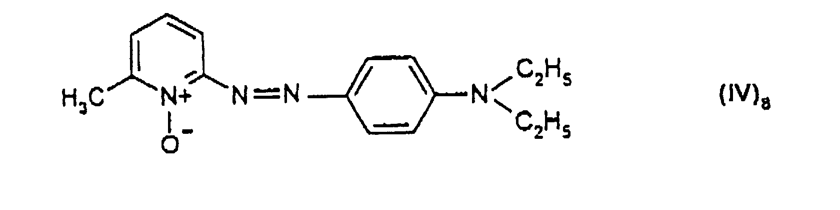

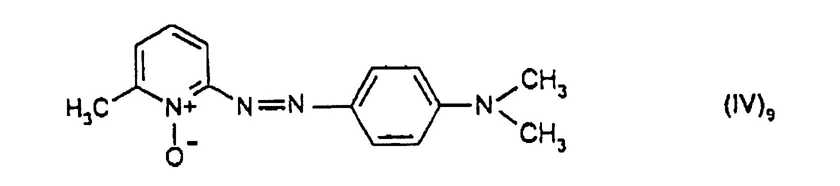

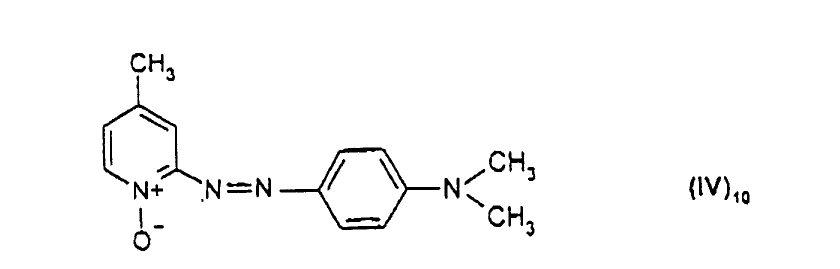

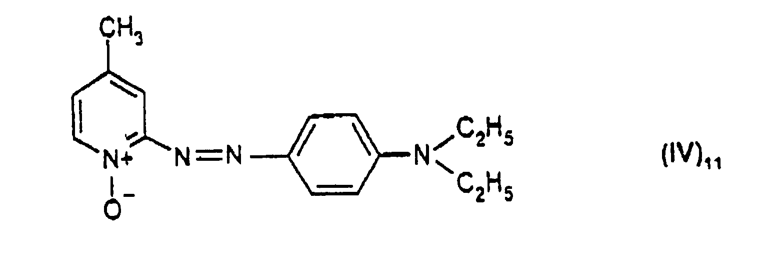

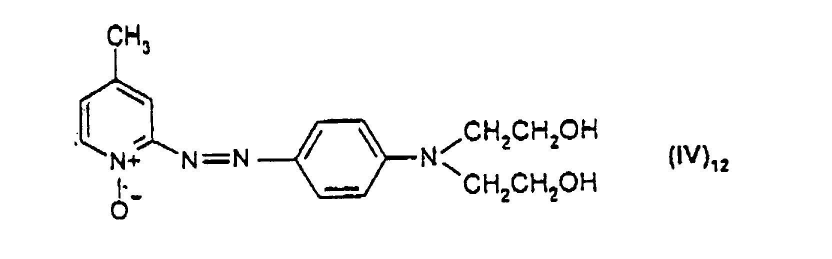

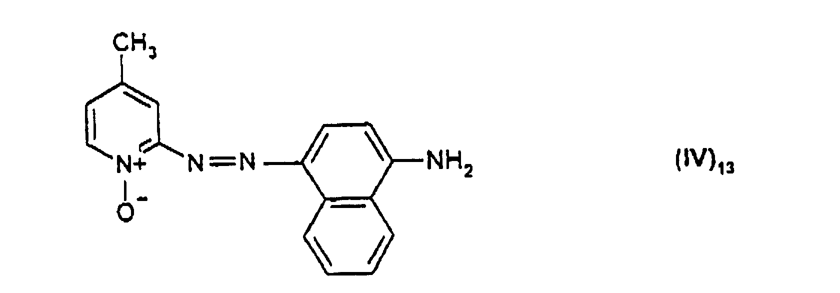

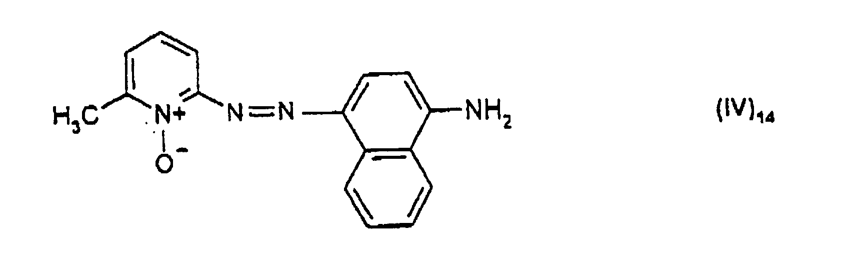

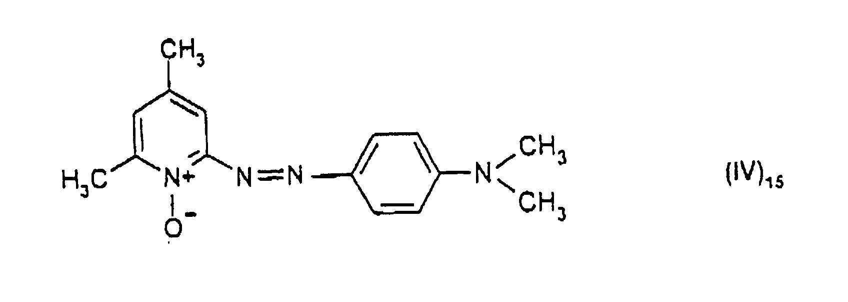

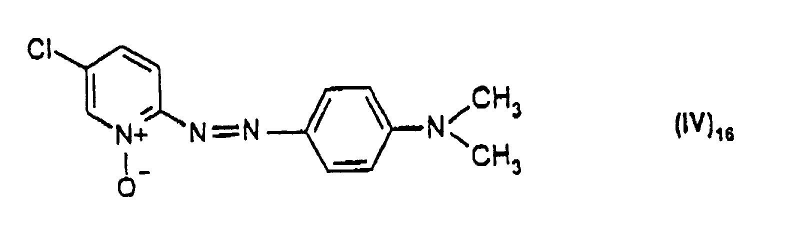

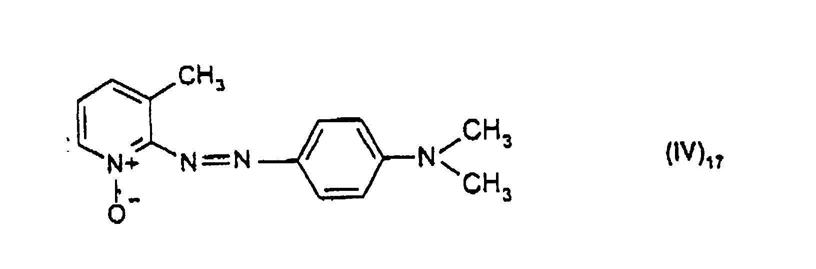

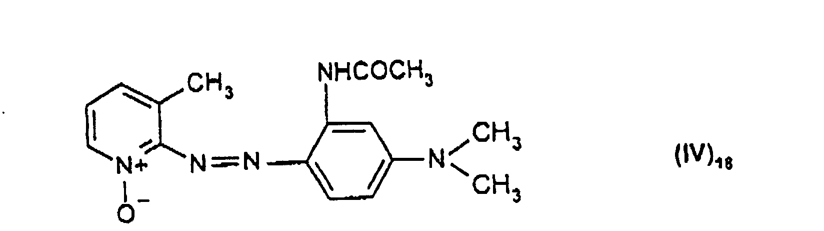

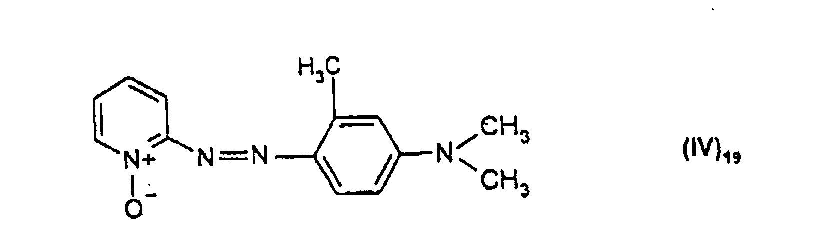

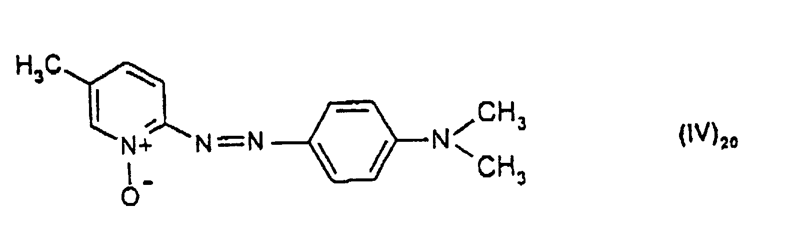

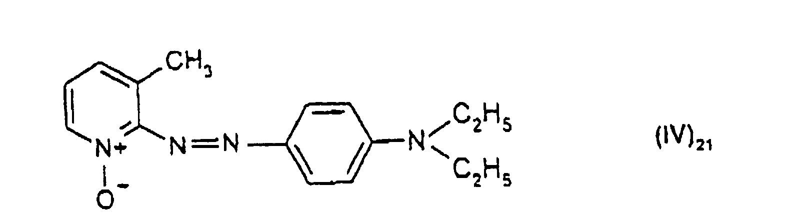

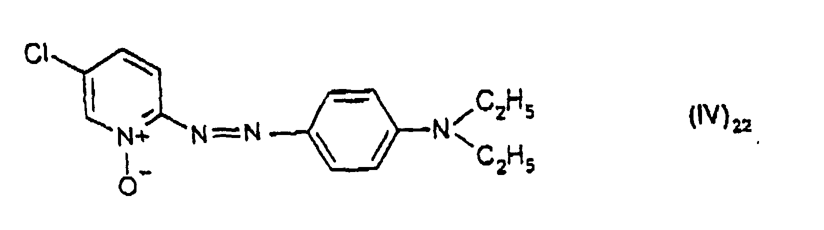

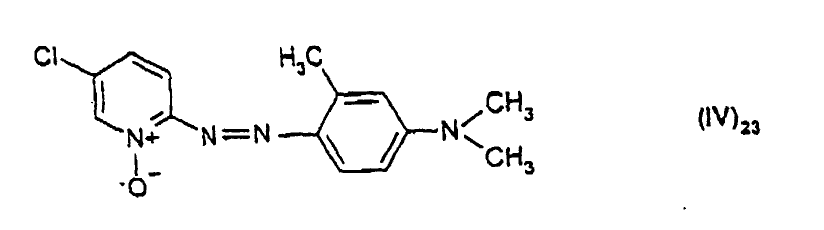

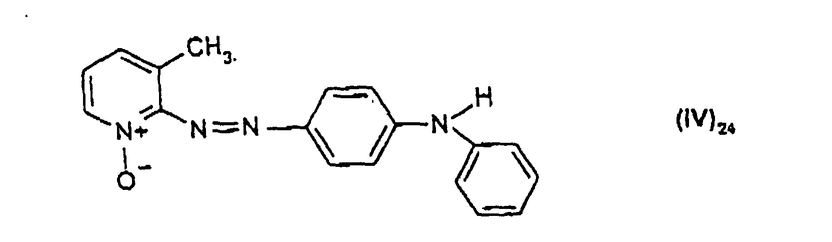

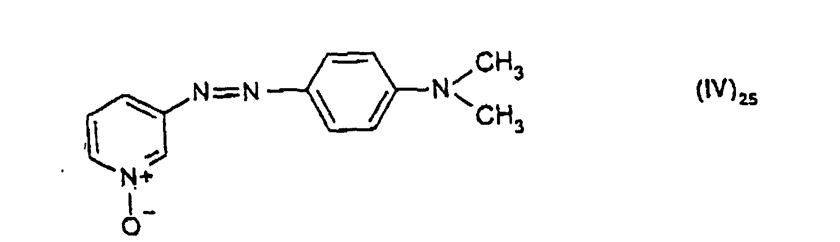

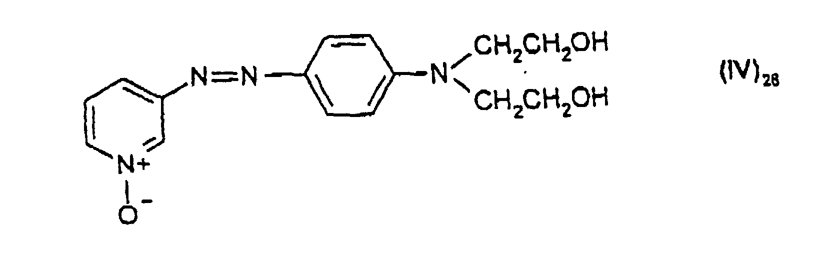

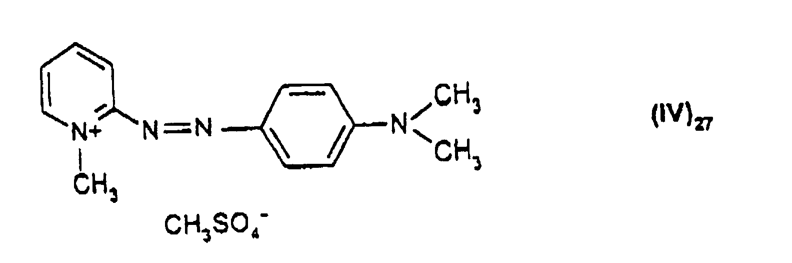

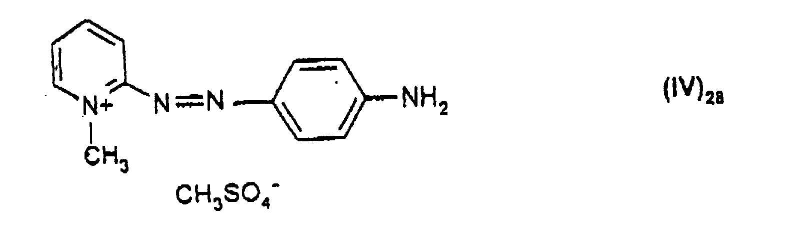

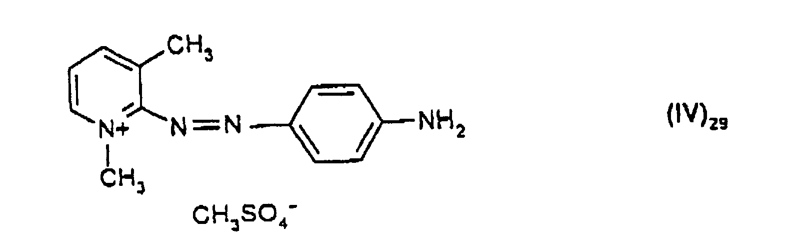

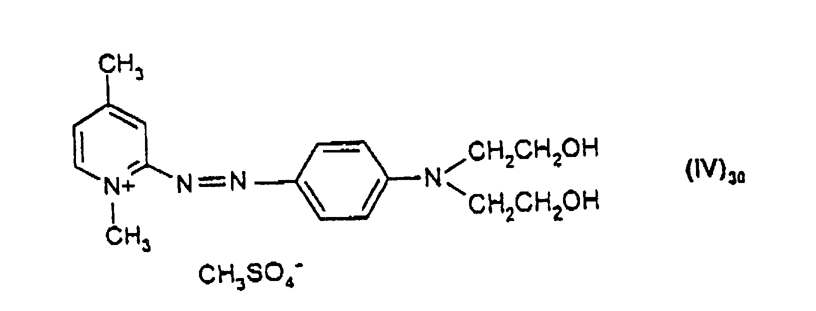

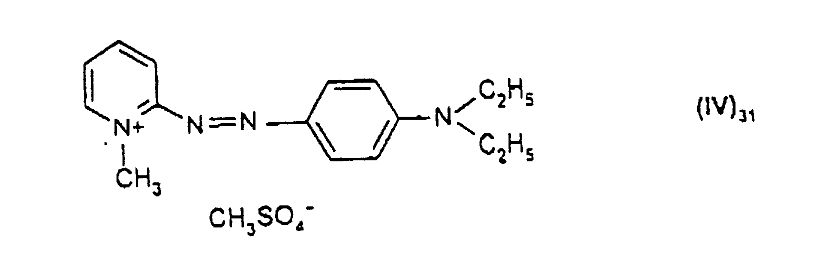

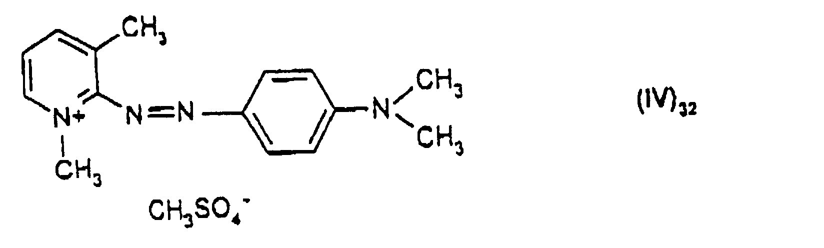

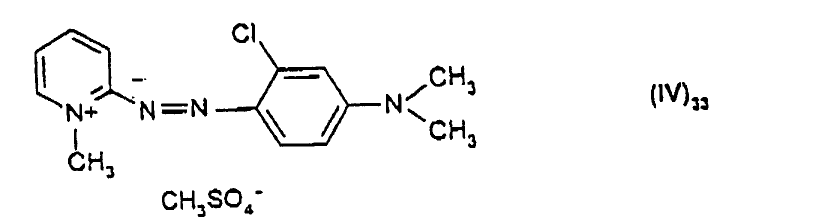

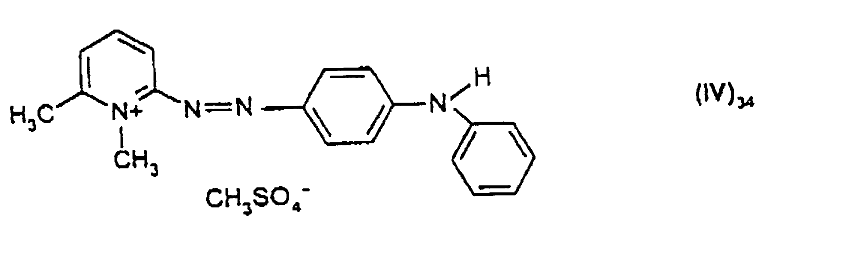

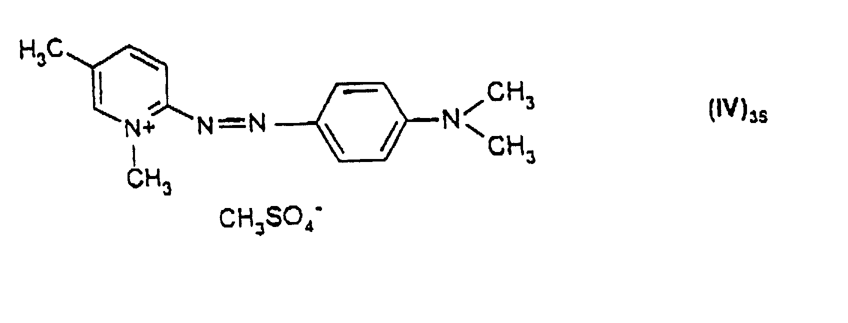

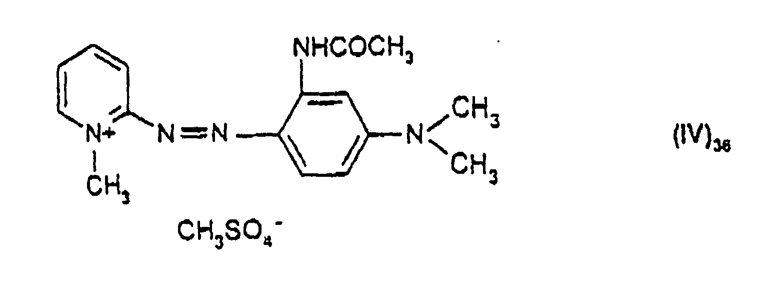

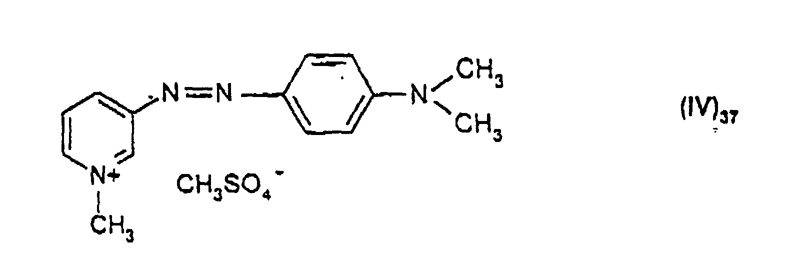

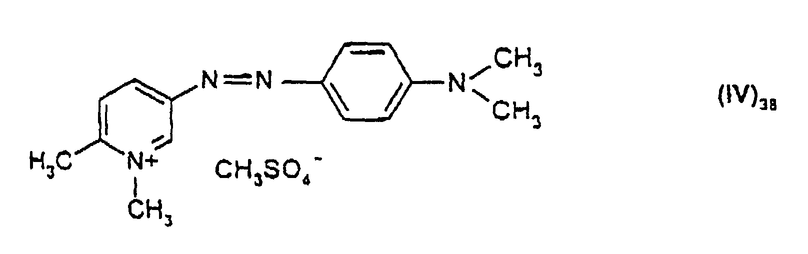

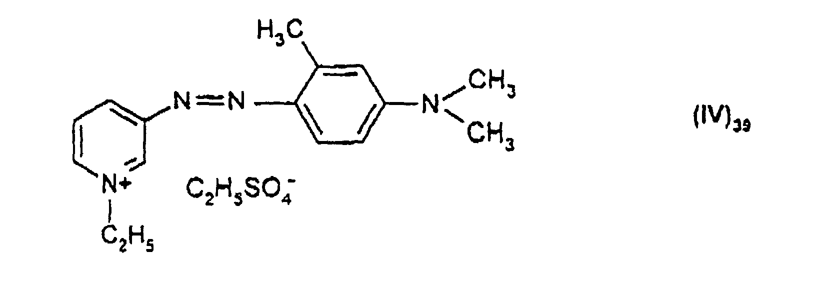

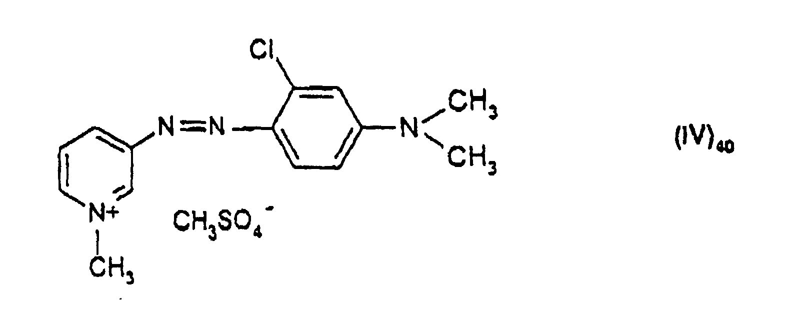

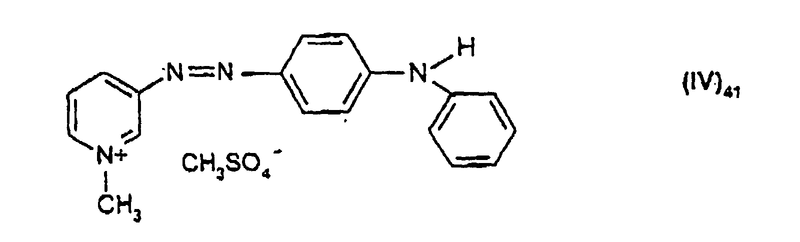

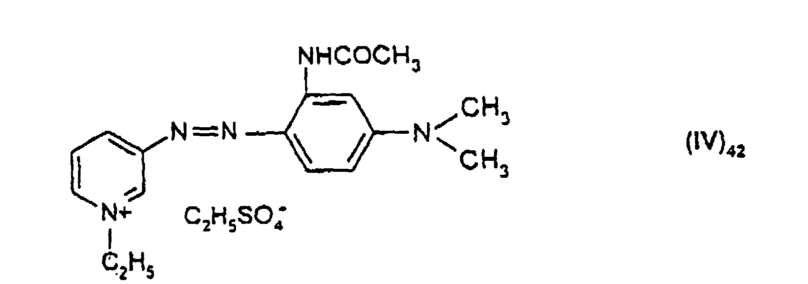

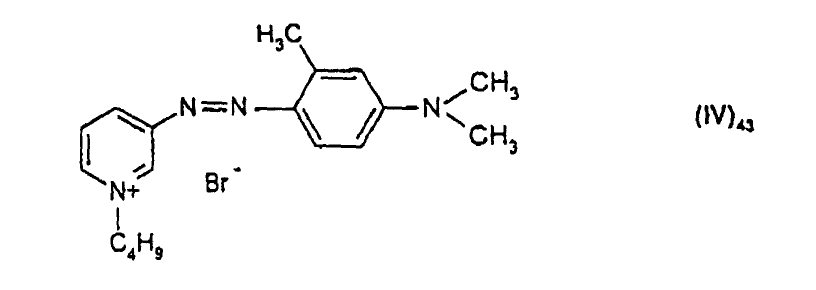

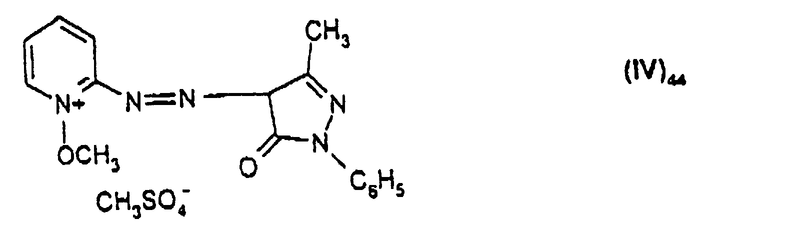

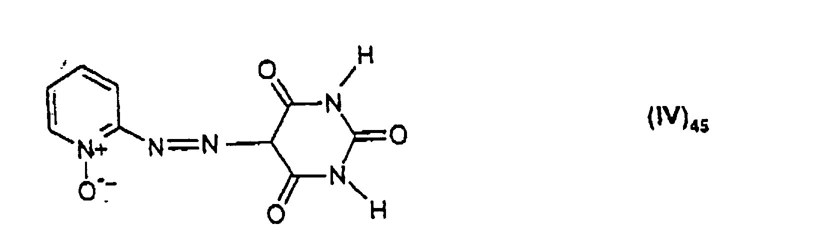

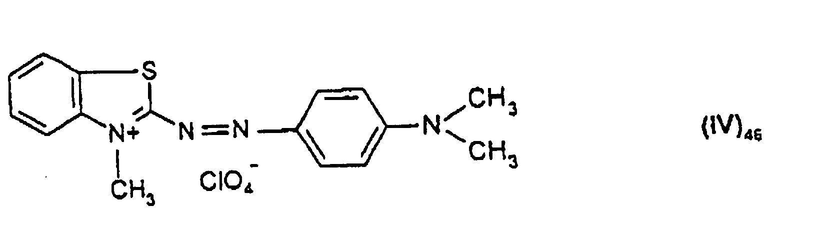

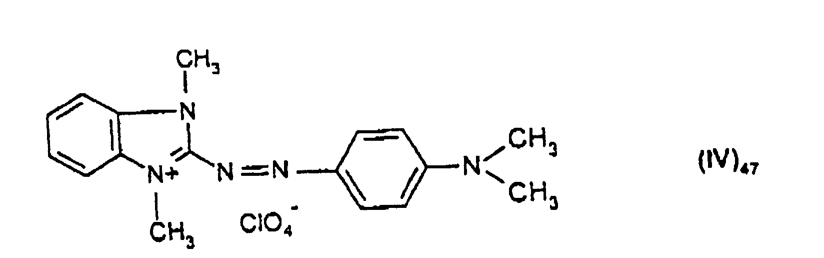

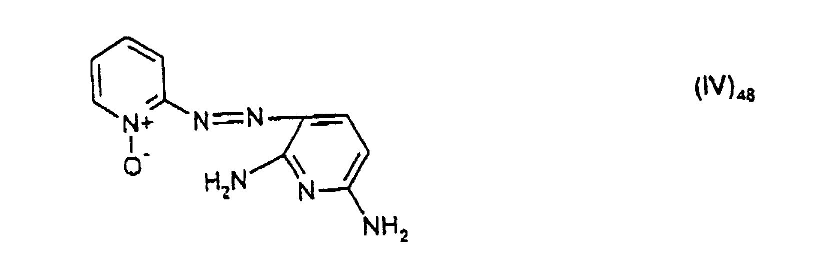

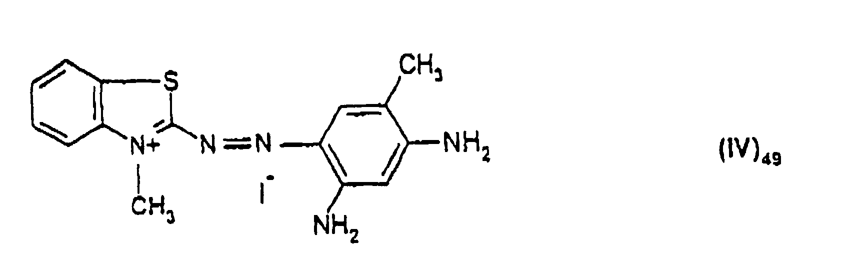

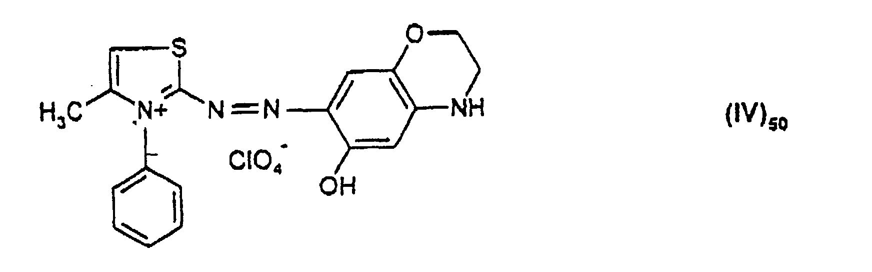

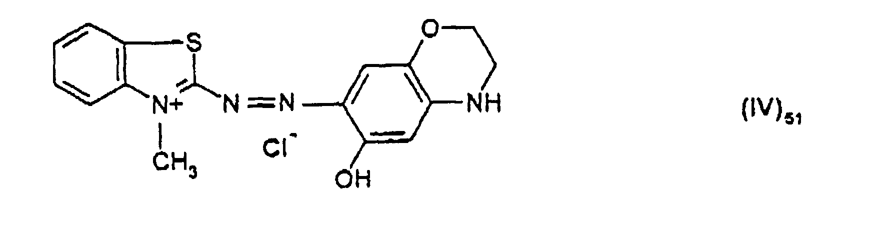

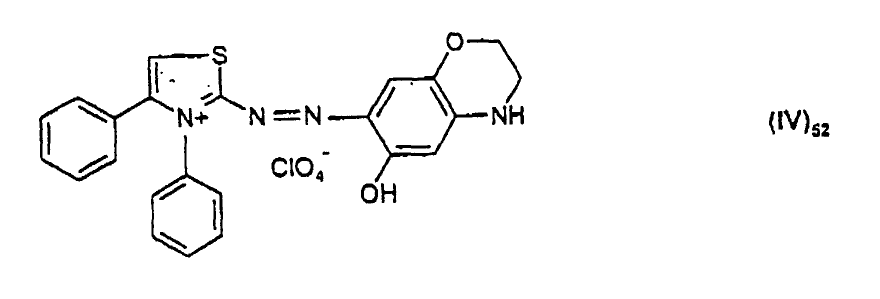

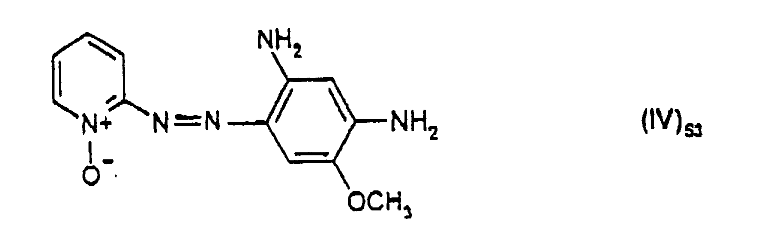

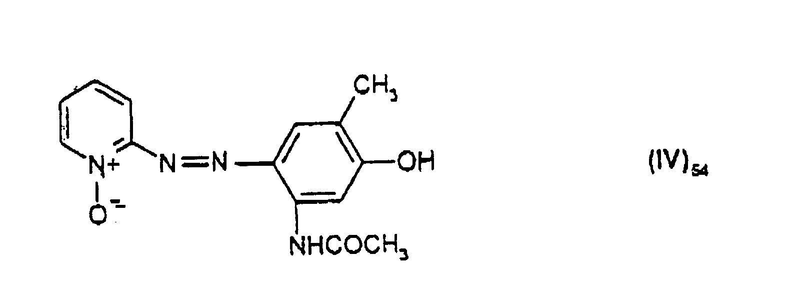

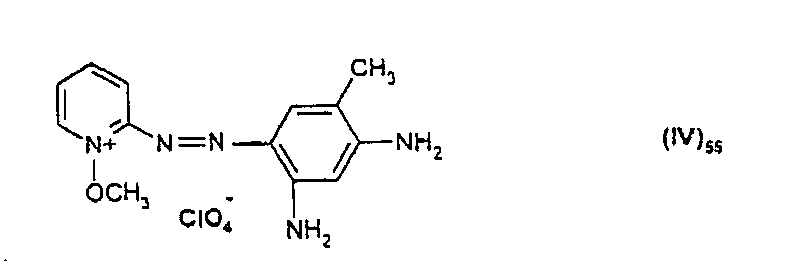

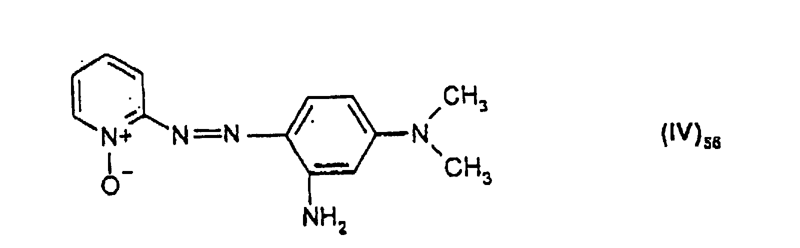

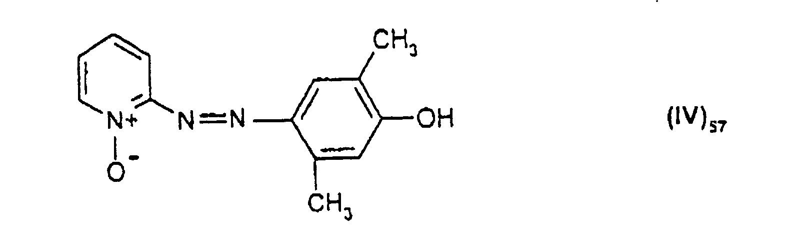

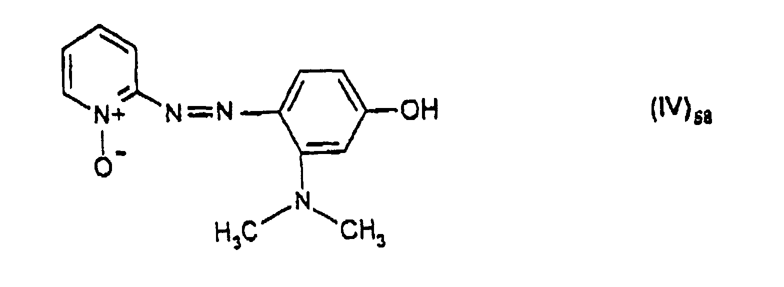

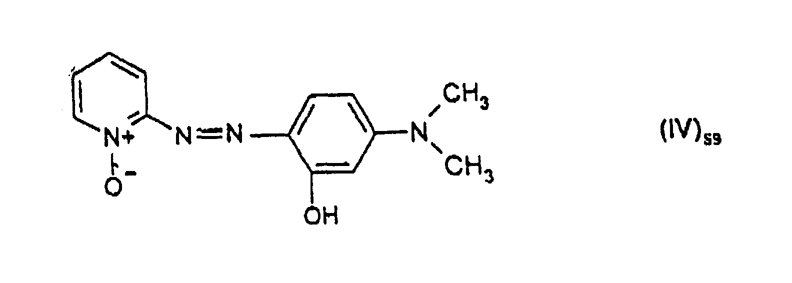

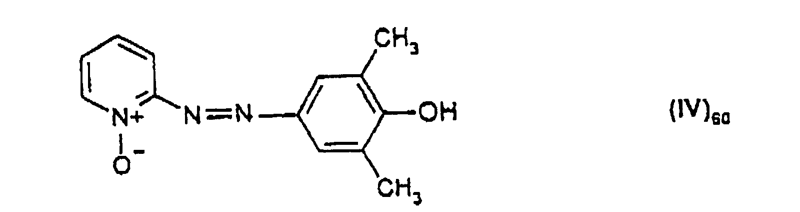

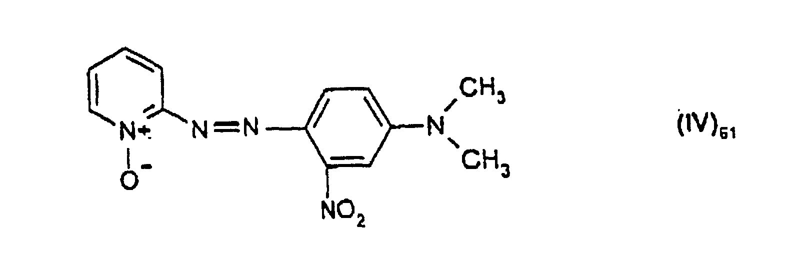

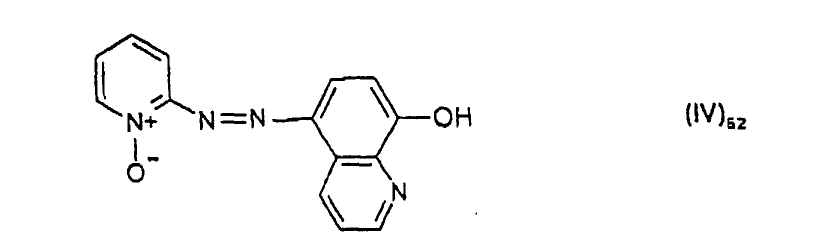

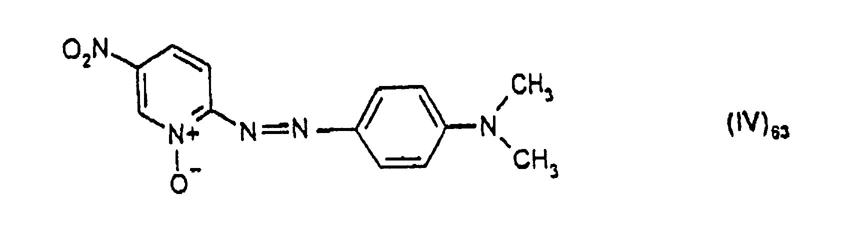

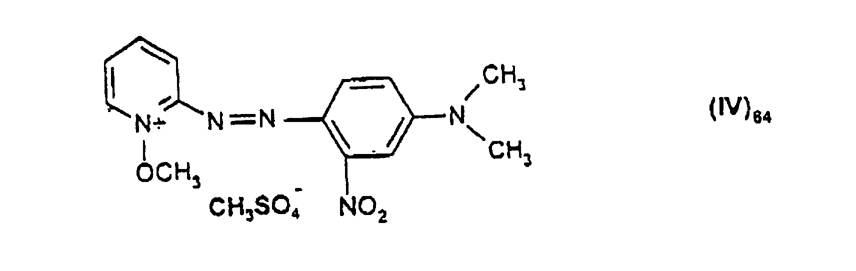

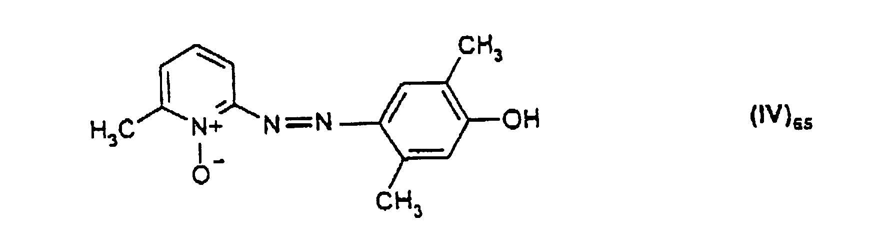

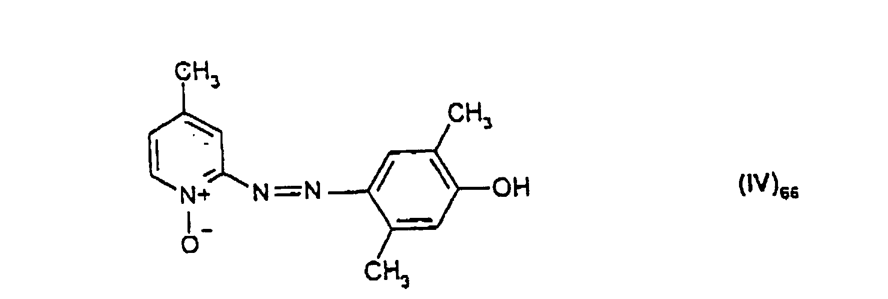

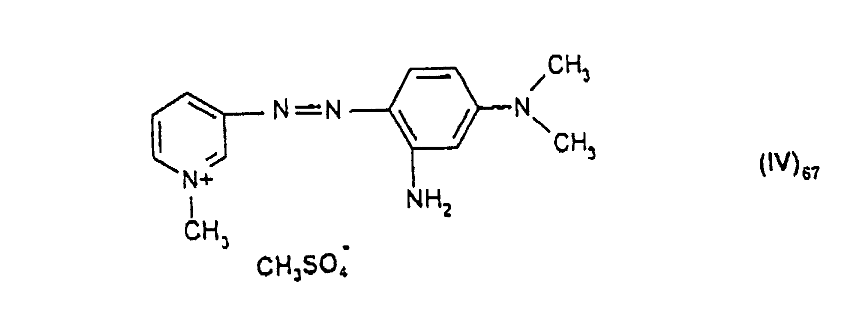

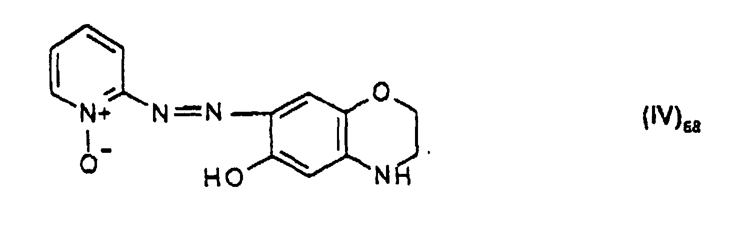

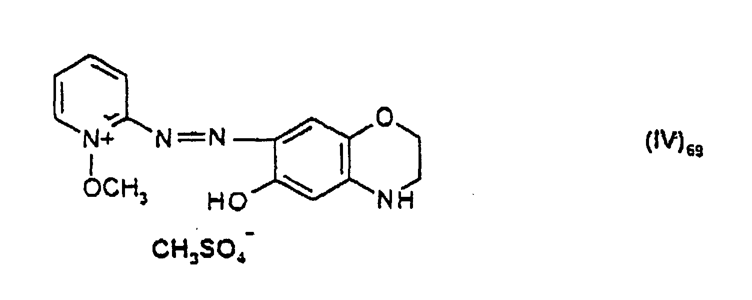

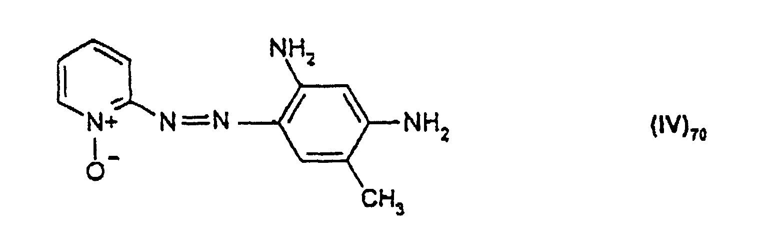

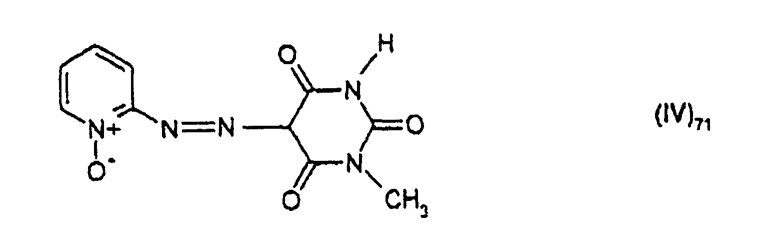

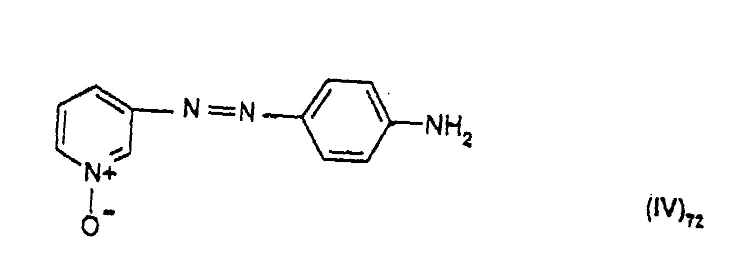

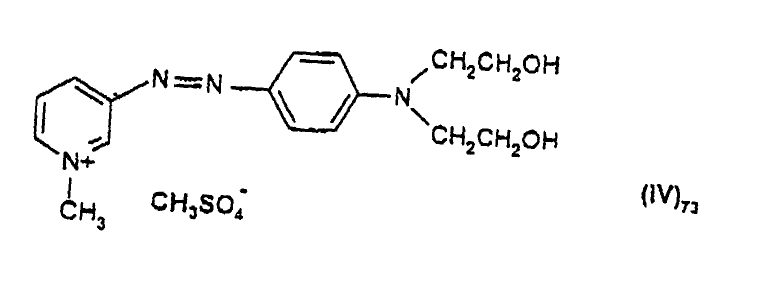

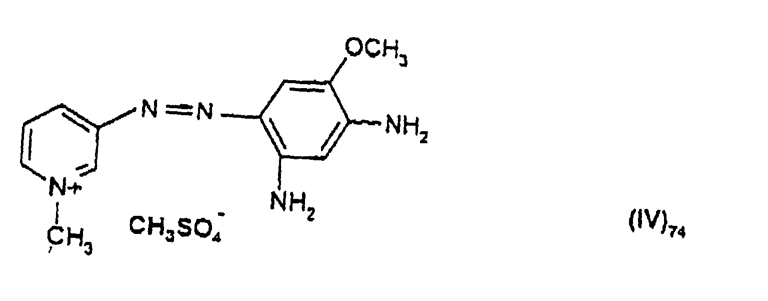

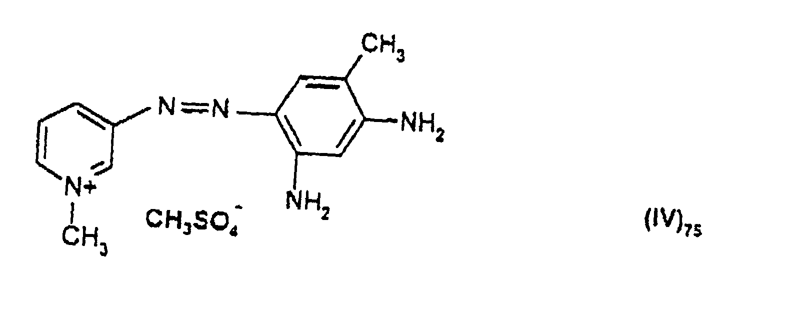

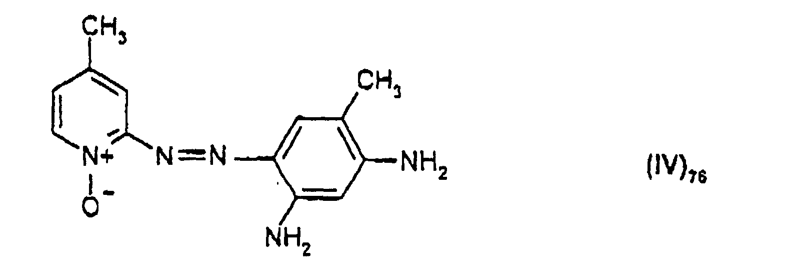

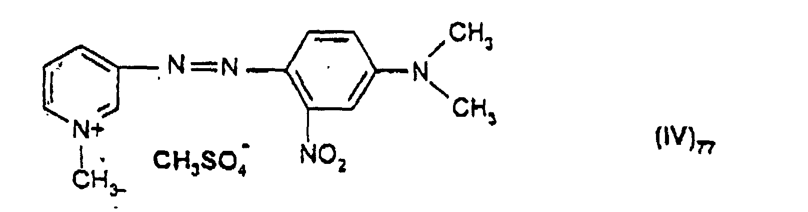

9. Zusammensetzung nach einem der vorhergehenden Ansprüche, **dadurch gekennzeichnet, dass** der oder die kationische(n) Direktfarbstoff(e) der Formeln (I), (II), (III), (III') oder (IV) 0,001 bis 10 Gew.-% des Gesamtgewichts der Zusammensetzung ausmachen.

10. Zusammensetzung nach Anspruch 9, **dadurch gekennzeichnet, dass** der oder die kationische(n) Direktfarbstoff(e) der Formeln (I), (II), (III), (III') oder (IV) 0,005 bis 5 Gew.-% des Gesamtgewichts der Zusammensetzung ausmachen.

11. Zusammensetzung nach einem der vorhergehenden Ansprüche, **dadurch gekennzeichnet, dass** der nichtionische grenzflächenaktive Stoff (ii) vom Typ Alkylpolyglycosid eine Verbindung der folgenden allgemeinen Formel (V) ist: 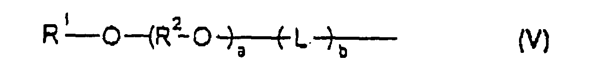 wobei in der Formel die Gruppe R₁ eine geradkettige oder verzweigte Alkyl und/oder Alkenylgruppe mit etwa 8 bis 24 Kohlenstoffatomen oder eine Alkylphenylgruppe, deren geradkettige oder verzweigte Alkylgruppe etwa 8 bis 24 Kohlenstoffatome aufweist, bedeutet, die Gruppe R₂ eine Alkylengruppe mit 2 bis 4 Kohlenstoffatomen ist, die Gruppe L einen reduzierten Zucker mit 5 bis 6 Kohlenstoffatomen bedeutet und a einen Wert im Bereich von 0 bis 10 und b einen Wert im Bereich von 1 bis 15 bezeichnet.

12. Zusammensetzung nach Anspruch 11, **dadurch gekennzeichnet, dass** in der Formel (V) die Gruppe R₁ eine geradkettige oder verzweigte Alkyl oder Alkenylgruppe mit 9 bis 14 Kohlenstoffatomen ist, a den Wert Null hat, L Glucose bedeutet und b einen Wert von 1 bis 4 bedeutet.

13. Zusammensetzung nach einem der vorhergehenden Ansprüche, **dadurch gekennzeichnet, dass** der nichtionische grenzflächenaktive Stoff (ii) vom Typ der Zuckerfettsäureester oder Alkylzuckerfettsäureester (ii)₂ ein Zucker-C₄₋₂₂-fettsäureester oder ein Alkyl(C₁₋₄)zucker-C₄₋₂₂-fettsäureester ist.

14. Zusammensetzung nach Anspruch 13, **dadurch gekennzeichnet, dass** es sich um Glucose-C₄₋₂₂-fettsäuremonoester oder Saccharose-C₄₋₂₂-fettsäuremonoester handelt.

15. Zusammensetzung nach Anspruch 13, **dadurch gekennzeichnet, dass** es sich um einen Methylglucosid-C₄₋₂₂-fettsäureester, Ethylglucosid-C₄-₂₂-fettsäureester oder Butylglucosid-C₄₋₂₂-fettsäureester handelt.

16. Zusammensetzung nach Anspruch 15, **dadurch gekennzeichnet, dass** es sich um einen Butylglucosid-C₁₂-₁₈-fettsäureester oder Methylglucosid-monostearat handelt.

17. Zusammensetzung nach einem der vorhergehenden Ansprüche, **dadurch gekennzeichnet, dass** der nichtionische grenzflächenaktive Stoff vom Typ der nichtionischen Zuckeramide mit Fettkette (ii)₃ ausgewählt ist unter: (ii)₃ (a) den N-substituierten Aldonamiden und (ii)₃ (b) den mehrfach hydroxylierten Fettsäureamiden oder deren Gemischen.

18. Zusammensetzung nach Anspruch 17, **dadurch gekennzeichnet, dass** die N-substituierten Aldonamide (ii)₃ (a) unter den folgenden Verbindungen ausgewählt sind:
(i) N-substituierten Lactobionamiden, N-substituierten Maltobionamiden, N-substituierten Cellobionamiden, N-substituierten Mellibionamiden oder N-substituierten Gentiobionamiden;
(ii) N-Lactobionylaminosäureestern, N-Maltobionylaminosäureestern, N-Mellibionylaminosäureestern, N-Cellobionylaminosäureestern und N-Gentiobionylaminosäureestern;
(iii) N-(Alkyloxy)alkyl-lactobionamiden;
(iv) N-(Polyalkyloxy)alkyllactobionamiden, N-(Polyalkyloxy)alkylmaltobionamiden, N-(Polyalkyloxy)alkylcellobionamiden, N-(Polyalkyloxy)alkylmellibionamiden oder N-(Polyalkyloxy)alkylgentiobionamiden.

19. Zusammensetzung nach Anspruch 17, **dadurch gekennzeichnet, dass** die mehrfach hydroxylierten Fettamide (ii)₃ (b) der folgenden Formel (VI) entsprechen: 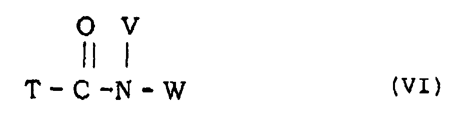 wobei
T eine C₅₋₃₁-Kohlenwasserstoffgruppe und vorzugsweise eine geradkettige Alkyl- oder Alkenylgruppe mit 7 bis 15 Kohlenstoffatomen oder deren Gemische bedeutet;
V Wasserstoff, eine C₁₋₄-Kohlenwasserstoffgruppe, 2-Hydroxyethyl, 2-Hydroxypropyl oder deren Gemische bedeutet, wobei V vorzugsweise eine C₁₋₄-Alkylgruppe und insbesondere Methyl bedeutet;
W eine Polyhydroxykohlenwasserstoffgruppe, die eine gerade Kohlenwasserstoffkette mit mindestens 3 Hydroxygruppen besitzt, die direkt an die Kette gebunden sind, oder ein alkoxyliertes Derivat dieser Gruppe bedeutet.

20. Zusammensetzung nach Anspruch 19, **dadurch gekennzeichnet, dass** W ein Derivat eines reduzierenden Zuckers ist, das durch reduzierende Aminierung erhalten wird, vorzugsweise Glycityl.

21. Zusammensetzung nach Anspruch 19 oder 20, **dadurch gekennzeichnet, dass** W unter den Gruppen der folgenden Formeln ausgewählt ist:
-CH₂-(CHOH)ₙ-CH₂OH;
-CH-(CH₂4H)-(CHOH)ₙ₋₁-CH₂OH;
-CH₂-(CHOH)₂(CHOR')(CHOH)-CH₂OH, wobei n eine ganze Zahl von 3 bis 5 bedeutet und R' Wasserstoff, ein cyclisches oder aliphatisches Monosaccharid oder eines seiner alkoxylierten Derivate.

22. Zusammensetzung nach Anspruch 21, **dadurch gekennzeichnet, dass** es sich um die Gruppe -CH₂-(CHOH)₄-CH₂OH handelt.

23. Zusammensetzung nach einem der Ansprüche 19 bis 22, **dadurch gekennzeichnet, dass** in der Formel (VI) die Gruppe T-CON= unter Cocamid, Stearamid, Oleamid, Lauramid, Myristiramid, Capricamid, Palmitamid und Talgamid ausgewählt ist.

24. Zusammensetzung nach einem der vorhergehenden, **dadurch gekennzeichnet, dass** der oder die nichtionische(n) grenzflächenaktive(n) Stoff(e) (ii) 0,05 bis 30 Gew.-% des Gesamtgewichts der Farbmittelzusaxnmensetzung ausmachen.

25. Zusammensetzung nach Anspruch 24, **dadurch gekennzeichnet, dass** der oder die nichtionische(n) grenzflächenaktive(n) Stoff(e) (ii) 0,1 bis 15 Gew.-% des Gesamtgewichts der Farbmittelzusammensetzung ausmachen.

26. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

27. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

28. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

29. Zusammensetzung nach Anspruch 28, **dadurch gekennzeichnet, dass** die Oxidationsbase(n) 0,0005 bis 12 Gew.-% des Gesamtgewichts der-Zusammensetzung ausmachen.

30. Zusammensetzung nach Anspruch 29, **dadurch gekennzeichnet, dass** die Oxidationsbase(n) 0,005 bis 6 Gew.-% des Gesamtgewichts der Zusammensetzung ausmachen.

31. Zusammensetzung nach einem der Ansprüche 28 bis 30, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

32. Zusammensetzung nach Anspruch 31, **dadurch gekennzeichnet, dass** der oder die Kuppler 0,0001 bis 10 Gew.-% des Gesamtgewichts der Farbmittelzusammensetzung ausmachen.

33. Zusammensetzung nach Anspruch 32, **dadurch gekennzeichnet, dass** der oder die Kuppler 0,005 bis 5 Gew.-% des Gesamtgewichts der Farbmittelzusammensetzung ausmachen.

34. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidationsmittel enthält.

35. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 34 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

36. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 34 während einer Zeitspanne, die zur Bildung der gewünschten Färbung ausreichend ist, aufgebracht wird, ohne dass nochmals gespült wird.

37. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) den in den vorhergehenden Ansprüchen definierten nichtionischen grenzflächenaktiven Stoff (ii) enthält.

38. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) den in den vorhergehenden Ansprüchen definierten nichtionischen grenzflächenaktiven Stoff (ii) enthält.

39. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 37 oder 38 und eine andere Abteilung die Zusammensetzung (B1) oder (B2) nach Anspruch 37 oder 38 enthält.

## Claims

1. Composition for dyeing keratinous fibres and in particular human keratinous fibres such as hair, containing in an appropriate dyeing medium, (i) at least one cationic direct dye chosen from the compounds of the following formulae (I), (II), (III), (III'), (IV):
**a) the compounds of the following formula (I)** : 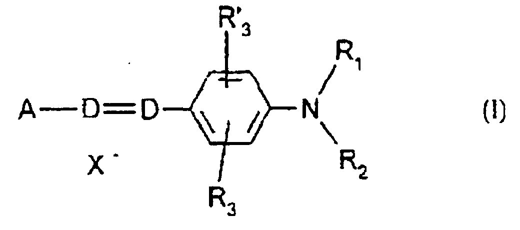 in which:
D represents a nitrogen atom or the -CH group,
R₁ and R₂, which are identical or different, represent a hydrogen atom; a C₁-C₄ alkyl radical which may be substituted with a -CN, -OH or -NH₂ radical or form with a carbon atom of the benzene ring an optionally oxygen-containing or nitrogen-containing heterocycle which may be substituted with one or more C₁-C₄ alkyl radicals; a 4'-aminophenyl radical,
R₃ and R'₃, which are identical or different, represent a hydrogen or halogen atom chosen from chlorine, bromine, iodine and fluorine, a cyano, C₁-C₄ alkyl, C₁-C₄ alkoxy or acetyloxy radical,
X⁻ represents an anion which is preferably chosen from chloride, methylsulphate and acetate,
A represents a group chosen from the following structures A₁ to A₁₈: 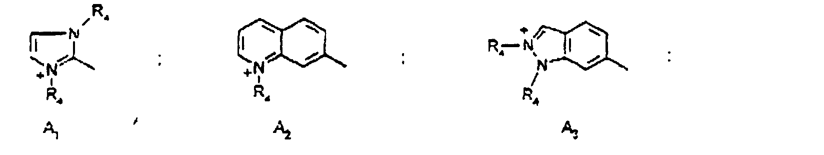 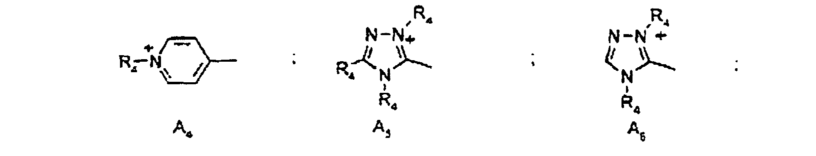 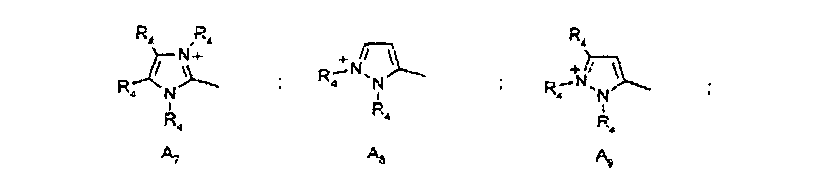 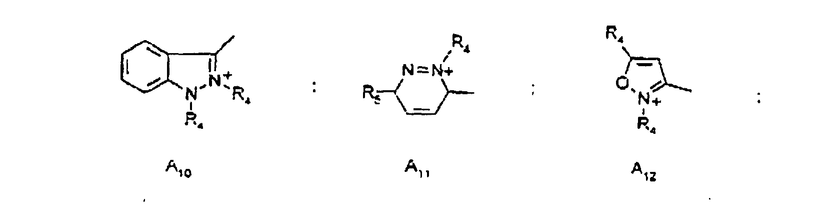 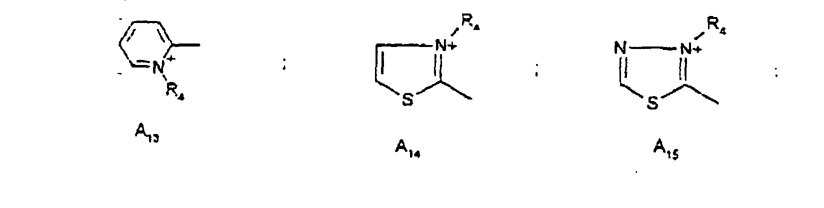 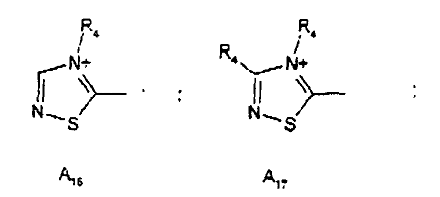 and 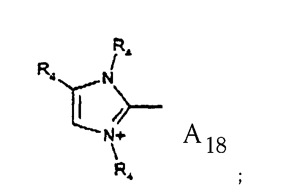 in which R₄ represents a C₁-C₄ alkyl radical which may be substituted with a hydroxyl radical and R₅ represents a C₁-C₄ alkoxy radical, with the proviso that when D represents -CH, A represents A₄ or A₁₃ and R₃ is different from an alkoxy radical, then R₁ and R₂ do not simultaneously denote a hydrogen atom;
**b) the compounds of the following formula (II):** 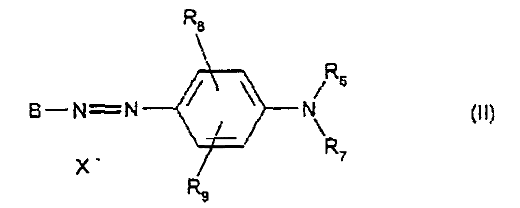 in which:
R₆ represents a hydrogen atom or a C₁-C₄ alkyl radical,
R₇ represents a hydrogen atom, an alkyl radical which may be substituted with a -CN radical or with an amino group, a 4'-aminophenyl radical or forms with R₆ an optionally oxygen-containing and/or nitrogen-containing heterocycle which may be substituted with a C₁-C₄ alkyl radical,
R₈ and R₉ , which are identical or different, represent a hydrogen atom, a halogen atom such as bromine, chlorine, iodine or fluorine, a C₁-C₄ alkyl or C₁-C₄ alkoxy radical, a -CN radical,
X⁻ represents an anion which is preferably chosen from chloride, methylsulphate and acetate,
B represents a group chosen from the following structures B1 to B6: 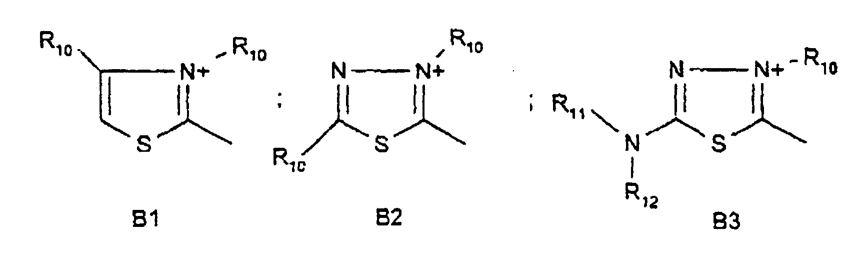 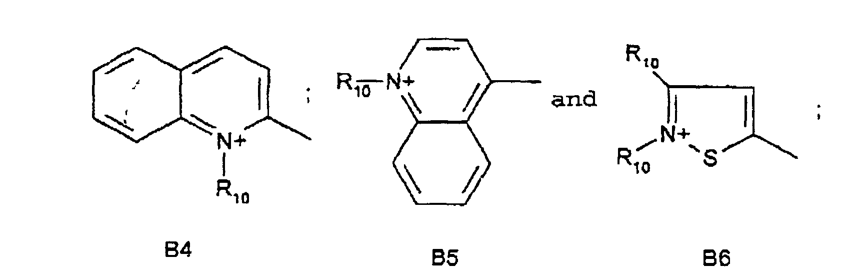
in which R₁₀ represents a C₁-C₄ alkyl radical, R₁₁ and R₁₂, which are identical or different, represent a hydrogen atom or a C₁-C₄ alkyl radical;
**c) the compounds of the following formulae (III) and (III'):** 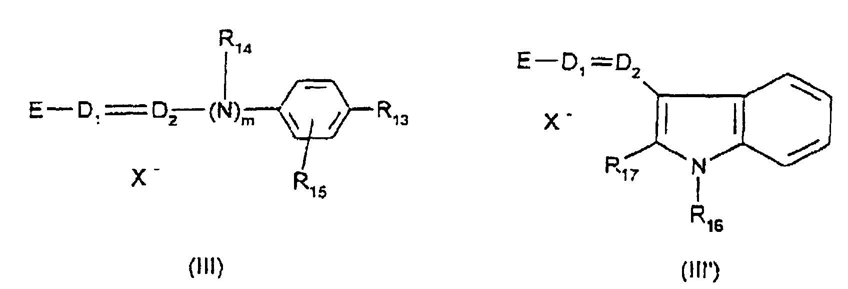 in which:
R₁₃ represents a hydrogen atom, a C₁-C₄ alkoxy radical, a halogen atom such as bromine, chlorine, iodine or fluorine or an amino radical,
R₁₄ represents a hydrogen atom, a C₁-C₄ alkyl radical or forms with a carbon atom of the benzene ring a heterocycle which is optionally oxygen-containing and/or substituted with one or more C₁-C₄ alkyl groups,
R₁₅ represents a hydrogen or halogen atom such as bromine, chlorine, iodine of fluorine,
R₁₆ and R₁₇, which are identical or different, represent a hydrogen atom or a C₁-C₄ alkyl radical,
D₁ and D₂, which are identical or different, represent a nitrogen atom or the -CH group,
m = 0 or 1,
it being understood that when R₁₃ represents an unsubstituted amino group, then D₁ and D₂ simultaneously represent a -CH group and m = 0,
X⁻ represents an anion which is preferably chosen from chloride, methylsulphate and acetate,
E represents a group chosen from the following structures E1 to E8: 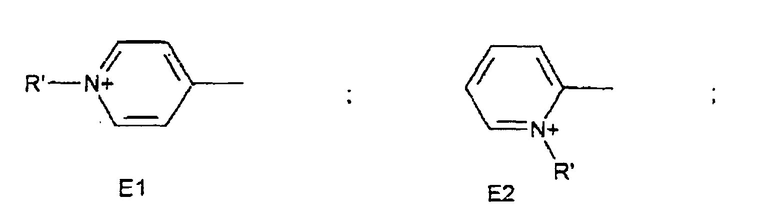 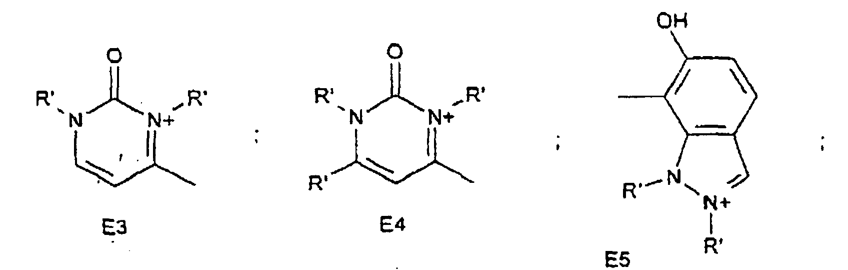 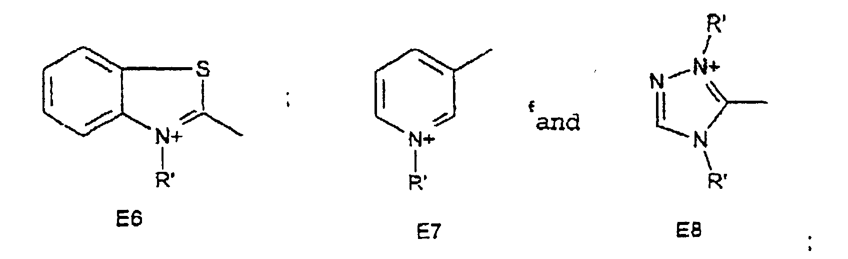
in which R' represents a C₁-C₄ alkyl radical;
when m = 0 and D₁ represents a nitrogen atom, then E may also denote a group having the following structure E9: 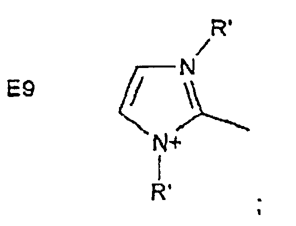 in which R' represents a C₁-C₄ alkyl radical,
**d) the compounds of the following formula (IV) :**
G-N=N-J (IV)
in which:
**the symbol G** represents a group chosen from the following structures G₁ to G₃: 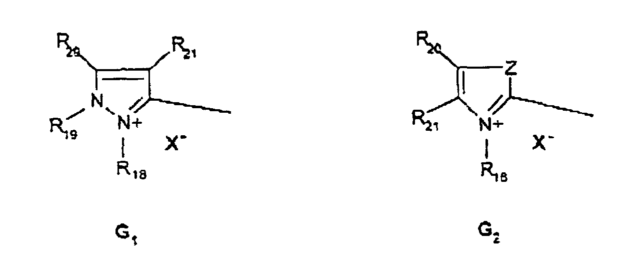 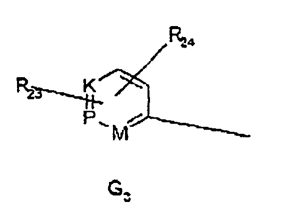 in which structures G₁ to G₃,
R₁₈ denotes a C₁-C₄ alkyl radical, a phenyl radical which may be substituted with a C₁-C₄ alkyl radical or a halogen atom chosen from chlorine, bromine, iodine and fluorine;
R₁₉ denotes a C₁-C₄ alkyl radical or a phenyl radical;
R₂₀ and R₂₁, which are identical or different, represent a C₁-C₄ alkyl radical, a phenyl radical, or form together in G₁ a benzene ring which is substituted with one or more C₁-C₄ alkyl, C₁-C₄ alkoxy or NO₂ radicals, or form together in G₂ a benzene ring which is optionally substituted with one or more C₁-C₄ alkyl, C₁-C₄ alkoxy or NO₂ radicals;
R₂₀ may denote, in addition, a hydrogen atom;
Z denotes an oxygen or sulphur atom or an -NR₁₉ group;
M represents a group -CH, -CR (R denoting C₁-C₄ alkyl), or -NR₂₂ (X⁻ ) ᵣ;
K represents a group -CH, -CR (R denoting C₁-C₄ alkyl) , Or -NR₂₂ (X⁻ ) ᵣ;
P represents a group -CH, -CR (R denoting C₁-C₄ alkyl), or -NR₂₂ (X⁻)ᵣ; r denotes zero or 1;
R₂₂ represents an O⁻ atom, a C₁-C₄ alkoxy radical or a C₁-C₄ alkyl radical;
R₂₃ and R₂₄, which are identical or different, represent a hydrogen or halogen atom chosen from chlorine, bromine, iodine and fluorine, a C₁-C₄ alkyl radical, a C₁-C₄ alkoxy radical or an -NO₂ radical;
X⁻ represents an anion which is preferably chosen from chloride, iodide, methylsulphate, ethylsulphate, acetate and perchlorate;
with the proviso that
if R₂₂ denotes O⁻, then r denotes zero;
if K or P or M denote -N-(C₁-C₄ alkyl)X⁻, then R₂₃ or R₂₄ is different from a hydrogen atom;
if K denotes -NR₂₂ (X⁻)_{r'} , then M = P = -CH, -CR;
if M denotes -NR₂₂(X⁻)r, then K = P = -CH, -CR;
if P denotes -NR₂₂(X⁻)ᵣ, then K = M and denote -CH or - CR;
if Z denotes a sulphur atom with R₃₁ denoting C₁-C₄ alkyl, then R₂₀ is different from a hydrogen atom;
if Z denotes -NR₂₂ with R₁₉ denoting C₁-C₄ alkyl, then at least one of the R₁₈, R₂₀ or R₂₁ radicals of G₂ is different from a C₁-C₄ alkyl radical;
**the symbol J** represents:
- **(a)** a group having the following structure J₁: 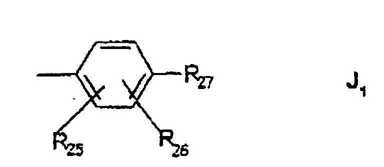 in which structure J₁,
R₂₅ represents a hydrogen atom, a halogen atom chosen from chlorine, bromine, iodine and fluorine, a C₁-C₄ alkyl radical, a C₁-C₄ alkoxy radical, a radical -OH, -NO₂, -NHR₂₈, -NR₂₉R₃₀, -NHCO(C₁-C₄alkyl), or forms with R₂₆ a 5- or 6-membered ring containing or otherwise one or more heteroatoms chosen from nitrogen, oxygen or sulphur;
R₂₆ represents a hydrogen atom, a halogen atom chosen from chlorine, bromine, iodine and fluorine, a C₁-C₄ alkyl or C₁-C₄ alkoxy radical, or forms with R₂₇ or R₂₈ a 5- or 6-membered ring containing or otherwise one or more heteroatoms chosen from nitrogen, oxygen or sulphur;
R₂₇ represents a hydrogen atom, an -OH radical, an -NHR₂₈ radical, an -NR₂₉R₃₀ radical;
R₂₈ represents a hydrogen atom, a C₁-C₄ alkyl radical, a C₁-C₄ monohydroxyalkyl radical, a C₂-C₄ polyhydroxyalkyl radical, a phenyl radical;
R₂₉ and R₃₀, which are identical or different, represent a C₁-C₄ alkyl radical, a C₁-C₄ monohydroxyalkyl radical, a C₂-C₄ polyhydroxyalkyl radical;
- **(b)** a 5- or 6- membered nitrogen-containing heterocycle group which is capable of containing other heteroatoms and/or carbonyl-containing groups and which may be substituted with one or more C₁-C₄ alkyl, amino or phenyl radicals,
and in particular a group having the following structure J₂: 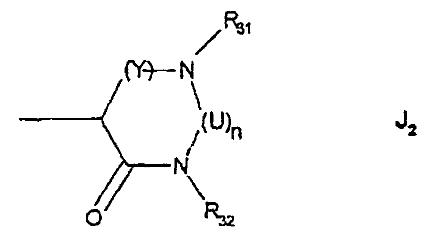 in which structure J₂,
R₃₁ and R₃₂, which are identical or different, represent a hydrogen atom, a C₁-C₄ alkyl radical, a phenyl radical;
Y denotes the -CO- radical or the radical 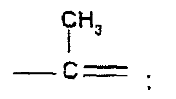
n = 0 or 1, with, when n denotes 1, U denotes the -CO- radical.
the said composition being **characterized in that** it contains, in addition,
(ii) at least one nonionic surfactant chosen from the group comprising:
**(ii)**_{**1**} - alkyl polyglucosides;
**(ii)**_{**2**}- sugar or alkyl sugar fatty acid esters;
**(ii)**_{**3**} - fatty sugar amides;
**(ii)**_{**4**} - mixtures thereof.

2. Composition according to Claim 1, **characterized in that** the cationic direct dyes of formula (I) are chosen from the compounds corresponding to the following structures (I1) to (I54):

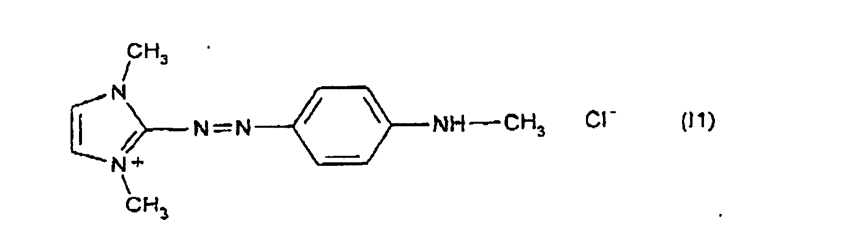

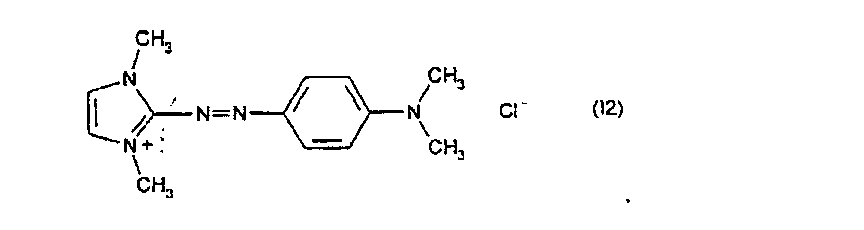

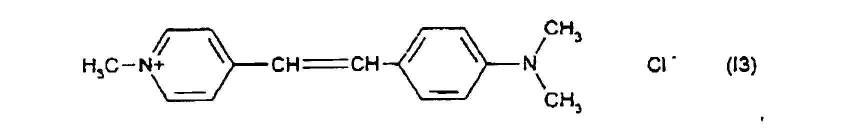

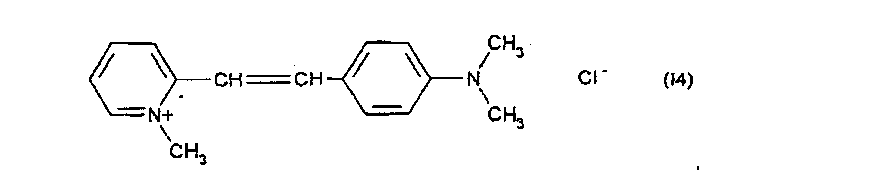

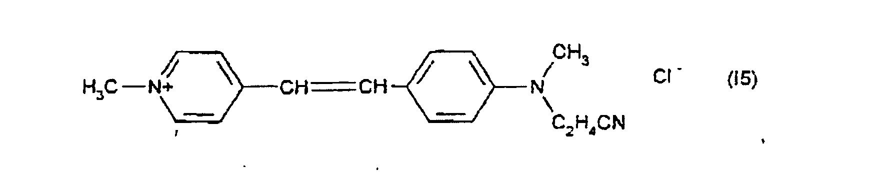

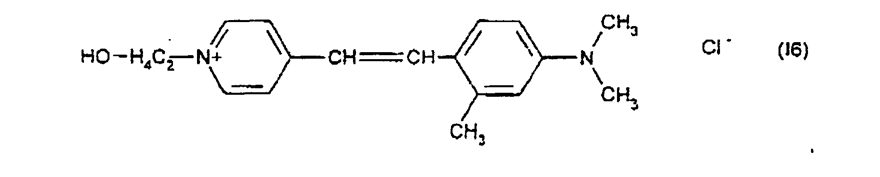

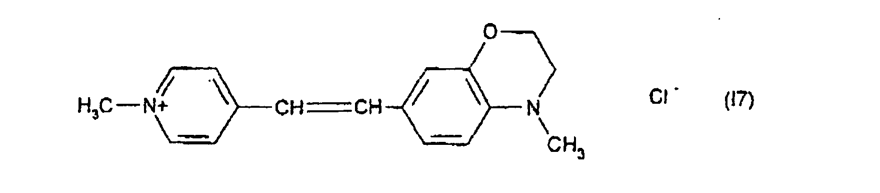

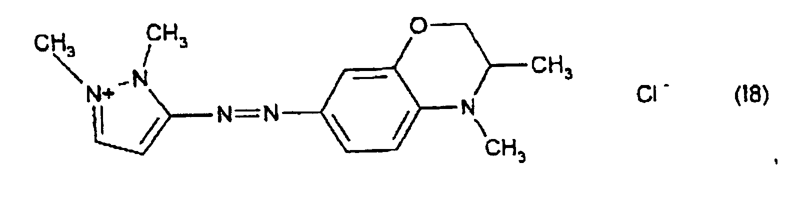

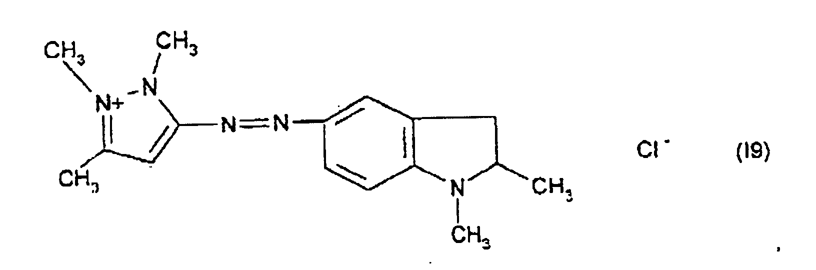

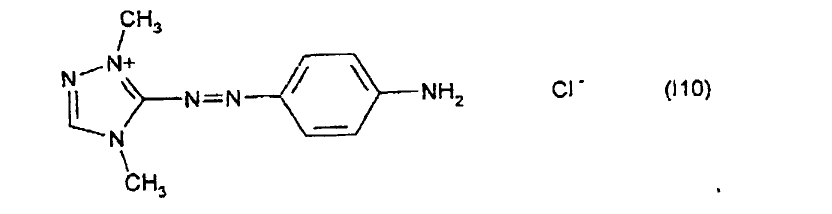

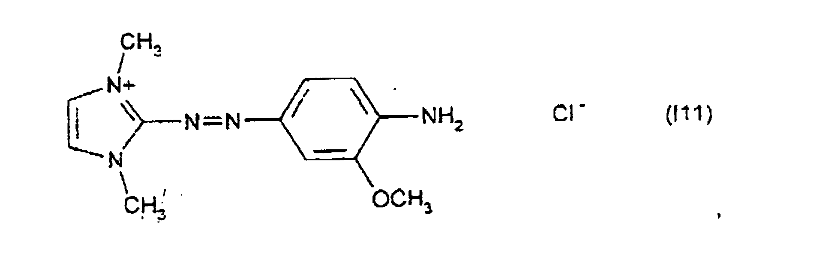

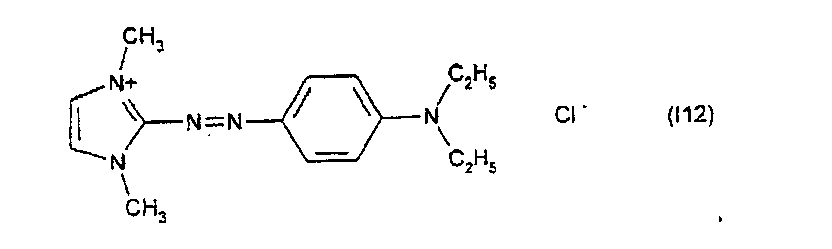

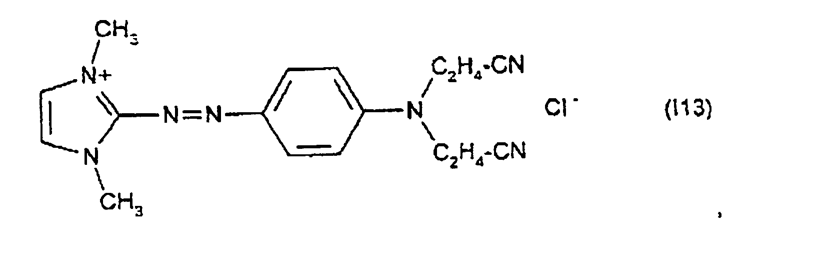

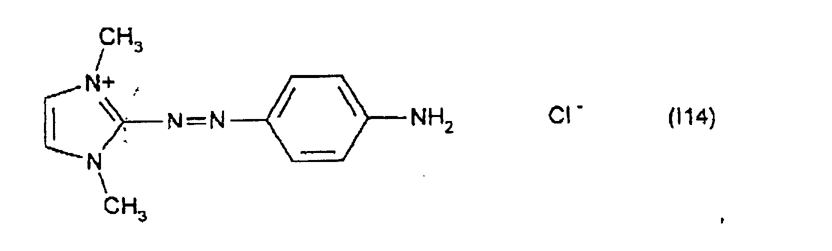

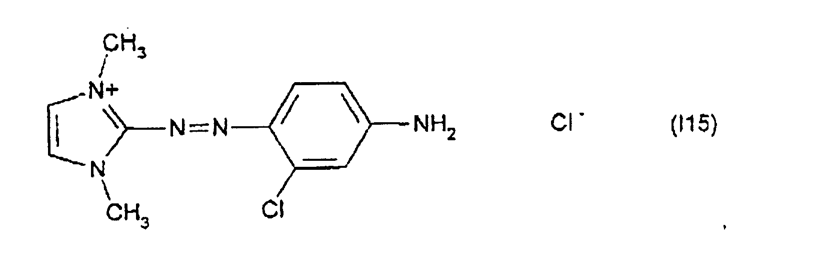

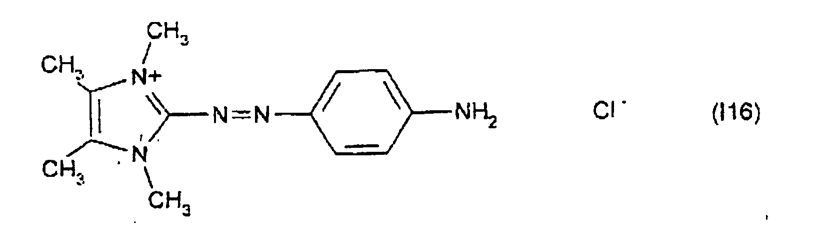

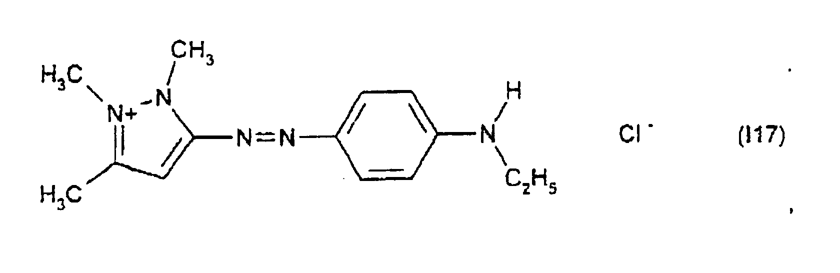

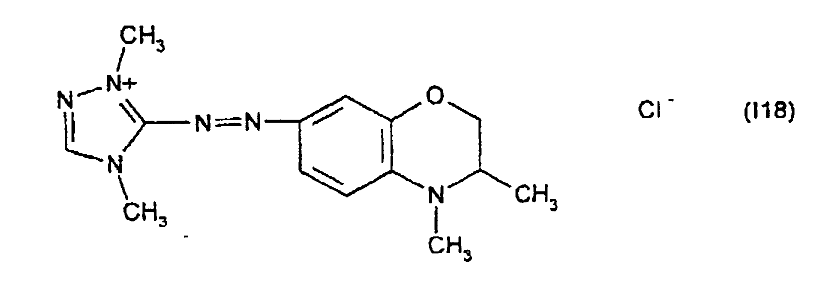

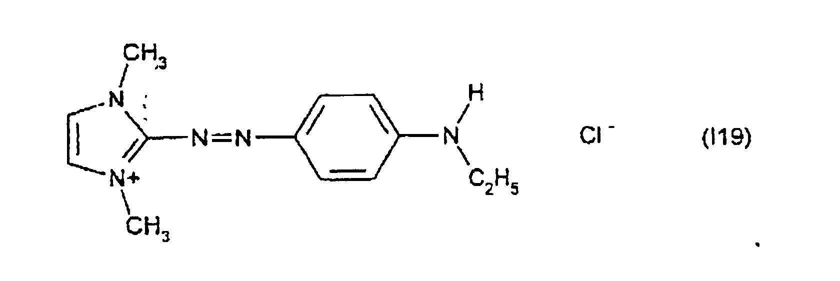

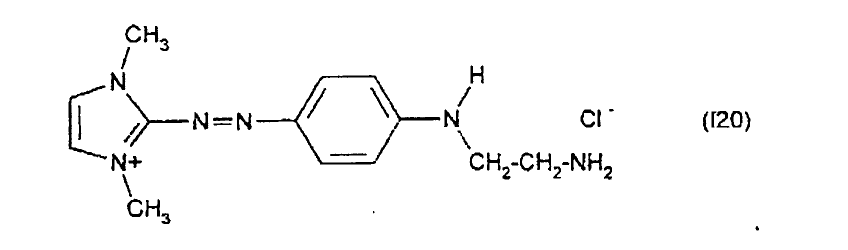

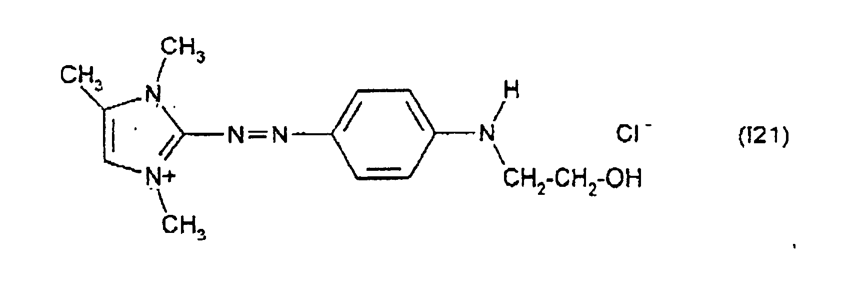

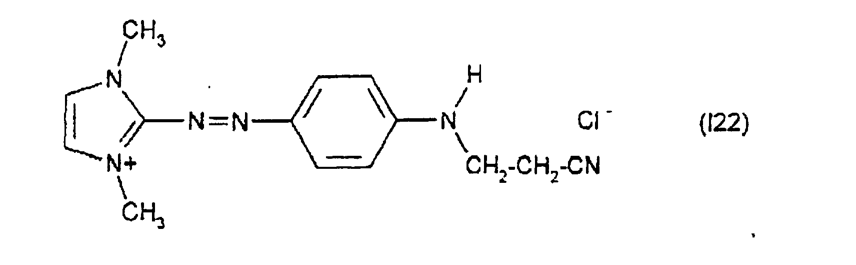

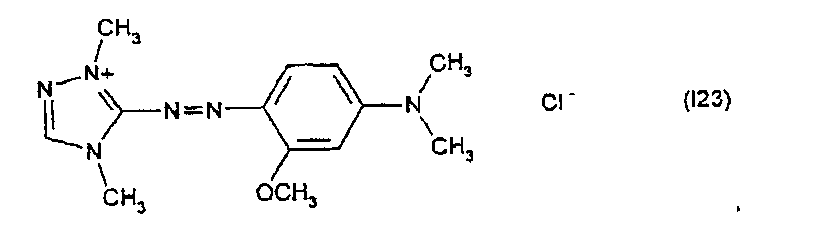

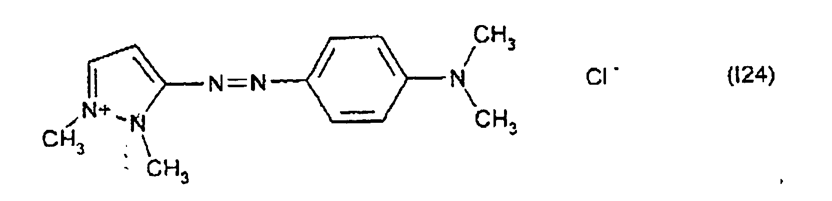

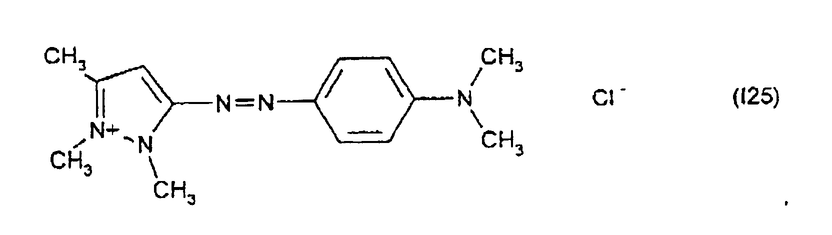

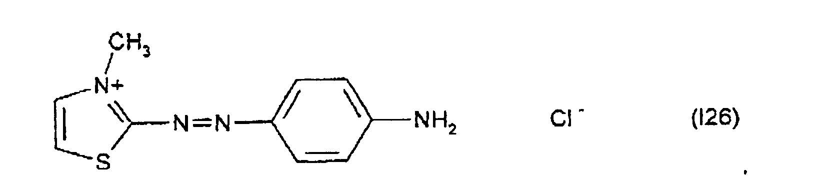

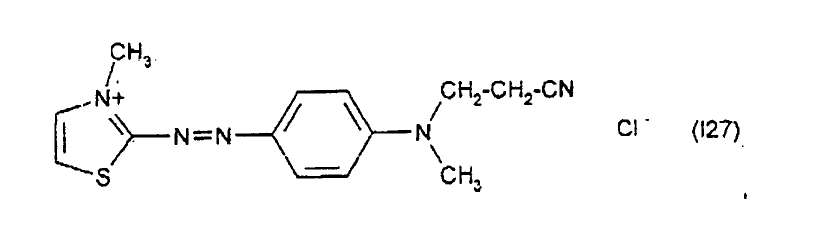

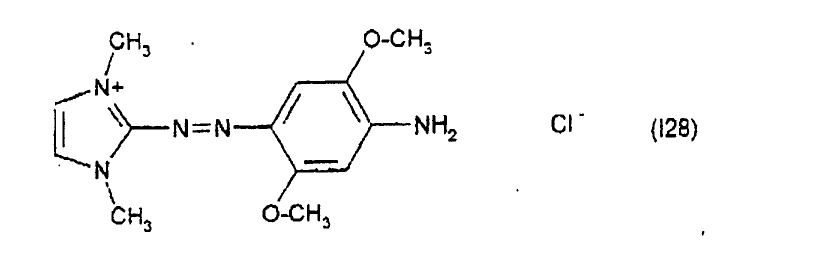

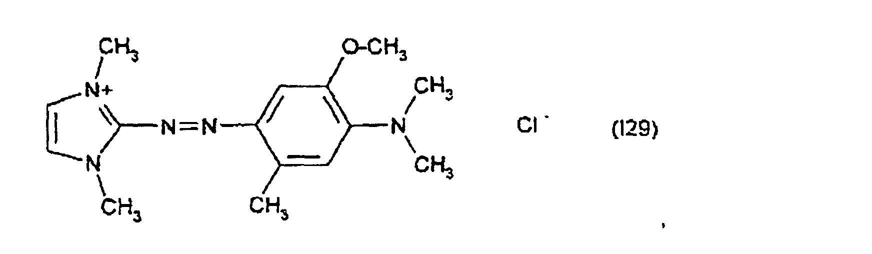

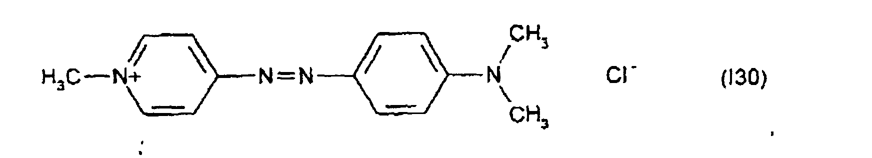

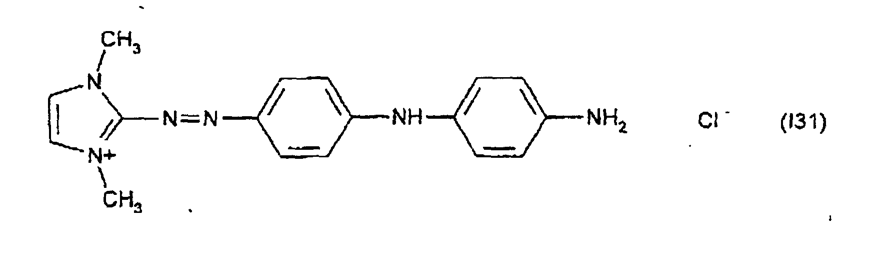

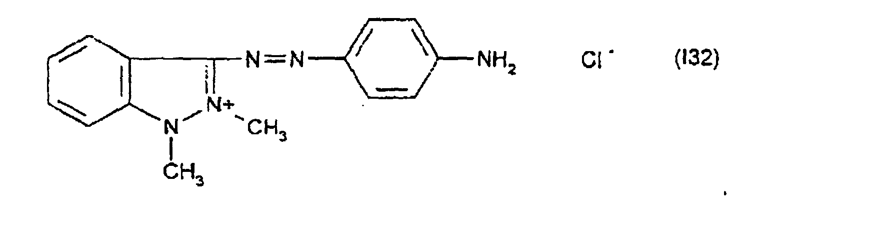

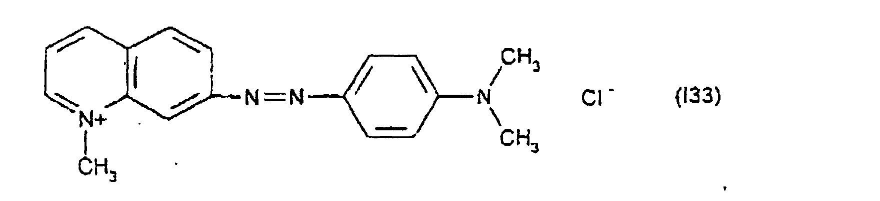

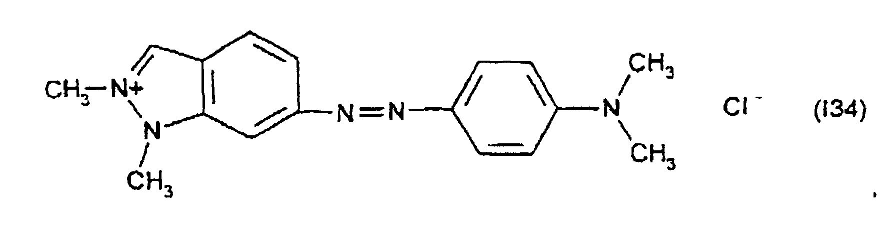

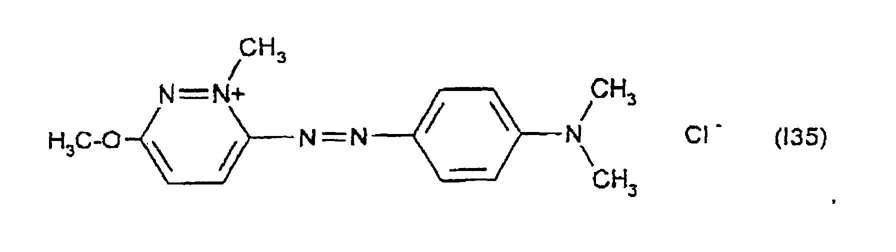

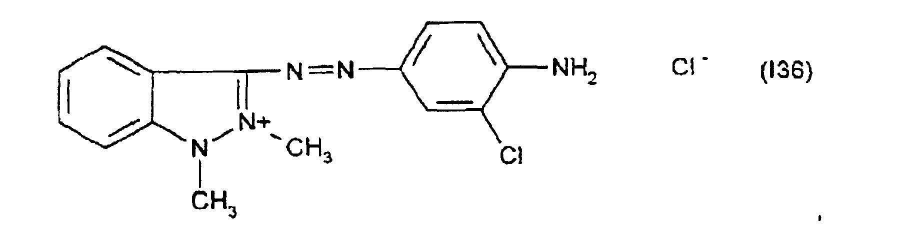

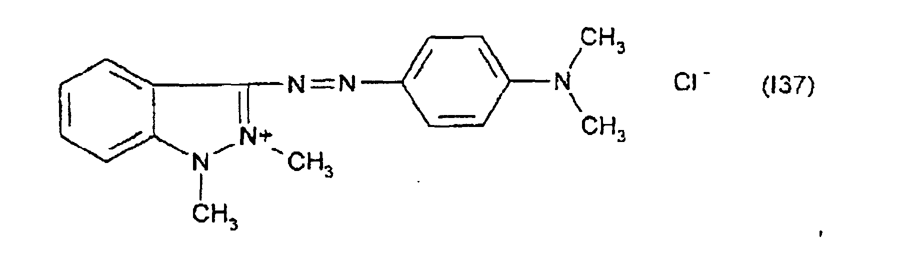

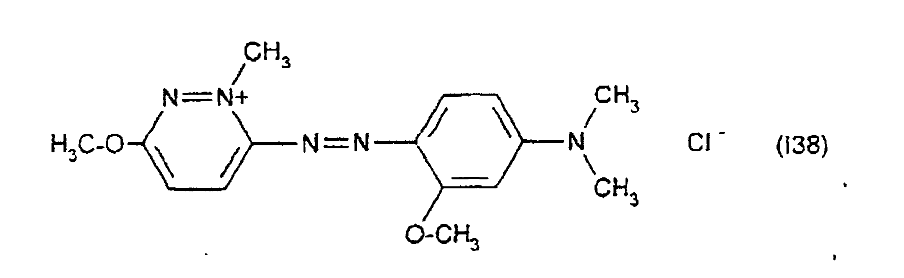

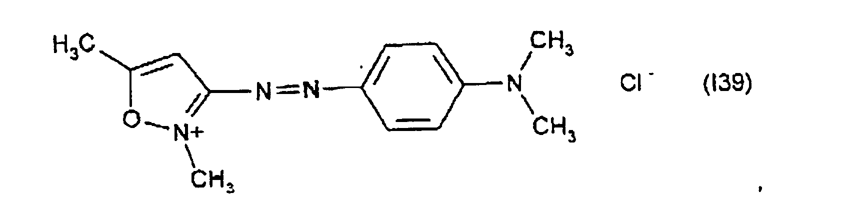

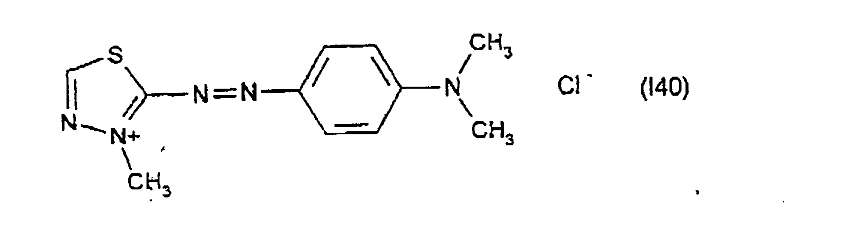

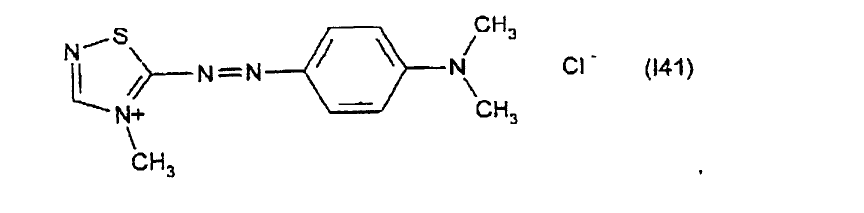

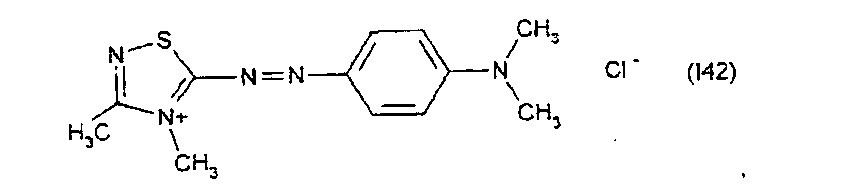

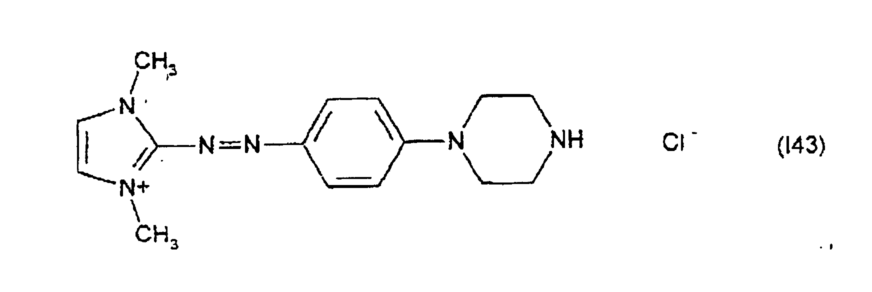

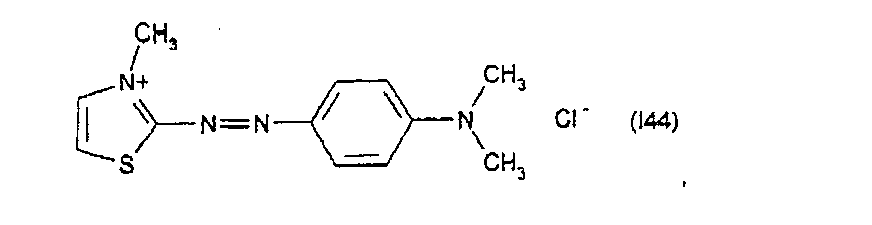

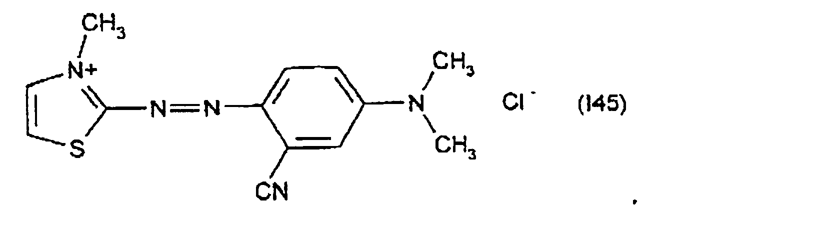

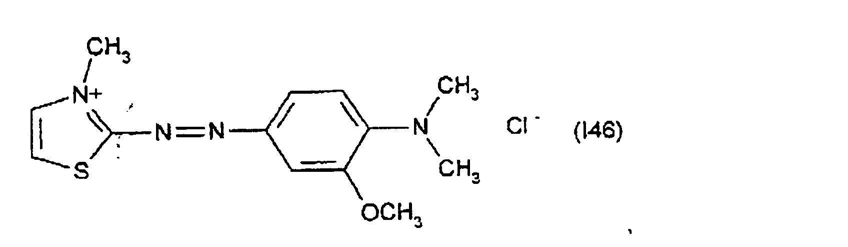

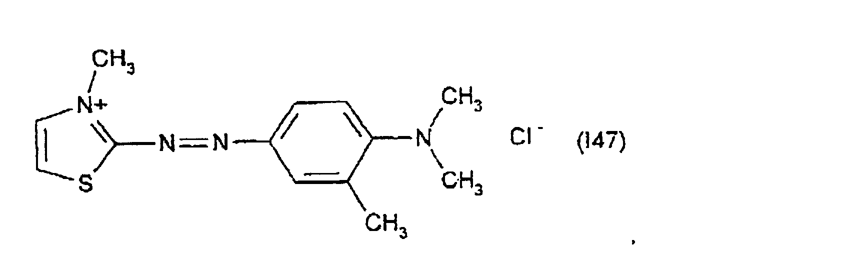

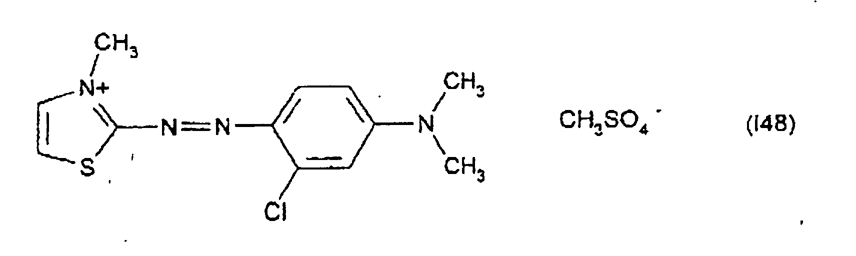

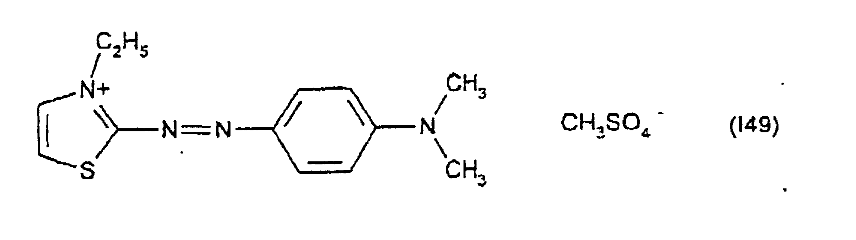

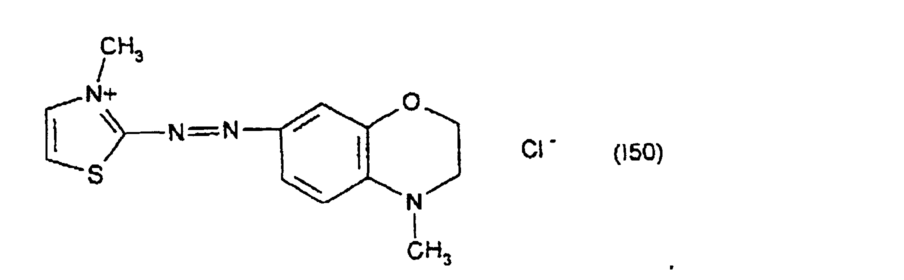

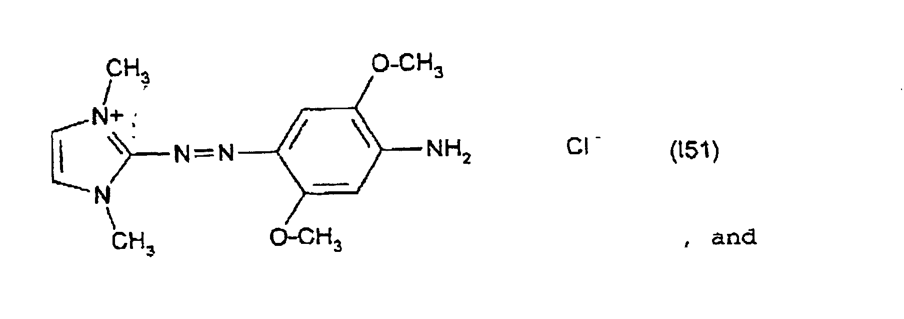

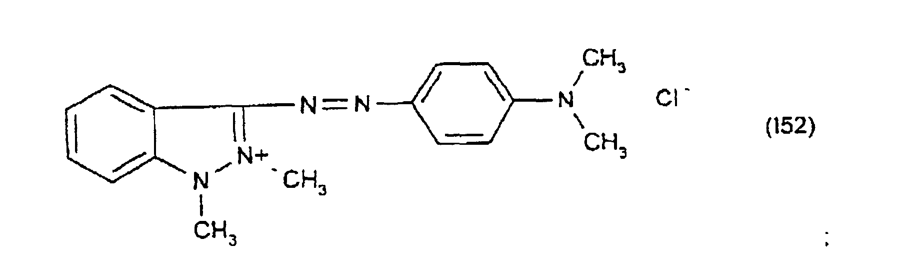

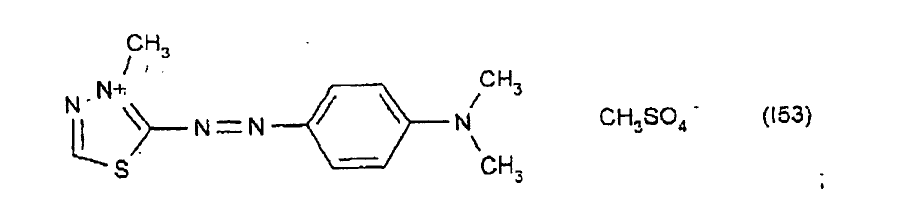

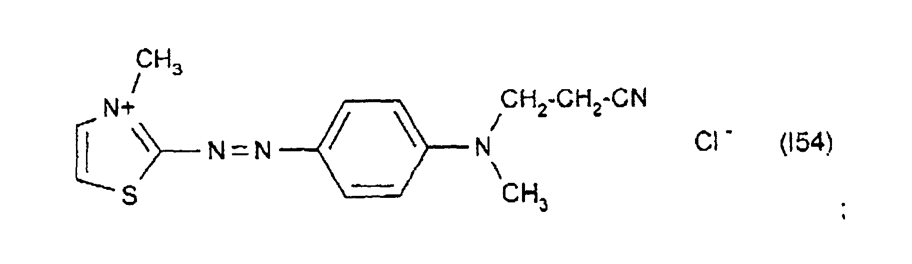

3. Composition according to Claim 2, **characterized in that** the cationic direct dyes correspond to the structures (I1), (I2), (I14), and (I31).

4. Composition according to Claim 1, **characterized in that** the cationic direct dyes of formula (II) are chosen from the compounds corresponding to the following structures (II1) to (II9): 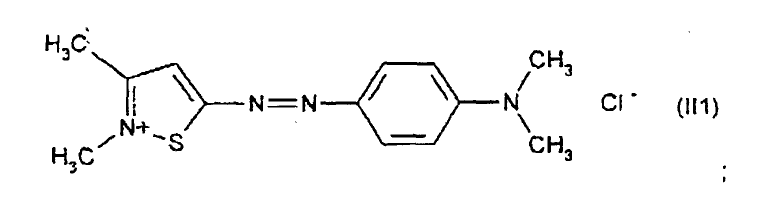 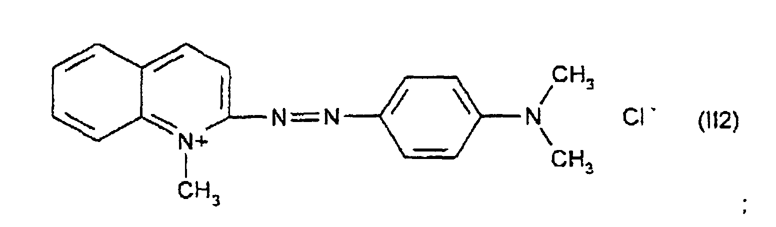 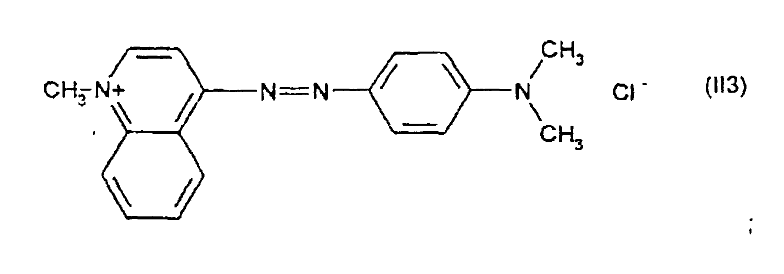 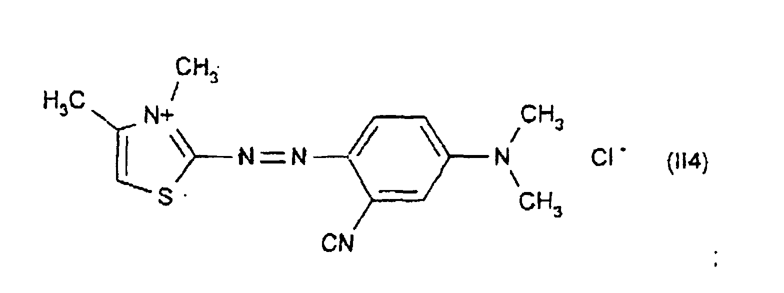 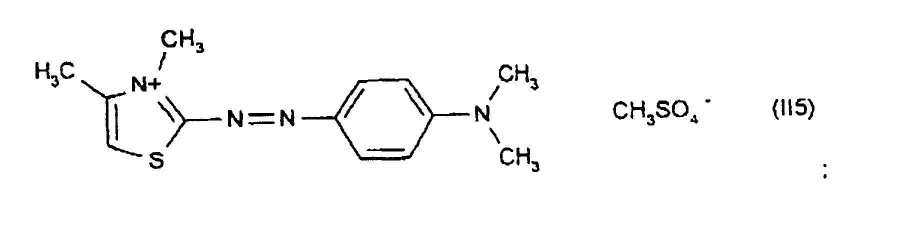 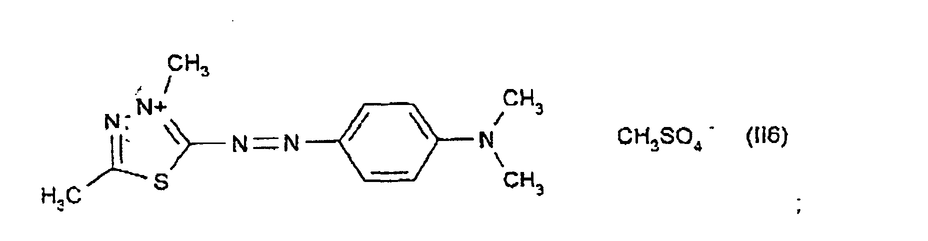 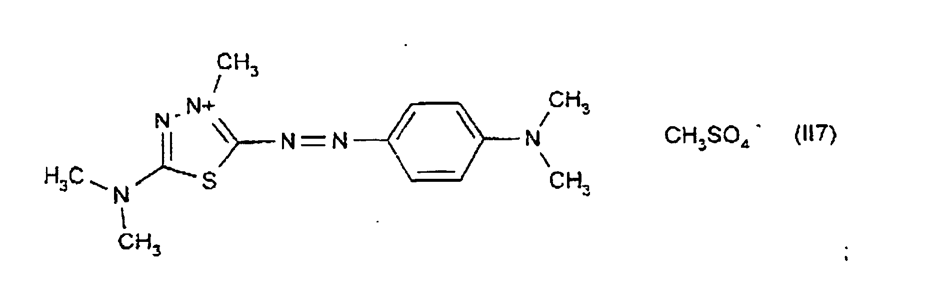 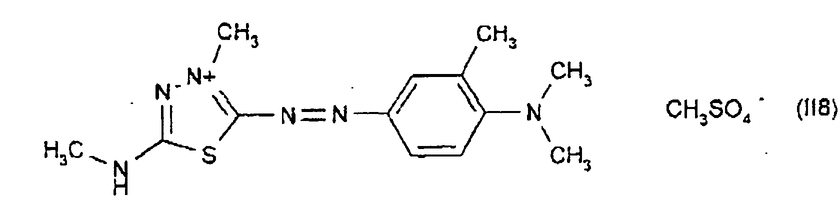 and

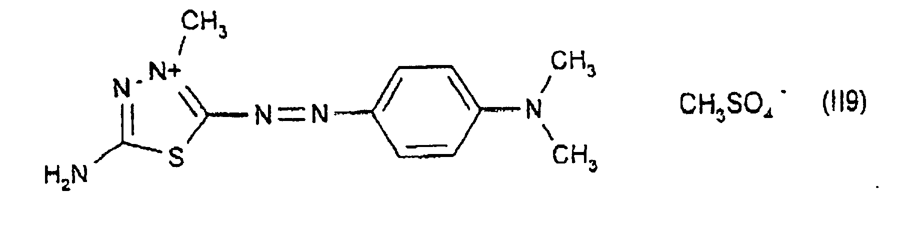

5. Composition according to Claim 1, **characterized in that** the cationic direct dyes of formula (III) are chosen from the compounds corresponding to the following structures (III1) to (III18): 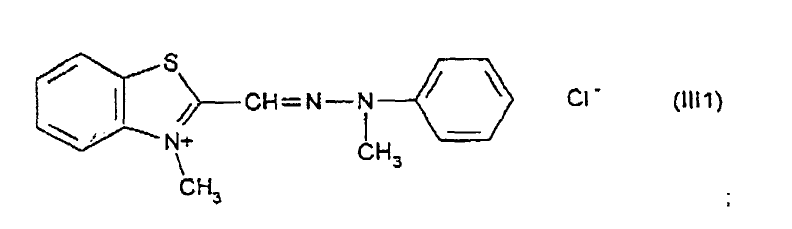 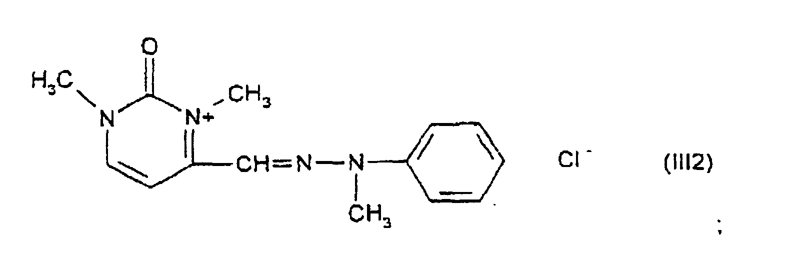 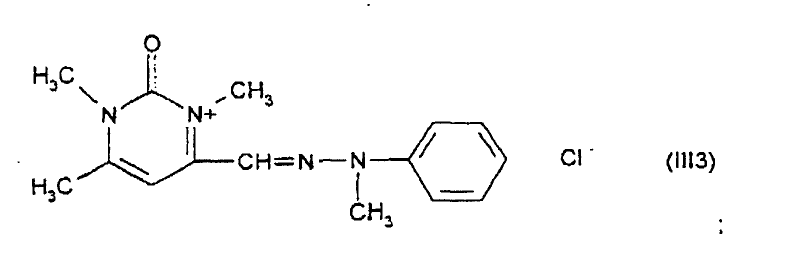 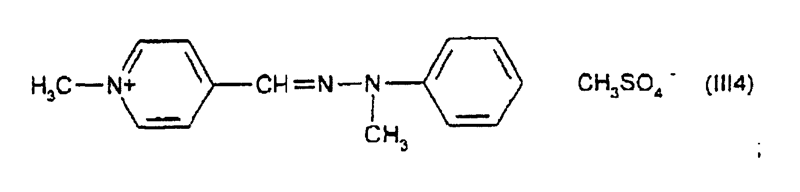 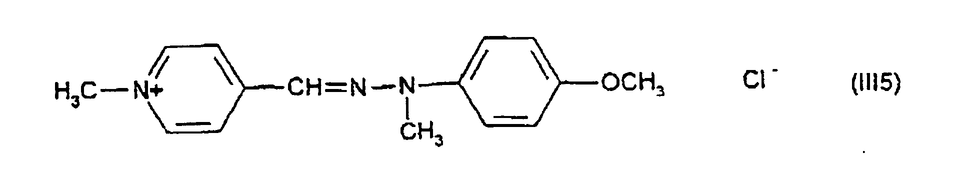 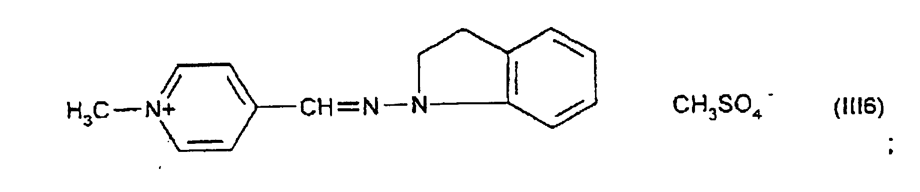 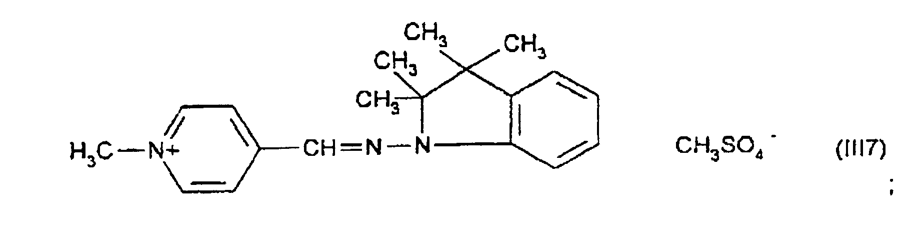 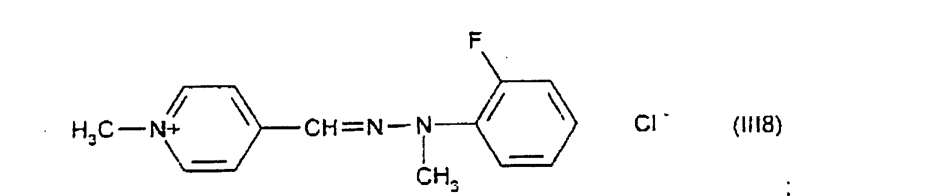 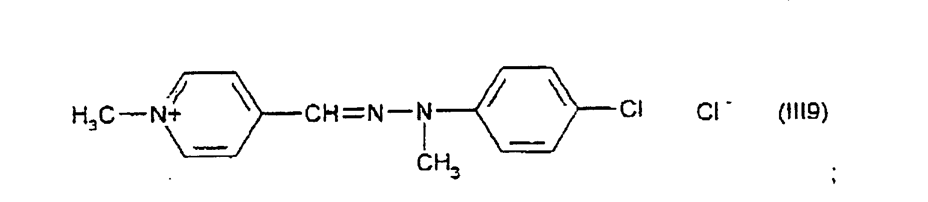 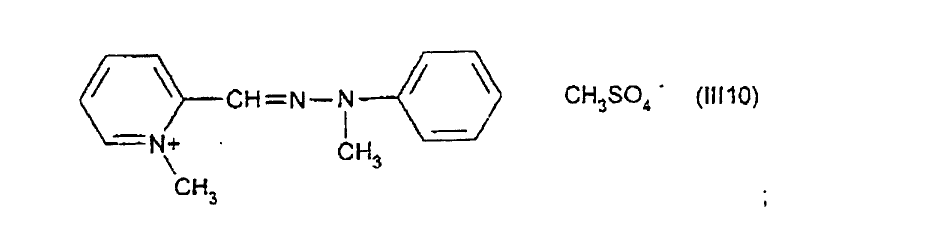 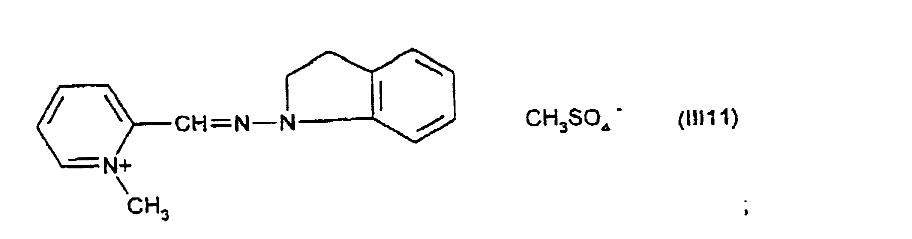 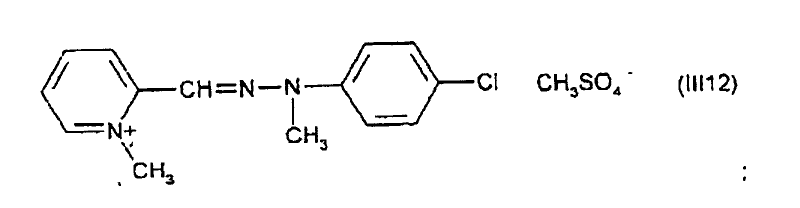 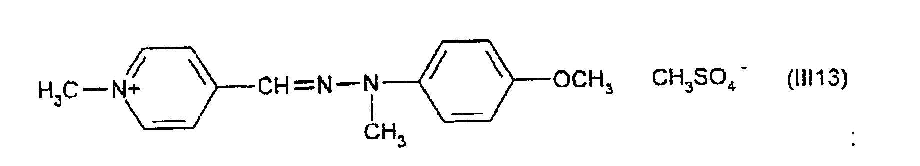 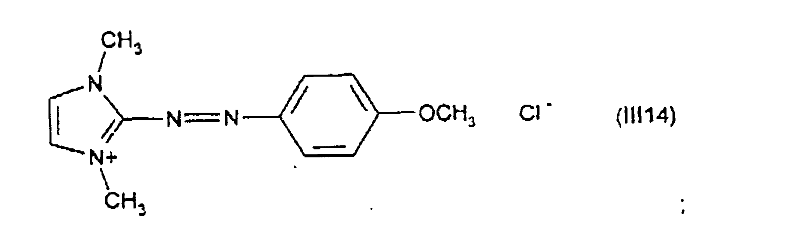 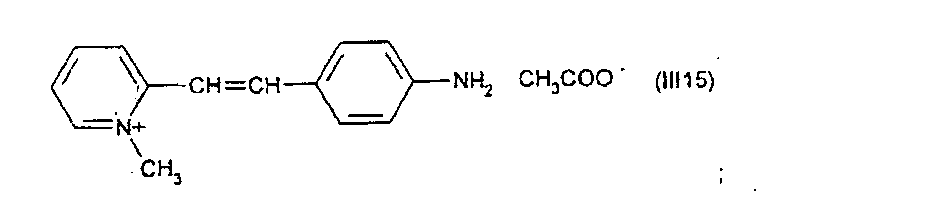 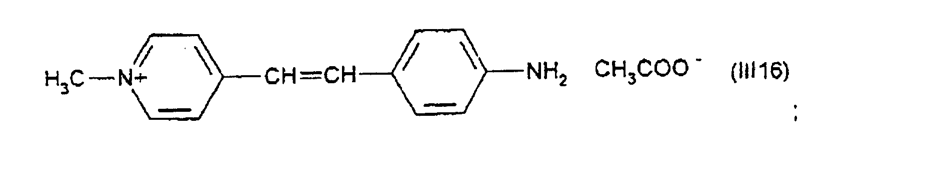 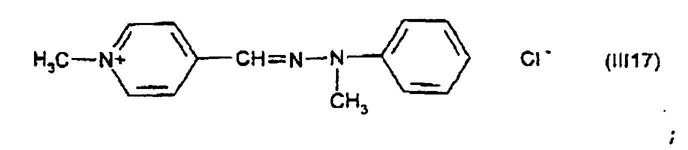 and

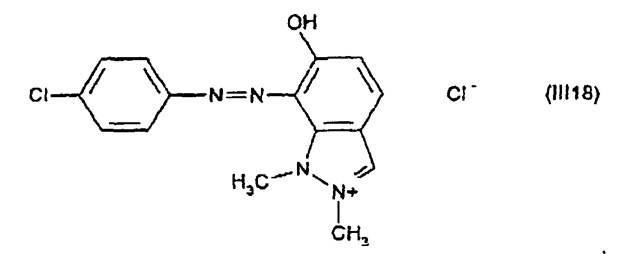

6. Composition according to Claim 5, **characterized in that** the cationic direct dyes of formula (III) are chosen from the compounds corresponding to the structures (III4), (III5) and (III13).

7. Composition according to Claim 1, **characterized in that** the cationic direct dyes of formula (III') are chosen from the compounds corresponding to the following structures (III'1) to (III'3): 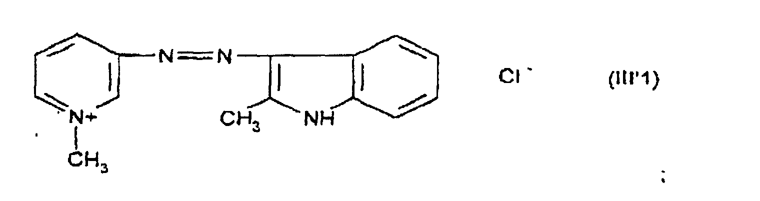 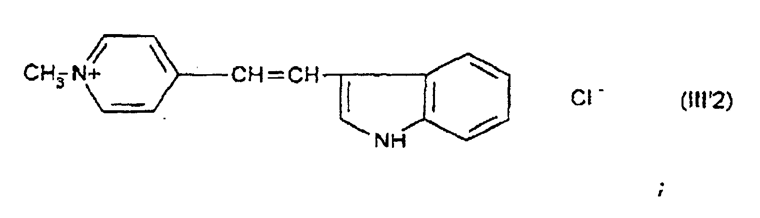 and

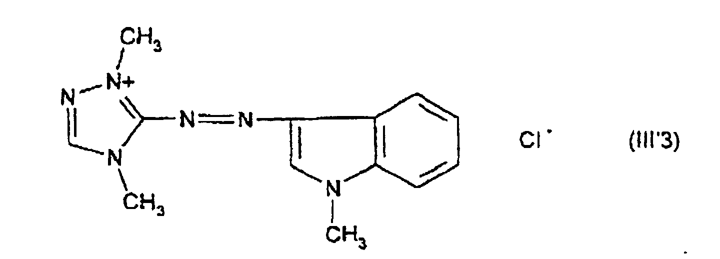

8. Composition according to Claim 1, **characterized in that** the cationic direct dyes of formula, (IV) are chosen from the compounds corresponding to the following structures (IV)₁ to (IV) ₇₇:

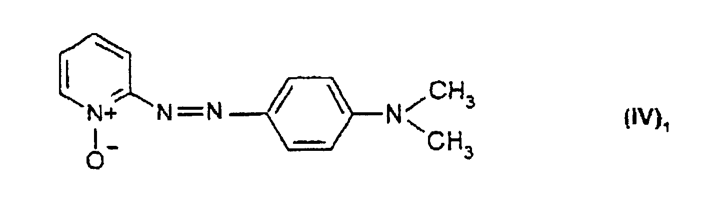

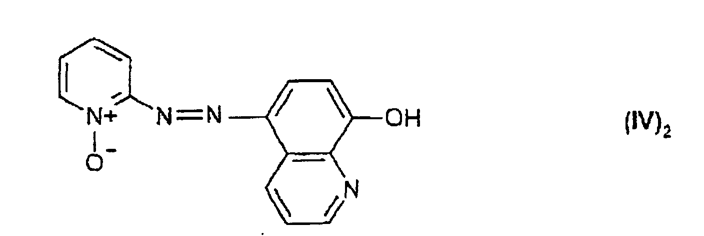

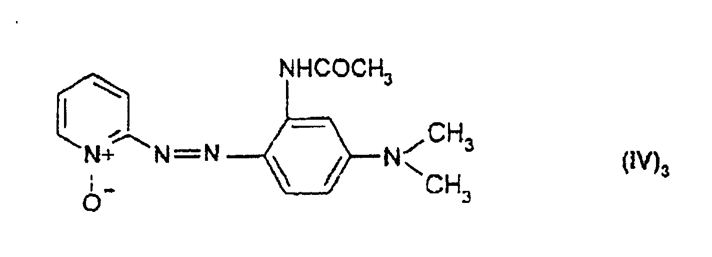

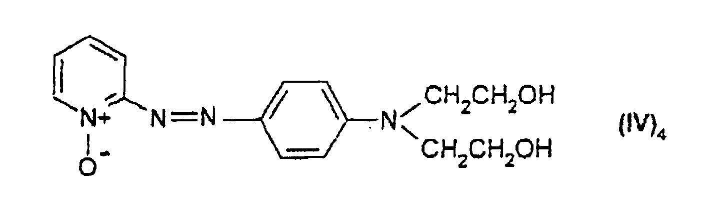

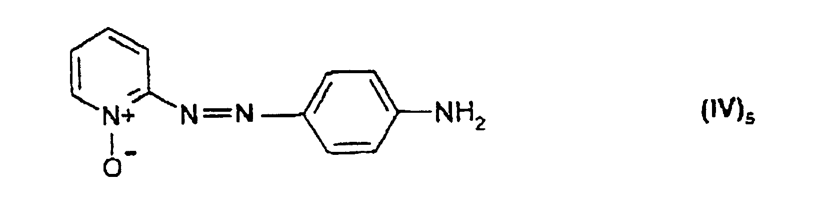

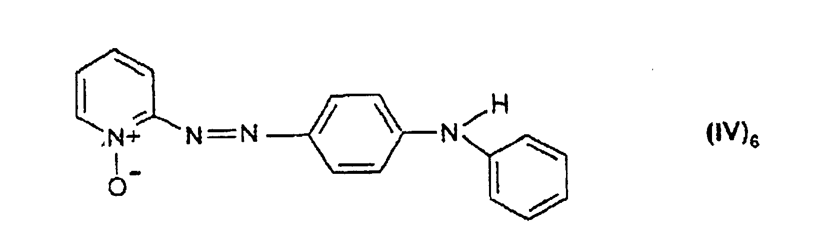

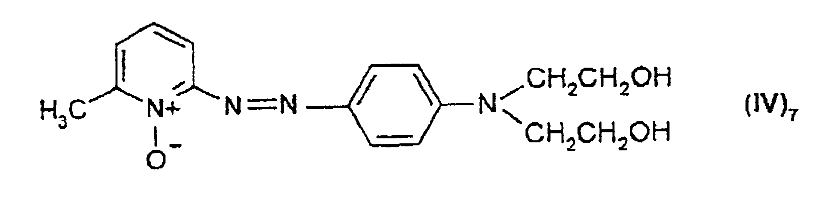

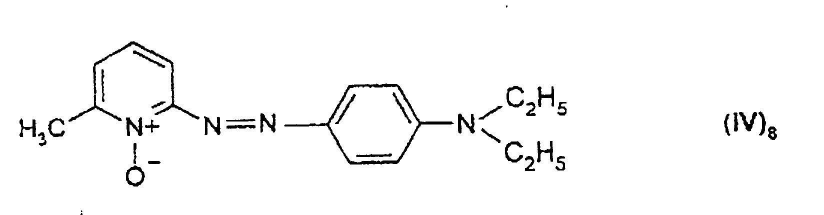

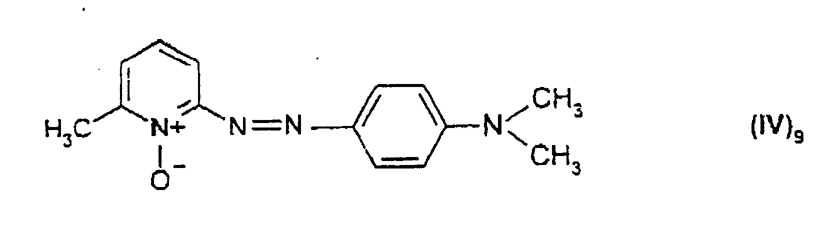

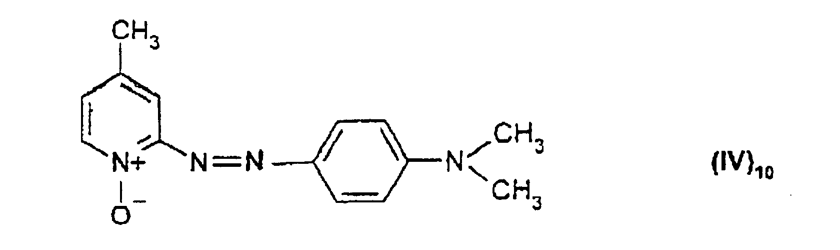

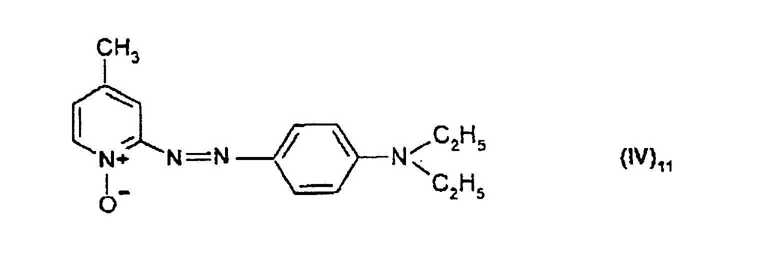

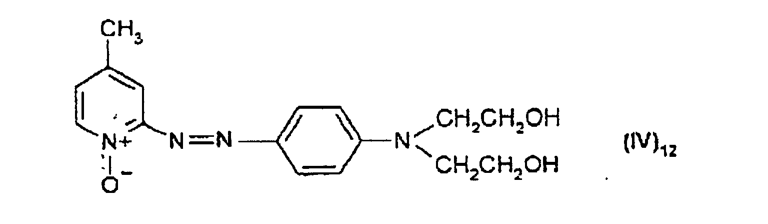

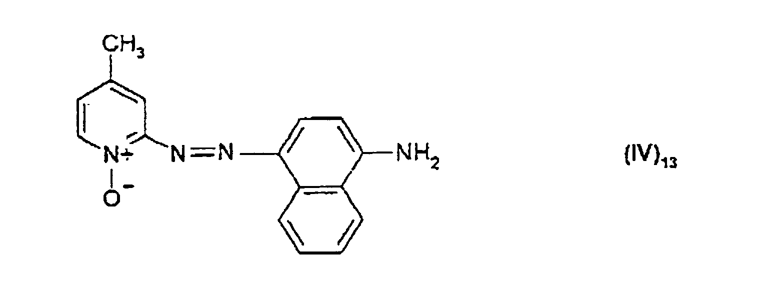

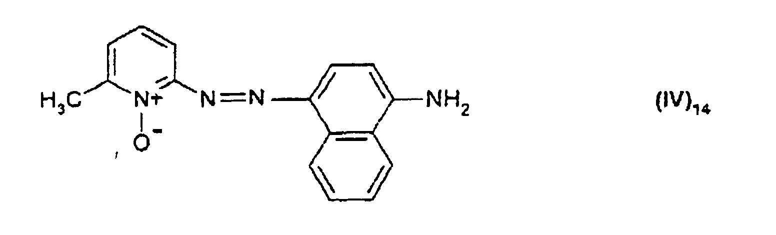

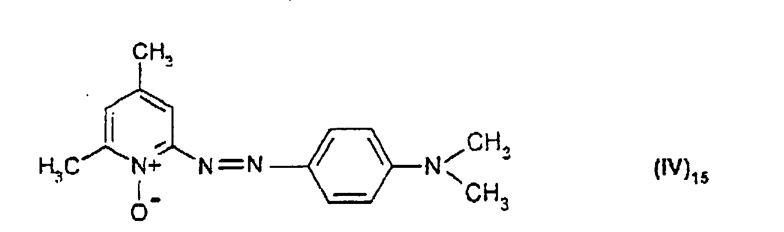

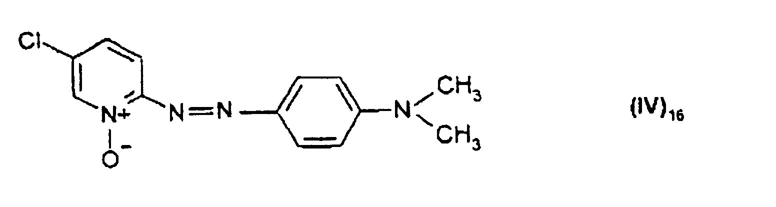

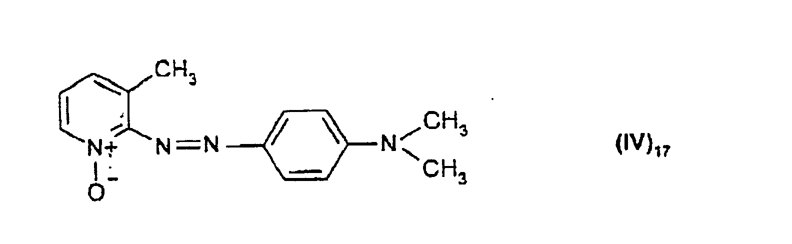

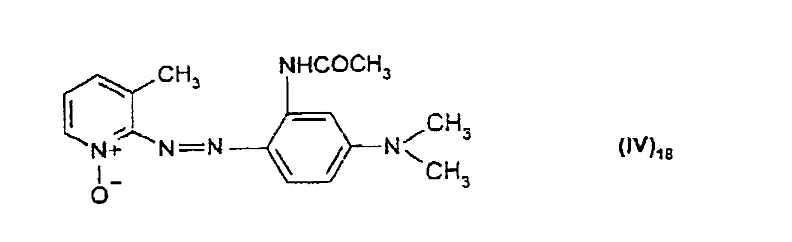

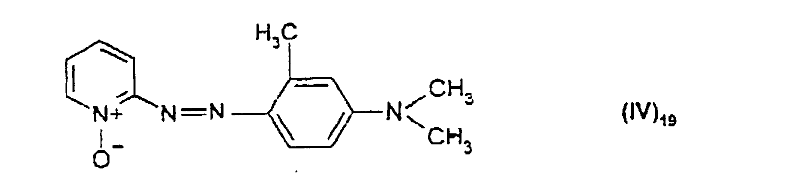

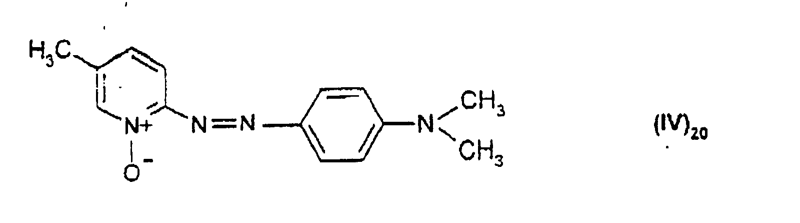

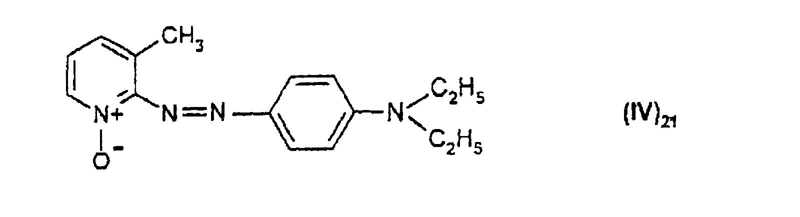

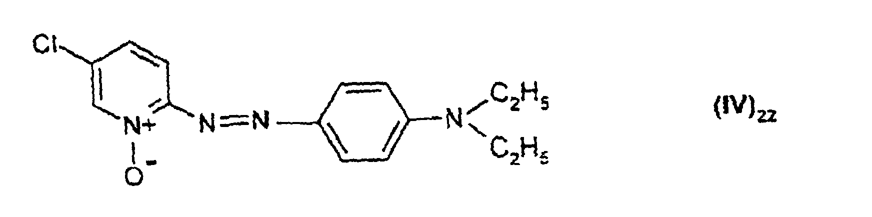

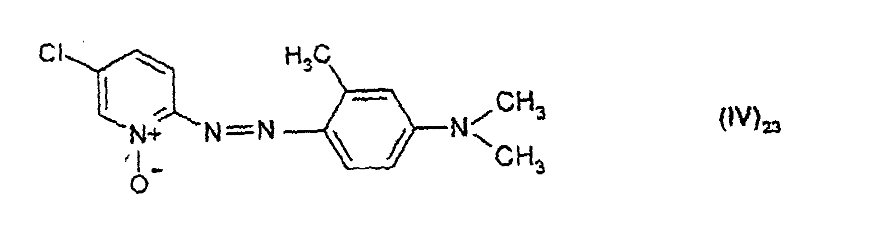

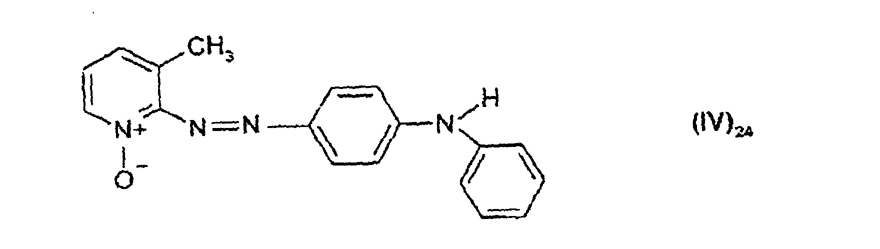

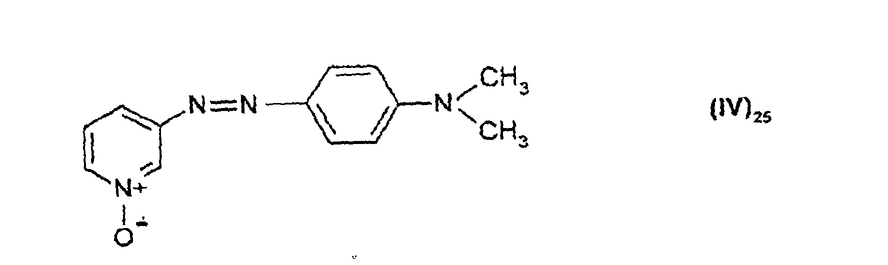

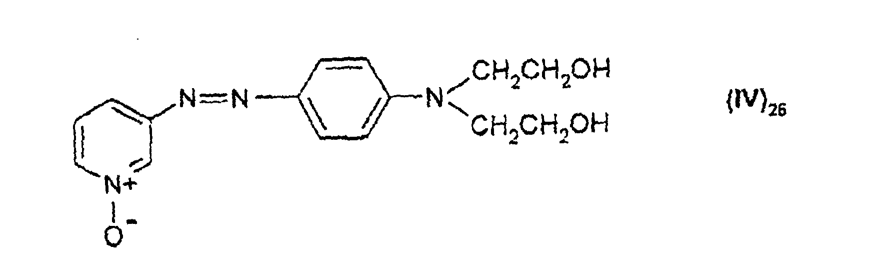

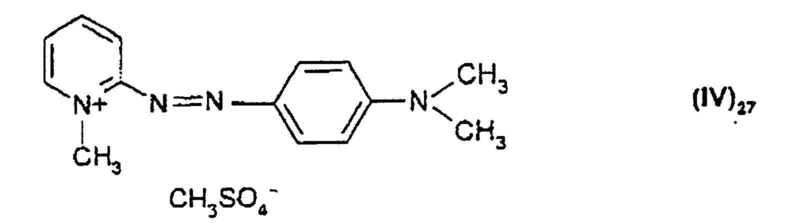

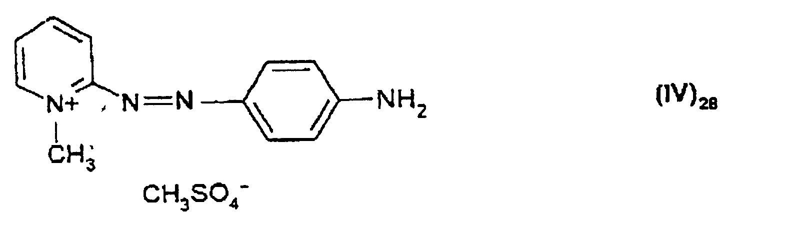

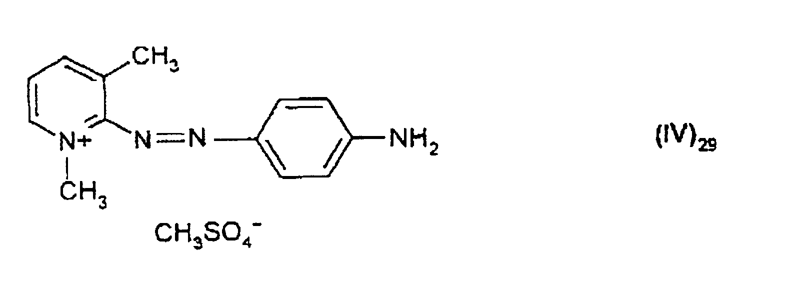

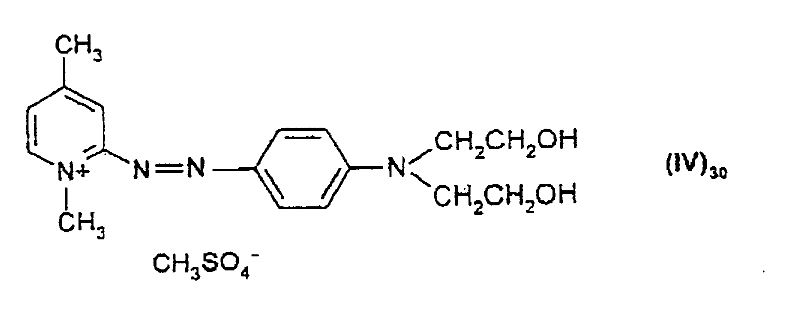

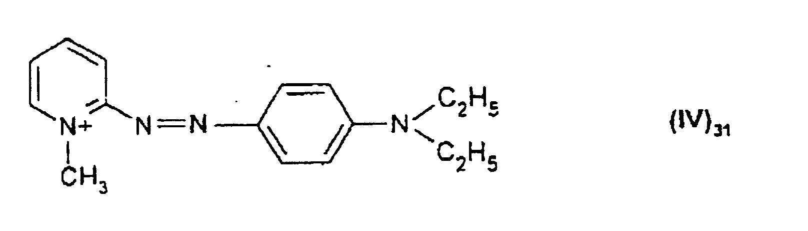

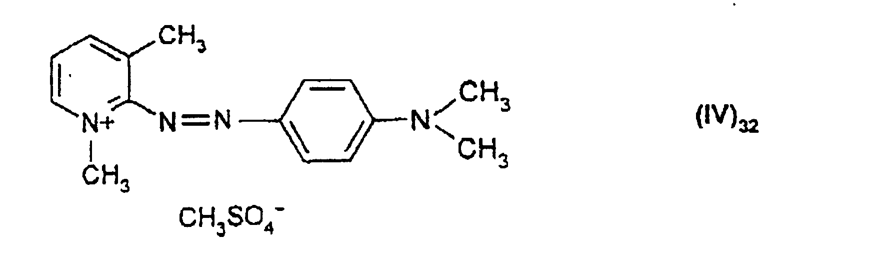

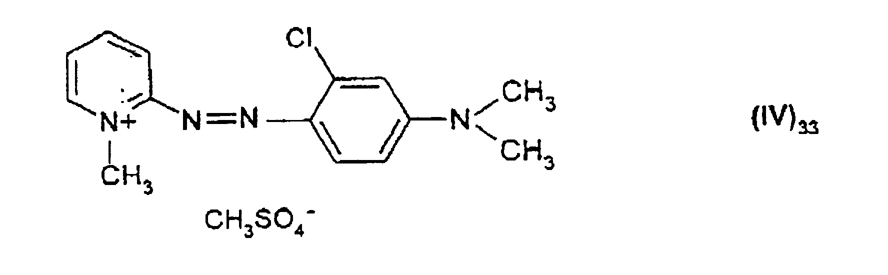

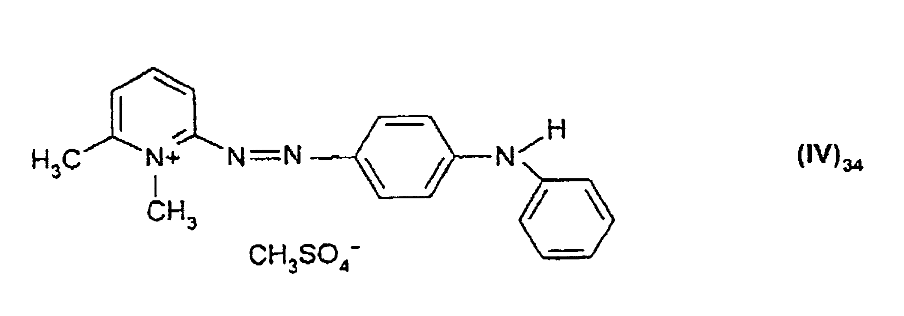

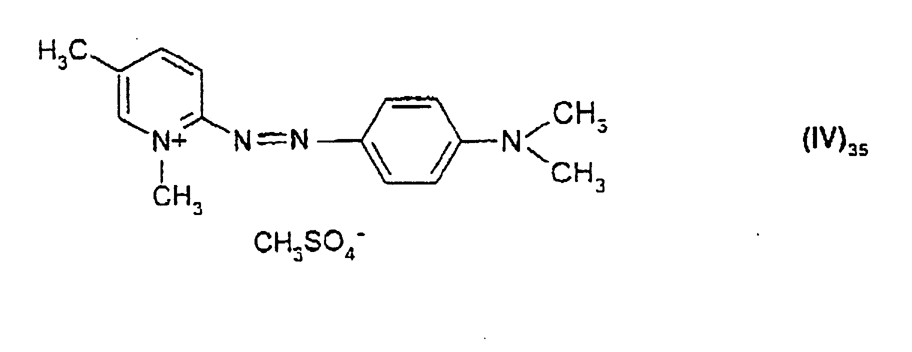

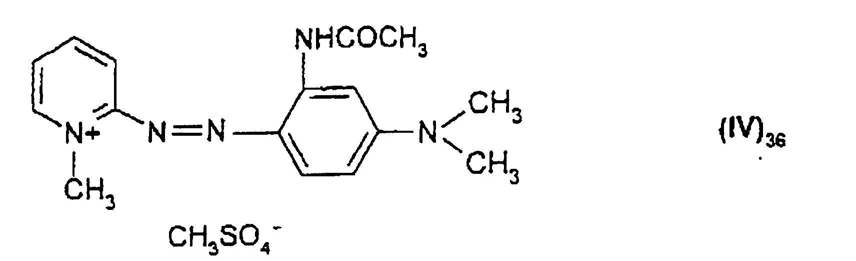

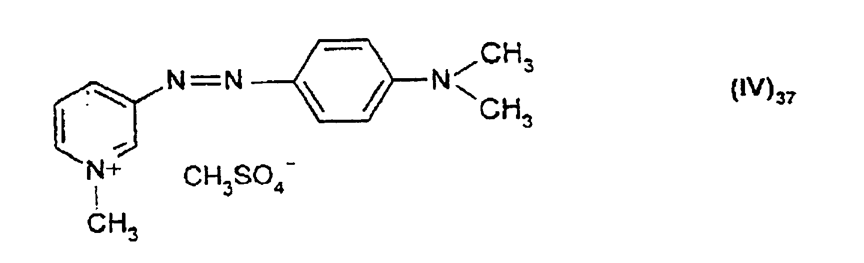

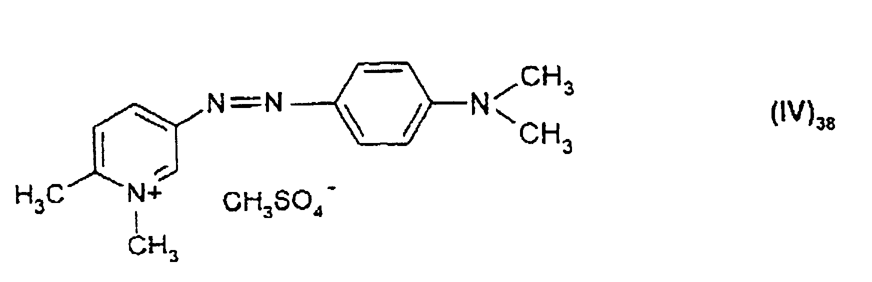

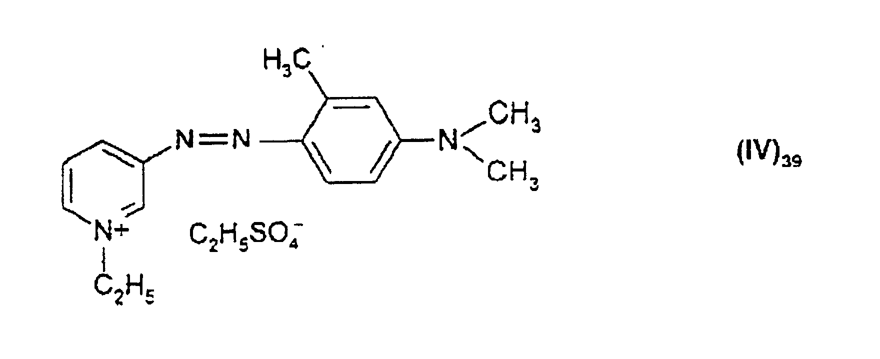

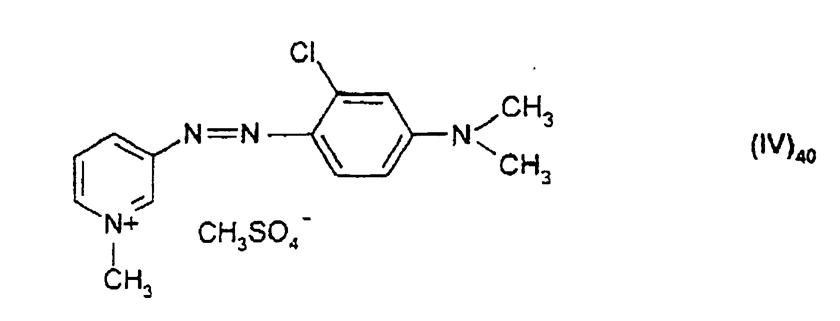

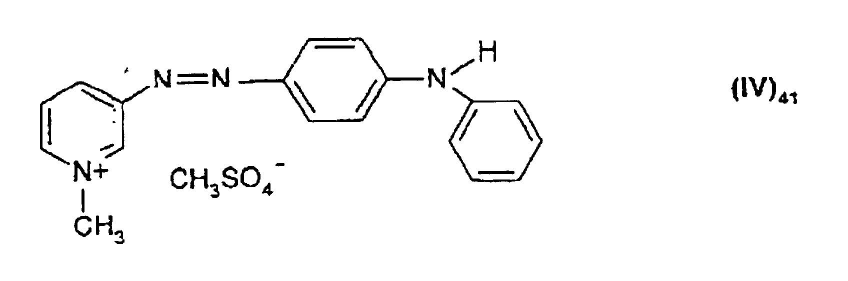

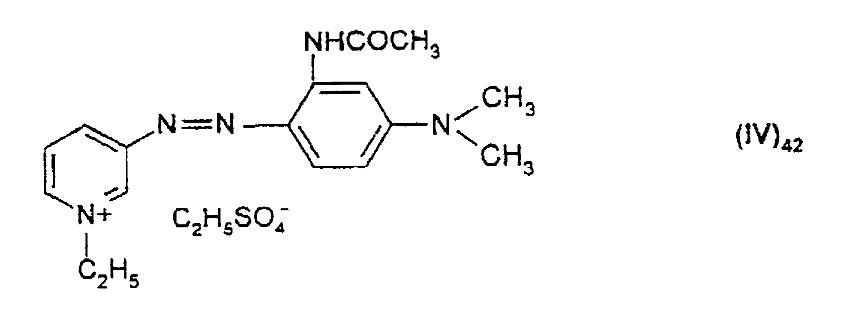

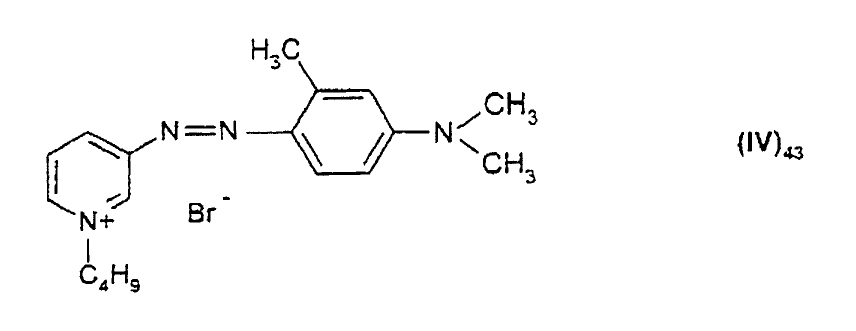

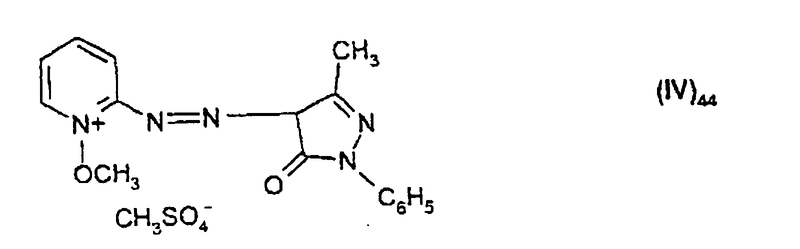

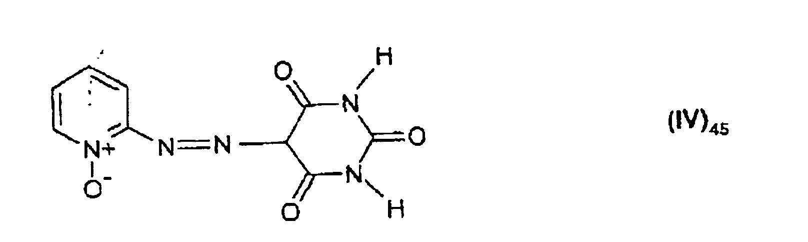

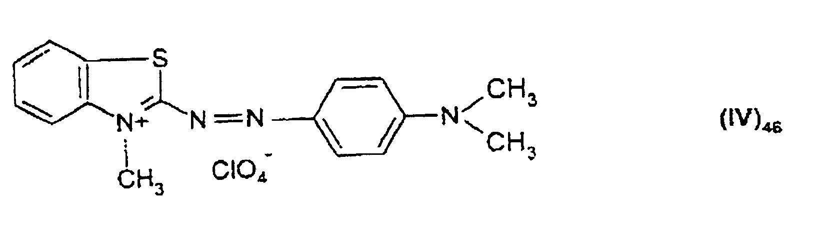

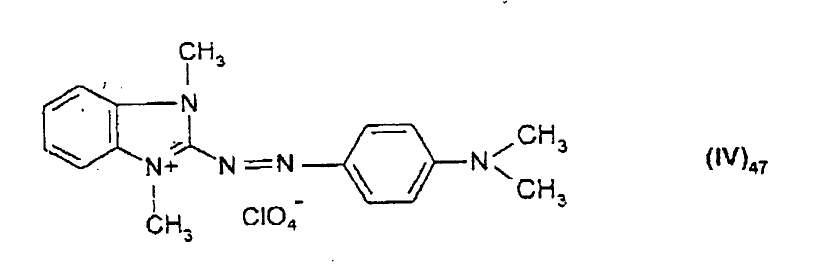

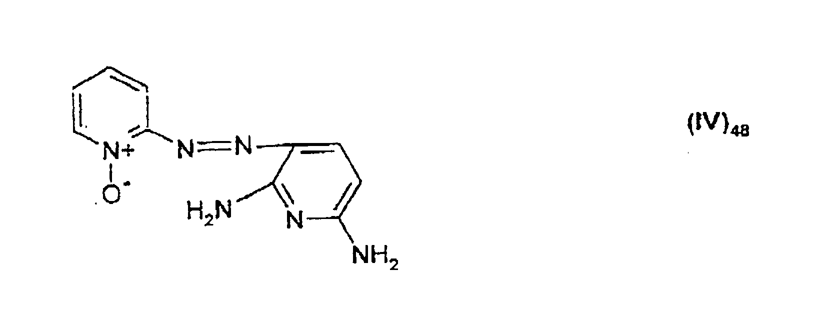

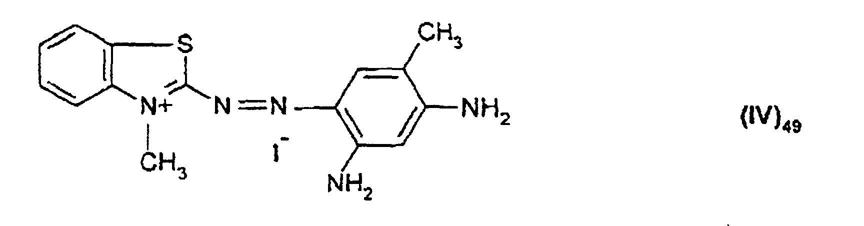

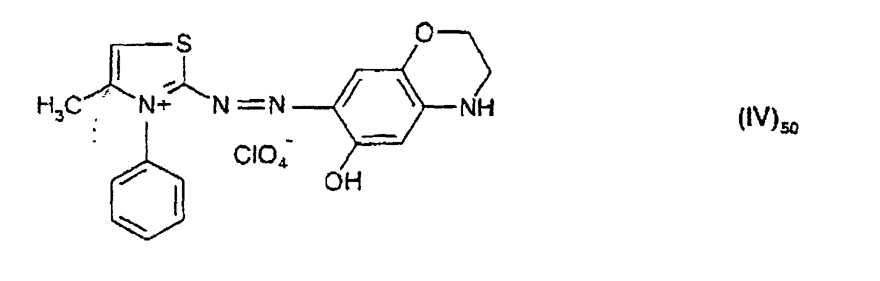

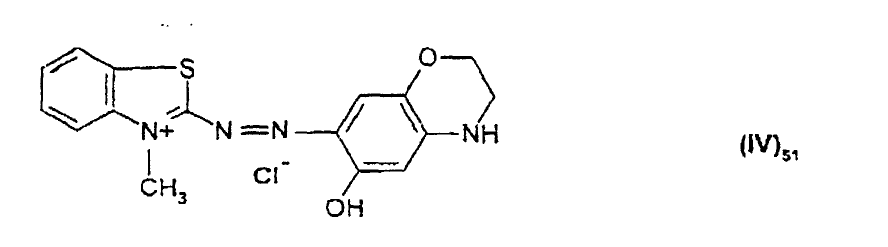

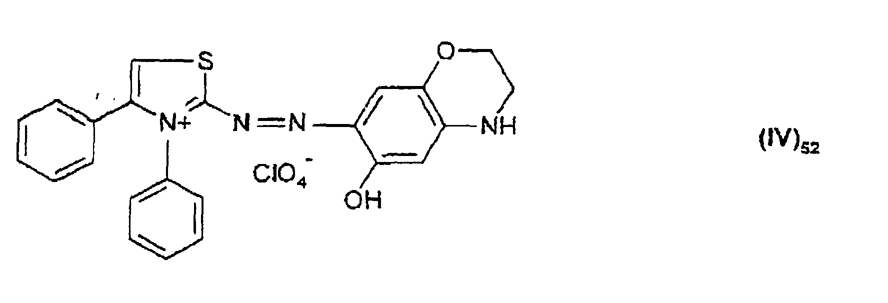

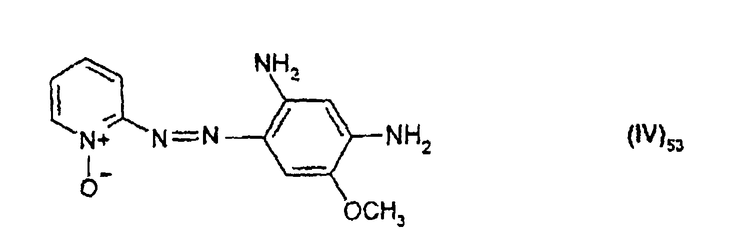

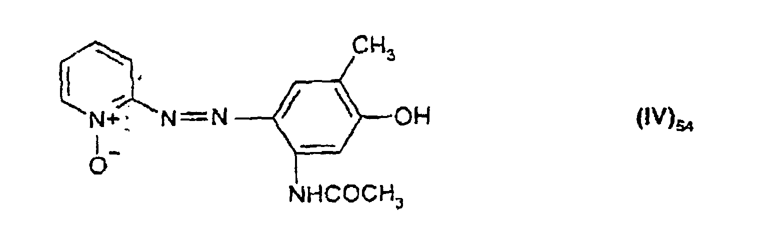

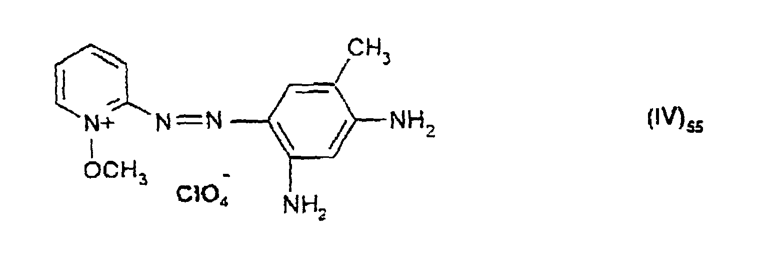

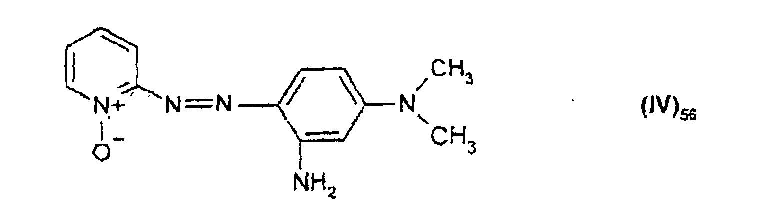

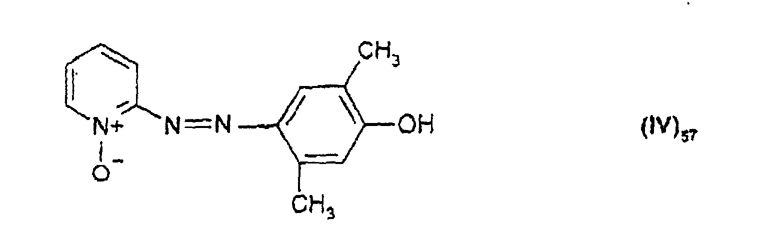

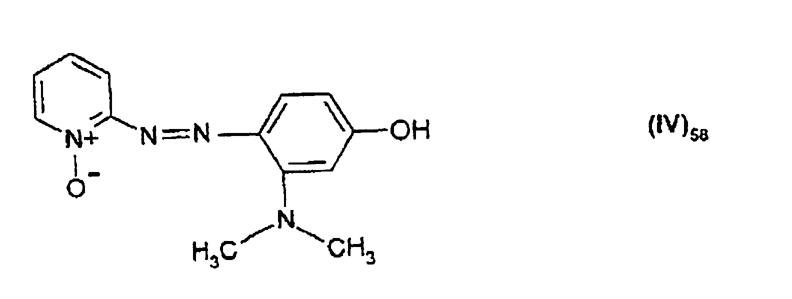

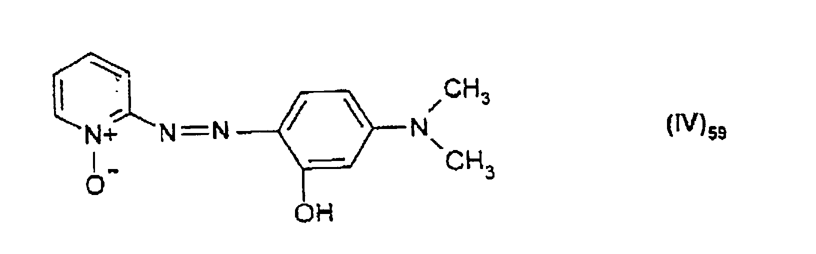

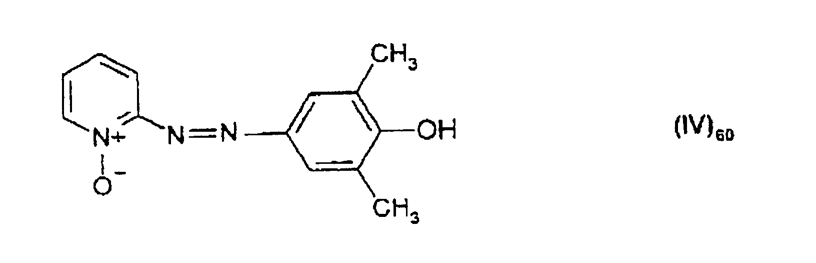

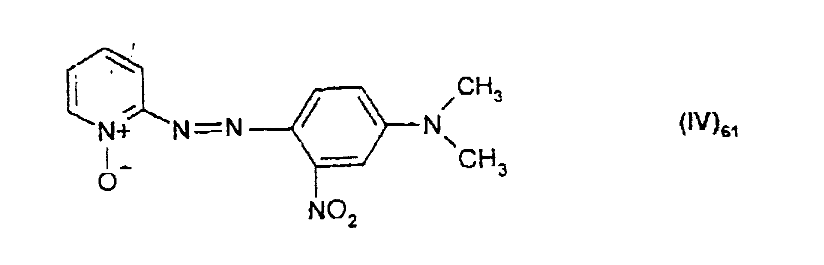

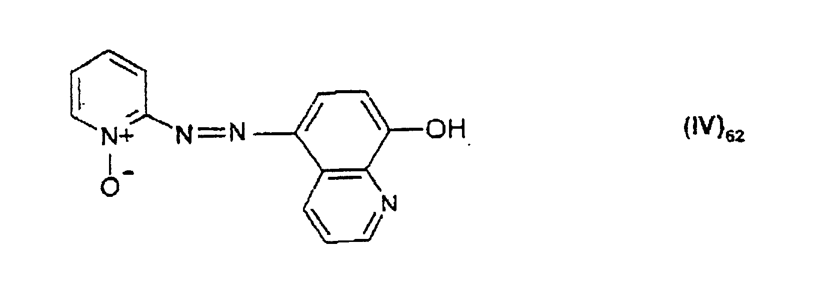

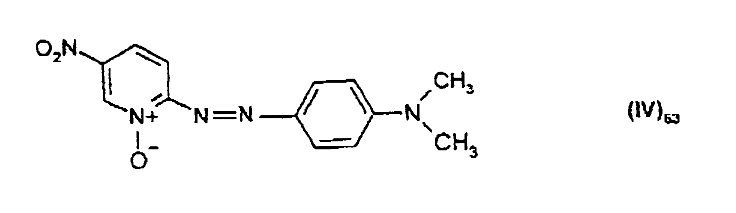

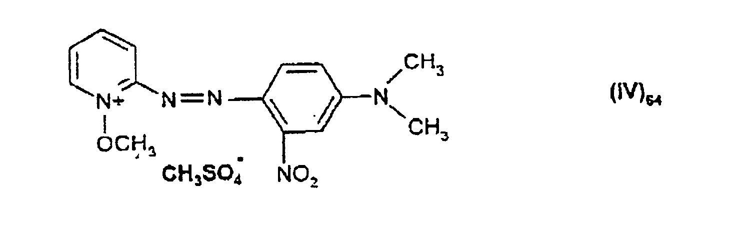

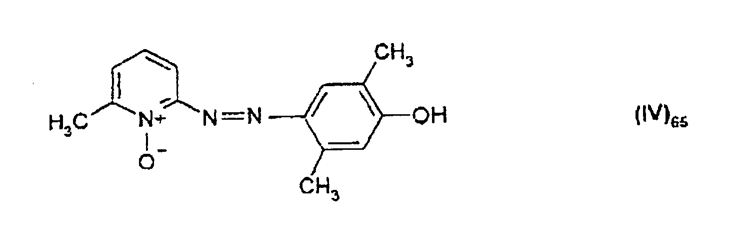

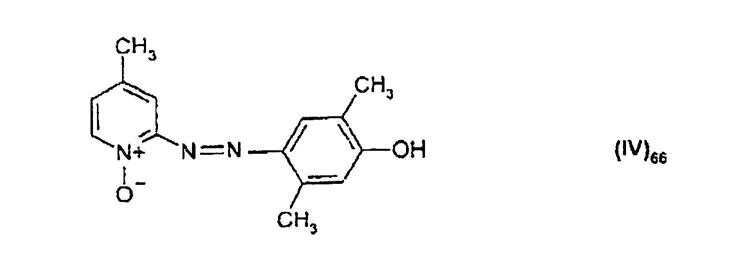

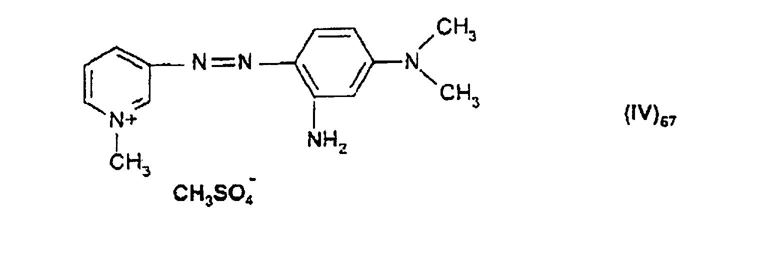

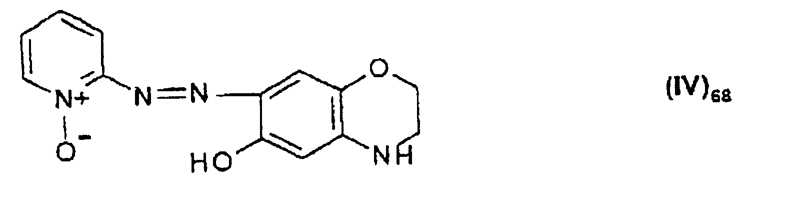

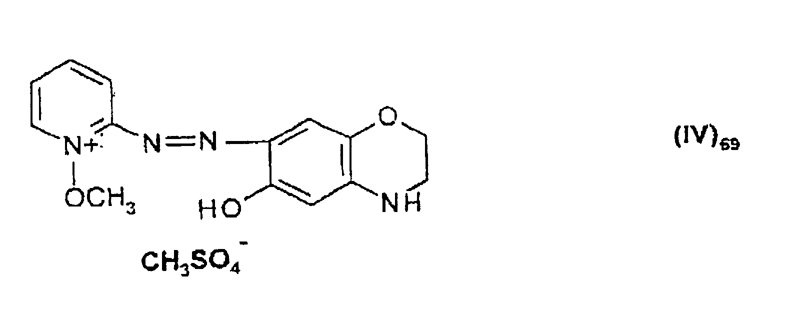

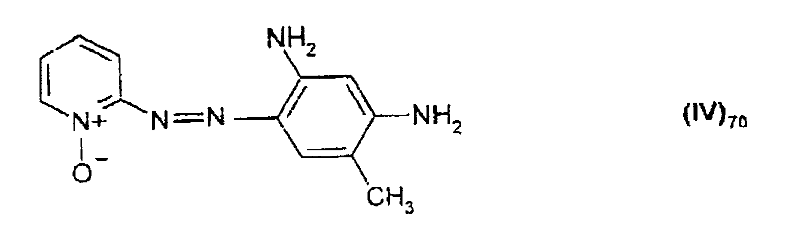

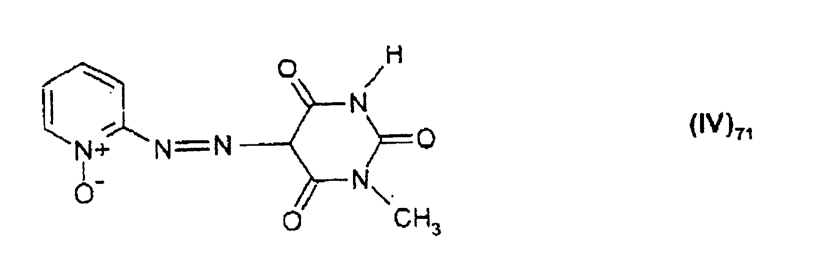

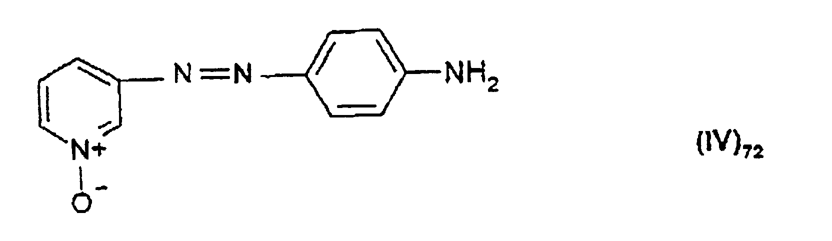

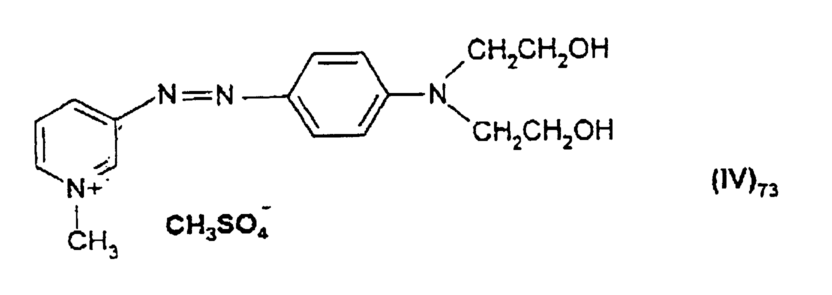

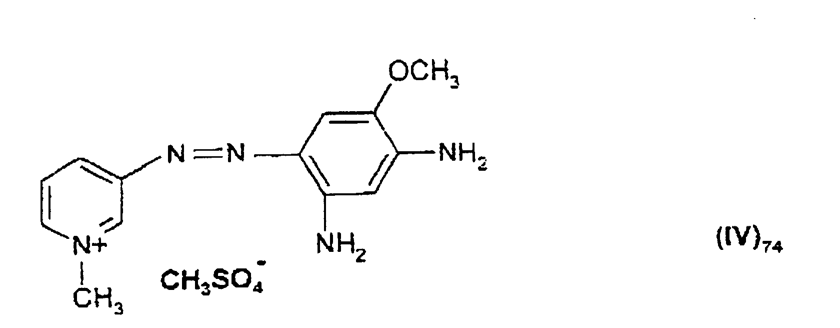

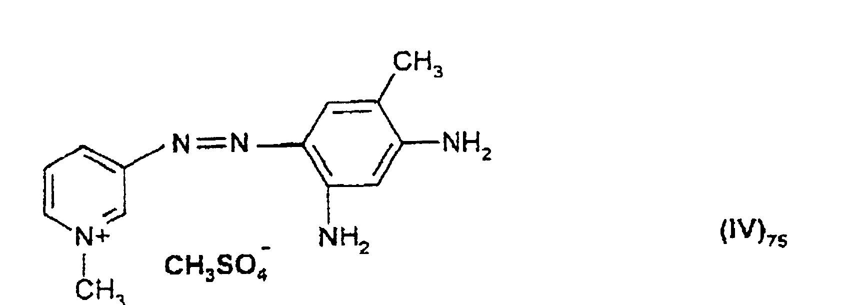

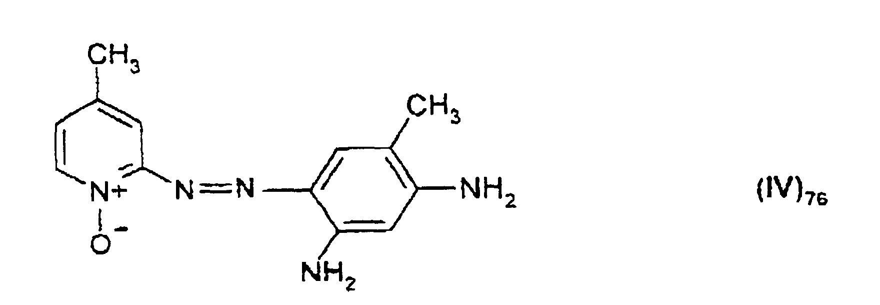

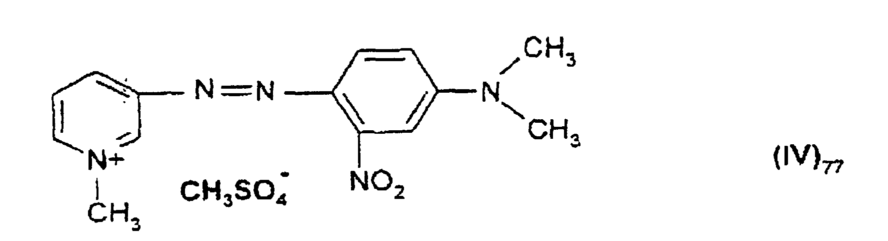

9. Composition according to any one of the preceding claims, **characterized in that** the cationic direct dye(s) of formulae (I), (II), (III), (III') or (IV) represent from 0.001 to 10% by weight of the total weight of the composition.

10. Composition according to Claim 9, **characterized in that** the cationic direct dye(s) of formulae (I), (II), (III), (III') or (IV) represent from 0.005 to 5% by weight of the total weight of the composition.

11. Composition according to any one of the preceding claims, **characterized in that** the alkyl polyglucoside-type nonionic surfactant (ii) is a compound of the following formula (V): 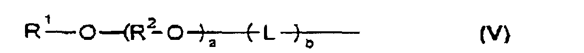 in which,
R¹ denotes a linear or branched alkyl and/or alkenyl radical comprising from about 8 to 24 carbon atoms, an alkylphenyl radical whose linear or branched alkyl group comprises from about 8 to 24 carbon atoms, R² denotes an alkylene radical comprising from 2 to 4 carbon atoms, L denotes a reduced sugar comprising from 5 to 6 carbon atoms, a denotes a value ranging from 0 to 10, and b denotes a value ranging from 1 to 15.

12. Composition according to Claim 11, **characterized in that** in formula (V), R¹ denotes a linear or branched alkyl and/or alkenyl radical comprising from 9 to 14 carbon atoms, a denotes zero, L denotes glucose, b denotes a value ranging from 1 to 4.

13. Composition according to any one of the preceding claims, **characterized in that** the nonionic surfactant (ii) of the sugar or alkyl sugar fatty acid ester type **(ii)**_{**2**} is a sugar C₄-C₂₂ fatty acid ester or a (C₁-C₄)alkyl sugar C₄-C₂₂ fatty acid ester.

14. Composition according to Claim 13, **characterized in that** it is glucose or sucrose C₄-C₂₂ fatty acid monoesters.

15. Composition according to Claim 13, **characterized in that** it is a methyl glucoside, ethyl glucoside or butyl glucoside C₄-C₂₂ fatty acid ester.

16. Composition according to Claim 15, **characterized in that** it is butyl glucoside C₁₂-C₁₈ fatty acid esters or methyl glucoside monostearate.

17. Composition according to any one of the preceding claims, **characterized in that** the nonionic surfactant of the fatty sugar amide type **(ii)**_{**3**} is chosen from **(ii)**_{**3**}**(a)** N-substituted aldonamides and (ii)₃(b) polyhydroxylated fatty acid amides or mixtures thereof.

18. Composition according to Claim 17, **characterized in that** the N-substituted aldonamides **(ii)**_{**3**}**(a)** are chosen from:
(i) N-substituted lactobionamides, N-substituted maltobionamides, N-substituted cellobionamides, N-substituted mellibionamides and N-substituted gentiobionamides;
(ii) N-lactobionylamino acid esters, N-maltobionylamino acid esters, N-mellibionylamino acid esters, N-cellobionylamino acid esters and N-gentiobionylamino acid esters;
(iii) N-(alkyloxy)alkyllactobionamides;
(iv) N-(polyalk-yloxy)alkyllactobionamides, N-(polyalkyloxy)alkylmaltobionamides, N-(polyalkyloxy)alkylcellobionamides, N-(polyalkyloxy)alkylmellibionamides or N-(polyalkyloxy)alkylgentiobionamides.

19. Composition according to Claim 17, **characterized in that** the polyhydroxylated fatty acid amide **(ii)**_{**3**}**(b)** is a compound of the following formula (VI): 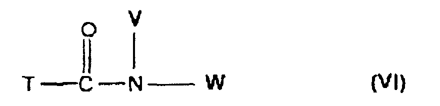 in which,
**T** denotes a C₅-C₃₁ hydrocarbon group, preferably a C₇-C₁₅ linear alkyl or alkenyl chain or mixtures thereof;
**V** denotes hydrogen, a C₁-C₄ hydrocarbon radical, 2-hydroxyethyl, 2-hydroxypropyl or mixtures thereof, preferably a C₁-C₄ alkyl and more particularly methyl:
**W** denotes a polyhydroxy hydrocarbon-containing group having a linear hydrocarbon chain with at least 3 hydroxyl groups directly attached to the chain, or an alkoxylated derivative of the said group.

20. Composition according to Claim 19, **characterized in that W** denotes a reducing sugar derivative obtained by reductive amination reaction and preferably a glycityl group.

21. Composition according to Claim 19 or 20, **characterized in that W** is chosen from the groups of the following formulae:
-(CH₂)-(CHOH)ₙ-CH₂OH ;
-CH-(CH₂OH)-(CHOH)ₙ₋₁-CH₂OH:
-CH₂-(CHOH)₂(CHOR') (CHOH)-CH₂OH in which n is an integer ranging from 3 to 5, R' denotes hydrogen, a cyclic or aliphatic monosaccharide or one of its alkoxylated derivatives.

22. Composition according to Claim 21, **characterized in that** it is the group -CH₂-(CHOH)₄-CH₂OH .

23. Composition according to any one of Claims 19 to 22, **characterized in that** in formula (VI), the group T-CON= is chosen from: cocamide, stearamide, oleamide, lauramide, myristiramide, capricamide, palmitamide or tallow amide.

24. Composition according to any one of the preceding claims, **characterized in that** the nonionic surfactant(s) (ii) represent from 0.05 to 30% by weight of the total weight of the dyeing composition.

25. Composition according to Claim 24, **characterised in that** the nonionic surfactant(s) represent from 0.1 to 15% by weight of the total weight of the dyeing composition.

26. Composition according to any one of the preceding claims, **characterized in that** the appropriate dyeing medium (or carrier) consists of water or of a mixture of water and of at least one organic solvent.

27. , Composition according to any one of the preceding claims, **characterized in that** it has a pH of between 2 and 11, and preferably between 5 and 10.

28. Composition according to any one of the preceding claims, **characterized in that** it is intended for oxidation dyeing and **in that** it contains one or more oxidation bases chosen from the para-phenylenediamines, the bis-phenylalkylenediamines, the para-aminophenols, the ortho-aminophenols and the heterocyclic bases.

29. Composition according to Claim 28, **characterized in that** the oxidation base(s) represent 0.0005 to 12% by weight of the total weight of the dyeing composition.

30. Composition according to Claim 29, **characterized in that** the oxidation base (s) represent 0.005 to 6% by weight of the total weight of the dyeing composition.

31. Composition according to any one of Claims 28 to 30, **characterized in that** it contains one or more couplers chosen from the the meta-phenylenediamines, the meta-aminophenols, the meta-diphenols and the 7 heterocyclic couplers.

32. Composition according to Claim 31, **characterized in that** the coupler(s) represent from 0.0001 to 10% by weight of the total weight of the dyeing composition.

33. Composition according to Claim 32, **characterized in that** the coupler(s) represent from 0.005 to 5% by weight of the total weight of the dyeing composition.

34. Composition according to any one of the preceding claims, **characterized in that** it is intended for direct lightening dyeing or oxidation dyeing and **in that** it then contains at least one oxidizing agent.

35. Method of dyeing keratinous fibres and in particular human keratinous fibres such as hair, **characterized in that** at least one dyeing composition as defined in any one of Claims 1 to 34 is applied to the fibres for a sufficient time to develop the desired colour, after which they are rinsed, optionally washed with shampoo, rinsed again and dried.

36. Method of dyeing keratinous fibres and in particular human keratinous fibres such as hair, **characterized in that** at least one dyeing composition as defined in any one of Claims 1 to 34 is applied to the fibres for a sufficient time to develop the desired colour, with no final rinsing.

37. Method of dyeing keratinous fibres and in particular human keratinous fibres such as hair, **characterized in that** it comprises a preliminary stage consisting of storing in a separate form, on the one hand, a composition (A1) comprising, in an appropriate dyeing medium, at least one cationic direct dye (i) as defined in the preceding claims and at least one oxidation base and, on the other hand, a composition (B1) containing, in an appropriate dyeing medium, at least one oxidizing agent, and then mixing them at the time of use before applying this mixture to the keratinous fibres, the composition (A1) or the composition (B1) containing the nonionic surfactant (ii) as defined in the preceding claims.

38. Method of dyeing keratinous fibres and in particular human keratinous fibres such as hair, **characterized in that** it comprises a preliminary stage consisting of storing in a separate form, on the one hand, a composition (A2) comprising, in an appropriate dyeing medium, at least one cationic direct dye (i) as defined in the preceding claims and, on the other hand, a composition (B2) containing, in an appropriate dyeing medium, at least one oxidizing agent, and then mixing them at the time of use before applying this mixture to the keratinous fibres, the composition (A2) or the composition (B2) containing the nonionic surfactant (ii) as defined in the preceding claims.

39. Multicompartment device or multicompartment dyeing "kit", **characterized in that** a first compartment contains composition (A1) or (A2) as defined in Claim 37 or 38 and a second compartment contains composition (B1) or (B2) as defined in Claim 37 or 38.
